# EUROPEAN PATENT APPLICATION

(11) **EP 4 074 709 A1**
(43) Date of publication of application: **19.10.2022**
(21) Application number: 20900635.2
(22) Date of filing: 11.12.2020
(51) Int. Cl.: C07D 417/14, A61K 31/427, A61K 31/4439, A61P 31/04

(54) **MONOCYCLIC BETA-LACTAM COMPOUND AND USE THEREOF**

(30) Priority: 13.12.2019 CN 201911281423
(71) Applicant: Evopoint Biosciences Co., Ltd., Suzhou, Jiangsu 215000 (CN)
(72) Inventor: WU, Yuchuan, Beijing 100176 (CN); LIU, Xiao, Beijing 100176 (CN); CHEN, Xi, Beijing 100176 (CN); HU, Yonghan, Suzhou, Jiangsu 215028 (CN); WANG, Wengui, Suzhou, Jiangsu 215028 (CN); ZHONG, Qifei, Beijing 100176 (CN); LI, Xin, Suzhou, Jiangsu 215028 (CN)
(74) Representative: Plasseraud IP
(86) International application number: PCT/CN2020/135825
(87) International publication number: WO 2021/115444

(57) **Abstract**

Provided is a monocyclic β-lactam compound as shown in general formula (I), or an ester, a stereoisomer or a pharmaceutically acceptable salt thereof. Also provided are a pharmaceutical composition containing the monocyclic β-lactam compound as shown in general formula (I), or an ester, a stereoisomer or a pharmaceutically acceptable salt thereof, and the use thereof in the preparation of a drug for treating bacterial infections.

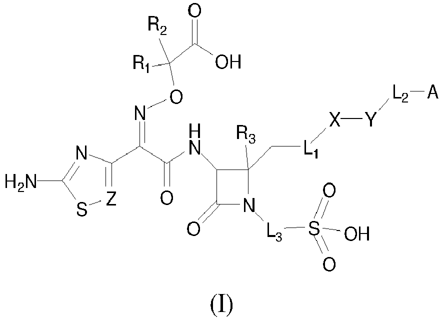

## Description

### TECHNICAL FIELD

The present invention belongs to the field of pharmaceutical chemistry, and relates to a novel monobactam compound and use thereof for treating bacterial infections.

### BACKGROUND

Since penicillin was widely used for treating bacterial infections in 1943, a complete production system for β-lactam antibiotics has been formed over more than 70 years of development. Nowadays, it has become an important part of the world pharmaceutical industry and has occupied the largest share of the global antibiotic market worth 42 billion dollars. However, as new antibiotic varieties are continuously brought into the market, the abuse phenomenon is becoming increasingly common. The bacterial resistance is a kind of natural evolution, and the abuse phenomenon is equivalent to applying strong selectivity to the bacterial resistance, so that the generation of drug-resistant strains is accelerated, some of bacteria are mutated into extremely resistant superbacteria that are resistant to a wide range of antibiotics. At present, 70% of clinically isolated strains are gram-negative bacteria, among which drug-resistant strains such as *Acinetobacter baumannii, Enterobacter* spp. and *Klebsiella pneumoniae* pose the greatest threat to human beings. Therefore, it is an urgent issue to be solved to develop drugs with good antibacterial activity against the drug-resistant strains.

The β-lactamase is the main mechanism by which the gram-negative bacteria are resistant to lactam antibiotics, and is an inactivated enzyme or a modified enzyme (β-lactam hydrolase) produced by bacteria. After passing through the outer membrane of the gram-negative bacteria via pore proteins, the antibiotics are hydrolyzed and inactivated by the β-lactamase secreted by the drug-resistant bacteria and cannot interact with target penicillin-binding protein (PBP). The β-lactamase produced by bacteria fall into two broad categories, namely enzymes with serine residues at the active site, and enzymes with metal ions at the active site, called metallo-β-lactamases (MBLs), or metalloenzymes for short.

This group of enzymes are mainly characterized by being capable of hydrolyzing carbapenems and other antibiotics, but having little effect on monobactam such as aztreonam. The active center of the enzymes requires the participation of metal zinc ions to exert the catalytic activity, so the enzymes are called metallo-β-lactamases. The substrate is a broad class of β-lactam antibiotics including carbapenems, the activity of which is not inhibited by common β-lactamase inhibitors such as clavulanic acid. The metallo-β-lactamases can be mediated by chromosomes and plasmids, can be detected in bacteria such as *Pseudomonas aeruginosa, Stenotrophomonas maltophilia, Serratia marcescens, Enterobacter* spp., *Klebsiella pneumoniae, Aeromonas hydrophila, Acinetobacter* spp., *Bacteroides fragilis,* and *Acinetobacter baumannii,* has wide distributed genera and regions, and is a hot spot of clinical attention. So far, there are 5 genotypes identified for bacterial acquired metallo-β-lactamases: IMP, VIM, SPM and GIM, SIM, among which IMP and VIM, the most important ones, are classified into many subtypes according to the difference in the genes and the translated amino acid sequences, now evolving to IMP-22 and VIM-12. The metallo-β-lactamases are increasingly expressed in drug-resistant strains, and no metallo-β-lactamase inhibitors are currently on the market or in mid-to-late stage of clinical studies.

Monobactam is a monocyclic β-lactam antibiotic that acts as a bactericide by inhibiting the synthesis of the bacterial cell wall with high affinity for penicillin-binding proteins (PBPs) on the cell membrane of gram-negative bacteria. Monobactam is stable against metallo-lactamases (class B enzymes) among β-lactamases, but the drug aztreonam is known to have no significant inhibitory effect against a variety of drug-resistant bacteria. Therefore, there is a need for novel antibacterial drugs, particularly antibacterial compounds capable of effectively combating gram-negative drug-resistant bacteria, and the present invention provides such compounds.

### SUMMARY

In one aspect, in an embodiment of the present invention, there is provided a monobactam compound of general formula (I): 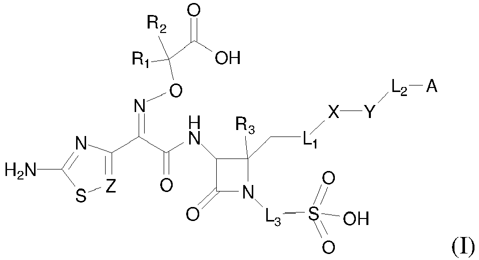 or an ester, a stereoisomer and a pharmaceutically acceptable salt thereof, wherein variables R₁, R₂, R₃, L₁, L₂, L₃, X, Y, A and Z are as defined herein.

In a further embodiment of the present invention, provided is a pharmaceutical composition comprising the compound of formula (I) or a pharmaceutically acceptable salt thereof, and a pharmaceutically acceptable carrier.

In a still further embodiment of the present invention, provided is a method for treating bacterial infections comprising administering to a subject in need thereof a therapeutically effective amount of the compound of formula (I) or a pharmaceutically acceptable salt thereof, or the pharmaceutical composition comprising the compound of formula (I) or a pharmaceutically acceptable salt thereof and a pharmaceutically acceptable carrier.

### Definitions

As used in the specification of the present invention, the following words and phrases are generally considered to have the meanings as set forth below, unless indicated in the context in which the words or phrases are used.

The term "alkyl" as used herein refers to a linear or branched saturated hydrocarbon having the indicated number of carbon atoms. For example, C₁-₆ alkyl refers to alkyl groups including, but not limited to, methyl, ethyl, propyl, isopropyl, butyl, sec-butyl, *tert-butyl,* pentyl, isopentyl, neopentyl, hexyl, isohexyl, and neohexyl. An alkyl group may be unsubstituted or optionally substituted with one or more substituents described herein.

The term "alkylene" refers to a divalent branched or unbranched alkane chain. For example, -C₁₋₄ alkylene- refers to alkylene groups including, for example, methylene (-CH2-), ethylene (-CH2CH2-), propylene isomers (for example, -CH2CH2CH2- and -CH(CH3)CH2-), butylene (for example, -CH2CH2CH2CH2-, -CH2CH(CH3)CH2-, -CH(CH3)CH2CH2- and - C(CH3)2CH2-). An alkylene group may be unsubstituted or optionally substituted with one or more substituents described herein.

The term "hydroxy" refers to the group -OH.

The term "alkoxy" refers to the group R-O-, wherein R is the alkyl as defined herein. In some embodiments, alkoxy groups include, for example, methoxy, ethoxy, n-propoxy, isopropoxy, n-butoxy, tert-butoxy, sec-butoxy, n-pentoxy, n-hexoxy, 1,2-dimethylbutoxy, and the like.

The term "cycloalkyl" refers to a cycloalkyl group of 3-20, preferably 3-12, more preferably 4-8, and most preferably 3-6 carbon atoms having a monocyclic ring or polycyclic fused rings. Such cycloalkyl groups include, for example, monocyclic structures such as cyclopropyl, cyclobutyl, cyclopentyl and cyclooctyl, or polycyclic structures such as adamantyl and bicyclo[2.2.1]heptane, or cycloalkyl groups fused to aryl groups such as indane, if the point of attachment is through a cycloalkyl group.

The term "aryl" includes substituted or unsubstituted aromatic groups in which each ring atom is carbon. Preferably, the ring is a 6-14 membered ring, and more preferably, is a 6-membered ring. Aryl groups include, but are not limited to, phenyl. The term "aryl" also includes "polycyclyl" and "polycyclic" systems having two or more rings in which two or more atoms are common to two adjoining rings (for example, the rings are "fused"), wherein at least one of the rings is aromatic, for example, the other rings may be cycloalkyl groups, cycloalkenyl groups, cycloalkynyl groups, or aromatic rings. In some preferred embodiments, the polycyclic ring has 2-3 rings. In certain preferred embodiments, the polycyclic ring system has two rings, wherein both rings are aromatic. Each ring of the polycyclic ring may be substituted or unsubstituted. In certain embodiments, each ring of the polycyclic ring contains 3-10 carbon atoms, preferably 5-7 carbon atoms, in the ring. For example, aryl groups include, but are not limited to, phenyl (benzene), tolyl, anthracenyl, fluorenyl, indenyl, azulenyl, and naphthyl, as well as benzo-fused carbocyclic moieties such as 5,6,7,8-tetrahydronaphthyl. In some embodiments, the aryl group is a monocyclic aromatic group. In some embodiments, the aryl group is a bicyclic aromatic group. In some embodiments, the aryl group is a tricyclic aromatic group.

The term "heterocycloalkyl" as used herein refers to a 3-18 membered, preferably 3-10 membered, and more preferably 3-6 membered substituted or unsubstituted saturated or partially saturated non-aromatic ring structure, which comprises at least one heteroatom, preferably one to three heteroatoms, and more preferably one or two heteroatoms. In certain embodiments, the ring structure may have two rings. In some embodiments, the two rings may have two or more atoms in common, for example, the rings are "fused". Heterocycloalkyl groups include, for example, piperidine, piperazine, pyrrolidine, morpholine, lactones, lactams, and the like. Heterocycles are described in Paquette, Leo A.; "Principles of Modern Heterocycle Chemistry" (W. A. Benjamin, New York, 1968), especially chapters 1, 3, 4, 6, 7 and 9; *"*The Chemistry of Heterocyclic Compounds, A series of Monographs" (John Wiley & Sons, New York, 1950), especially volumes 13, 14, 16, 19 and 28; and J. Am. Chem. Soc. (1960) 82:5566. Examples of heterocycloalkyl groups include, but are not limited to, tetrahydrofuran, dihydrofuran, tetrahydrothiene, tetrahydropyran, dihydropyran, tetrahydrothiopyran, thiomorpholine, thiaoxane, homopiperazine, azetidine, oxetane, thietane, homopiperidine, piperidine, piperazine, pyrrolidine, morpholine, oxepane, thiepane, azapine, diazepine, thiazepine, 2-pyrroline, 3-pyrroline, indoline, 2*H*-pyran, 4*H*-pyran, dioxane, 1,3-dioxolane, pyrazoline, dithiane, dithiolane, dihydropyran, dihydrothiene, dihydrofuran, pyrazolidinyl, imidazoline, imidazolidine, 3-azabicyclo[3.1.0]hexane, 3-azabicyclo[4.1.0]heptane, and azabicyclo[2.2.2]hexane. Spiro moieties are also included within the scope of the definition herein. Examples of heterocyclic groups in which the ring atoms are partially substituted with oxo (=O) are pyrimidinone and 1,1-dioxo-thiomorpholine.

The term "heteroaryl" as used herein refers to a substituted or unsubstituted aromatic monocyclic ring structure comprising at least one heteroatom (e.g., O, N or S), preferably one to three, and more preferably one or two heteroatoms, preferably a 5-7 membered ring, more preferably a 5-6 membered ring. When two or more heteroatoms are present in the heteroaromatic ring, they may be identical or different. The term "heteroaryl" also includes "polycyclyl" and "polycyclic" systems having two or more rings in which two or more atoms are common to two adjoining rings (for example, the rings are "fused"), wherein at least one of the rings is heteroaromatic, for example, the other rings may be cycloalkyl groups, cycloalkenyl groups, cycloalkynyl groups, aryl groups, heteroaromatic rings and/or aromatic rings. In some preferred embodiments, the polycyclic heteroaryl group has 2-3 rings. In certain embodiments, preferably, the polycyclic heteroaryl group has two rings, wherein both rings are aromatic. In certain embodiments, each ring of the polycyclic ring contains 3-10 atoms in the ring, preferably 5-6 atoms in the ring. For example, heteroaryl groups include, but are not limited to, pyrrole, furan, thiophene, imidazole, oxazole, thiazole, pyrazole, pyridine, pyrazine, pyridazine, quinoline, pyrimidine, indolizine, indole, indazole, benzimidazole, benzothiazole, benzofuran, benzothiophene, cinnoline, phthalazine, quinazoline, carbazole, phenoxazine, quinoline, purine, and the like. In some embodiments, the heteroaryl group is a monocyclic aromatic group. In some embodiments, the heteroaryl group is a bicyclic aromatic group.

The term "amino" refers to the group -NH2.

The term "halogen" refers to fluorine, chlorine, bromine and iodine.

The term "pharmaceutically acceptable salt" refers to a pharmaceutically acceptable organic or inorganic salt of the compound of the present invention. Exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisate, fumarate, arginine, gluconate, glucuronate, saccharate, formate, benzoate, glutamate, methanesulfonate (mesylate), ethanesulfonate, benzenesulfonate, *p*-toluenesulfonate, and pamoate; or ammonium salts (e.g., primary amine salts, secondary amine salts, tertiary amine salts, and quaternary ammonium salts), and metal salts (e.g., sodium salts, potassium salts, calcium salts, magnesium salts, manganese salts, iron salts, zinc salts, copper salts, lithium salts, and aluminum salts).

The term "pharmaceutically acceptable" as used herein means that the substance or composition must be compatible chemically and/or toxicologically with the other ingredients comprising a preparation, and/or the mammal being treated therewith.

The term "treating" as used herein refers to therapeutic treatments and prophylactic or preventative or protective measures, which aim to prevent or slow down (alleviate) an undesired pathological change or disorder. For purposes of the present invention, beneficial or desired clinical results include, but are not limited to, alleviation of symptoms, reduction in disease severity, delay or slowing of disease progression, amelioration or palliation of the disease state, and remission (whether partial or total), whether detectable or undetectable.

The term "therapeutically effective amount" as used herein means that an amount of the compound of the present invention that (i) treats or prevents a disease or disorder described herein, (ii) alleviates or eliminates one or more diseases or disorders described herein, or (iii) prevents or delays the onset of one or more symptoms of a disease or disorder described herein.

### DETAILED DESCRIPTION

In an embodiment, the present invention provides a compound of formula (I):
A compound of formula (I): 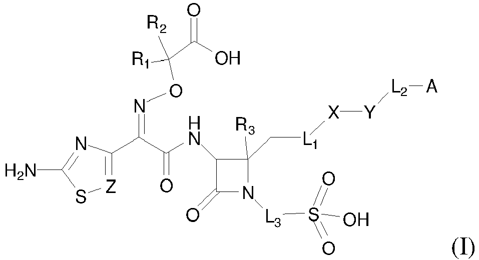 wherein R₁ and R₂ are each independently selected from H, C₁-C₆ alkyl and C₁-C₆ haloalkyl; or R₁ and R₂, together with the carbon atom to which they are attached, form C₃-C₆ cycloalkyl, or R₁ and R₂, together with the carbon atom to which they are attached, form C₃-C₆ heterocycloalkyl; wherein the C₃-C₆ cycloalkyl and C₃-C₆ heterocycloalkyl are optionally substituted with C₁-C₆ alkyl, C₁-C₆ haloalkyl, halogen or hydroxy;
R₃ is selected from H, C₁-C₆ alkyl and halogen, wherein the C₁-C₆ alkyl is optionally substituted with halogen or hydroxy;
L₁ is absent or is selected from -O-T-, -O-C(=O)-T-, -O-S(=O)ᵣ-T-, -S(=O)ᵣ-T-, -S-CH₂-C(O)-T-, -NH-C(=O)-T-, -NH-S(O)ᵣ-T-, -NH-C(=N-CN)-T, -NH-C(=NH)-T-, -NH-T-, -(CH₂)ₚ-S(O)ᵣ-T-, -(CH₂)ₚ-C(O)-T-, -(CH₂)ₚ-S-T- and -(CH₂)ₚ-C(=NH)-T-, wherein the T is absent or is selected from -(CH₂)ₚ-, -(CH₂)ₚ-NH-(CH₂)_{q}-, -(CH₂)ₚ-O-(CH₂)_{q}-, -(CH₂)ₚ-C(=O)-NH-(CH₂)_{q}-, - (CH₂)ₚ-NH-C(=O)-(CH₂)_{q}-, -CH(CH₃)-NH-C(=O)-(CH₂)_{q}-, -(CH₂)ₚ-NH-C(=O)-NH-(CH₂)_{q}-, - CH(CH₃)-NH-C(=O)-NH-(CH₂)_{q}-, -CH(CH₃)-NH-C(=O)-NH-(CH₂)_{q}-, and -(CH₂)ₚ-C(=O)-(CH₂)_{q}-;
wherein the r is 1 or 2; wherein the p and q are 0, 1 or 2;
X is selected from 5-6 membered heteroaryl, 6-10 membered aryl, 4-8 membered cycloalkyl and 4-8 membered heterocycloalkyl; wherein the 5-6 membered heteroaryl or 4-8 membered heterocycloalkyl contains 1, 2 or 3 heteroatoms selected from N, O and S as ring atoms, and the 5-6 membered heteroaryl, 6-10 membered aryl, 4-8 membered cycloalkyl or 4-8 membered heterocycloalkyl is optionally substituted with P, wherein the P is selected from C₁₋₄ alkyl, C₁₋₄ haloalkyl, C₁₋₄ alkoxy, halogen, NR4R4' and hydroxy, wherein the C₁₋₄ alkyl is optionally further substituted with hydroxy, C₁₋₄ alkoxy or NR4R4';
Y is absent or is selected from 5-6 membered heteroaryl, 6-10 membered aryl, 4-8 membered cycloalkyl and 4-8 membered heterocycloalkyl; wherein the 5-6 membered heteroaryl or 4-8 membered heterocycloalkyl contains 1, 2 or 3 heteroatoms selected from N, O and S as ring atoms, and the 5-6 membered heteroaryl, 6-10 membered aryl, 4-8 membered cycloalkyl or 4-8 membered heterocycloalkyl is optionally substituted with Q, wherein the Q is selected from halogen, C₁₋₄ alkyl, NR4R4', hydroxy, C₁₋₄ haloalkyl, C₁₋₄ alkoxy, -C₁₋₄ alkylene-N(R₅)₂, -C₁₋₄ alkylene-NR₅-C(=NR₅)-N(R₅)₂, -C₁₋₄ alkylene-NR₅-CR₅(=NR₅), -C₁₋₄ alkylene (C₁₋₄ alkylene-N(R₅))₂, and -C₁₋₄ alkylene-N⁺(R₇)₃, wherein the C₁₋₄ alkyl is optionally substituted with hydroxy, C₁₋₄ alkoxy or NR4R4', and the C₁₋₄ alkylene is optionally substituted with NR4R4', hydroxy or halogen;
R₄ and R₄' are each independently selected from H and C₁₋₄ alkyl;
R₅ is selected from H and C₁₋₄ alkyl optionally substituted with -OR₆ or -NR₆R₆'; or two R₅ on the same N may form 4-6 membered heterocycloalkyl optionally substituted with C₁₋₄ alkyl, C₁₋₄ alkoxy, hydroxy, amino or oxo, by cyclization;
R6 and R₆' are each independently selected from H and C₁₋₄ alkyl optionally substituted with hydroxy, C₁₋₄ alkoxy or NR4R4';
R₇ is selected from H, C₁₋₄ alkyl and -CH(-C₁₋₄ alkylene-N(R₅)₂)₂;
L2 is absent or is selected from -(NR₄)ₘ-C₁₋₄ alkylene-(NR4)n-, -(NR₄)ₘ-C₁₋₄ alkylene-NR4-C(=NR₄)-NR₄-, and -C₁₋₄ alkylene-NR₄-C₁₋₄ alkylene-; wherein m is 0 or 1, n is 1 or 2, and the C₁₋₄ alkylene is optionally substituted with C₁₋₄ alkyl, NH2, or -C₁₋₄ alkylene-NH₂;
A is selected from H, M and -(CO)-M, wherein the M is selected from 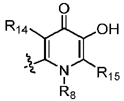 and 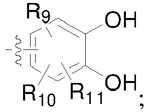 wherein R₈ is selected from H, C₁-C₆ alkyl, hydroxy, and C₁₋₄ alkoxy; R₉, R₁₀ and R₁₁ are each independently selected from H, halogen, CN and OR₁₂, wherein R₁₂ is H, C₁-C₆ alkyl, C₁-C₆ haloalkyl, C₁-C₆ alkylcarbonyl, or -C(O)-NH₂; R₁₄ and R₁₅ are each independently selected from: H, C₁₋₆ alkyl, halogen, cyano and OR₁₆, wherein R₁₆ is selected from H and C₁-C₆ alkyl;
L₃ is absent or is -O-;
Z is selected from N and CR₁₃, wherein R₁₃ is selected from H and halogen;
or an ester, a stereoisomer and a pharmaceutically acceptable salt thereof.

In a particular embodiment, the present invention provides compounds as shown below: 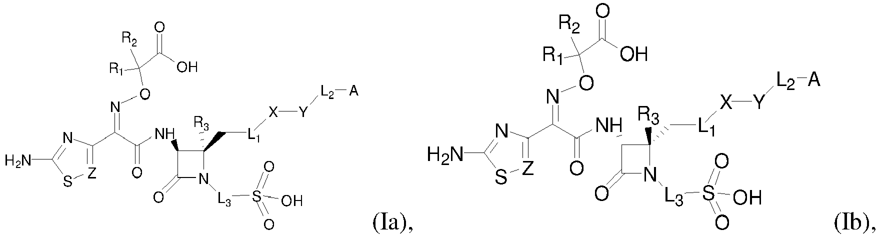 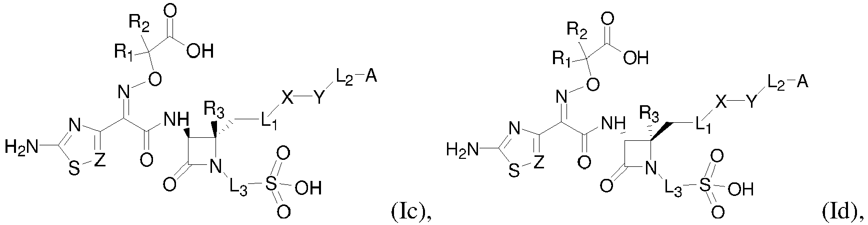 or an ester, a stereoisomer and a pharmaceutically acceptable salt thereof.

In a preferred embodiment, R₃ is selected from H and C₁-C₆ alkyl, wherein the C₁-C₆ alkyl is optionally substituted with halogen;
X is selected from 5-6 membered heteroaryl, 6-10 membered aryl, 4-8 membered cycloalkyl and 4-8 membered heterocycloalkyl; wherein the 5-6 membered heteroaryl or 4-8 membered heterocycloalkyl has at least 1 of heteroatoms selected from an N atom, and the 5-6 membered heteroaryl, 6-10 membered aryl, 4-8 membered cycloalkyl or 4-8 membered heterocycloalkyl is optionally substituted with P, wherein the P is selected from C₁₋₄ alkyl, C₁₋₄ haloalkyl, C₁₋₄ alkoxy, halogen, NR₄R₄' and hydroxy, wherein the C₁₋₄ alkyl is optionally further substituted with hydroxy, C₁₋₄ alkoxy or NR₄R₄', and R₄ and R₄' are each independently selected from H and C₁₋₄ alkyl.

In a preferred embodiment, R₃ is selected from H and C₁-C₆ alkyl, wherein the C₁-C₆ alkyl is optionally substituted with halogen;
X is selected from 5-membered heteroaryl and phenyl; wherein the 5-membered heteroaryl has at least 1 of heteroatoms selected from an N atom, and the 5-membered heteroaryl or phenyl is optionally substituted with P, wherein the P is selected from C₁₋₄ alkyl, C₁₋₄ haloalkyl, C₁₋₄ alkoxy, halogen, NR4R4' and hydroxy, wherein the C₁₋₄ alkyl is optionally further substituted with hydroxy, C₁₋₄ alkoxy or NR4R4', and R₄ and R₄' are each independently selected from H and C₁₋₄ alkyl.

In a preferred embodiment, R₃ is selected from H, methyl and halomethyl;
X is selected from oxazolyl, isoxazolyl, thiazolyl, isothiazolyl, imidazolyl, pyrazolyl, triazolyl, oxadiazolyl, thiadiazolyl and phenyl; wherein the groups are optionally substituted with P, wherein the P is selected from C₁₋₄ alkyl, C₁₋₄ haloalkyl, C₁₋₄ alkoxy, halogen, NR4R4' and hydroxy, wherein the C₁₋₄ alkyl is optionally further substituted with hydroxy, C₁₋₄ alkoxy or NR₄R₄', and R₄ and R₄' are each independently selected from H and C₁₋₄ alkyl.

In a preferred embodiment, R₁ and R₂ are each independently selected from H, methyl and halomethyl, or R₁ and R₂, together with the carbon atom to which they are attached, form cyclopropane, cyclobutane, or cyclopentane; wherein the cyclopropane, cyclobutane or cyclopentane is optionally substituted with C₁-C₆ alkyl, C₁-C₆ haloalkyl, halogen or hydroxy.

In a preferred embodiment, R₁ and R₂ are each methyl, or R₁ and R₂, together with the carbon atom to which they are attached, form cyclopropane, cyclobutane, or cyclopentane; wherein the cyclopropane, cyclobutane or cyclopentane is optionally substituted with C₁-C₆ alkyl, C₁-C₆ haloalkyl, halogen or hydroxy;
R₃ is selected from H, methyl and CH₂F;
X is selected from oxazolyl, isoxazolyl, thiazolyl and phenyl, wherein the groups are optionally substituted with P, wherein the P is selected from C₁₋₄ alkyl and NR4R4', wherein R₄ and R₄' are each independently selected from H and C₁₋₄ alkyl;
Y is absent or is selected from 5-6 membered heteroaryl, phenyl and 4-6 membered heterocycloalkyl; wherein the 5-6 membered heteroaryl or 4-6 membered heterocycloalkyl contains 1 or 2 N atoms as ring atoms, and the 5-6 membered heteroaryl, phenyl or 4-6 membered heterocycloalkyl is optionally substituted with Q, wherein the Q is selected from halogen, C₁₋₄ alkyl, NR4R4', hydroxy, C₁₋₄ haloalkyl, C₁₋₄ alkoxy, -C₁₋₄ alkylene-N(R₅)₂, -C₁₋₄ alkylene-NR₅-C(=NR₅)-N(R₅)₂, -C₁₋₄ alkylene-NR₅-CR₅(=NR₅), -C₁₋₄ alkylene (C₁₋₄ alkylene-N(R₅))₂, and -C₁₋₄ alkylene-N^{÷}(R₇)₃, wherein the C₁₋₄ alkyl is optionally substituted with hydroxy, C₁₋₄ alkoxy or NR4R4', and the C₁₋₄ alkylene is optionally substituted with NR4R4', hydroxy or halogen;
R₄ and R₄' are each independently selected from H and C₁₋₄ alkyl;
R₅ is selected from H and C₁₋₄ alkyl optionally substituted with -OR₆ or -NR₆R₆'; or two R₅ on the same N may form 4-6 membered heterocycloalkyl optionally substituted with C₁₋₄ alkyl, C₁₋₄ alkoxy, hydroxy, amino or oxo, by cyclization;
R6 and R₆' are each independently selected from H and C₁₋₄ alkyl optionally substituted with hydroxy, C₁₋₄ alkoxy or NR4R4';
R₇ is selected from H, C₁₋₄ alkyl and -CH(-C₁₋₄ alkylene-N(R₅)₂)₂.

In a preferred embodiment, X is selected from oxazolyl, isoxazolyl, thiazolyl and phenyl, wherein the groups are optionally substituted with P, wherein the P is methyl;
Y is absent or is selected from pyrazolyl, pyridinyl, pyrrolyl, azetidinyl, pyrimidinyl and phenyl, wherein the groups are optionally substituted with Q, wherein the Q is selected from halogen, C₁₋₄ alkyl, NR4R4', hydroxy, C₁₋₄ haloalkyl, C₁₋₄ alkoxy, -C₁₋₄ alkylene-N(Rs)2, -C₁₋₄ alkylene-NR₅-C(=NR₅)-N(R₅)₂, -C₁₋₄ alkylene-NR₅-CR₅(=NR₅), -C₁₋₄ alkylene (C₁₋₄ alkylene-N(R₅))₂, and -C₁₋₄ alkylene-N^{÷}(R₇)₃, wherein the C₁₋₄ alkyl is optionally substituted with hydroxy, C₁₋₄ alkoxy or NR4R4', and the C₁₋₄ alkylene is optionally substituted with NR4R4', hydroxy or halogen;
R₄ and R₄' are each independently selected from H and C₁₋₄ alkyl;
R₅ is selected from H and C₁₋₄ alkyl optionally substituted with -OR₆ or -NR₆R₆'; or two R₅ on the same N may form 4-6 membered heterocycloalkyl optionally substituted with C₁₋₄ alkyl, C₁₋₄ alkoxy, hydroxy, amino or oxo, by cyclization;
R6 and R₆' are each independently selected from H and C₁₋₄ alkyl optionally substituted with hydroxy, C₁₋₄ alkoxy or NR4R4';
R₇ is selected from H, C₁₋₄ alkyl and -CH(-C₁₋₄ alkylene-N(R₅)₂)₂;
A is selected from H, M and -(CO)-M, wherein the M is selected from 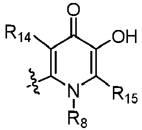 and 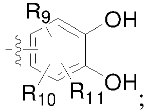 wherein R₈ is hydroxy; R₉, R₁₀ and R₁₁ are each independently selected from H and halogen; R₁₄ and R₁₅ are each independently selected from H, C₁₋₃ alkyl, halogen, cyano and OR₁₆, wherein R₁₆ is selected from H and C₁-C₃ alkyl.

In a preferred embodiment, R₁ and R₂ are each methyl, or R₁ and R₂, together with the carbon atom to which they are attached, form cyclopropane, cyclobutane or cyclopentane;
R₃ is selected from H, methyl and CH2F;
L₁ is -NH-C(O)-;
Y is absent or is selected from 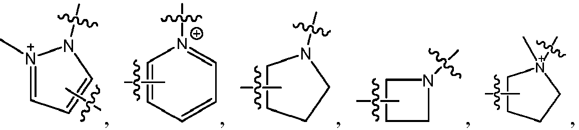 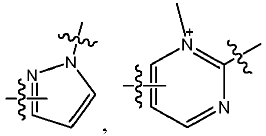 and phenyl;
L2 is absent or is selected from -(NR₄)ₘ-C₁₋₄ alkylene-(NR4)n-, -(NR₄)ₘ-C₁₋₄ alkylene-NR4-C(=NR₄)-NR₄-, and -C₁₋₄ alkylene-NR₄-C₁₋₄ alkylene-; wherein m is 0 or 1, n is 1 or 2, and the C₁₋₄ alkylene is optionally substituted with C₁₋₄ alkyl, NH2, or -C₁₋₄ alkylene-NH₂;
A is selected from H, M and -(CO)-M, wherein the M is selected from 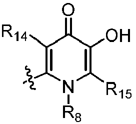 and 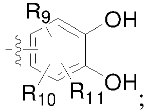 wherein R₈ is hydroxy; R₉, R₁₀ and R₁₁ are each independently selected from H and halogen; R₁₄ and R₁₅ are each independently selected from H and C₁₋₃ alkyl;
L₃ is absent;
Z is CH, N or CCl.

In a preferred embodiment, R₁ and R₂ are each methyl, or R₁ and R₂, together with the carbon atom to which they are attached, form cyclopropane, cyclobutane or cyclopentane;
R₃ is selected from H, methyl and CH2F;
L₁ is -NH-C(O)-;
X is selected from 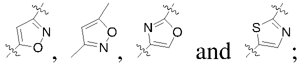
Y is absent or is selected from pyrazolyl, pyridinyl, pyrrolyl, azetidinyl, pyrimidinyl and phenyl, wherein the groups are optionally substituted with Q, wherein the Q is selected from halogen, C₁₋₄ alkyl, NR4R4', hydroxy, C₁₋₄ haloalkyl, C₁₋₄ alkoxy, -C₁₋₄ alkylene-N(Rs)2, -C₁₋₄ alkylene-NR₅-C(=NR₅)-N(R₅)₂, -C₁₋₄ alkylene-NR₅-CR₅(=NR₅), -C₁₋₄ alkylene (C₁₋₄ alkylene-N(R₅))₂, and -C₁₋₄ alkylene-N^{÷}(R₇)₃, wherein the C₁₋₄ alkyl is optionally substituted with hydroxy, C₁₋₄ alkoxy or NR4R4', and the C₁₋₄ alkylene is optionally substituted with NR4R4', hydroxy or halogen;
R₄ and R₄' are each independently selected from H and C₁₋₄ alkyl;
Rs is selected from H and C₁₋₄ alkyl optionally substituted with -OR₆ or -NR₆R₆'; or two R₅ on the same N may form 4-6 membered heterocycloalkyl optionally substituted with C₁₋₄ alkyl, C₁₋₄ alkoxy, hydroxy, amino or oxo, by cyclization;
R6 and R₆' are each independently selected from H and C₁₋₄ alkyl optionally substituted with hydroxy, C₁₋₄ alkoxy or NR4R4';
R₇ is selected from H, C₁₋₄ alkyl and -CH(-C₁₋₄ alkylene-N(R₅)₂)₂;
L2 is absent or is selected from -(NH)ₘ-C₁₋₄ alkylene-(NH)n-, -(NH)ₘ-C₁₋₄ alkylene-NH-C(=NH)-NH-, and -C₁₋₄ alkylene-NH-C₁₋₄ alkylene-; wherein m is 0 or 1, n is 1 or 2, and the C₁₋₄ alkylene is optionally substituted with C₁₋₄ alkyl, NH2, or -C₁₋₄ alkylene-NH2;
A is selected from H, M and -(CO)-M, wherein M is selected from 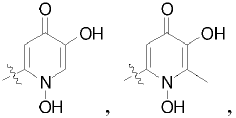 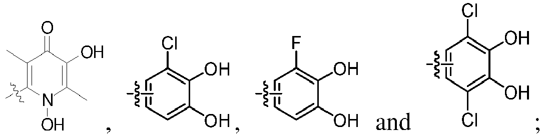
L₃ is absent;
Z is CH, N or CCl.
In a preferred embodiment, R₁ and R₂ are each methyl, or R₁ and R₂, together with the carbon atom to which they are attached, form cyclopropane, cyclobutane or cyclopentane;
R₃ is selected from H, methyl and CH2F;
L₁ is -NH-C(O)-;
X is selected from 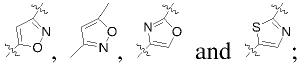
Y is absent or is selected from the following groups: 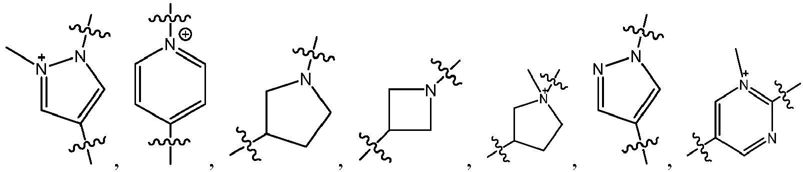 and phenyl;
L₂ is absent or is selected from -C₁₋₄ alkylene-NH-, -C₁₋₄ alkylene-(NH)₂-, -NH-C₁₋₄ alkylene-NH-, -C₁₋₄ alkylene-NH-C(=NH)-NH-, and -C₁₋₄ alkylene-NH-C₁₋₄ alkylene-; wherein the C₁₋₄ alkylene is optionally substituted with NH2 or -CH2-NH2;
A is selected from H, M and -(CO)-M, wherein M is selected from 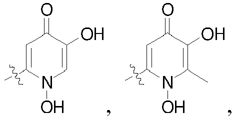 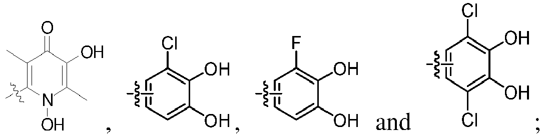
L₃ is absent;
Z is CH, N or CCl.

In a preferred embodiment, the compound of the present invention is selected from: 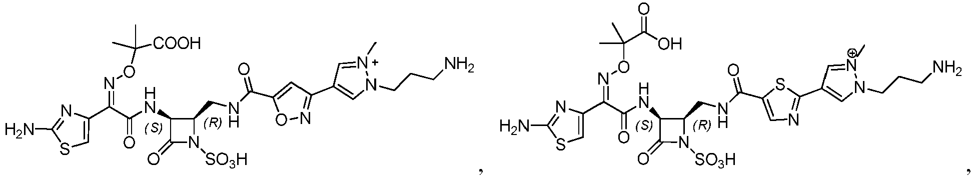 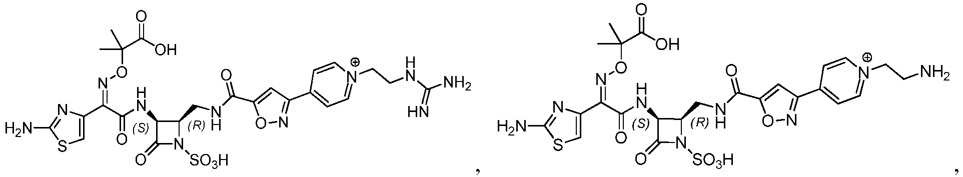 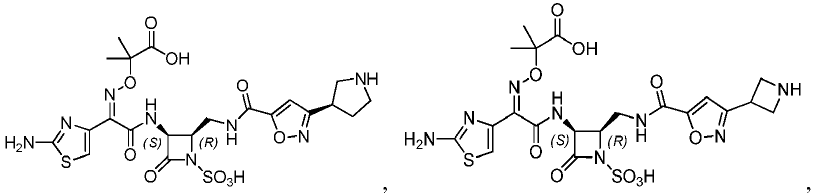 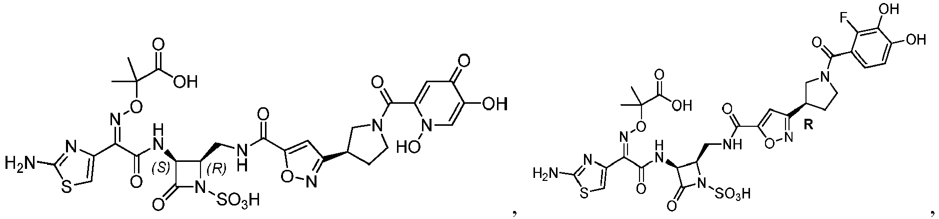 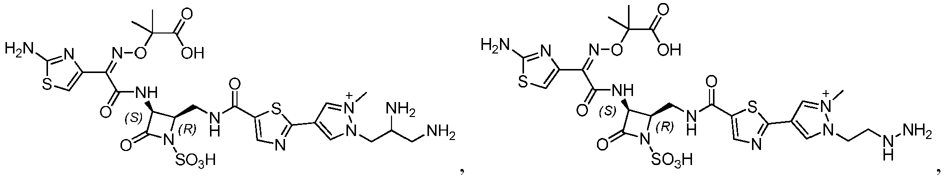 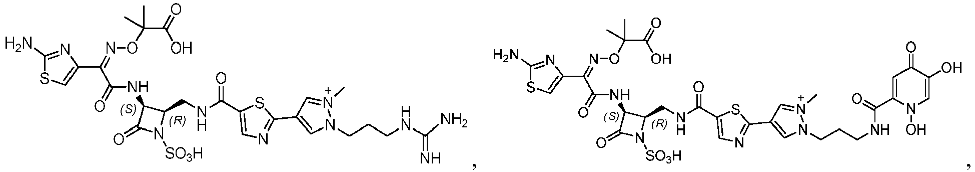 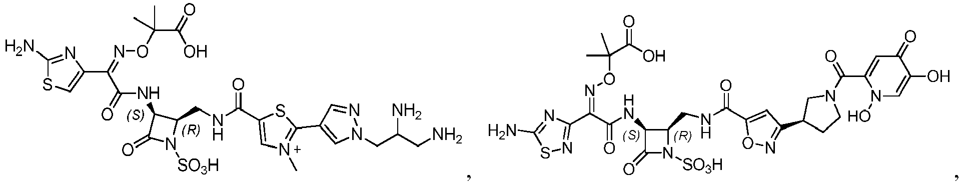 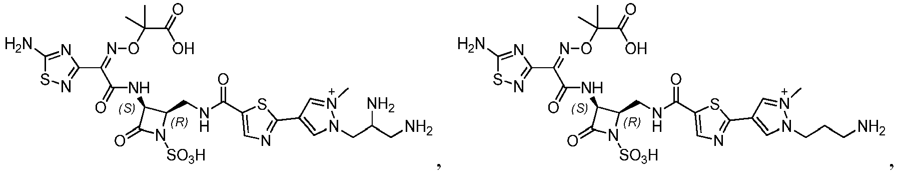 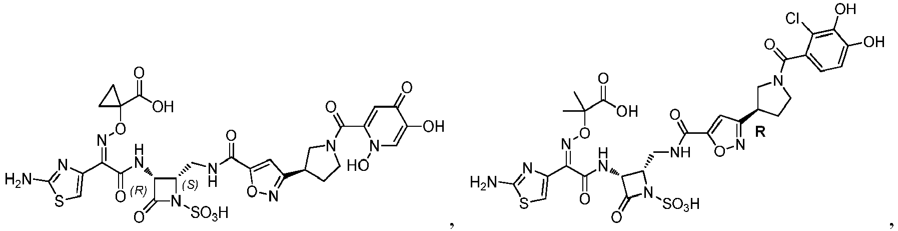 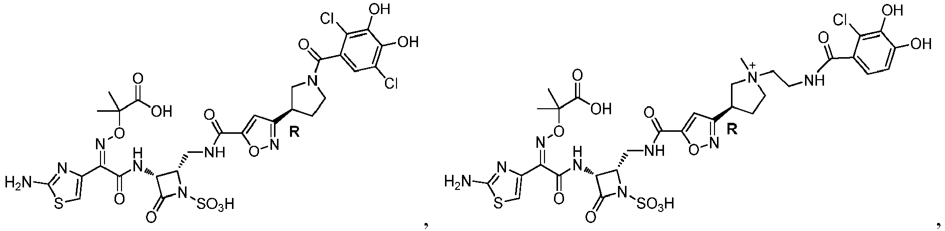 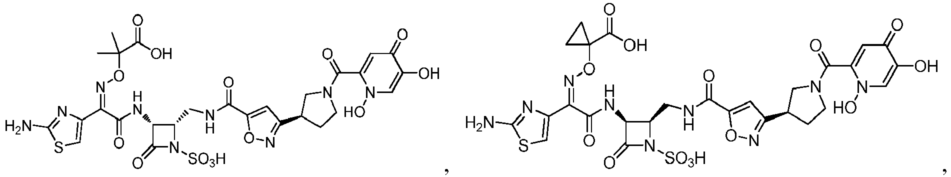 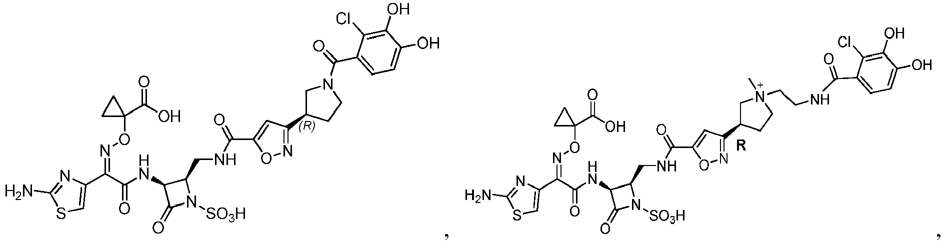 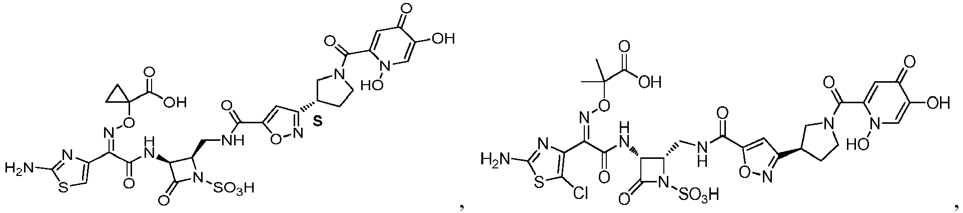 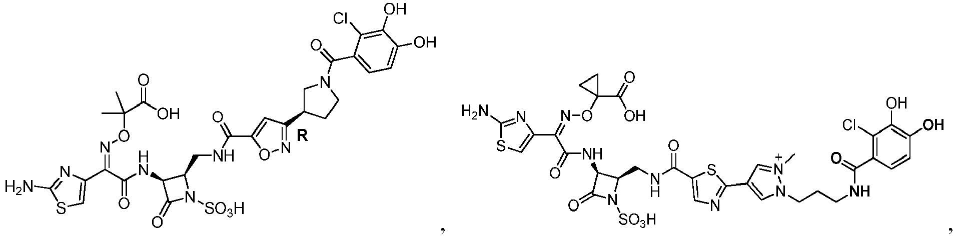 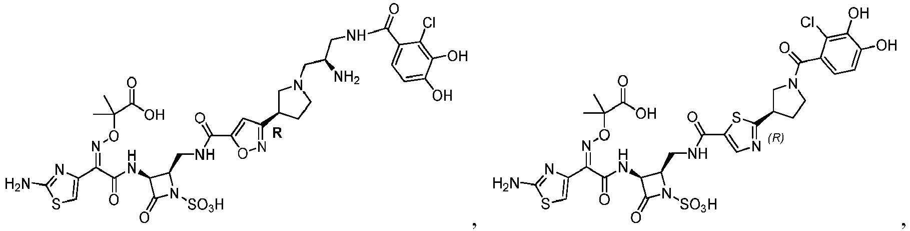 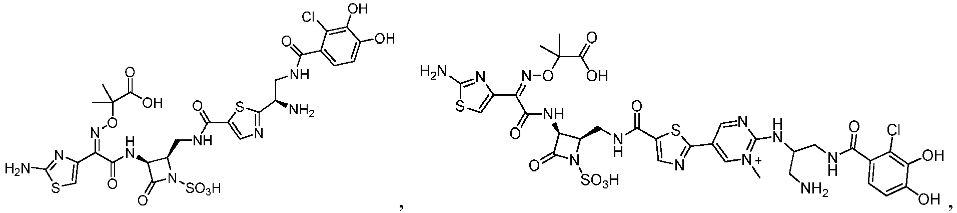 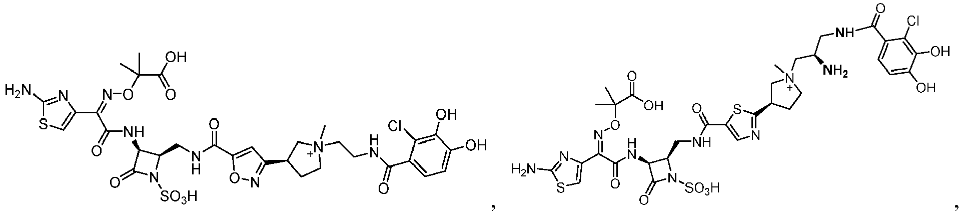 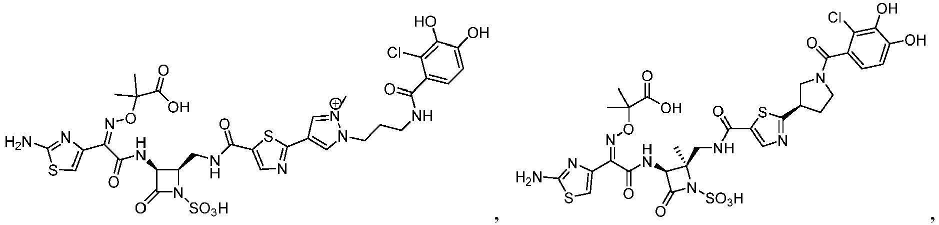 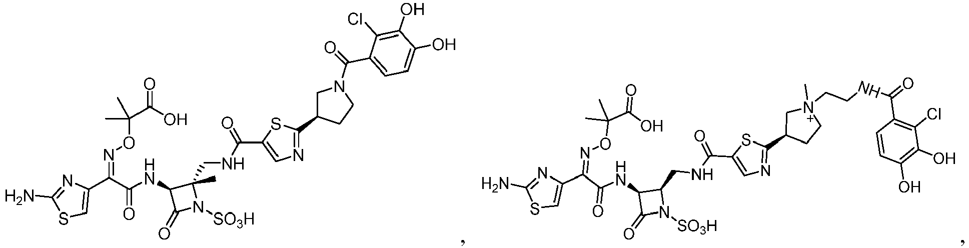 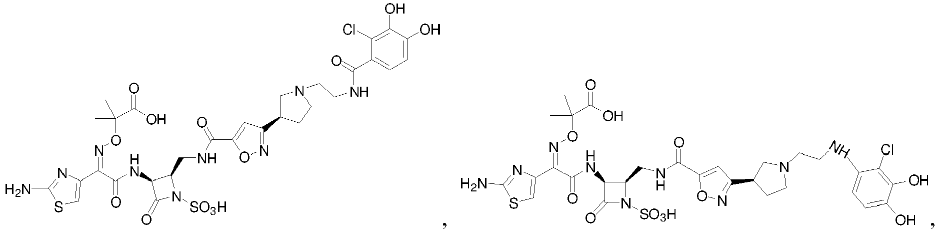 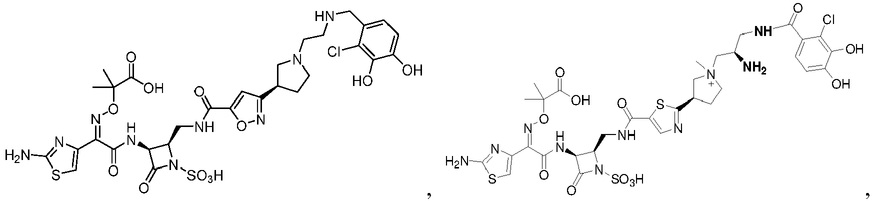 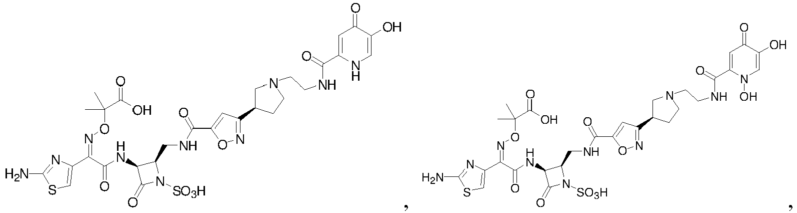 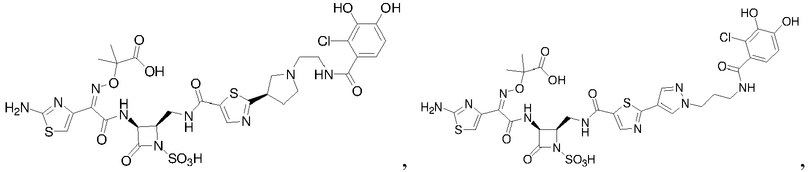 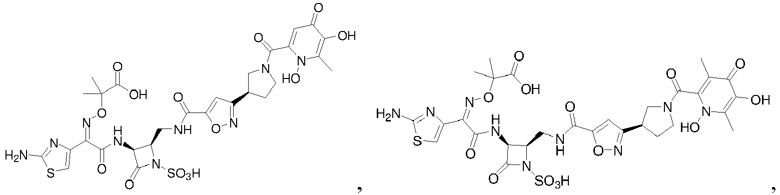 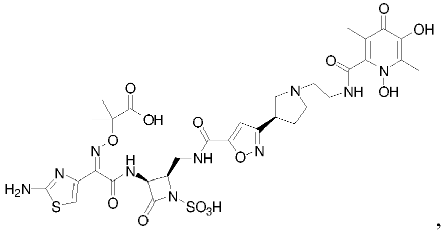 and 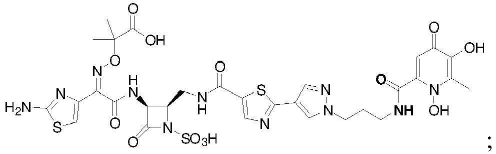 or an ester, a stereoisomer and a pharmaceutically acceptable salt thereof.

In another aspect, the present invention provides a pharmaceutical composition comprising the compound of formula (I) according to the present invention or an ester, a stereoisomer and a pharmaceutically acceptable salt thereof, and a pharmaceutically acceptable carrier. The pharmaceutically acceptable carrier may be a solid or a liquid. The solid carrier may be one or more substances used as excipients, diluents, sweeteners, solubilizers, lubricants, binders, tablet disintegrating agents, stabilizers, preservatives, or encapsulating materials. The liquid carrier may be a solvent or a liquid dispersion medium. Suitable solid carriers include, but are not limited to, for example, cellulose, glucose, lactose, mannitol, magnesium stearate, magnesium carbonate, sodium carbonate, sodium saccharin, sucrose, dextrin, talc, starch, pectin, gelatin, tragacanth, acacia, sodium alginate, methylparaben, methylcellulose, sodium carboxymethylcellulose, low-melting-point wax, cocoa butter, and the like. Suitable liquid carriers include, but are not limited to, water, ethanol, polyols (e.g., glycerol, propylene glycol, liquid polyethylene glycols, and the like), vegetable oils (e.g., peanut oil, cottonseed oil, safflower oil, sesame oil, olive oil, corn oil, and soybean oil), glycerides, agar, pyrogen-free water, isotonic saline, Ringer's solutions, and mixtures thereof.

The pharmaceutical composition of the present invention may additionally comprise one or more β-lactam antibiotics and β-lactamase inhibitors. The β-lactam antibiotics described herein can include penicillins, cephalosporins, monobactams, carbapenems, and penemase inhibitors. Specifically, the β-lactam antibiotics can include penicillin, oxacillin, cloxacillin, dicloxacillin, flucloxacillin, ampicillin, pivampicillin, amoxicillin, carbenicillin, furbenicillin, sulbenicillin, ticarcillin, piperacillin, mecillinam, cephalothin, cephaloridine, cefazolin, cefradine, cefuroxime, cefaclor, cefotaxime, ceftriaxone, ceftazidime, cefoperazone, cefoxitin, imipenem, aztreonam, and the like. The β-lactamase inhibitors can include various β-lactamase inhibitors which have been reported or used clinically, such as clavulanic acid, tazobactam, avibactam, diazaspirobicyclo[3.2. 1]octane compounds.

The pharmaceutical composition of the present invention may be administered to a patient or subject in need of treatment by any suitable route of administration, including oral administration, parenteral administration (including subcutaneous, intramuscular, intravenous, and intradermal administration), nasal spray administration, topical administration, rectal administration, intranasal administration, buccal administration, vaginal administration or administration via an implantable reservoir. In some embodiments, the pharmaceutical composition provided may be intravenously and/or intraperitoneally administered.

In a further aspect, the present invention provides use of the compound of formula (I) of the present invention, or an ester, a stereoisomer and a pharmaceutically acceptable salt thereof, in the manufacture of a medicament for treating and preventing bacterial infections.

In addition, the present invention also provides a method for inhibiting bacteria or treating and/or preventing a disease caused by bacterial infections, which comprises administering to a patient or subject a therapeutically and/or prophylactically effective amount of the compound of formula (I) of the present invention, or an ester, a stereoisomer or a pharmaceutically acceptable salt thereof.

In a preferred embodiment, the bacteria are one or more of Escherichia coli, Acinetobacter baumannii, Klebsiella pneumoniae, Pseudomonas aeruginosa, Enterobacter cloacae, Citrobacter spp., Citrobacter freundii, Proteus vulgaris (including Proteus mirabilis), Shigella spp., Serratia marcescens, Moraxella catarrhalis, Neisseria gonorrhoeae, Neisseria meningitidis, Campylobacter spp., Helicobacter pylori, Vibrio cholerae, Haemophilus influenzae, Mycobacterium spp., Bacteroides fragilis, Bacillus cereus, and Stenotrophomonas maltophilia.

In an embodiment, the disease caused by the bacteria is selected from: respiratory tract infection (upper respiratory tract infection such as pharyngitis, lower respiratory tract infection including trachitis, bronchitis, and pneumonia caused by *Enterobacter* spp. and *Serratia marcescens* such as community-acquired pneumonia, ventilator-acquired pneumonia, hospitalacquired pneumonia, and bronchiectasis), pulmonary tuberculosis and pulmonary infection complicated by pulmonary fibrosis, urinary tract infection (including uncomplicated and complicated pyelonephritis, recurrent cystitis, complicated urinary tract infection, and uncomplicated urinary tract infection), central nervous system infection (encephalitis, meningitis, and encephalopyosis), ear infection (otitis externa and otitis media), intra-abdominal infection including peritonitis, cardiovascular infection (blood infection such as septicemia or bacteremia, endocarditis, myocarditis, and pericarditis), skin or soft tissue infection, bone and joint infections (arthritis and osteomyelitis), genital infections (genital ulcers, vaginitis and cervicitis), ocular infection (conjunctivitis, keratitis, and endophthalmitis), oral infection (including gingivitis, periodontitis, and pulpitis), and the like. The compound of formula (I) of the present invention, or an ester, a stereoisomer or a pharmaceutically acceptable salt thereof can be especially used for treating an infection caused by aztreonam-resistant bacteria.

When required, the compound or pharmaceutical composition of the present invention can be provided in a package or with a dispensing device containing one or more unit dosage forms. For example, the package may comprise a metal or a plastic foil, or a glass and a rubber stopper (e.g., in a vial). The package or dispensing device may be accompanied by instructions for use of drugs.

The dosage depends on various factors including the age, weight and condition of a patient and the route of administration. The exact dosage to be administered is left to the discretion of the attending physician. The actual dosage levels and time frame of active ingredients of the pharmaceutical composition of the present invention may be varied so as to obtain an amount of the active ingredient that, for a particular patient, composition, and route of administration, may achieve the desired therapeutic response without posing toxicity to the patient. Typically, the medicament or pharmaceutical composition of the present invention is administered at a dose sufficient to reduce or eliminate symptoms associated with bacterial infection.

The present invention is further illustrated by the following specific examples, which are not intended to limit the present invention. Various modifications and variations may be made by those skilled in the art in light of the teachings without departing from the spirit and scope of the present invention.

### Preparation Examples

### Example 1. Synthesis of Compound 1-1

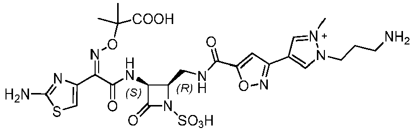

### Synthesis of compound 3

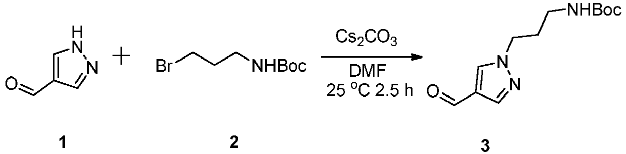

To 160 mL of N,N-dimethylformamide were added 1*H*-pyrazole-4-carbaldehyde (20 g, 208.3 mmol), *N*-Boc-3-aminopropyl bromide (49.56 g, 208.16 mmol) and cesium carbonate (68 g, 208.16 mmol). The reaction mixture was stirred at room temperature for 2.5 h, diluted with 800 mL of ethyl acetate, and washed twice with 500 mL of sodium chloride. The organic phase was dried over anhydrous sodium sulfate and concentrated to dryness by rotary evaporation to give a colorless oil (50 g), which was directly used in the next step. m/z (ES⁺), [M+H]⁺ = 254.

### Synthesis of compound 4

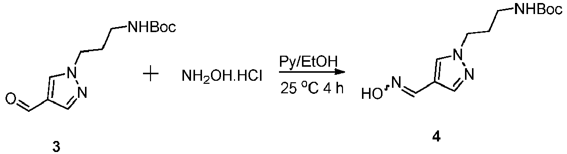

To 400 mL of ethanol were added compound 3 (crude, 50 g, 208 mmol), hydroxylamine hydrochloride (20.84 g, 300 mmol) and pyridine (47.44 g, 600 mmol). The reaction mixture was stirred at room temperature for 4 h and concentrated to dryness by rotary evaporation. The residue was directly purified by silica gel column chromatography (PE : EA = 0-50%) to give compound 4 as a white solid (40 g, 71.6%). m/z (ES⁺), [M+H]⁺ = 269.

### Synthesis of compound 6

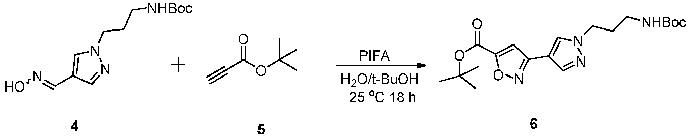

To 160 mL of tert-butanol and 80 mL of water were added compound 4 (20 g, 74.62 mmol) and *tert-butyl* propiolate (25.2 g, 200 mmol), followed by (bis(trifluoroacetoxy)iodo)benzene (56 g, 133.32 mmol) in batches. The reaction mixture was stirred at room temperature overnight. 800 mL of ethyl acetate was added to the reaction mixture, and the resulting mixture was washed with 800 mL of water. The organic phase was washed with 600 mL of a saturated aqueous solution of sodium chloride, dried over anhydrous sodium sulfate, and concentrated to dryness by rotary evaporation. The residue was purified by silica gel column chromatography (PE : EA = 0-50%) to give compound 6 as a pale oil (3 g, 10.3%). m/z (ES⁺), [M+H]⁺ = 393.

### Synthesis of compound 7

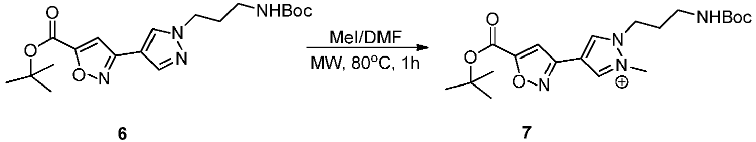

Compound 6 was added to 12 mL of N,N-dimethylformamide and 6 mL of iodomethane. The reaction mixture was microwaved at 80°C for 5 h. After reaction, the reaction mixture was cooled, and a solid precipitated. The solid was collected by filtration to give yellow compound 7 (2.5 g, 80.6%). m/z (ES⁺), [M+H-56]⁺ = 351.

### Synthesis of compound 8

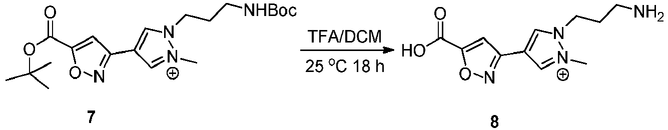

Trifluoroacetic acid (3 mL) was added dropwise to compound 7 (2.5 g, 6.1 mmol) in dichloromethane (6 mL). The reaction mixture was stirred at room temperature overnight, and a solid precipitated. The mixture was filtered to give a yellow solid, which was directly used in the next step. m/z (ES⁺), [M+H-56]⁺ = 251.

### Synthesis of compound 9

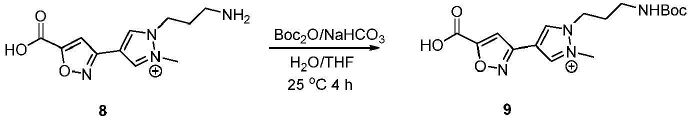

Compound 8 (about 6.1 mmol) was added to a mixture of water (40 mL) and tetrahydrofuran (20 mL), and then the pH of the mixture was adjusted to about 8 with sodium bicarbonate, followed by the addition of Boc anhydride (2.5 g). The reaction mixture was stirred at room temperature for 4 h and concentrated to dryness by rotary evaporation. The resulting solid was purified by reversed-phase chromatography to give white compound 9. (1 g, 46.5% for 2 steps). m/z (ES⁺), [M+H]⁺ = 352.

### Synthesis of compound 11

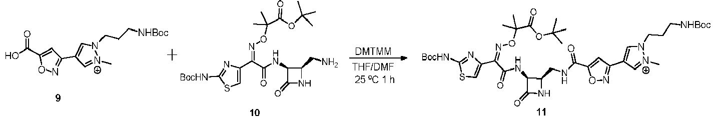

Compound 10 (315 mg, 0.6 mmol) and compound 9 (252 mg, 0.72 mmol) were added to tetrahydrofuran (6 mL) and *N*,*N*-dimethylformamide (4 mL), and DMTMM (200 mg, 0.96 mmol) was added to the reaction mixture. The reaction mixture was stirred at room temperature under nitrogen for 1.5 h and purified by reversed-phase chromatography to give compound 11 as a white solid (196 mg, 38%). m/z (ES+), [M+H]+ = 860; acid, HPLC tR = 0.935 min.

### Synthesis of compound 12

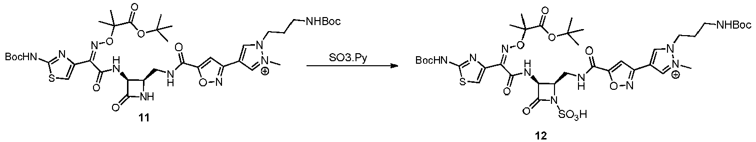

Sulfur trioxide pyridine complex (200 mg, 1.14 mmol, 5 eq) was added to a solution of compound 11 (196 mg, 0.228 mmol, 1.00 eq) in *N*,*N*-dimethylformamide (3 mL). The reaction mixture was stirred at room temperature under nitrogen overnight and purified by reversed-phase chromatography to give compound 12 as a white solid (66 mg, 30.8%). m/z (ES+), [M]+ = 939; acid, HPLC tR = 2.170 min.

### Synthesis of Compound 1-1

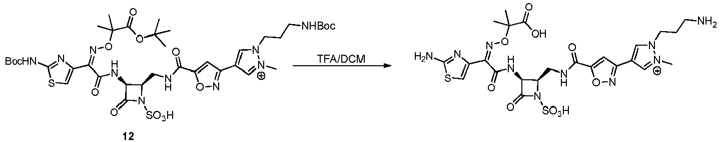

Trifluoroacetic acid (1 mL) was added dropwise at 0 °C to compound 12 (60 mg, 0.06 mmol, 1.00 eq) in dichloromethane (4 mL). The reaction mixture was stirred at 0 °C for 6 h, blow-dried with nitrogen, and washed four times with diethyl ether by centrifugation. The resulting solid was purified by preparative chromatography (column: Xselect CSH OBD column 30 × 150 mm 5 µm, n; mobile phase A:; mobile phase B: ACN; flowrate: 60 mL/min; gradient: from 1 B to 15 B over 7 min; 254/220 nm;) to give an off-white solid (26.9 mg, 61.6%). m/z (ES+), [M+H]+ = 683; acid, HPLC tR = 0.833 min. 1H NMR (D₂O, 400 MHz): δ (ppm) 1.39 (s, 6H), 2.31 (p, J = 7.7 Hz, 2H), 2.96-3.17 (m, 2H), 3.77 (d, J = 6.4 Hz, 2H), 4.15 (s, 3H), 4.53-4.60 (m, 3H), 5.31 (t, 1H), 7.06 (s, 1H), 7.23 (s, 1H), 8.73 (s, 1H), 8.80 (s, 1H).

### Example 2. Synthesis of Compound 1-2

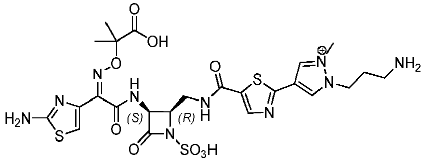

### Synthesis of compound 2

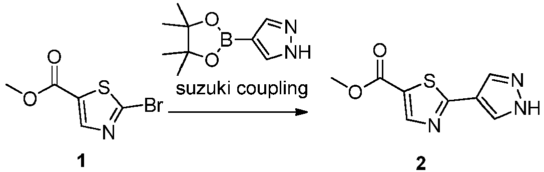

To dioxane (50 mL) and water (2.5 mL) were added methyl 2-bromothiazole-5-carboxylate (8 g, 36 mmol, 1 eq), 4-pyrazoleboronic acid pinacol ester (8.389 g, 43 mmol, 1.2 eq), tetrakis(triphenylphosphine)palladium (0.416 g, 0.36 mmol, 0.01 eq) and potassium carbonate (9.9 g, 72 mmol, 2 eq). The reaction mixture was refluxed under nitrogen for 2 h. Water was added to the reaction mixture, which was then extracted with ethyl acetate. The organic phase was dried over anhydrous sodium sulfate and concentrated to dryness by rotary evaporation. The residue was purified by silica gel column chromatography to give compound 2 (3 g, crude). m/z (ES+), [M+H]+ = 210; acid, HPLC tR = 0.871 min.

### Synthesis of compound 3

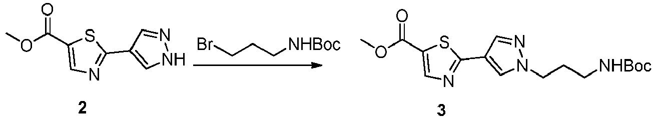

To *N*,*N*-dimethylformamide (30 mL) were added compound 2 (3 g, crude), *N*-Boc-3-aminopropyl bromide (3.4 g, 0.014 mmol, 1 eq) and cesium carbonate (4.66 g, 0.014 mmol, 1 eq). The reaction mixture was stirred at room temperature for 3 h, diluted with water, and extracted with ethyl acetate. The organic phase was dried over anhydrous sodium sulfate and concentrated to dryness by rotary evaporation. The residue was purified by column chromatography with petroleum ether/ethyl acetate to give compound 3 (1.5 g, 11% for two steps). m/z (ES+), [M]+ = 366.9; base, HPLC tR = 0.227 min.

### Synthesis of compound 4

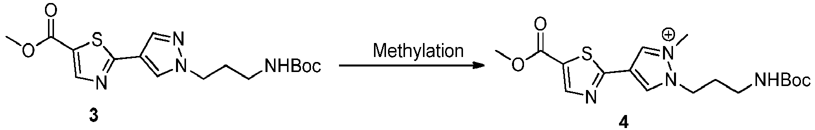

Compound 3 (0.5 g, 1.36 mmol) was added to iodomethane (1 mL) and *N,N-*dimethylformamide (2.5 mL). The reaction mixture was microwaved at 80 °C for 3 h and concentrated to dryness by rotary evaporation. The crude product was purified by reversed-phase chromatography to give compound **4** (0.2 g, 38.6%). m/z (ES+), [M]+ = 381; acid, HPLC tR = 0.737 min.

### Synthesis of compound 5

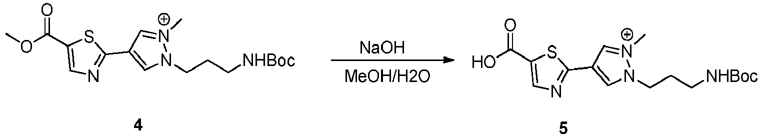

Aqueous sodium hydroxide solution (0.7 mL, 2 M) was added to a solution of compound 4 (0.2 g, 1.36 mmol) in methanol (1 mL). The reaction mixture was stirred at room temperature for 1 h and adjusted to pH 7 with citric acid, and a solid precipitated. The solid was purified by reversed-phase chromatography to give compound 5 as a yellow solid (0.15 g, 81%). m/z (ES+), [M]+ = 367; acid, HPLC tR = 0.967 min.

### Synthesis of compound 6

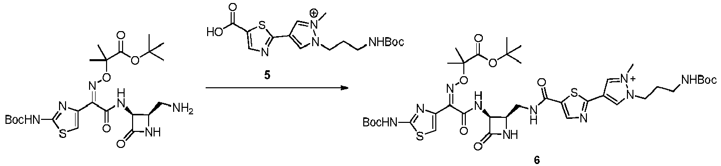

Amine (400 mg, 0.76 mmol) and compound 5 (545 mg, 1.48 mmol) were added to tetrahydrofuran (8 mL) and N,N-dimethylformamide (5 mL), and DMTMM (545 mg, 1.97 mmol) was added under nitrogen. The reaction mixture was stirred at room temperature under nitrogen for 2 h and purified by reversed-phase chromatography to give compound 6 as a white solid (20 mg, 3%). m/z (ES+), [M]+ = 876; acid, HPLC tR = 1.383 min.

### Synthesis of compound 7

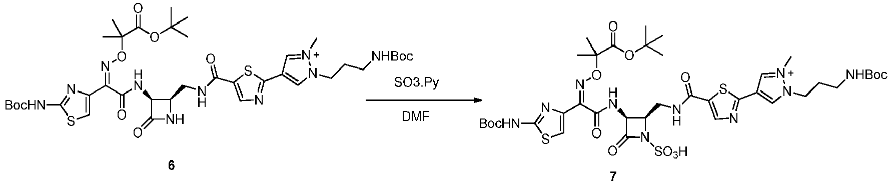

Sulfur trioxide pyridine complex (72 mg, 0.45 mmol, 20 equiv) was added to compound 6 (20 mg, 0.022 mmol, 1.00 equiv) in *N*,*N*-dimethylformamide (3 mL). The reaction mixture was stirred at room temperature under nitrogen overnight and purified by reversed-phase chromatography to give compound 7 as a white solid (10 mg, 50%). m/z (ES+), [M]+ = 956; acid, HPLC tR = 2.487 min.

### Synthesis of compound 1-2

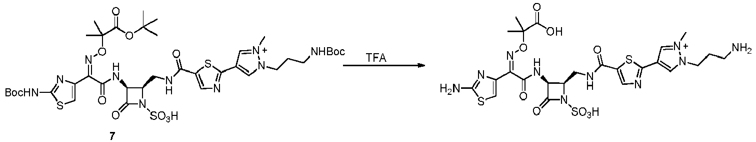

Trifluoroacetic acid (2 mL) was added dropwise at 0 °C under nitrogen to compound 7 (10 mg, 0.047 mmol) in dichloromethane (2 mL). The reaction mixture was stirred at 0 °C for 5 h and purified by preparative high-pressure liquid chromatography (column: Sun Fire C18 OBD preparative column, 100 Å, 5 µm, 19 mm × 250 mm; mobile phase A: water (0.1% FA), mobile phase B: ACN; flowrate: 60 mL/min; gradient: from 1 B to 26 B over 7 min; 254/220 nm; RT1: 2.42, 6.30; RT2:) to give a white compound (1.4 mg, 19.1% ). 1H NMR (D₂O, 400 MHz): δ (ppm) 1.43 (d, J = 2.6 Hz, 6H), 1.51 (d, J = 4.1 Hz, 1H), 2.40 (p, J = 7.4 Hz, 2H), 3.11-3.24 (m, 2H), 3.64-3.86 (m, 1H), 4.23 (s, 2H), 4.57 (q,J = 6.3 Hz, 1H), 4.65 (t, J = 7.5 Hz, 2H), 5.35 (d, J = 5.7 Hz, 1H), 6.91 (s, 1H), 8.22 (s, 1H), 8.43 (s, 1H), 8.87 (d, J = 29.0 Hz, 2H).

### Example 3. Synthesis of Compound 1-3

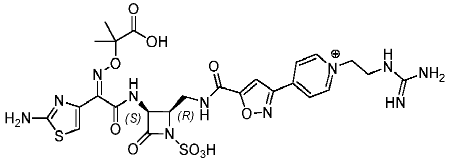

### Synthesis of compound 2

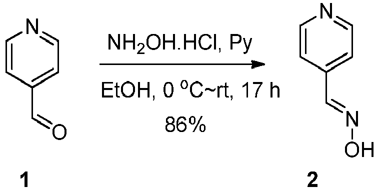

Pyridine (23.7 g, 0.3 mol) was added to a solution of 4-pyridinecarboxaldehyde (10.7 g, 0.1 mol) and hydroxylamine hydrochloride (10.3 g, 0.15 mol) in ethanol (70 mL). The reaction mixture was stirred at room temperature overnight and filtered. The solid was washed with ethyl acetate. The white solid was dried to give compound 2 (12 g, 98.0 mmol), which was directly used in the next step without purification. m/z (ES+), [M+H]+ = 123; acid, HPLC tR = 0.182 min.

### Synthesis of compound 4

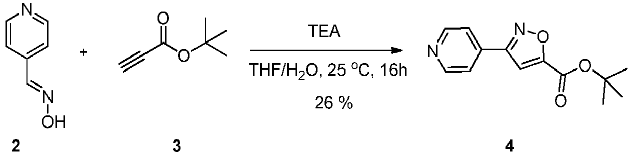

Compound 2 (4.8 g, 39.3 mmol) and *tert-butyl* propiolate (7.56 g, 60 mmol) were added to a mixture of tetrahydrofuran (80 mL) and water (40 mL), and an aqueous solution of NaOCl (61 mL) was added dropwise to the mixture (over 1 h). The reaction mixture was stirred at room temperature overnight. Water (60 mL) was added to the mixture, which was then extracted with ethyl acetate (200 mL × 3). The organic phase was washed with saturated brine (100 mL × 3), dried over anhydrous sodium sulfate, and concentrated to dryness by rotary evaporation. The residue was purified by column chromatography (EA/PE = 1/4 to 1/1) to give compound 4 as a white solid (2 g, 21%). (ES+), [M+H]+ = 247; acid, HPLC tR = 0.804 min.

### Synthesis of compound 6

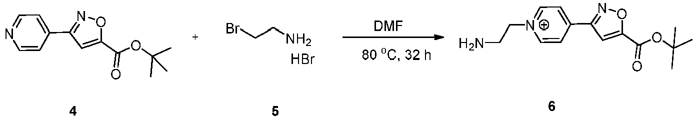

2-Bromoethylamine hydrobromide was added to compound 4 (1.8 g, 7.3 mmol) in *N,N-*dimethylformamide (6 mL) (under nitrogen). The reaction mixture was stirred at 80 °C for 22 h and cooled to room temperature. Dichloromethane (20 mL) and methyl *tert-butyl* ether (30 mL) were added, and a solid precipitated. The solid was collected by filtration and washed with methyl *tert-butyl* ether (40 mL) to give a pale brown crude product 6. m/z (ES+), [M]+ = 290; acid, HPLC tR = 1.326 min.

### Synthesis of compound 7

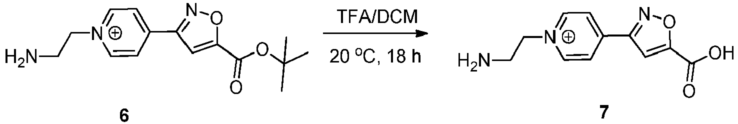

Trifluoroacetic acid (6 mL) was added to compound 6 (crude, 3.8 g, 7.3 mmol) in dichloromethane (15 mL). The reaction mixture was allowed to react at room temperature overnight. Methyl *tert-butyl* ether (70 mL) was added to the reaction mixture, and a solid precipitated. The solid was collected by filtration to give brown compound 7 (3.0 g). m/z (ES+), [M]+ = 234; acid, HPLC tR = 0.366 min.

### Synthesis of compound 8

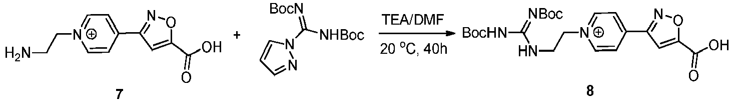

Compound 7 (crude, 1.5 g, 3.65 mmol) was dissolved in *N*,*N*-dimethylformamide (36 mL), and triethylamine (5.06 g, 50 mmol) and *N*,*N*-di-Boc-1*H*-1-guanylpyrazole (2.03 g, 6.57 mmol) were added at 0 °C under nitrogen to the reaction mixture. The reaction mixture was allowed to react at room temperature for 40 h. A cold solution of NaH₂PO₄/NaCl (1:1, 50 mL) was added to the reaction mixture, and a solid precipitated. The solid was collected by filtration and washed with MTBE/PE (1:1, 100 mL) to give yellow compound 8 (1.39 g, 80%). m/z (ES+), [M]+ = 476; acid, HPLC tR = 0.827 min. 1H NMR (methanol-*d*₄, 300 MHz): δ (ppm) 1.28 (s, 9H), 1.54 (s, 9H), 3.36 (s, 2H), 4.03 (t, J = 5.3 Hz, 2H), 7.46 (s, 1H), 8.57 (d, J = 6.5 Hz, 2H), 9.13 (d, J = 6.5 Hz, 2H).

### Synthesis of compound 9

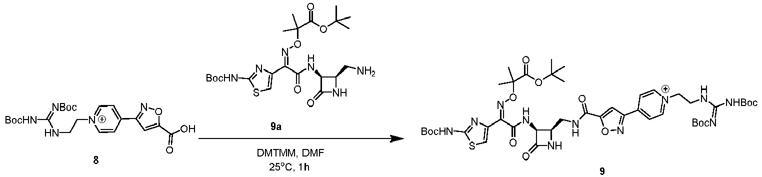

Amine (315 mg, 0.6 mmol) and compound 8 (342 mg, 0.72 mmol) were added to tetrahydrofuran (5 mL) and N,N-dimethylformamide (3 mL), and DMTMM (166 mg, 0.6 mmol) was added to the mixture under nitrogen. The reaction mixture was stirred at room temperature for 1 h. Icy NaH₂PO₄ (20 mL) was added to the reaction mixture, which was then extracted with ethyl acetate (20 mL × 2). The organic phase was washed with water (40 mL), and the aqueous phase was extracted with ethyl acetate (20 mL). The ethyl acetate phases were combined, washed with saturated brine (40 mL), dried over anhydrous sodium sulfate, and concentrated to dryness by rotary evaporation. The residue was and purified by reversed-phase chromatography to give compound 9 as a pale yellow solid (177 mg, 30%). m/z (ES-), [M-H] = 984; acid, HPLC tR = 1.191 min.

### Synthesis of compound 10

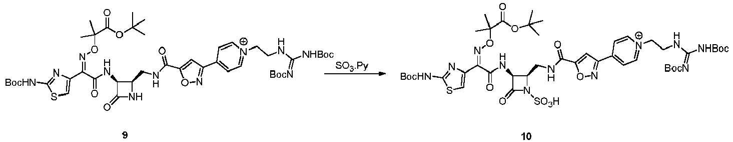

Sulfur trioxide pyridine complex (200 mg, 1.14 mmol, 6.3 eq) was added to compound 9 (177 mg, 0.18 mmol, 1.00 eq) in *N*,*N*-dimethylformamide (3 mL). The reaction mixture was stirred at room temperature under nitrogen overnight and purified by reversed-phase chromatography to give compound 10 as a white solid (60 mg, 31.4%). m/z (ES-), [M-H] = 1064; acid, HPLC tR = 1.383 min.

### Synthesis of compound 1-3

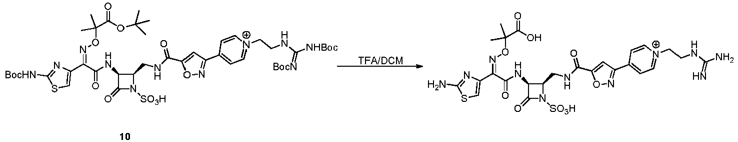

Trifluoroacetic acid (1 mL) was added dropwise at 0 °C under nitrogen to compound 10 (60 mg, 0.056 mmol, 1.00 eq) in dichloromethane (4 mL). The reaction mixture was stirred at 0 °C for 6 h and blow-dried with nitrogen to remove the solvent. The mixture was washed 4 times with diethyl ether (10 mL) by centrifugation. The resulting solid was purified by preparative high-pressure liquid chromatography (column: Xselect CSH OBD column 30 × 150 mm 5 µm, n; mobile phase A:; mobile phase B: ACN; flowrate: 60 mL/min; gradient: from 1 B to 15 B over 7 min; 254/220 nm; RT1: 6.63; RT2:; volume of injection: mL; numbers of performance:;) to give a compound as a white solid (14.3 mg). m/z (ES+), [M]+ = 708; acid, HPLC tR = 0.952 min. 1H NMR (D₂O, 400 MHz): δ (ppm) 1.40 (s, 6H), 3.72-3.91 (m, 4H), 4.56 (t, J = 6.1 Hz, 1H), 4.79 (t, J = 5.5 Hz, 2H), 5.31 (d, J = 5.7 Hz, 1H), 6.97 (s, 1H), 7.59 (s, 1H), 8.51 (d, J = 6.5 Hz, 2H), 8.96 (d, J = 6.6 Hz, 2H).

### Example 4. Synthesis of Compound 1-4

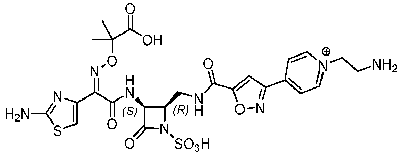

### Synthesis of compound 2

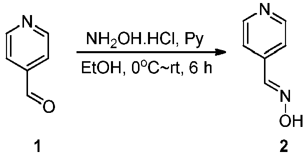

Pyridine (23.7 g, 0.30 mol) was added to compound 1 (10.7 g, 0.10 mol) and hydroxylamine hydrochloride (10.3 g, 0.15 mol) in ethanol (70 mL). The reaction mixture was stirred at room temperature for 6 h. The resulting solid was collected by filtration, washed with ethyl acetate, and dried to give compound 2 as a white solid (10 g, 82% yield), which was directly used in the next step. m/z (ES+), [M+H]⁺ = 123; ¹H NMR (300 MHz, DMSO-*d*₆) δ 12.82 (s, 1H), 8.94-8.86 (m, 2H), 8.43 (s, 1H), 8.20-8.09 (m, 2H).

### Synthesis of compound 3

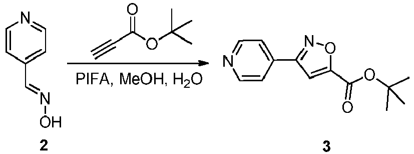

(Bis(trifluoroacetoxy)iodo)benzene (26.4 g, 82.0 mmol) was added in batches at 0 °C to compound 2 (5 g, 41.0 mmol) and *tert-butyl* propiolate (13 g, 102.5 mmol) in methanol (60 mL) and water (30 mL). The reaction mixture was stirred at room temperature for 2 h and concentrated to remove methanol by rotary evaporation. Water (30 mL) was added to the solution, which was then extracted with ethyl acetate (100 mL × 3). The organic phase was washed with saturated brine (100 mL × 2), dried over anhydrous sodium sulfate, and concentrated to dryness by rotary evaporation. The residue was purified by silica gel column chromatography (ethyl acetate/petroleum ether = 1/4 to 1/1) to give compound 3 as a white solid (2.3 g, 23%). m/z (ES⁺), [M+H]⁺ = 247.

### Synthesis of compound 4

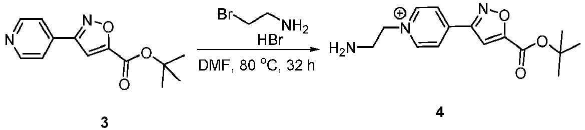

2-Bromoethylamine hydrobromide (4.2 g, 20.5 mmol) was added under nitrogen to compound 3 (1.0 g, 4.1 mmol) in *N*,*N*-dimethylformamide (10 mL). The reaction mixture was stirred at 80 °C for 32 h and cooled to room temperature. Dichloromethane (15 mL)/methyl *tert-*butyl ether (20 mL) was added to the mixture, and a solid precipitated. The solid was washed with methyl *tert-butyl* ether (40 mL) to give compound 4 as a pale brown solid, which was directly used in the next step.

### Synthesis of compound 5

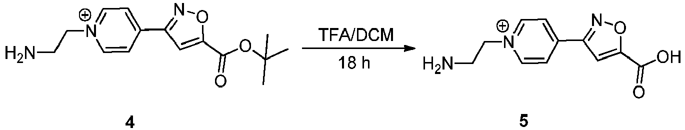

Trifluoroacetic acid (5 mL) was added dropwise to compound 4 (1 g, 3.45 mmol, crude) in dichloromethane (10 mL). The reaction mixture was stirred at room temperature overnight (under nitrogen). The organic phase was concentrated to dryness by rotary evaporation, and the residue was crystallized from dichloromethane/methanol to give compound 5 as a solid (660 mg, 69.4% yield for 2 steps). m/z (ES⁺), [M]+ = 234.

### Synthesis of compound 6

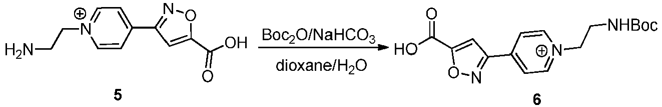

Sodium bicarbonate (438 mg, 5.21 mmol) and di-*tert*-butyl dicarbonate (2.27 g, 10.43 mmol) were added at 0 °C to a mixture of compound 5 (610 mg, 2.61 mmol) in tetrahydrofuran/water (1:2, 15 mL). The reaction mixture was stirred at room temperature for 2 h and concentrated by rotary evaporation to remove the solvent. The residue was purified by reversed-phase chromatography to give compound 6 as a white solid (530 mg, 61%). m/z (ES⁺), [M]+ = 334.

### Synthesis of compound 7

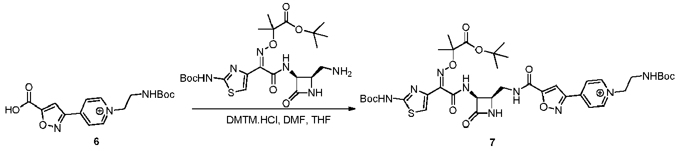

4-(4,6-Dimethoxytriazin-2-yl)-4-methylmorpholinium hydrochloride (DMTMM) (74.3 mg, 252 µmol) was added to compound 6 (56 mg, 168 µmol) and amine (88.2 mg, 168 µmol) in tetrahydrofuran (2.5 mL) and N,N-dimethylformamide (1.5 mL). The reaction mixture was stirred at room temperature under nitrogen for 2 h, added to a cold aqueous solution of sodium dihydrogen phosphate (10 mL), and extracted with ethyl acetate (10 mL × 2). The organic phase was washed with water (20 mL), and the aqueous phase was reversely extracted with ethyl acetate (10 mL). The organic phases were combined, washed with saturated brine (20 mL), dried over anhydrous sodium sulfate, and concentrated to dryness by rotary evaporation. The residue was purified by reversed-phase chromatography to give compound 7 as a white solid (135 mg, 95%). m/z (ES⁺), [M]+ = 842.

### Synthesis of compound 8

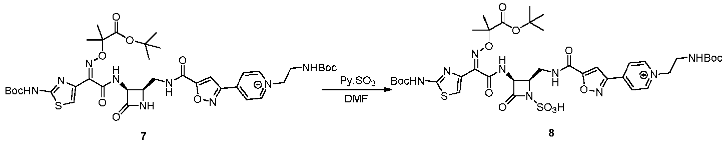

Sulfur trioxide pyridine complex (255 mg, 1.6 mmol) was added to compound 7 (135 mg, 0.16 mmol) in *N*,*N*-dimethylformamide. The reaction mixture was stirred at 45 °C under nitrogen for 2 h and purified by reversed-phase chromatography to give compound 8 as a white solid (88 mg, 95%). m/z (ES⁺), [M+H]⁺ = 924.

### Synthesis of compound 1-4

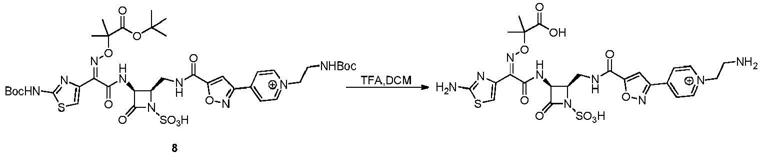

Trifluoroacetic acid (2 mL) was added dropwise at 0 °C under nitrogen to compound 8 (50 mg, 0.054 mmol) in dichloromethane (2 mL). The reaction mixture was stirred at 0 °C for 6 h and blow-dried with nitrogen. The solid was lyophilized and purified by preparative high-pressure liquid chromatography to give a product as a white solid (13.9 mg, 38.5%). m/z (ES⁺), [M+H]⁺ = 667; ¹H NMR (400 MHz, D₂O) δ 9.02 (d, J = 7.0 Hz, 2H), 8.52 (d, J = 6.8 Hz, 2H), 7.59 (s, 1H), 6.93 (s, 1H), 5.30 (d, J = 5.7 Hz, 1H), 4.96 (t, J = 6.3 Hz, 2H), 4.56 (d, J = 5.9 Hz, 1H), 3.78 (d, J = 6.7 Hz, 2H), 3.66 (q, J = 7.5, 6.9 Hz, 2H), 1.39 (s, 6H).

### Example 5. Synthesis of Compound 1-5

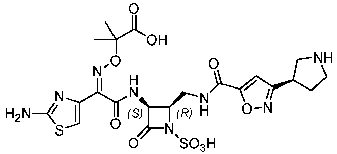

### Synthesis of compound 2

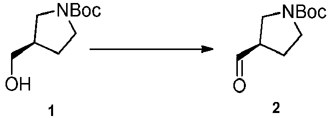

SO3•Py (24 g, 149.1 mmol, 3 eq) and Et3N (34 mL, 302.5 mmol, 6.5 eq) were successively added under N2 at 0 °C to a mixture of *tert-butyl* (*R*)-3-(hydroxymethyl)pyrrolidine-1-carboxylate (10 g, 49.7) in DMSO (40 mL) and DCM (20 mL). The reaction mixture was stirred at room temperature for 2 h, then diluted with NaHCO₃ (a saturated aqueous solution, 200 mL), and extracted with diethyl ether (400 mL × 2). The organic phases were combined and washed successively with HCl (1 mol/L), NaHCO₃ (a saturated aqueous solution) and NaCl (a saturated aqueous solution). Then the organic solvent was removed by rotary evaporation under reduced pressure to give compound 2 as a crude product (9.37 g, crude).

### Synthesis of compound 3

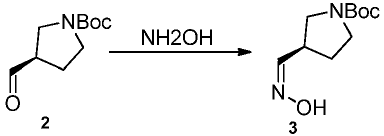

Compound 2 (9.37 g, 0.045 mol, 1 eq), hydroxylamine hydrochloride (4.68 g, 0.0678 mol, 1.5 eq) and pyridine (10.7 g, 0.135 mol, 3 eq) were added to ethanol (100 mL). The reaction mixture was stirred at room temperature overnight, concentrated by rotary evaporation to remove the solvent, diluted with water, and extracted with ethyl acetate (100 mL). The organic layer was dried over anhydrous sodium sulfate and filtered. The filtrate was concentrated by rotary evaporation to give a crude product, which was further purified by silica gel column chromatography (petroleum ether and ethyl acetate) to give compound 3 as a yellow oil (8.5 g, 85%).

m/z (ES -), [M-H] = 213; base, HPLC tR = 1.069 min.

### Synthesis of compound 4

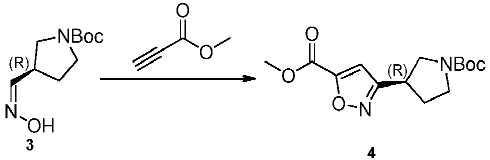

Compound 3 (9 g, 42 mmol, 1 eq), methyl propiolate (8.8 g, 105 mmol, 2.5 eq) and pyridine (10.7 g, 0.135 mol, 3 eq) were added to ethanol (100 mL). The reaction mixture was stirred at room temperature overnight, concentrated by rotary evaporation to remove the solvent, diluted with water, and extracted with ethyl acetate (100 mL). The organic layer was dried over anhydrous sodium sulfate and filtered. The filtrate was concentrated by rotary evaporation to give a crude product, which was further purified by silica gel column chromatography (petroleum ether and ethyl acetate) to give compound 4 as a brown oil (7 g, 67.5%). m/z (ES+), [M+MeCN+Na]+ = 360; acid, HPLC tR = 0.988 min.

### Synthesis of compound A-11

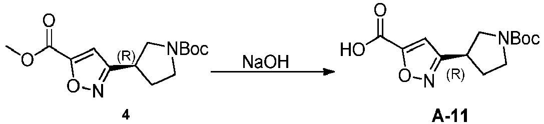

A solution of NaOH (0.81 g, 0.02 mol, 2 eq) in water (8 mL) was added to a solution of compound 4 (3 g, 0.01 mol) in MeOH (20 mL). The resulting mixture was stirred at room temperature for 2 h and acidified with HCl. The solvent was removed, and the crude product was purified by reversed-phase column chromatography with acetonitrile and purified water to give compound A-11 as a yellow oil (1.36 g, 45.9%). m/z (ES-), [M-H]- = 281; acid, HPLC tR = 0.967 min.

### Synthesis of compound 6

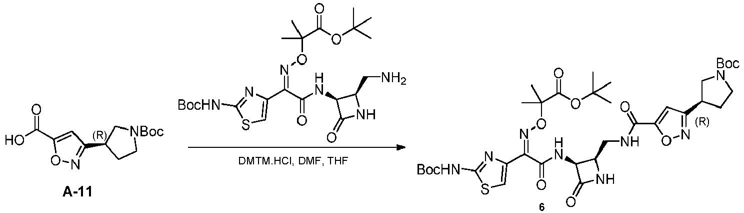

A-11 (0.174 g, 6.178 mmol, 1.3 eq) and 4-(4,6-dimethoxytriazin-2-yl)-4-methylmorpholinium hydrochloride (0.210 g, 7.129 mol, 1.5 eq) were added to a mixture of *tert*-butyl((((*Z*)*-*2-(((2*R*,3*S*)-2-(aminomethyl)-4-oxoazetidin-3-yl)amino)*-*1-(2-((*tert*butoxycarbonyl)amino)thiazol-4-yl)-2-oxoethyl)amino)-2-methylpropionate (0.25 g, 4.75 mmol, 1 eq) in DMF and THF (3:5). The resulting mixture was stirred at room temperature for 2 h, concentrated, and added to a cold, half-saturated solution of NaH₂PO₄. The mixture was extracted with ethyl acetate, and the organic phases were combined, washed with saturated brine, dried over anhydrous sodium sulfate, and filtered. The filtrate was concentrated by rotary evaporation. The mixture was further purified by silica gel chromatography to give compound 6 as a yellow oil (0.3 g, 79%). m/z (ES+), [M+H] + = 791; acid, HPLC tR = 1.130 min.

### Synthesis of compound 7

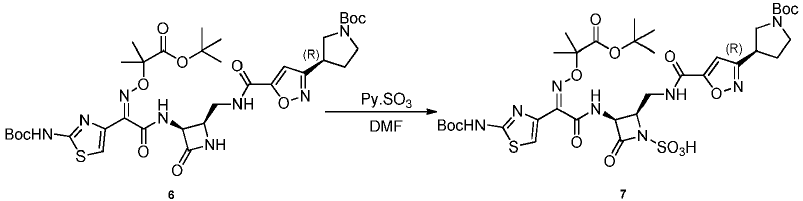

SO₃•Py (0.25 g, 1.5 mmol, 6 eq) was added under nitrogen to a solution of compound 6 (0.2 g, 0.25 mmol, 1 eq) in DMF (0.210 g, 7.129 mol, 1.5 eq). The resulting mixture was stirred at 45 °C for 2 h and purified by preparative-HPLC with H₂O and MeCN. The product fractions were directly lyophilized to give the title compound 7 as a white solid (0.1 g, 40%). m/z (ES-), [M-H]⁻ = 869; base, HPLC tR = 1.269 min.

### Synthesis of compound 1-5

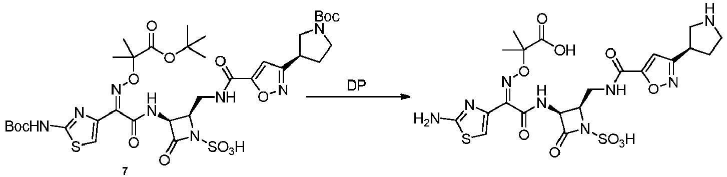

TFA (0.5 mL) was added at 0 °C to a solution of compound 7 (0.05 g, 40%) in DCM (1 mL). The mixture was stirred at 0 °C for 3 h and blow-dried with nitrogen to remove the solvent and thus to give a crude product, which was then purified by preparative HPLC (Xselect CSH OBD column, 30 × 150 mm) using a mixture of water (containing 0.1% FA) and MeCN as the eluent. The fractions containing the desired compound were lyophilized to give compound I-5 as an off-white solid (4.8 mg).

m/z (ES+), [M+H]+ = 615; acid, HPLC tR = 0.593 min. 1H NMR (400 MHz, D₂O) δ 6.99 (d, J = 15.2 Hz, 2H), 5.35 (d, J = 5.7 Hz, 1H), 4.63 (d, 1H), 3.81-3.66 (m,4H), 3.50-3.37 (m, 3H), 2.47 (dt, J = 13.7, 7.0 Hz, 1H), 2.18 (dd, J = 13.5, 7.6 Hz, 1H), 1.42 (s, 6H).

### Example 6. Synthesis of Compound 1-6

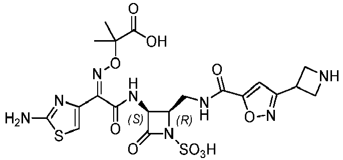

### Synthesis of compound 2

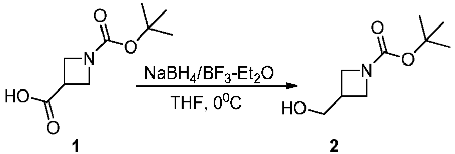

Sodium borohydride (5.67 g, 0.149 mol) was added in batches at 0 °C to a solution of *N-*Boc-3-carboxylic acid azetidine (10 g, 0.0497 mol) in tetrahydrofuran (40 mL). The reaction mixture was stirred at 0 °C for 30 min, and then a solution of boron trifluoride etherate (24 mL) was added (dropwise over 1 h). The reaction mixture was stirred under nitrogen for 16 h, quenched with ice water, and extracted with ethyl acetate (50 mL × 3). The organic phase was concentrated to dryness by rotary evaporation, and the residue was purified by silica gel column chromatography (ethyl acetate/petroleum ether =1/3 to 1/1) to give compound 2 as a pale yellow oil (2.5 g, 27%). m/z (ES⁺), [M]+ = 187.

### Synthesis of compound 3

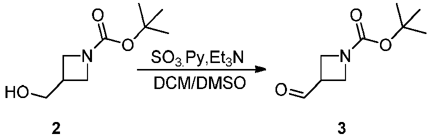

Sulfur trioxide pyridine complex (6.4 g, 0.04 mol) and triethylamine (8.1 g, 0.08 mol) were added at 0 °C under nitrogen to compound 2 (2.5 g, 0.0134 mol) in dichloromethane/dimethyl sulfoxide (5:1, 60 mL). The reaction mixture was stirred at room temperature for 1 h, added to an icy aqueous solution of sodium bicarbonate, and extracted with diethyl ether (100 mL × 3). The organic phase was washed successively with 1 mol of hydrochloric acid, a saturated aqueous solution of sodium bicarbonate and saturated brine, dried over anhydrous sodium sulfate, and concentrated to dryness by rotary evaporation. The residue was purified by column chromatography to give compound 3 as a pale yellow oil (1.5 g, 61%). m/z (ES⁺), [M]⁻ = 184.

### Synthesis of compound 4

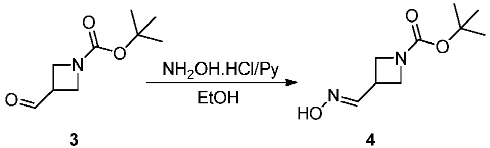

Pyridine (2.61 g, 0.033 mol) was added at 0 °C to a solution of compound 3 (2.1 g, 0.011 mol) and hydroxylamine hydrochloride (1.17 g, 0.017 mol) in ethanol (20 mL). The reaction mixture was stirred at room temperature for 6 h and concentrated by rotary evaporation to remove the solvent. The residue was purified by silica gel column chromatography to give compound 4 as a pale yellow solid (1.0 g, 45.5%). m/z (ES⁺), [M]⁻ = 199.30.

### Synthesis of compound 5

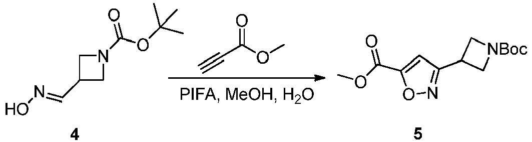

(Bis(trifluoroacetoxy)iodo)benzene (3.22 g, 10 mmol) was added in batches at 0 °C to a solution of compound **4** (1 g, 5 mmol) and methyl propiolate (1.05 g, 12.5 mmol) in methanol (10 mL) and water (5 mL). The reaction mixture was stirred at room temperature for 2 h, and the organic phase was concentrated. Water (20 mL) was added, and the mixture was extracted with ethyl acetate (100 mL × 3). The organic phase was washed with saturated brine (50 mL × 2), dried over anhydrous sodium sulfate, and concentrated to dryness by rotary evaporation. The residue was purified by silica gel column chromatography (ethyl acetate/petroleum ether = 1/4 to 1/1) to give compound 5 as a pale yellow solid (800 mg, 57%). m/z (ES⁺), [M+H]⁺ = 283.

### Synthesis of compound 6

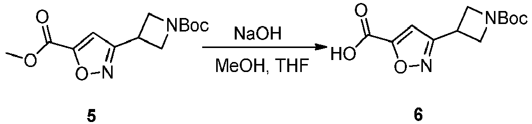

2 mol of an aqueous solution of sodium hydroxide (213 mg, 5.32 mmol) was added at 0 °C to compound 5 (750 mg, 2.66 mmol) in tetrahydrofuran/methanol (9 mL, 2:1). The reaction mixture was warmed to room temperature, allowed to react for 1 h, and concentrated by rotary evaporation to remove the solvent. Water was added, and the pH was adjusted to 5 with 1 mol of hydrochloric acid. The resulting solid was collected by filtration to give compound 6 as a yellow solid (690 mg, 96%). m/z (ES⁺), [M+H]⁺ = 269.

### Synthesis of compound 7

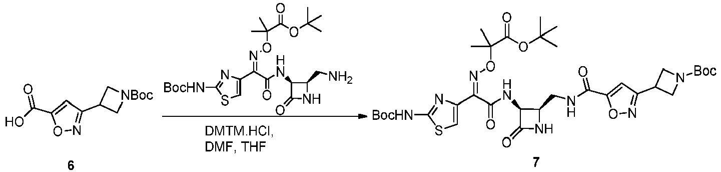

4-(4,6-Dimethoxytriazin-2-yl)-4-methylmorpholinium hydrochloride (300 mg, 1.017 mmol) was added to compound 6 (200 mg, 0.746 mmol) and amine (357 mg, 0.678 mmol) in tetrahydrofuran (5 mL) and N,N-dimethylformamide (3 mL). The reaction mixture was stirred under nitrogen for 2 h, added to an icy aqueous solution of sodium dihydrogen phosphate (10 mL), and extracted with ethyl acetate (10 mL × 2). The organic phase was washed with water (20 mL), and the aqueous phase was reversely extracted with ethyl acetate (10 mL). The organic phases were combined, washed with saturated brine (20 mL), dried over anhydrous sodium sulfate, and concentrated to dryness by rotary evaporation. The residue was purified by reversed-phase chromatography to give compound 7 as a white solid (205 mg, 38.9%). m/z (ES⁺), [M+H]⁺ = 777.

### Synthesis of compound 8

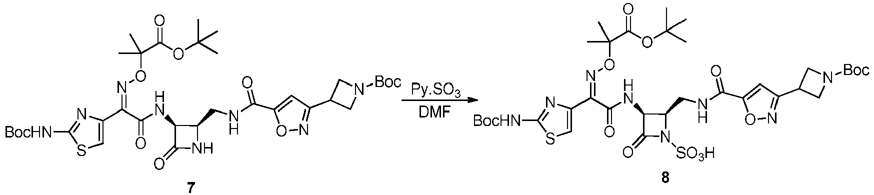

Sulfur trioxide pyridine complex (318 mg, 2.0 mmol) was added to compound 7 (155 mg, 0.2 mmol) in *N*,*N*-dimethylformamide (2 mL). The reaction mixture was stirred at 45 °C under nitrogen for 2 h and purified by reversed-phase chromatography to give compound 8 as a white solid (88 mg, 95%). m/z (ES⁺), [M+H]⁺ = 857.

### Synthesis of compound 1-6:

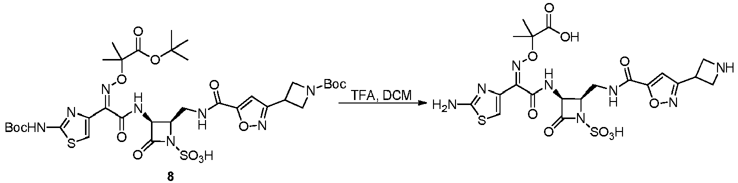

Trifluoroacetic acid (2 mL) was added dropwise at 0 °C under nitrogen to compound 8 (50 mg, 0.058 mmol) in dichloromethane (2 mL). The reaction mixture was stirred at 0 °C for 6 h and blow-dried. The solid was lyophilized and purified by reversed-phase column chromatography to give a compound as a white solid (10.4 mg, 29.8%).

m/z (ES⁺), [M+H]⁺ = 601. ¹H NMR (400 MHz, D₂O) δ 6.99 (d, J = 8.8 Hz, 2H), 5.32 (d, J = 5.7 Hz, 1H), 4.42 (s, 1H), 4.29-4.40 (m, 5H), 3.84-3.73 (m, 2H), 1.40 (s, 6H).

### Example 7. Synthesis of Compound 1-7

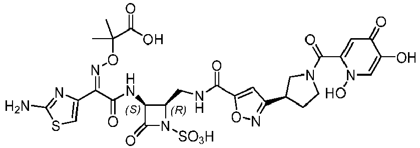

### Synthesis of compound 2

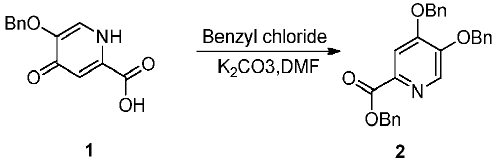

Benzyl chloride (1.93 g, 15.3 mmol) was added dropwise to 3-benzyloxy-4-pyridone-6-carboxylic acid (1.5 g, 6.1 mmol) and potassium carbonate (2.07 g, 15 mmol) in *N,N-*dimethylformamide (20 mL). The reaction mixture was reacted at 80 °C overnight and cooled to room temperature. Ice water was added, and a solid precipitated. The solid was collected by filtration and purified by silica gel column chromatography to give compound 2 as a yellow oil (4 g, crude).

m/z (ES+), [M+H]+ = 426; acid, HPLC tR = 1.371 min.

### Synthesis of compound 3

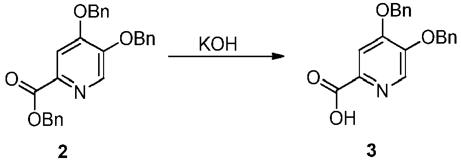

A 1 mol/L aqueous solution of potassium hydroxide (11 mL, 11 mmol) was added dropwise to compound 2 (3.7 g, 8.7 mmol) in tetrahydrofuran (50 mL). The reaction mixture was allowed to react at room temperature for 3 h. Water (100 mL) was added to the reaction mixture, and the pH was adjusted to 2-3 with 1 N hydrochloric acid. A solid precipitated, and was collected by filtration, washed with water (3 × 25 mL), and dried to give compound 3 as a white solid.

m/z (ES+), [M+H]+ = 336.4; acid, HPLC tR = 0.773 min. 1H NMR (400 MHz, DMSO-*d*6) δ 5.32 (d, *J* = 1.8 Hz, 4H), 7.47-7.29 (m, 7H), 7.46 (dd, *J* = 6.6, 1.7 Hz, 3H), 7.74 (s, 1H), 8.35 (s, 1H), 12.88 (s, 1H).

### Synthesis of compound 4

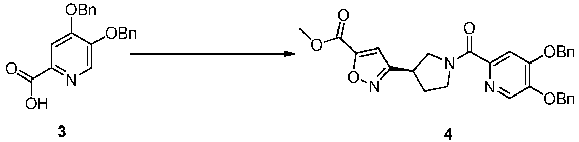

To *N*,*N*-dimethylformamide (10 mL) were added compound 3 (800 mg, 2.4 mmol), amine (1.4g, 7.14 mmol), HATU (1.1 g, 2.89 mmol) and DIEA (1.44 g, 10.89 mmol). The reaction mixture was allowed to react at room temperature for 2 h, followed by the addition of ethyl acetate (20 mL)/water (20 mL). The organic phase was washed with saturated brine (20 mL) and concentrated. The residue was purified by column chromatography (PE : EA = 0-50%) to give compound 4 as a white solid (910 mg, 79%).

m/z (ES+), [M+H]+ = 514; acid, HPLC tR = 1.476 min. 1H NMR (methanol-*d*₄, 300 MHz): δ (ppm) 2.47-2.70 (m, 1H), 2.80 (dt, *J* = 12.3, 6.1 Hz, 1H), 4.02-4.33 (m, 3H), 4.35 (dd, *J* = 3.5, 1.3 Hz, 3H), 4.38-4.52 (m, 3H), 5.45-5.86 (m,4H), 7.61 (d, *J* = 17.4 Hz, 1H), 7.72-8.03 (m, 10H), 8.67 (s, 1H).

### Synthesis of compound 5

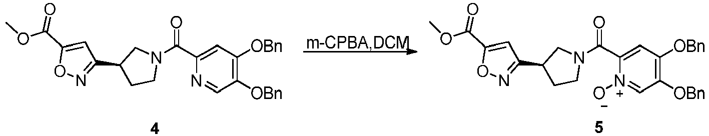

m-CPBA (350 mg, 2 mmol) was dissolved at 0 °C in dichloromethane (10 mL), and the resulting solution was added dropwise to compound 4 (510 mg, 1 mmol) in dichloromethane (5 mL). The reaction mixture was allowed to react at room temperature for 2 h. Dichloromethane (20 mL) and saturated sodium bicarbonate (20 mL) were added to the reaction mixture. The organic phase was washed with sodium chloride (20 mL) and concentrated. The residue was purifed by preparative thin layer chromatography (DCM : MeOH = 10:1) to give compound 5 as a white solid (320 mg, 61%). m/z (ES+), [M+H]+ = 530; acid, HPLC tR = 1.245 min.

### Synthesis of compound 6

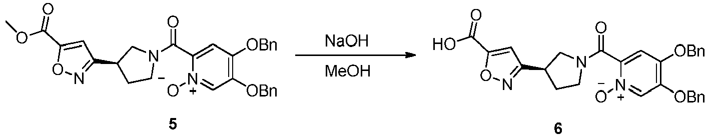

An aqueous solution of sodium hydroxide (4 mL, 2 M) was added dropwise to compound 5 (310 mg, 0.58 mmol) in methanol (6 mL). The reaction mixture was allowed to react at room temperature for 1 h and concentrated by rotary evaporation to remove methanol. The aqueous phase was adjusted to pH 2.5 with citric acid and extracted with ethyl acetate. The organic phase was dried over anhydrous sodium sulfate and concentrated to dryness by rotary evaporation to give compound 6 as a white solid (280 mg). m/z (ES+), [M+H]+ = 516; acid, HPLC tR = 1.061 min.

### Synthesis of compound 7

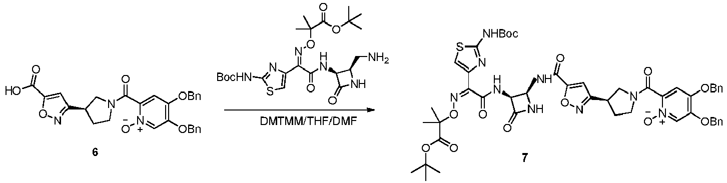

Compound 6 (110 mg, 0.21 mmol) and amine (100 mg, 0.19 mmol) were added to tetrahydrofuran (1.6 mL) and N,N-dimethylformamide (1 mL), and DMTMM (100 mg, 0.34 mmol) was added under nitrogen to the mixture. The reaction mixture was stirred at room temperature for 2 h and purified by reversed-phase chromatography to give compound 7 as a white solid (130 mg, 54.6 %). m/z (ES+), [M]+ = 1024; acid, HPLC tR = 1.679 min.

### Synthesis of compound 8

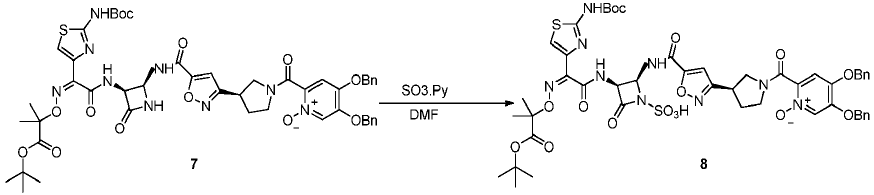

Sulfur trioxide pyridine complex (200 mg, 1.14 mmol, 11 equiv) was added to compound 7 (108 mg, 0.1 mmol) in *N*,*N*-dimethylformamide (3 mL). The reaction mixture was stirred at room temperature under nitrogen overnight and purified by reversed-phase chromatography to give pale yellow compound 8 (56 mg, 50%). m/z (ES+), [M]+ = 1104; acid, HPLC tR = 1.579 min.

### Synthesis of compound 1-7

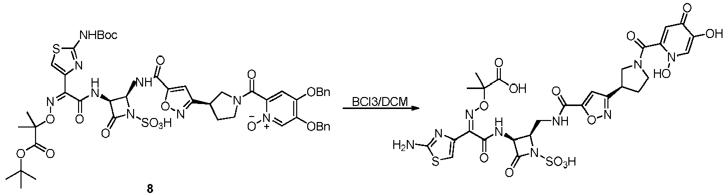

Boron trichloride (1 mL, 1 mol/L in dichloromethane) was added dropwise at 0 °C under nitrogen to compound 8 (92 mg, 0.083 mmol) in dichloromethane (7 mL). The reaction mixture was stirred at 0 °C for 1 h and blow-dried with nitrogen to remove the solvent. Diethyl ether was added, and a solid precipitated. The solid was centrifuged with diethyl ether and purified by preparative high-pressure liquid chromatography to give a white solid (12.7 mg, 19.85%).

¹H NMR (400 MHz, D₂O) δ 1.43 (s, 3H), 1.44 (s, 3H), 2.16 (dq, *J* = 20.4, 6.8, 5.7 Hz, 1H), 2.42 (ddd, *J* = 25.8, 12.6, 6.2 Hz, 1H), 3.35-3.54 (m, 2H), 3.54-3.88 (m, 5H), 4.53-4.60 (m, 1H), 5.37 (t, *J* = 5.7 Hz, 1H), 6.90-7.09 (m, 3H), 7.87 (d, *J* = 4.6 Hz, 1H). m/z (ES+), [M+H]⁺ = 768; [M-H]⁻ = 766; acid, HPLC tR = 1.391 min.

### Example 8. Synthesis of Compound 1-8

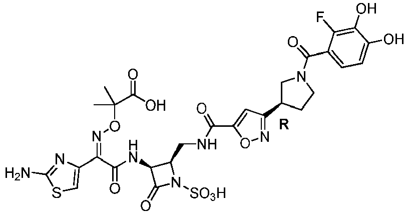

### Synthesis of compound 1

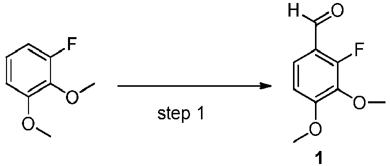

To a solution of hexamethylenetetramine (2.2 g, 16 mmol) in TFA (10 mL) being heated was added dropwise at 80 °C a solution of 1-fluoro-2,3-dimethoxybenzene (1.20 g, 7.8 mmol) in TFA (10 mL). The mixture was heated for another hour and then concentrated *in vacuo.* Ice/water (30 mL) was added until the mixture became cloudy. The mixture was stirred for 15 min, then made basic with solid sodium carbonate, stirred for 20 min, and then extracted with ethyl acetate. The organic phase was washed with water, dried, and evaporated *in vacuo.* The residue was purified by silica gel column chromatography using a mixture of ethyl acetate (0-50%) and petroleum ether as the eluent to give compound 1 as a white solid (1.1 g). m/z (ES+), [M+H]+ = 185; acid, HPLC tR = 1.040 min. 1H NMR (400 MHz, chloroform-d) δ 10.19 (s, 1H), 7.59 (dd, J = 8.8, 7.3 Hz, 1H), 6.80 (dd, J = 8.8, 1.6 Hz, 1H), 3.95 (d, J = 1.4 Hz, 6H).

### Synthesis of compound 2

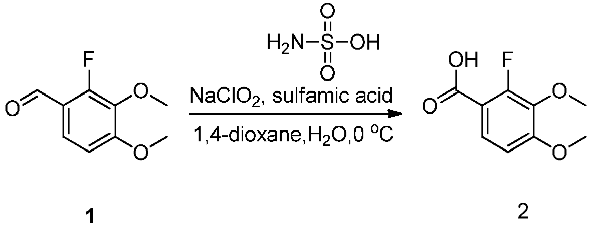

To a solution of 2-fluoro-3,4-dimethoxybenzaldehyde (1.6 g, 8.69 mmol) in 1,4-dioxane (15 mL) were added at 0 °C a solution of sulfamic acid (1.8 g, 19.1 mmol) in water (7 mL) and a solution of NaClO₂ (1.72 g, 19.1 mmol) in water (7 mL). The reaction mixture was stirred at 0 °C for 30 min and then quenched with an aqueous solution of sodium bisulfite (20 mL). The aqueous layer was extracted with ethyl acetate, and the extracts were combined, washed with water and brine, dried over anhydrous sodium sulfate, and filtered. The filtrate was concentrated *in vacuo* to give 2-fluoro-3,4-dimethoxybenzoic acid as an off-white solid (1.9 g, crude). m/z (ES+), [M-H]+ = 199; acid, HPLC tR = 0.917 min.

### Synthesis of compound 3

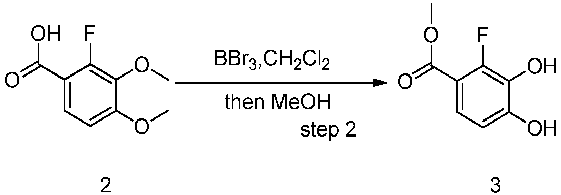

BBr3 (5.9 g, 23.7 mmol) was added dropwise at 0 °C to a solution of 2-fluoro-3,4-dimethoxybenzoic acid (1.9 g, 9.5 mmol) in dichloromethane (20 mL). The reaction mixture was stirred at room temperature for 2 h and then slowly poured into MeOH (270 mL). After the resulting mixture was stirred at room temperature for 2 days, ice water was added thereto, and then methanol was removed by rotary evaporation *in vacuo.* The resulting solid precipitate was collected by filtration, washed with water, and dried *in vacuo* to give 2-fluoro-3,4-dihydroxybenzoate (1.3 g, 80%). [M-H]+ = 185; acid, HPLC tR = 0.832 min.

### Synthesis of compound 4

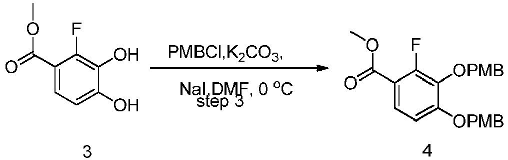

To a solution of methyl 2-fluoro-3,4-dihydroxybenzoate (1 g, 5.3 mmol) in DMF (5 mL) were added K₂CO₃ (1.85 g, 13 mmol), NaI (0.8 g, 5.3 mmol) and PMBCl (1.9 g, 12 mmol). The reaction mixture was stirred at 50 °C for 3.5 h, and then ice water was added thereto. The aqueous layer was extracted with ethyl acetate, and the extracts were combined, washed with brine, dried over anhydrous sodium sulfate, and filtered. The filtrate was concentrated *in vacuo* to give a crude product, which was further purified by silica gel column chromatography using a mixture of ethyl acetate (0-50%) and petroleum ether as the eluent to give compound 4 as a white solid (1.6 g, 70%). [M+Na]+ = 449; acid, HPLC tR = 1.422 min.

### Synthesis of compound 5

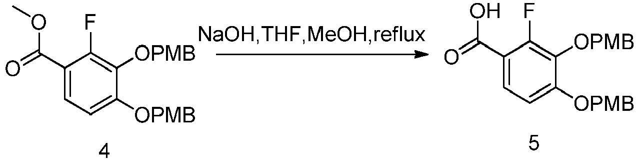

To a mixture of compound 4 (1 g, 2.3 mmol) in THF (5 mL), MeOH (5 mL) and H₂O (5 mL) was added NaOH (0.187 g). The resulting mixture was stirred at reflux for 1.5 h. The solvent was evaporated *in vacuo,* and HCl (2 mol/L) was added until the pH 3-4. The solid precipitate was collected by filtration, washed with water, and dried *in vacuo* to give 2-fluoro-3,4-bis((4-methoxybenzyl)oxy)benzoic acid (0.7 g, 73%).

m/z (ES+), [M+Na]+ = 435; acid, HPLC tR = 0.825 min.

### Synthesis of compound 6

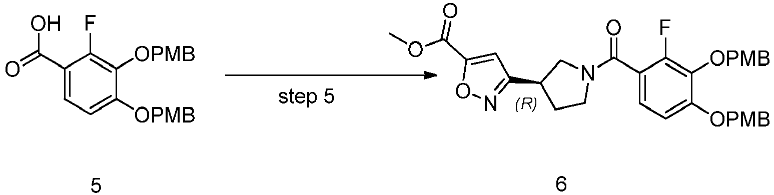

To a solution of 2-fluoro-3,4-bis((4-methoxybenzyl)oxy)benzoic acid (0.2 g, 0.485 mmol) and methyl (*R*)-3-(pyrrolidin-3-yl)isoxazole-5-carboxylate hydrochloride (0.095 g, 2.33 mmol) in THF (3 mL) were added HOBt (0.13 g, 0.97 mmol), TEA (0.196 g, 1.94 mmol) and EDC•HCl (0.186 g, 0.97 mmol). The reaction mixture was stirred at room temperature for 3 h and diluted with water and ethyl acetate. The organic layer was separated, and the aqueous layer was extracted with ethyl acetate. The extracts were combined, washed with brine, dried over anhydrous MgSO₄, and filtered. The filtrate was concentrated *in vacuo.* The resulting residue was purified by silica gel chromatography to give methyl (*R*)-3-(1-(2-fluoro-3,4-bis((4-methoxybenzyl)oxy)benzoyl)pyrrolidin-3-yl)isoxazole-5-carboxylate as a white solid (180 mg, 73%).

m/z (ES+), [M+H]+ = 591; acid, HPLC tR = 1.751 min

### Synthesis of compound 7

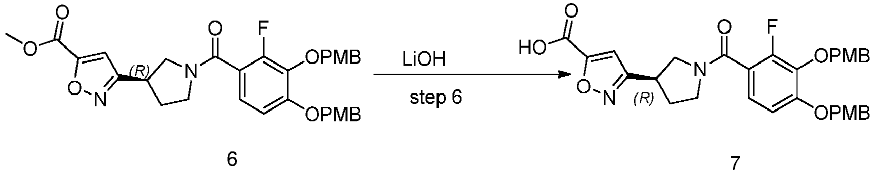

LiOH (0.024 g) was added to a solution of methyl (*R*)-3-(1-(2-fluoro-3,4-bis(4-methoxybenzyloxy)benzoyl)pyrrolidin-3-yl)isoxazole-5-carboxylate (200 mg, 0.338 mmol) in MeOH, THF and H₂O (0.5 mL : 0.5 mL : 0.5 mL). The reaction mixture was stirred at room temperature for 2 h. MeOH and THF were removed, and the mixture was acidified with HCl (1 mol/L) to pH 2.5. The solid precipitate was collected by filtration, washed with water, and dried *in vacuo* to give (R)-3-(1-(2-fluoro-3,4-bis((4-methoxybenzyl)oxy)benzoyl)pyrrolidin-3-yl)isoxazole-5-carboxylic acid as a white solid (152 mg, 77.9%).

m/z (ES+), [M+H]+ = 577; base, HPLC tR = 0.979 min.

### Synthesis of compound 8

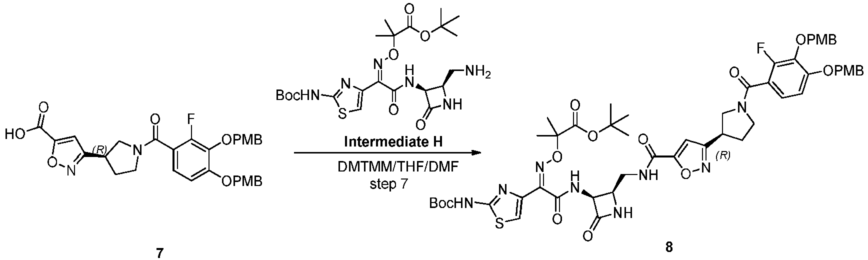

To a 10 mL round-bottom flask were added a solution of (*R*)-3-(1-(2-fluoro-3,4-bis((4-methoxybenzyl)oxy)benzoyl)pyrrolidin-3-ylisoxazole-5-carboxylic acid (109 mg, 0.019 mmol) and 2-((((*Z*)-2-((((2*S*,3*R*)-2-(aminomethyl)-4-oxoazetidin-3-yl)amino)-1-(2-(((*tert-*butoxycarbonyl)amino)thiazol-4-yl)-2-oxoethylidene)amino)oxy)-2-methylpropionate (100 mg, 0.019 mmol) in THF (0.8 mL) and DMF (0.5 mL). DMTMM (84 mg, 0.0285 mmol) was added under nitrogen. The reaction mixture was stirred at room temperature for 2 h and purified by flash (MeCN and H₂O) to give 2-((((*Z*)-1-)-(2-((*tert*-butoxycarbonylcarbonyl)amino)thiazol-4-yl)-2-((((2S),3R)-2-(((3-((R)-1-(2-fluoro-3,4-bis((4-methoxybenzyl)oxy)benzoyl)pyrrolidinyl-3-yl)isoxazole-5-carboxamido)methyl)-4-oxoazetidin-3-yl)amino)-2-oxoethylidene)amino)oxy)-2-methylpropionate as a white solid (43 mg, 20.8%). m/z (ES+), [M]+ = 1085; acid; HPLC tR = 0.977 min.

### Synthesis of compound 9

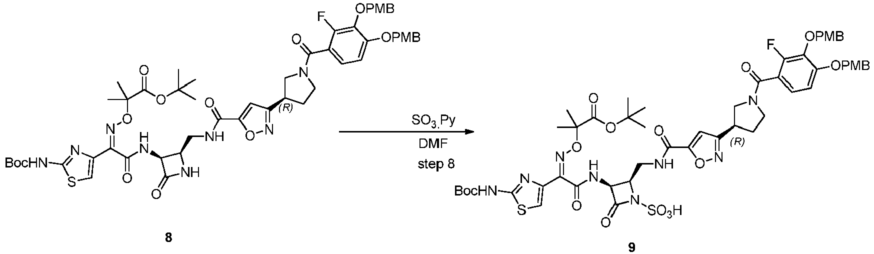

To a solution of compound 8 (43 mg, 0.0396 mmol) in DMF (1 mL) was added sulfur trioxide pyridine (62.6 mg, 0.396 mmol, 10 eq). The resulting solution was stirred under nitrogen at 45 °C for 3 h. The residue was purified by flash (MeCN and H₂O) to give compound 9 as a white solid (35 mg). m/z (ES+), [M]+ = 1165; acid, HPLC tR = 1.518 min.

### Synthesis of compound 1-8

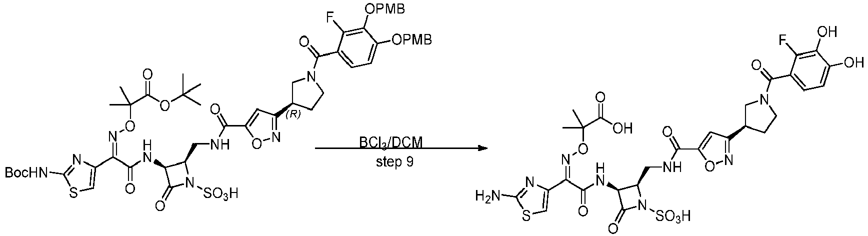

BrCl3 (0.4 mL) was added at 0 °C to compound 9 (35 mg) in dichloromethane (2 mL). The resulting mixture was stirred at 0 °C for 4 h and blow-dried with nitrogen. The solid was washed with diethyl ether (3 times). The crude product was further purified by preparative HPLC (under the following conditions: column: Xbridge shied RP18 OBD column, 19 × 250 mm, 10 µm; mobile phase A: water (0.1% FA); mobile phase B: ACN; flowrate: 25 mL/min; gradient: from 20 B to 35 B over 8 min; 254/220 nm; RT1: 7.70; RT2:) to give 2-(((((*Z*)-1-(2-aminothiazol-4-yl)-2-(((((2*S*,3*R*)-2-((3-((*R*)-1-(2-fluoro-3,4-dihydroxybenzoyl)pyrrolidin-3-y )isoxazole-5-carboxamido)methyl)-4-oxo-1-sulfoazetidin-3-yl)amino)-2-oxoethylidene)amino)oxy)-2-methylpropionic acid as a off-white solid (10.8 mg).

m/z (ES+), [M+H]+ = 769; acid, HPLC tR = 0.997 min. ¹H NMR (400 MHz, DMSO-*d*₆) δ 6.94 (d, *J* = 28.0 Hz, 1H), 6.85-6.54 (m, 3H), 5.18 (dd, *J* = 5.7, 4.6 Hz, 1H), 4.17 (s, 1H), 3.83 (s, 1H),3.70 (s, 1H), 3.61 (d, *J* = 12.6 Hz, 1H), 3.58-3.40 (m, 4H), 3.36 (t, *J* = 6.7 Hz, 1H), 2.39-2.21 (m, 1H), 2.13-1.93 (m, 1H), 1.46-1.35 (m, 6H).

### Example 9. Synthesis of Compound 1-9

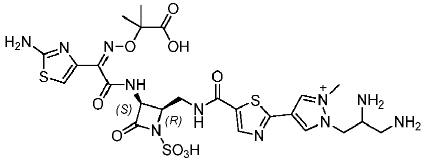

### Synthesis of compound A-2

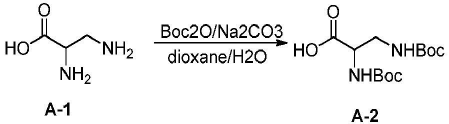

Compound A-1 (10 g, 56.5 mmol), Boc₂O (30.5 g, 143.5 mmol) and Na₂CO₃ (17.97 g, 169.5 mmol) were added to a solvent mixture of dioxane (150 mL)/H₂O (150 mL). The reaction system was stirred at room temperature for 10 h, concentrated, adjusted to pH 3 with a 2 N aqueous solution of hydrochloric acid, and extracted with ethyl acetate. The organic phase was washed with saturated brine, dried over anhydrous sodium sulfate, and filtered. The filtrate was concentrated to dryness by rotary evaporation to give a crude product, which was directly used in the next step without further purification. [M+H]+ = 305; acid, HPLC tR = 1.315 min.

### Synthesis of compound A-3

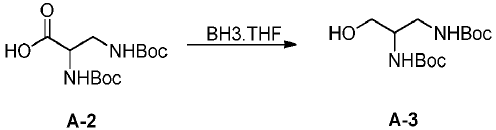

Compound A-2 (crude product from step 1) was dissolved in 150 mL of tetrahydrofuran, and BH3.THF (100 mL, 100 mmol) was added dropwise at 0 °C. The reaction mixture was stirred at room temperature for 5 h, then allowed to react overnight, quenched with H₂O (50 mL), and stirred for 30 min. The organic phase was separated from the reaction mixture, dried, and concentrated to give a crude product as an oil, which was purified by silica gel column chromatography to give A-3 as a colorless oil (4 g, 14.3% yield for two steps). m/z (ES+), [M+H]+ = 291; acid, HPLC RT = 0.754 min. 1H NMR (300 MHz, chloroform-d) δ 1.45 (d, J = 2.7 Hz, 18H), 3.09-3.43 (m, 2H), 3.54 (t, J = 12.6 Hz, 2H), 3.69 (dd, J = 2.9, 11.6 Hz, 1H), 5.02 (d, J = 42.8 Hz, 2H).

### Synthesis of compound A-4

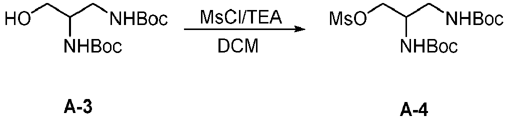

Compound A-3 (4 g, 19.1 mmol, 1 eq) was dissolved in dichloromethane (100 mL), and MsCl (2.8 g, 24.82 mmol) was added dropwise at 0 °C. The reaction mixture was stirred for 5 min, and then triethylamine (5.4 mL, 38.2 mmol) was added dropwise. The reaction mixture was stirred at 0 °C for 2 h, and the reaction was completed as indicated by TLC. The reaction mixture was diluted with dichloromethane and washed with saturated brine. The organic phase was dried and concentrated to give a crude product, which was directly used in the next step without further purification.

### Synthesis of compound 2

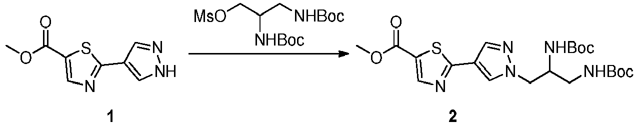

Compound 1 (2 g, 9.6 mmol, 1 eq) was dissolved in DMF (100 mL), and C_{S2}CO₃ (6.24 g, 19 mmol, 2 eq) and 2,3-bis((*tert*-butoxycarbonyl)amino)propyl methanesulfonate (7 g, 19 mmol, 2 eq) were successively added. The reaction mixture was stirred at 40 °C for 4 h. Water (50 mL) was added, and the reaction mixture was extracted 3 times with 100 mL of ethyl acetate. The organic phase was distilled under reduced pressure, and the residue was purified by silica gel column chromatography to give compound 2 as a yellow solid (2.1 g, 45.6%). m/z (ES+), [M+1]+ = 482.2; acid, HPLC tR = 1.343 min.

### Synthesis of compound 3

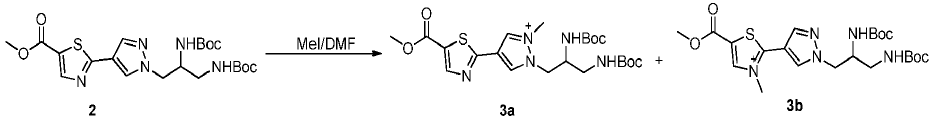

Compound 2 (2.1 g, 4.37 mmol,1 eq) was dissolved in DMF (10 mL), and MeI (6.2 g, 43.7 mmol, 10 eq) was added. Then the reaction mixture was stirred in a microwave tube at 80 °C for 1.5 h and finally purified by HPLC using a C-18 column (0-50%, 0.5‰ aqueous formic acid solution and acetonitrile) to give a mixture of compounds 3a and 3b as a yellow solid (900 mg, 41.6%). m/z (ES+), [M]+ = 496.2; acid, HPLC tR = 1.089 min. Isomer 2: m/z (ES+), [M]+ = 496.2; acid, HPLC tR = 1.089 min. ¹H NMR (400 MHz, DMSO-*d*₆) δ 1.14 (s, 9H), 1.40 (s, 9H), 3.19 (d, *J* = 7.0 Hz, 2H), 3.88 (s, 3H), 4.04 (q, *J* = 7.6 Hz, 1H), 4.25 (s, 3H), 4.40 (t, *J* = 13.0 Hz, 1H), 4.76 (d, *J* = 14.2 Hz, 1H), 8.57 (s, 1H), 9.34 (s, 1H), 9.38 (s, 1H).

### Synthesis of compound 4

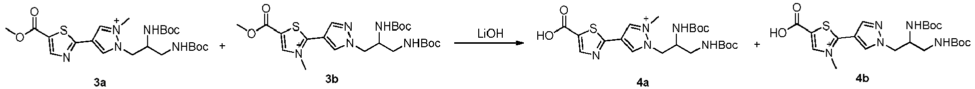

Mixture 3(3a and 3b, 900 mg, 1.81 mmol, 1.00 eq) was dissolved in tetrahydrofuran and water (10 mL : 5 mL), and LiOH (435 mg, 18.1 mmol, 10 eq) was added. The reaction mixture was stirred at room temperature for 1 h and concentrated to dryness by rotary evaporation under reduced pressure to give mixture 4 as a yellow solid (4a and 4b, 1.2 g, crude), which was directly used without further purification. m/z (ES+), [M]+ = 482.1; acid, HPLC tR = 1.058 min.

### Synthesis of compound 5

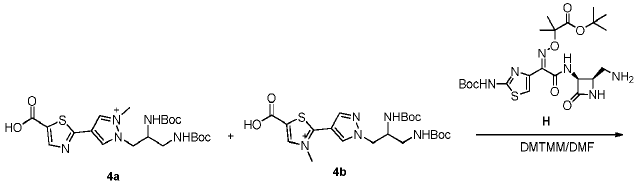

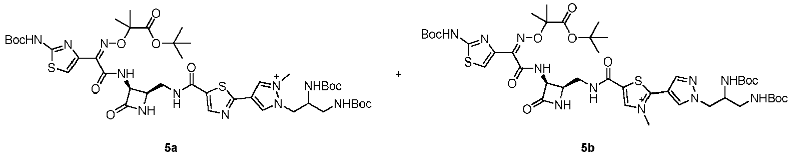

Mixture 4 (4a and 4b, 454 mg, 0.860 mmol, 1 eq), reactant H (500 mg, 1.04 mmol, 1.2 eq) and DMTMM (406 mg, 1.38 mmol, 1.6 eq) were dissolved in DMF/THF (8:5, 10 mL). The reaction mixture was stirred at room temperature for 2 h, and the product was purified by HPLC using a C-18 column (0-60%, 0.5‰ aqueous formic acid solution and acetonitrile) to give compound 5 as a yellow solid (300 mg, 32%). m/z (ES+), [M-H]+ = 990.4; base, HPLC tR = 0.953 min.

### Synthesis of compound 6

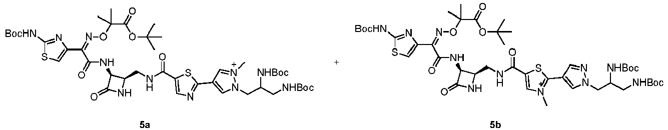

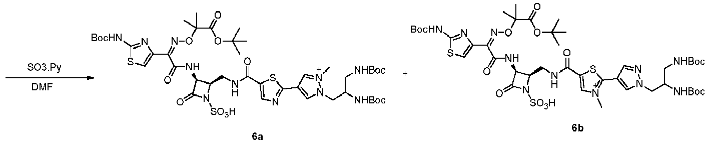

Mixture 5 (5a and 5b, 200 mg, 0.202 mmol, 1 eq) and SO₃.Py (192 mg, 1.212 mmol, 6 eq) were dissolved in DMF (4 mL). The reaction mixture was stirred at 36 °C overnight. The pH of the reaction mixture was adjusted to 3 with HCl (2 M), and the product was purified by HPLC (0-100%, 0.5‰ aqueous formic acid solution and acetonitrile) to give mixture 6 as a white solid (6a and 6b, 80 mg, 37%). m/z (ES+), [M]+ = 1071.6; base, HPLC tR = 1.210 min.

### Synthesis of compound 1-9 and compound 1-13

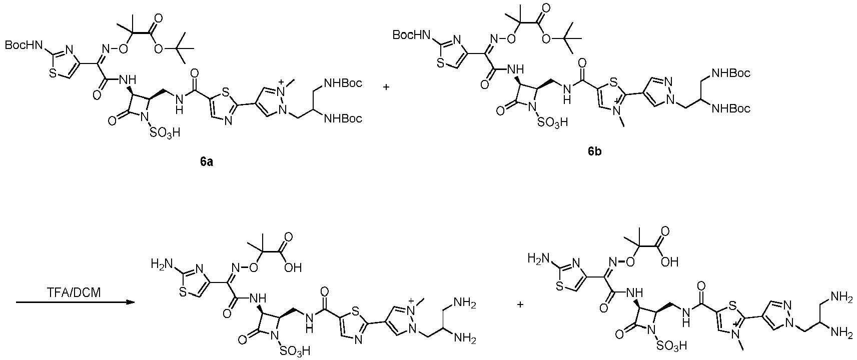

TFA (2 mL) was added dropwise at 0 °C to a solution of mixture 6 (6a and 6b, 80 mg, 0.035 mmol) in DCM (4 mL). The reaction mixture was stirred at 0 °C for 5 h. After the reaction was completed, the reaction mixture was dried under a stream of nitrogen, and the residue was quenched and centrifuged in diethyl ether. The previous step was repeated 3 times. The residue was lyophilized and purified by HPLC to give compounds **1-9** and **1-13.**

**Compound 1-9:** m/z (ES+), [M]+ = 715; acid, HPLC RT = 3.301 min. ¹H NMR (300 MHz, D₂O) δ 1.43 (s, 6H), 3.02 (dd, *J* = 10.0, 13.1 Hz, 1H), 3.30 (dd, *J* = 3.8, 13.1 Hz, 1H), 3.60 (s, 1H), 3.78 (d, *J* = 6.4 Hz, 2H), 4.23 (s, 3H), 4.48-4.63 (m, 2H), 5.34 (d, *J* = 5.6 Hz, 1H), 6.96 (s, 1H), 8.23 (s, 1H), 8.86 (s, 1H), 8.92 (s, 1H).

### Example 10. Synthesis of Compound I-10

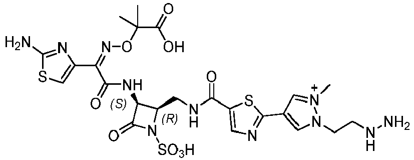

### Synthesis of compound 2:

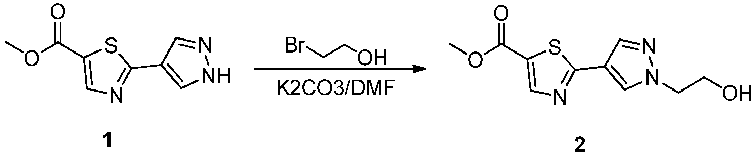

Cesium carbonate (6 g, 19.12 mmol) and 2-bromoethan-1-ol (3.58 g, 28.7 mmol) were added to a solution of compound 1 (2 g, 9.56 mmol) in *N*,*N*-dimethylformamide (30 mL). The reaction mixture was heated to 50 °C, allowed to react overnight, cooled to room temperature, diluted with ice water, and then extracted with ethyl acetate. The organic layer was washed with saturated brine, dried over anhydrous magnesium sulfate, and filtered. The filtrate was concentrated. The crude product was purified by column chromatography to give compound 2 as a yellow solid (1.1 g). m/z (ES⁺), [M+H]⁺ = 254.

### Synthesis of compound 3:

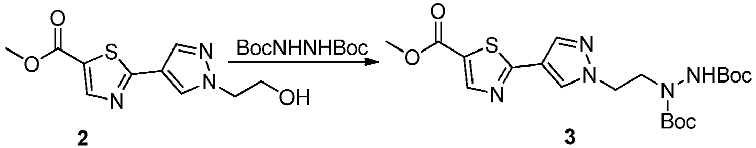

Compound 2 (2 g, 7.9 mmol), di-*tert*-butyl 1,2-dihydroazodicarboxylate (2.75 g, 11.8 mmol) and triphenylphosphane (4.13 g, 15.8 mmol) were added at 0 °C under nitrogen to tetrahydrofuran (15 mL), and diisopropyl azodicarboxylate (3.19 g, 15.8 mmol) was added. The reaction mixture was stirred at room temperature under nitrogen for 2 h. The residue was purified by column chromatography to give compound 3 as a pale yellow oil (0.7 g, 20.9%). m/z (ES⁺), [M+H]⁺ = 468.

### Synthesis of compound 4:

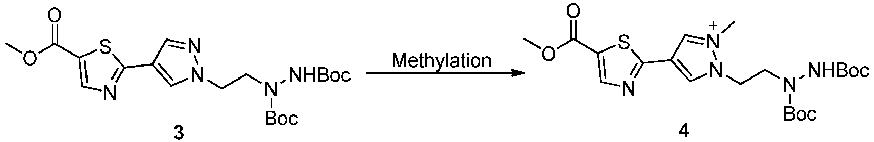

To a solution of compound 3 (0.6 g, 1.49 mmol) in *N*,*N*-dimethylformamide (3 mL) were added iodomethane (1.8 g, 12.6 mmol) and potassium carbonate (0.88 g, 7.23 mmol). The mixture was microwaved at 80 °C for 4 h. After the reaction was completed, the mixture was filtered, and the filtrate was concentrated. The resulting crude product was washed with dichloromethane to give compound 4 as a yellow solid (50 mg). m/z (ES⁺), [M]⁺ = 482.

### Synthesis of compound 5:

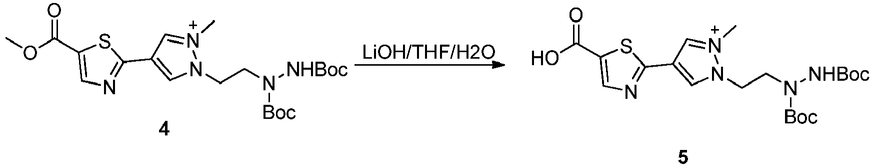

To a solution of compound 4 (50 mg, 1.03 mmol) in tetrahydrofuran (0.5 mL)/methanol (1 mL) was added an aqueous solution of lithium hydroxide. The reaction mixture was stirred at 0 °C for 2 h. After the reaction was completed, the reaction system was concentrated to give compound 5 as a crude product (90 mg). m/z (ES⁺), [M]⁺ = 468.

### Synthesis of compound 6:

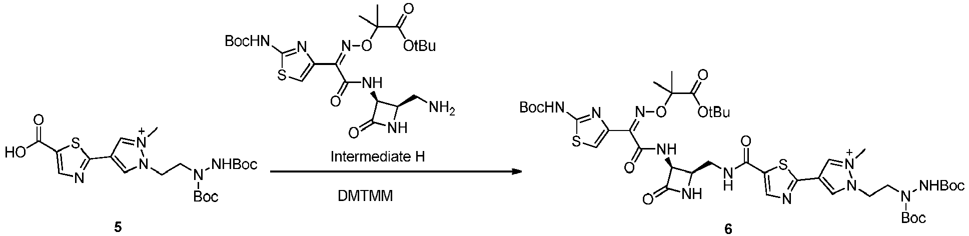

To a solution of compound 5 (50 mg, 0.106 mmol) in *N,N-*dimethylformamide/tetrahydrofuran (0.5 mL : 0.7 mL) were added compound H (112 mg, 0.213 mmol) and 4-(4,6-dimethoxytriazin-2-yl)-4-methylmorpholinium hydrochloride (DMTMM) (62.8 mg, 0.213 mmol). The mixture was stirred at room temperature for 4 h. The crude product was purified by column chromatography to give 6 as a colorless oil (40 mg). m/z (ES+), [M]+ = 976.

### Synthesis of compound 7:

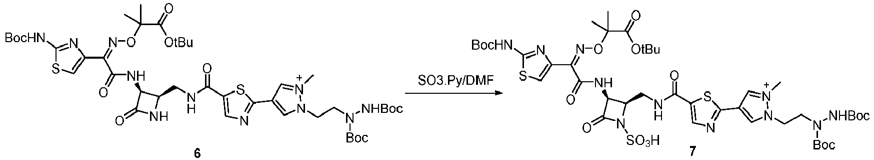

To a solution of compound 6 (40 mg) in *N*,*N*-dimethylformamide (1 mL) was added sulfur trioxide pyridine complex (60 mg). The reaction mixture was stirred at 0 °C for 4 h. The crude product was purified by column chromatography to give compound 7 (15 mg).

### Synthesis of compound 1-10:

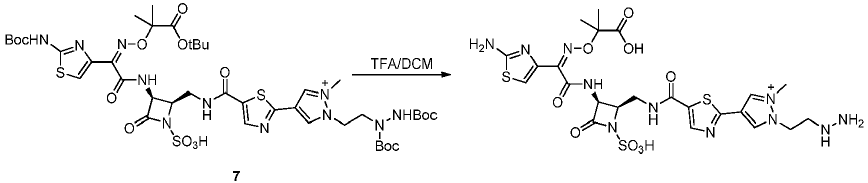

To a solution of compound 7 (40 mg, 0.106 mmol) in dichloromethane (1 mL) was added trifluoroacetic acid (0.3 mL). The reaction mixture was stirred at 0 °C for 3 h and purified by reversed-phase column chromatography to give compound 1-10 (0.5 mg). m/z (ES+), [M+H]⁺ = 701.

### Example 11. Synthesis of Compound 1-11

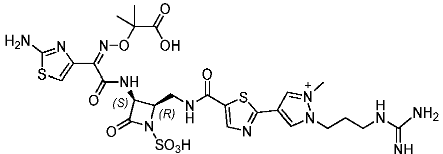

### Synthesis of compound 2

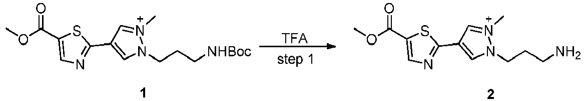

Compound 1 (1 g, 2.62 mmol) was dissolved in dichloromethane (10 mL), and trifluoroacetic acid (5 mL) was slowly added dropwise. The reaction mixture was stirred at room temperature for 2 h. After the reaction was completed, the reaction mixture was concentrated to give compound 2 as a yellow oily crude product (1.51 g), which was directly used in the next step without purification. Compound 1 includes two configurations, one of which disappeared during the hydrolysis in step 3. The presence of both configurations can be detected in the analysis in step 2. m/z (ES+), [M]+ = 281; acid, HPLC RT = 0.174 min.

### Synthesis of compound 3

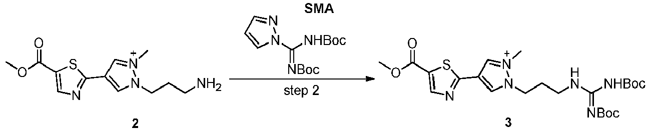

Compound 2 (1.51 g, 2.69 mmol) was dissolved in acetonitrile, and triethylamine (1.09 g, 10.76 mmol) was added dropwise, followed by the slow addition of starting material A (874 mg, 2.82 mmol). The reaction mixture was stirred at 40 °C for 2 h and concentrated to dryness by rotary evaporation to give compound 3 as a yellow oily crude product (3 g), which was used in the next step without purification. m/z (ES+), [M]+ = 523; acid, HPLC RT = 1.104 min. ¹H NMR (400 MHz, methanol-*d*₄) δ 1.52 (d, J = 1.6 Hz, 18H), 2.26 (dt, J = 33.5, 6.7, 6.7 Hz, 2H), 3.34-3.61 (m, 5H), 4.00 (s, 2H), 4.26 (s, 3H), 7.77 (d, J = 1.8 Hz, 1H), 8.27-8.31 (m, 1H), 8.32 (d, J = 0.8 Hz, 1H).

### Synthesis of compound 4

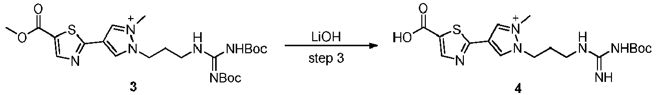

Compound 3 (3 g, 5.74 mmol) was dissolved in methanol, and a solution of lithium hydroxide (413 mg, 17.21 mmol) in water (5 mL) was slowly added dropwise to the above methanol solution. The reaction mixture was stirred at room temperature for 2 h and concentrated, and then a small amount of water was added. The mixture was adjusted to pH 3-4 with hydrochloric acid (1 M) and lyophilized to give crude compound 4 as a yellow solid (2 g), which was directly used in the next step without purification. During the workup of the hydrolysis reaction, it was found that one Boc protecting group had been removed. m/z (ES+), [M]+=409; acid, HPLC RT=0.763 min. ¹H NMR (400 MHz, methanol-*d*₄) δ 1.55 (s, 9H), 2.36 (d, J = 29.7 Hz, 2H), 3.47-3.77 (m, 4H), 4.32 (s, 3H), 7.90 (s, 1H), 8.20 (s, 1H), 8.23 (d, J = 1.3 Hz, 1H).

### Synthesis of compound 5

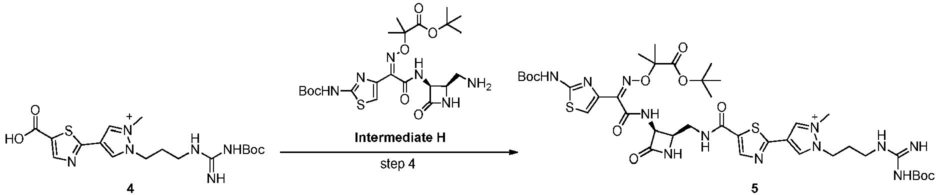

Compound 4 (1 g, 1.14 mmol) was dissolved in *N*,*N*-dimethylformamide/tetrahydrofuran (6 mL/9 mL), and starting material B (500 mg, 0.95 mmol) and DMTMM (560 mg, 1.9 mmol) were added slowly. The reaction mixture was stirred at room temperature for 6 h and purified directly through a C18 column to give compound 5 as a yellow solid (200 mg, 40.6% yield). m/z (ES+), [M]+=917; base, HPLC RT=2.055 min. ¹H NMR (400 MHz, methanol-*d*₄) δ 1.49-1.57 (m, 33H), 2.32 (d, J = 8.4 Hz, 2H), 3.49 (t, J = 6.7, 6.7 Hz, 2H), 3.59 (dd, J = 14.0, 6.1 Hz, 1H), 3.78 (dd, J = 14.0, 6.9 Hz, 1H), 4.12 (q, J = 6.4, 6.3, 6.3 Hz, 1H), 4.27 (s, 3H), 4.65 (t, J = 7.4, 7.4 Hz, 2H), 5.23 (dd, J = 5.8, 2.9 Hz, 1H), 7.39 (d, J = 3.9 Hz, 1H), 8.29 (s, 1H), 9.01 (s, 1H), 9.07 (d, J = 14.1 Hz, 1H).

### Synthesis of compound 6

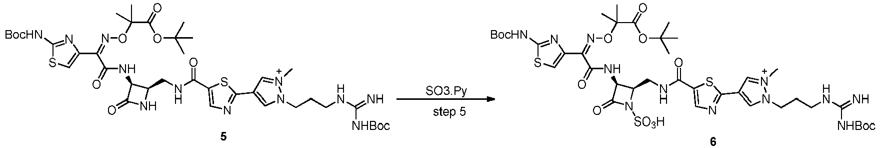

Compound 5 (200 mg, 0.218 mmol) was dissolved in *N*,*N*-dimethylformamide (3 mL), and sulfur trioxide pyridine complex (345 mg, 2.18 mmol) was slowly added. The reaction mixture was stirred at 40 °C overnight and purified directly through a C18 column to give compound 6 as a yellow solid (35 mg, 20.5% yield). m/z (ES+), [M]+ =998; base HPLC RT = 2.015 min.

### Synthesis of compound 1-11

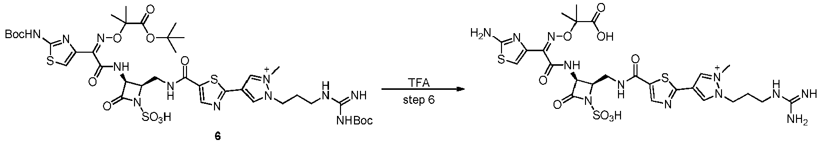

Compound 8 (35 mg, 0.035 mmol) was dissolved in dichloromethane (4 mL), and the system was purged three times with nitrogen. Trifluoroacetic acid (2 mL) was slowly added dropwise at 0 °C under nitrogen. The reaction mixture was stirred at 0 °C for 7 h. After the reaction was completed, the reaction mixture was blow-dried with nitrogen. The residue was washed three times with diethyl ether by centrifugation. The crude product was lyophilized and purified by preparative high-pressure liquid chromatography to give compound 1-11 as a pale yellow solid (7.7 mg, 29.6% yield). m/z (ES+), [M]+ = 741; acid, HPLC RT = 5.916 min. ¹H NMR (400 MHz, D₂O) δ 1.41 (s, 6H), 2.22-2.32 (m, 2H), 3.33 (t, J = 6.7 Hz, 2H), 3.80 (d, J = 6.4 Hz, 2H), 4.19 (s, 3H), 4.60 (t, J = 6.5 Hz, 3H), 5.36 (d, J = 5.7 Hz, 1H), 6.97 (s, 1H), 8.24 (s, 1H), 8.82 (d, J = 17.2 Hz, 2H).

### Example 12. Synthesis of Compound 1-12

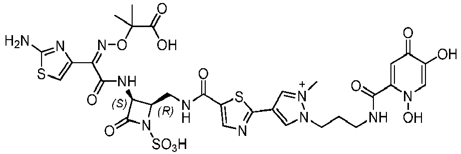

### Synthesis of compound 2

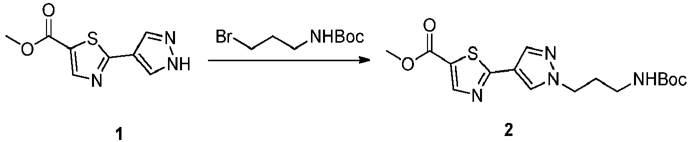

Compound 1 (3.13 g, 15 mmol, 1.00 eq) was dissolved in *N*,*N*-dimethylformamide (30 mL), and *N*-Boc-3-aminopropyl bromide (3.6 g, 15 mmol, 1.00 eq) and potassium carbonate (6 g, 15 mmol, 1 eq) were added to the reaction mixture, which was then allowed to react at room temperature for 3 h. The solid was filtered out. 100 mL of water was added to the organic phase, and the mixture was extracted with ethyl acetate (2 × 200 mL). The organic phase was dried over anhydrous sodium sulfate and concentrated to dryness by rotary evaporation to give compound 2 as a pale oil (5 g, 91.2% yield). m/z (ES+), [M+H]+ = 367; acid, HPLC tR = 0.986 min. 1H NMR (methanol-*d*₄, 400 MHz): δ (ppm) 1.47 (s, 9H), 2.08 (dd, J = 13.9, 7.1 Hz, 2H), 3.11 (t, J = 6.6 Hz, 2H), 3.94 (s, 3H), 4.28 (t, J = 6.9 Hz, 2H), 8.05 (d, J = 0.8 Hz, 1H), 8.34 (d, J = 5.5 Hz, 2H).

### Synthesis of compound 3

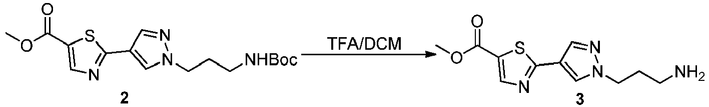

Trifluoroacetic acid (10 mL) was added dropwise to compound 2 (2.4 g, 6.55 mmol, 1.00 eq) in dichloromethane (20 mL). The reaction mixture was allowed to react at room temperature for 2 h and concentrated to give compound 3 as a yellow oil (4 g, crude). m/z (ES+), [M+H]+ = 267; acid, HPLC tR = 0.833 min.

### Synthesis of compound 4

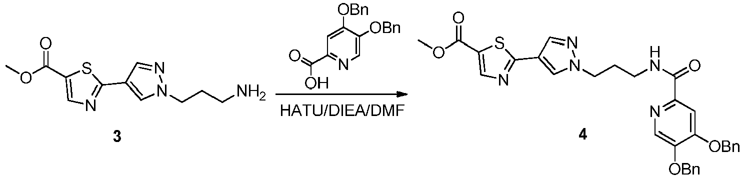

Compound 3 (4 g, crude, 6.55 mmol, 1.00 eq), HATU (3.7 g, 13.11 mmol, 2eq), acid (2.2 g, 6.55 mmol, 1. eq) and DIEA (5.1 g, 40 mmol, 6 eq) were added at 0 °C to *N,N-*dimethylformamide (20 mL). The reaction mixture was allowed to react at room temperature for 2 h. 50 mL of water was added to the reaction mixture, which was then extracted with ethyl acetate (2 × 100 mL). The organic phase was concentrated to dryness by rotary evaporation, and the residue was purified by column chromatography (PE/EA = 1/1 to 0/1) to give white compound 4 (2.5 g, 65.4% for 2 steps). m/z (ES+), [M+H]+ = 584; acid, HPLC tR = 1.069 min. 1H NMR (methanol-d4, 300 MHz): δ (ppm) 2.34 (p, J = 6.5 Hz, 2H), 3.60 (t, J = 6.3 Hz, 2H), 3.97 (s, 3H), 4.42 (t, J = 6.5 Hz, 2H), 5.35 (d, J = 5.1 Hz, 4H), 7.37-7.67 (m, 10H), 7.84 (d,J = 1.8 Hz, 2H), 8.05 (d, J = 0.7 Hz, 1H), 8.21 (s, 1H), 8.32-8.43 (m, 1H).

### Synthesis of compound 5

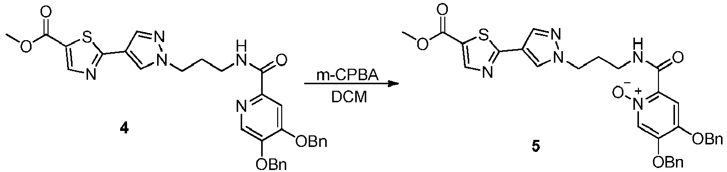

A solution of m-CPBA (1 g, 5.8 mmol, 6 eq) in dichloromethane (20 mL) was added dropwise at 0 °C to compound 4 (500 mg, 0.86 mmol) in dichloromethane (5 mL). The reaction mixture was allowed to react at room temperature for 5 h and then at 45 °C for another 2 h. The reaction mixture was concentrated, and the residue was purified by preparative thin layer chromatography to give white compound 5 (160 mg, 31.2%). m/z (ES+), [M+H]+ = 600; acid, HPLC tR = 1.918 min.

### Synthesis of compound 6

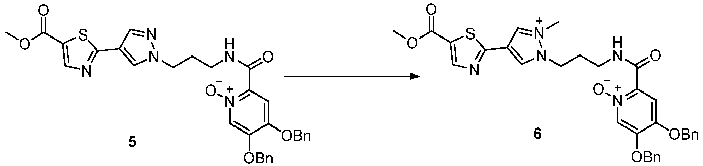

Compound 5 (450 mg, 0.75 mmol, 1.00 eq) was added to a mixture of *N,N-*dimethylformamide (6 mL) and iodomethane (3 mL). The reaction mixture was microwaved at 80 °C for 2 h and directly purified by reversed-phase chromatography to give white compound 6 (130 mg, 28.2%). m/z (ES+), [M]+ = 614; acid, HPLC tR = 1.142 min.

### Synthesis of compound 7

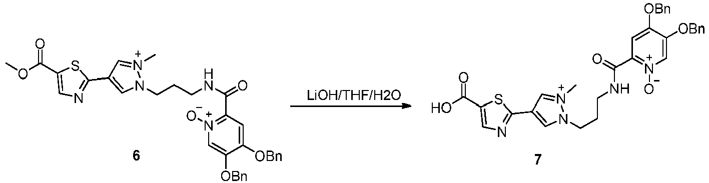

A solution of lithium hydroxide (50 mg, 1.78 mmol, 9 eq) in water (2 mL) was added dropwise to compound 6 (130 mg, 0.21 mmol) in methanol (5 mL). The reaction mixture was allowed to react at room temperature for 2 h, concentrated by rotary evaporation to remove methanol, adjusted to pH 2.5 with 1 N hydrochloric acid, and filtered to give a solid, which was then dried to give white compound 7 (200 mg, crude). m/z (ES+), [M]+ = 600; acid, HPLC tR = 1.490 min.

### Synthesis of compound 8

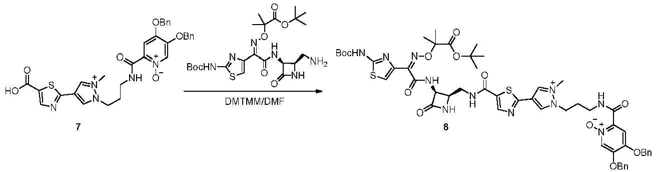

Compound 7 (200 mg, crude, 0.21 mmol) and amine (110 mg, 0.21 mmol) were added to tetrahydrofuran (1.6 mL) and *N,N*-dimethylformamide (1 mL), and DMTMM (100 mg, 0.34 mmol) was added under nitrogen to the mixture. The reaction mixture was stirred at room temperature for 2 h and purified by reversed-phase chromatography to give compound 8 as a white solid (110 mg, 47.5%). m/z (ES-), [M-H] = 1108; acid, HPLC tR = 2.375 min, 1H NMR (methanol-*d*₄, 400 MHz): δ (ppm) 1.47 (s, 9H), 1.52 (d, J = 6.9 Hz, 16H), 2.36 (s, 2H), 3.51-3.69 (m, 3H), 3.74 (dd, J = 14.0, 7.0 Hz, 1H), 3.95-4.12 (m, 1H), 4.26 (s, 3H),4.67 (s, 2H), 5.19 (d, J = 4.5 Hz, 1H), 5.25 (d, J = 10.9 Hz, 3H), 7.32-7.55 (m, 10H), 7.78 (s, 1H), 8.08 (s, 1H), 8.29 (s, 2H), 8.89 (s, 1H), 9.07 (s, 1H).

### Synthesis of compound 9

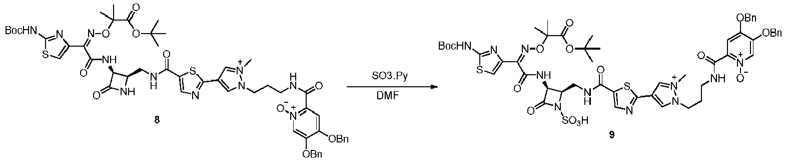

Sulfur trioxide pyridine complex (200 mg, 1.14 mmol, 11 eq) was added to compound 8 (110 mg, 0.1 mmol, 1.00 eq) in *N,N*-dimethylformamide (3 mL). The reaction mixture was stirred at room temperature under nitrogen overnight and purified by reversed-phase chromatography to give compound 9 as a pale yellow solid (30 mg, 25.4%). m/z (ES+), [M]+ = 1189; acid, HPLC tR = 1.137 min.

### Synthesis of compound 1-12

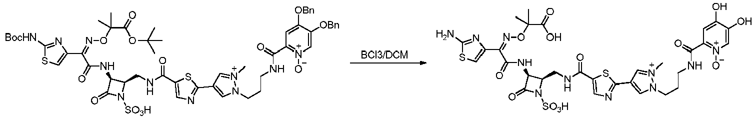

Boron trichloride (0.25 mL, 10.00 eq) was added dropwise at 0 °C under nitrogen to compound 9 (30 mg, 0.025 mmol, 1.00 equiv) in dichloromethane (2 mL). The reaction mixture was stirred at 0 °C for 4 h and blow-dried with nitrogen. Diethyl ether was added, and a solid precipitated. The solid was collected by centrifugation, washed several times with diethyl ether, and purified by preparative high-pressure liquid chromatography (: Atlantis preparative T3 OBD column,, 19 × 250 mm 10 u; mobile phase A: water (0.1% FA); mobile phase B: ACN; flowrate: 25 mL/min; gradient: from 5 B to 33 B over 12 min; 254/220 nm) to give compound 1-12 as a white solid (7.6 mg, 35.3%). m/z (ES+), [M]+ = 852; acid, HPLC tR = 0.557 min. 1H NMR (D₂O, 400 MHz): δ (ppm) 1.53 (d, J = 3.8 Hz, 6H), 2.43 (s, 2H), 3.72 (s, 2H), 3.89 (s, 2H), 4.21 (s, 3H), 4.30 (d, J = 3.7 Hz, 3H), 5.45 (t, J = 4.8 Hz, 1H), 7.07 (d, J = 3.9 Hz, 1H),7.40 (d, J = 4.6 Hz, 1H), 7.76 (d, J = 4.3 Hz, 1H), 8.22 (d, J = 4.3 Hz, 1H), 8.83 (d, J = 37.6 Hz, 2H).

### Example 13. Synthesis of Compound 1-13

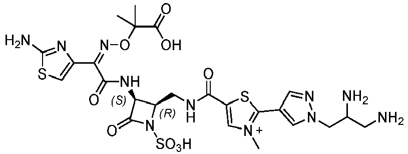

Reference was made to Example 9 for synthesis method.

**Compound 1-13:** m/z (ES+), [M]⁺ = 715; acid, HPLC RT = 5.196 min. ¹H NMR (400 MHz, D₂O) δ 1.51 (s, 6H), 3.24-3.50 (m, 2H), 3.77 (d, *J* = 9.4 Hz, 2H), 4.17-4.18 (m, 4H), 4.60-4.68 (m, 2H), 4.70-4.77 (m, 1H), 5.35 (dd, J = 3.0, 6.1 Hz, 1H), 7.04 (d, J = 4.0 Hz, 1H), 8.28 (d, *J* = 3.2 Hz, 1H), 8.50 (d, *J* = 3.9 Hz, 1H), 8.65 (s, 1H).

### Example 14. Synthesis of Compound 1-14

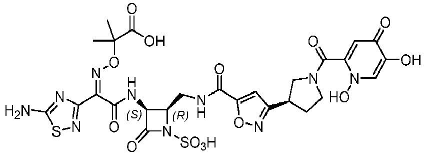

### Synthesis of compound 2:

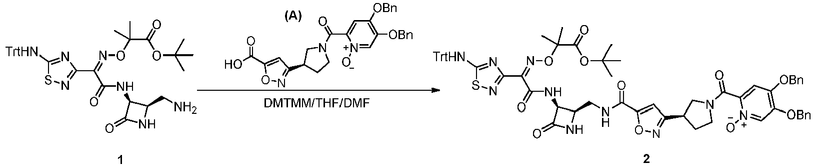

Compound 1 (100 mg, 0.149 mmol) and compound A (115 mg, 0.224 mmol) were dissolved in *N,N*-dimethylformamide (1.5 mL), and 4-(4,6-dimethoxytriazin-2-yl)-4-methylmorpholinium hydrochloride (88 mg, 0.298 mmol) was added slowly. The reaction mixture was stirred at room temperature under nitrogen for 2 h, then quenched with a solution of potassium hydrogen phosphate (10 mL), and extracted with ethyl acetate (10 mL × 3). The organic phase was washed with saturated brine (20 mL), dried over anhydrous sodium sulfate, and concentrated. The residue was purified by reversed-phase column chromatography and lyophilized to give compound 2 as a white solid (78 mg, 44.8%). m/z (ES⁺), [M]⁺ = 1167.

### Synthesis of compound 3:

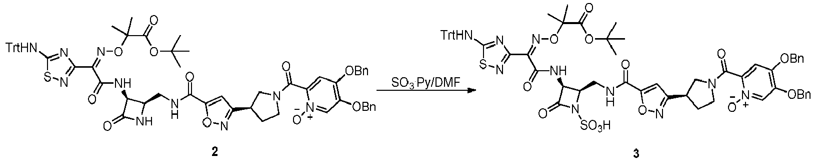

Compound 2 (78 mg, 0.0668 mmol) was dissolved in *N,N*-dimethylformamide (4 mL), and sulfur trioxide pyridine complex (106 mg, 0.668 mmol) was slowly added. The reaction mixture was stirred at 45 °C under nitrogen for 2 h, purified by reversed-phase column chromatography, and lyophilized to give compound 3 as a white solid (70 mg, 84%). m/z (ES+), [M]⁺ = 1247.

### Synthesis of compound 1-14:

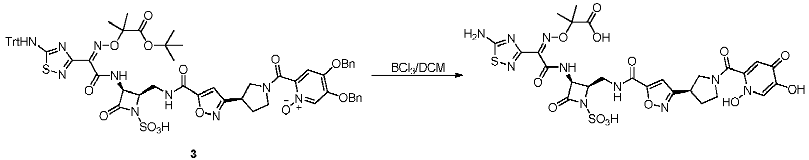

Compound 3 (70 mg, 0.056 mmol) was dissolved in dichloromethane (5 mL), and a solution of boron trichloride in dichloromethane (1 M, 66 mg, 0.561 mmol) was slowly added dropwise in an ice bath under nitrogen. The reaction mixture was stirred in the ice bath for 4 h and blow-dried with nitrogen after the reaction was completed. The residue was washed three times with diethyl ether by centrifugation. The resulting crude product was purified by reversed-phase column chromatography and lyophilized to give compound **1-14** as a white solid (5.2 mg, 12.1 %). m/z (ES⁺), [M+H]⁺ = 769. ¹H NMR (400 MHz, D₂O) δ 7.85 (d, J = 5.1 Hz, 1H), 6.94 (d, J = 7.8 Hz, 2H), 5.32 (s, 1H), 4.53 (s, 1H), 3.97-3.89 (m, 1H), 3.58-3.70 (m, 6H), 3.41 (s, 1H), 2.41 (m, 1H), 2.11 (m, 1H), 1.48 (d, J = 3.6 Hz, 6H).

### Example 15. Synthesis of Compound 1-15

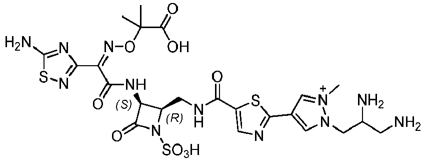

### Synthesis of compound 2:

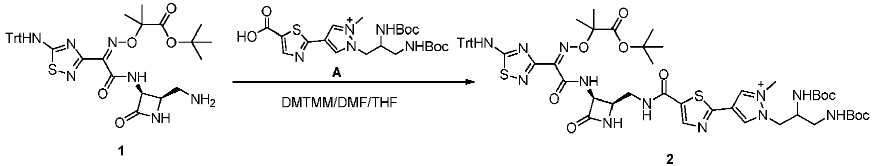

Compound 1 (150 mg, 0.224 mmol) was dissolved in tetrahydrofuran (2.5 mL), and then a solution of compound A (162 mg, 0.336 mmol) in N,N-dimethylformamide (1.5 mL) and 4-(4,6-dimethoxytriazin-2-yl)-4-methylmorpholinium hydrochloride (132 mg, 0.448 mmol) were successively added under nitrogen. The reaction mixture was stirred at room temperature for 2 h. After the reaction was completed, the reaction mixture was concentrated by rotary evaporation to remove the solvent, and a saturated solution of sodium dihydrogen phosphate (10 mL) was added. The mixture was extracted twice with ethyl acetate (10 mL × 2). The organic phase was washed with water (20 mL), and the aqueous phase was extracted with ethyl acetate (10 mL). The organic phases were combined, washed with saturated brine (20 mL), dried over anhydrous sodium sulfate, and concentrated to dryness by rotary evaporation *in vacuo* to give a crude product, which was then purified by reversed-phase column chromatography to give compound 2 as a white solid (50 mg, 19.7%).

### Synthesis of compound 3:

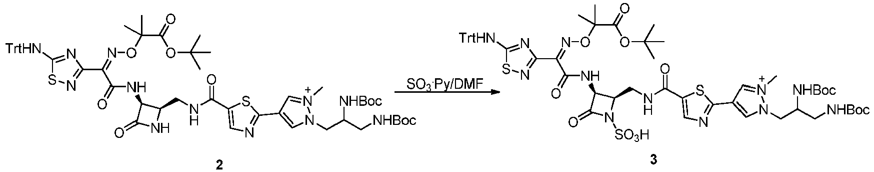

Compound 2 (50 mg, 0.0441 mmol) was dissolved in dry *N,N*-dimethylformamide (4 mL), and sulfur trioxide pyridine complex (70 mg, 0.441 mmol) was added. The reaction mixture was stirred under nitrogen at 45 °C for 2 h. After the reaction was completed, the reaction mixture was directly purified by reversed-phase column chromatography to give compound 3 as a white solid (40 mg, 74.8%). m/z (ES⁺), [M+H]⁺ = 1214.

### Synthesis of compound 1-15:

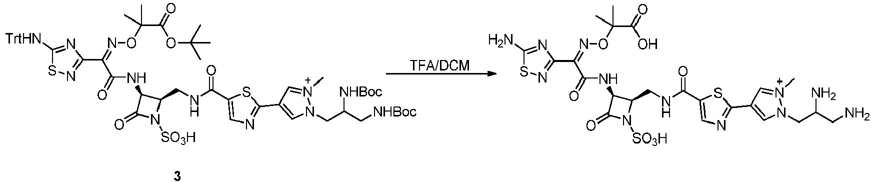

Compound 3 (40 mg, 0.033 mmol) was dissolved in dichloromethane (2 mL), and trifluoroacetic acid (2 mL) was added dropwise at 0 °C under nitrogen. The reaction mixture was stirred at 0 °C for 6 h, concentrated to dryness by rotary evaporation, and lyophilized to give a crude product, which was then purified by reversed-phase column chromatography to give product 1-15 as a white solid (3.8 mg, 16.1%). m/z (ES⁺), [M]⁺ = 716; ¹H NMR (400 MHz, D₂O) δ 8.86 (s, 1H), 8.81 (s, 1H), 8.15 (s, 1H), 5.28 (d, *J* = 5.5 Hz, 1H), 4.52-4.41 (m, 3H), 4.18 (s, 3H), 3.83 (dd, *J* = 14.3, 6.5 Hz, 1H), 3.57 (dd, *J* = 14.3, 6.9 Hz, 1H), 3.50 (s, 1H), 3.29-3.20 (m, 1H), 2.99-2.89 (m, 1H), 1.41 (s, 6H).

### Example 16. Synthesis of Compound 1-16

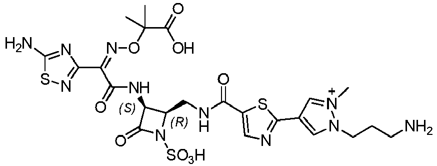

### Synthesis of compound 2:

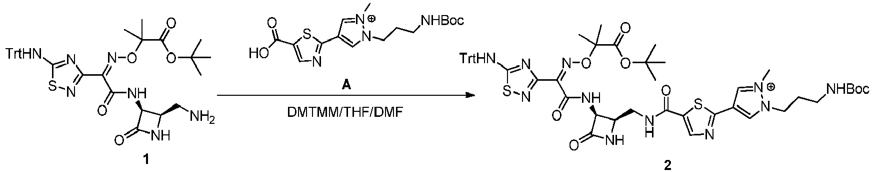

Compound 1 (210 mg, 0.314 mmol) was dissolved in tetrahydrofuran (5.0 mL), and a solution of compound A (173 mg, 0.471 mmol) in *N,N*-dimethylformamide (3.0 mL) and 4-(4,6-dimethoxytriazin-2-yl)-4-methylmorpholinium hydrochloride (185 mg, 0.628 mmol) were successively added under nitrogen. The reaction mixture was stirred at room temperature for 2 h. After the reaction was completed, the reaction mixture was concentrated by rotary evaporation to remove the solvent, and a saturated solution of sodium dihydrogen phosphate (10 mL) was added. The mixture was extracted twice with ethyl acetate (10 mL × 2). The organic phase (20 mL) was washed with water, and the aqueous phase was extracted with ethyl acetate (10 mL). The organic phases were combined, washed with saturated brine (20 mL), dried over anhydrous sodium sulfate, and concentrated to dryness by rotary evaporation *in vacuo* to give a crude product, which was then purified by reversed-phase column chromatography to give compound 2 as a white solid (53 mg, 16.6%). m/z (ES⁺), [M-H]⁺ = 1018.

### Synthesis of compound 3:

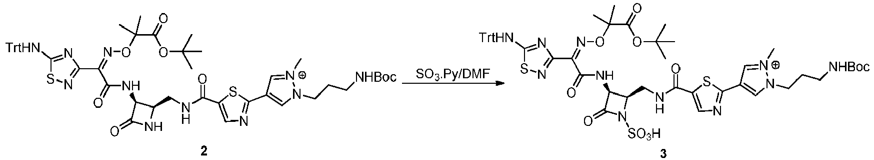

Compound 2 (50 mg, 0.0441 mmol) was dissolved in dry *N,N*-dimethylformamide (4 mL), and sulfur trioxide pyridine complex (78 mg, 0.49 mmol) was added. The reaction mixture was allowed to react under nitrogen at 45 °C for 2 h. After the reaction was completed, the reaction mixture was purified by reversed-phase column chromatography to give compound 3 as a white solid (25 mg, 46.5%). m/z (ES⁺), [M-H]⁺ = 1098.

### Synthesis of compound 1-16:

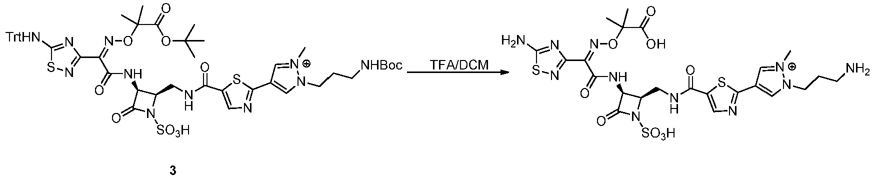

Compound 3 (25 mg, 0.023 mmol) was dissolved in dichloromethane (2 mL), and trifluoroacetic acid (2 mL) was added dropwise at 0 °C under nitrogen. The reaction mixture was stirred at 0 °C for 6 h, concentrated to dryness by rotary evaporation, and lyophilized to give a crude product, which was purified by reversed-phase column chromatography to give product 1-16 as a white solid (2.0 mg, 12.6%). m/z (ES⁺), [M+H]⁺ = 701. ¹H NMR (400 MHz, D₂O) δ 8.83 (s, 1H), 8.76 (s, 1H), 8.14 (s, 1H), 5.28 (d, *J* = 5.5 Hz, 1H), 4.58 (s, 2H), 4.50 (s, 1H),4.15 (s, 3H), 3.82 (dd, *J* = 14.3, 6.6 Hz, 1H), 3.57 (dd, *J* = 14.3, 6.9 Hz, 1H), 3.14-3.06 (m, 2H), 2.33 (p, *J* = 7.6 Hz, 2H), 1.40 (s, 6H).

### Example 17. Synthesis of Compound 1-17

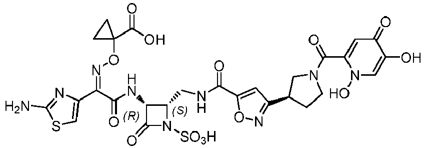

### Synthesis of compound 1:

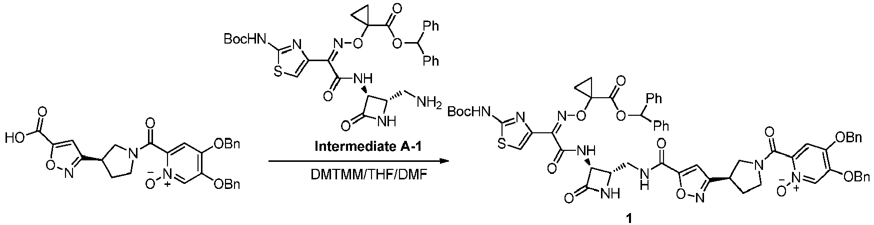

To a solution of (R)-4,5-bis(benzyloxy)-2-(3-(5-carboxyisoxazol-3-yl)pyrrolidine-1-carbonyl)pyridine 1-oxide (324 mg, 0.63 mmol) and compound A (1400 mg, 0.63 mmol) in tetrahydrofuran (3.2 mL) and N,N-dimethylformamide (2 mL) in a 10 mL round-bottom flask was added under nitrogen 4-(4,6-dimethoxytriazin-2-yl)-4-methylmorpholinium hydrochloride (278 mg, 0.945 mmol). The reaction mixture was stirred at room temperature for 2 h and purified by reversed-phase column chromatography to give compound **1** as a white solid (500 mg, 57%). m/z (ES⁺), [M+H]⁺ = 1132.

### Synthesis of compound 2:

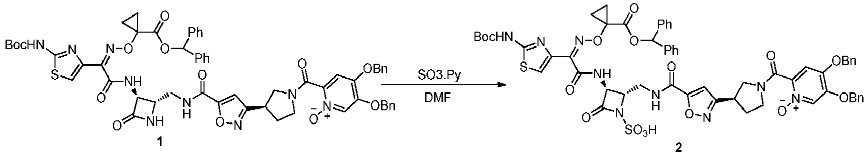

To a solution of compound 1 (500 mg, 0.442 mmol) in *N,N*-dimethylformamide (3 mL) in a 10 mL round-bottom flask was added sulfur trioxide pyridine (600 mg, 3.8 mmol). The resulting solution was stirred at 45 °C for 3 h. The reaction mixture was purified by reversed-phase column chromatography to give compound 2 as a pale yellow solid (350 mg, 65.4%).

m/z (ES⁺), [M+H]⁺ = 1212.

### Synthesis of compound 1-17:

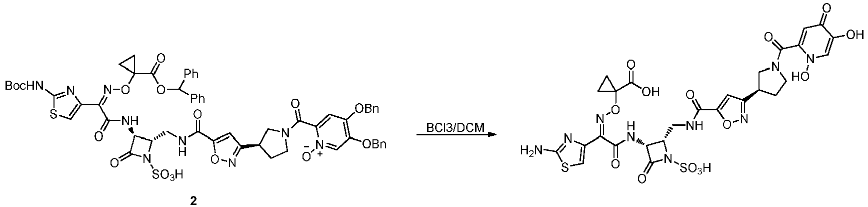

To a solution of compound 2 (340 mg, 0.28 mmol) in dichloromethane (10 mL) was added at 0 °C boron trichloride (5 mL, 5 mmol). The resulting mixture was stirred at 0 °C under nitrogen for 4 h. After the reaction was completed, the solution was blow-dried with nitrogen. The solid was washed three times with diethyl ether and centrifuged to give a crude product, which was then purified by reversed-phase column chromatography to give 1-17 as an off-white solid (61.8 mg). m/z (ES⁺), [M+H]⁺ = 766. ¹H NMR (400 MHz, D₂O): δ (ppm) 1.42 (t, J = 10.1 Hz, 4H), 2.20 (ddd, *J* = 21.0, 13.5, 7.1 Hz, 1H), 2.35-2.64 (m, 1H), 3.33-4.15 (m, 7H), 4.55-4.68 (m, 1H), 5.41 (t, *J* = 5.6 Hz, 1H), 6.86-7.13 (m, 2H), 7.16 (d, *J* = 3.1 Hz, 1H), 7.94 (d, *J* = 5.3 Hz, 1H).

### Example 18. Synthesis of Compound 1-18

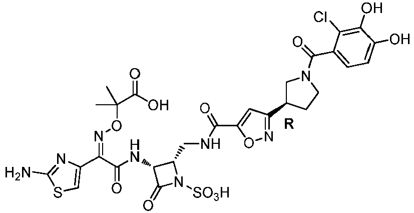

### Synthesis of compound 2

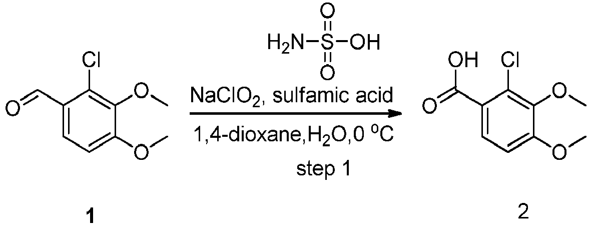

2-Chloro-3,4-dimethoxybenzaldehyde (20.06 g, 100 mmol) was dissolved in dioxane (300 mL), and a solution of sulfamic acid (21.36 g, 220 mmol) in water (200 mL) and a solution of NaClO₂ (19.9 g, 220 mmol) in water (100 mL) were successively added dropwise at 0 °C to the reaction mixture, which was then allowed to react at 0 °C for 30 min. The reaction mixture was quenched with a solution of sodium bisulfite (45.8 g, 440 mmol) in water (200 mL) and extracted with ethyl acetate. The organic phase was washed successively with water and saturated brine, dried over anhydrous sodium sulfate, and concentrated to dryness by rotary evaporation to give compound 2 (21.7 g, 100%). m/z (ES+), [M-H]+ = 215; acid, HPLC tR = 0.673 min. 1H-NMR (DMSO-*d*₆) δ: 13.04 (br s, 1H), 7.63 (d, *J* = 8.9 Hz, 1H), 7.11 (d, J = 8.9 Hz, 1H), 3.89 (s, 3H), 3.74(s, 3H).

### Synthesis of compound 3

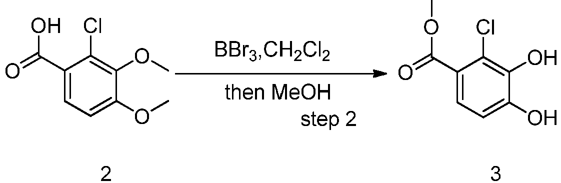

Boron tribromide (24 mL, 253 mmol) was added dropwise at 0 °C to compound 2 (21.7 g, 100 mmol) in dichloromethane (180 mL). The reaction mixture was allowed to react at room temperature for 2 h. Methanol (270 mL) was slowly added to the reaction mixture, which was then allowed to react for another two days, followed by the addition of ice water. Methanol was removed by rotary evaporation, and a solid precipitated. The solid was collected by filtration and dried to give compound 3 (18.9 g, 93%). [M+H]⁺ = 203; acid, HPLC tR = 0.987 min. 1H-NMR (DMSO-*d*₆) δ: 10.50 (br s, 1H), 9.39 (br s, 1H), 7.22 (d, *J* = 8.5 Hz, 1H), 6.78 (d, *J* = 8.5 Hz, 1H), 3.76 (s, 3H).

### Synthesis of compound 5

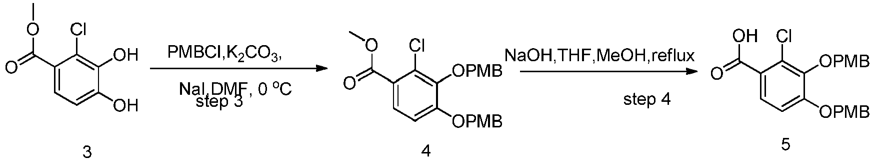

Potassium carbonate (32.4 g, 23.4 mmol), sodium iodide (14 g, 93.5 mmol) and 4-methoxybenzyl chloride (33.5 g, 215 mmol) were added to compound 3 (18.9 g, 93.5 mmol) in *N,N*-dimethylformamide (140 mL). The reaction mixture was allowed to react at 50 °C for 3.5 h. Water (500 mL) and IPE (200 mL) were added to the reaction mixture, and a solid precipitated. The solid was collected by filtration and dissolved in tetrahydrofuran (230 mL) and methanol (180 mL), followed by the addition of 2 mol/L sodium hydroxide (125 mL, 250 mmol) to the reaction mixture, which was then refluxed for 1.5 h. The solvent was removed by rotary evaporation. IPE and 2 mol/L hydrochloric acid (135 mL, 270 mmol) were added to the mixture, and a solid precipitated. The solid was collected by filtration and dried to give compound 5 (19.6 g, 48.5%). m/z (ES+), [M+Na]+ = 451; acid, HPLC tR = 1.393 min.

### Synthesis of compound 6

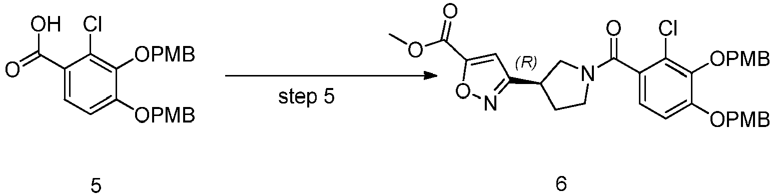

Compound 5 (1 g, 2.33 mmol), amine (541 mg, 2.33 mmol), HOBt (630 mg, 4.66 mmol), triethylamine (940 mg, 9.32 mmol) and EDC·HCl (895 mg, 4.66 mmol) were added to 50 mL of tetrahydrofuran. The reaction mixture was allowed to react at room temperature for 3 h, followed by the addition of water and ethyl acetate. The aqueous phase was separated and extracted with ethyl acetate. The organic phases were combined, washed with saturated brine, dried over anhydrous sodium sulfate, and concentrated to dryness by rotary evaporation. The residue was purified by column chromatography to give compound 6 as a yellow oil (720 mg, 52%). m/z (ES+), [M]⁺ = 607; acid, HPLC tR = 1.308 min.

### Synthesis of compound 7

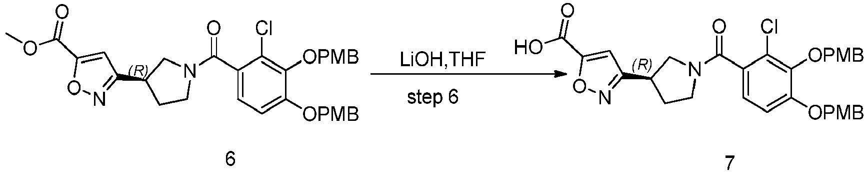

An aqueous solution of lithium hydroxide (4 mL, 2 mol/L) was added dropwise to compound 6 (440 mg, 0.73 mmol) in methanol (6 mL). The reaction mixture was allowed to react at room temperature for 2 h and concentrated by rotary evaporation to remove methanol. The aqueous phase was adjusted to pH 2.5 with hydrochloric acid (1 mol/L) and extracted with ethyl acetate. The organic phase was concentrated to give white compound 7 (650 mg, 92.4%). m/z (ES+), [M]⁺ = 593; acid, HPLC tR = 1.654 min.

### Synthesis of compound 8

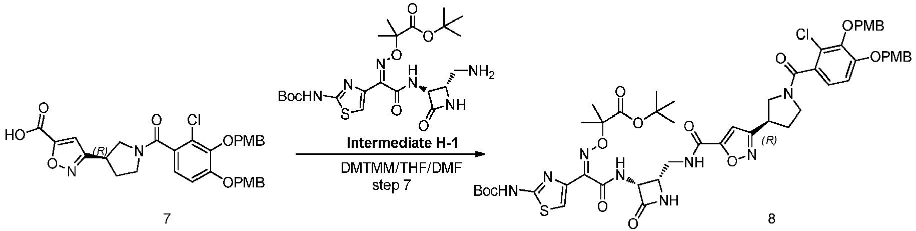

Compound 7 (323 mg, 0.54 mmol) and compound H-1 (286 mg, 0.54 mmol) were added to tetrahydrofuran (1.6 mL) and N,N-dimethylformamide (1 mL), and DMTMM (240 mg, 0.81 mmol) was added under nitrogen to the mixture. The reaction mixture was stirred at room temperature for 2 h and purified by reversed-phase chromatography to give compound 8 as a white solid (350 mg, 53.8%). m/z (ES+), [M]⁺ = 1101; acid, HPLC tR = 1.996 min.

### Synthesis of compound 9

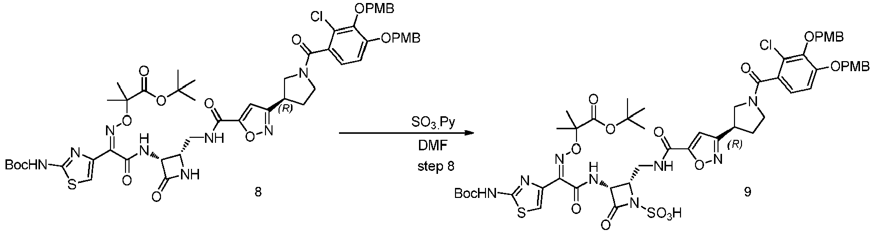

Sulfur trioxide pyridine complex (350 mg, 2.21 mmol, 7 eq) was added to compound 8 (350 mg, 0.318 mmol, 1.00 eq) in *N,N*-dimethylformamide (3 mL). The reaction mixture was stirred at 45 °C under nitrogen for 3 h and purified by reversed-phase chromatography to give compound 9 as a pale yellow solid (270 mg, 72%). m/z (ES+), [M]⁺ = 1181; acid, HPLC tR = 1.200 min.

### Synthesis of compound 1-18

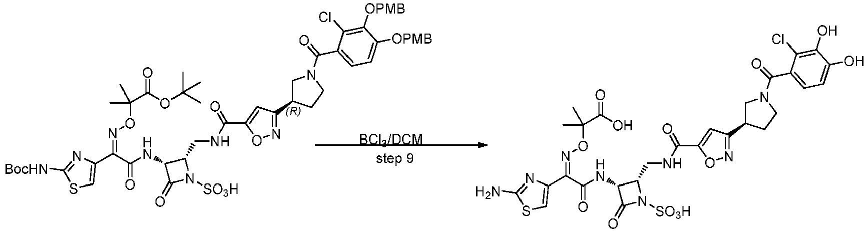

Boron trichloride (0.63 mL, 0.63 mmol) was added dropwise at 0 °C under nitrogen to compound 9 (50 mg, 0.042 mmol, 1.00 eq) in dichloromethane (2 mL). The reaction mixture was stirred at 0 °C for 4 h and blow-dried with nitrogen to remove the solvent. The residue was washed three times with diethyl ether by centrifugation. The solid was purified by preparative high-pressure liquid chromatography (XSelect CSH preparative C18 OBD column, 19 × 250 mm, 5 µm; mobile phase A: water (0.1% FA); mobile phase B: ACN; flowrate: 25 mL/min; gradient: from 5 B to 30 B over 10 min; 254/220 nm;) to give 1-18 as a white solid (7.0 mg). m/z (ES+), [M]⁺ = 785; acid, HPLC tR = 0.673 min. ¹H NMR (D₂O, 400 MHz): δ (ppm) 1.11-1.62 (m, 6H), 2.00-2.48 (m, 2H), 3.25-3.39 (m, 2H), 3.54-3.95 (m, 5H), 4.45-4.60 (m, 1H), 5.33 (t, *J*=5.9 Hz, 1H), 6.67-6.95 (m, 3H), 6.98-7.08 (m, 1H).

### Example 19. Synthesis of Compound 1-19

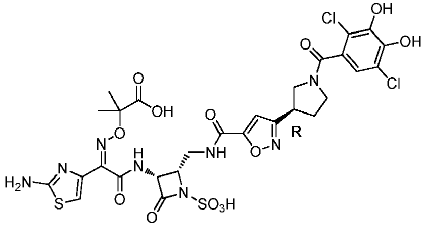

### Synthesis of compound 2

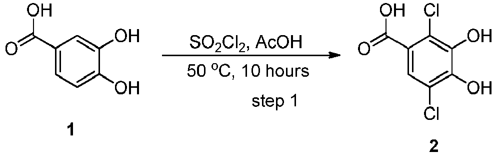

Sulphonyl chloride (10.51 g, 77.8 mmol) was added dropwise to a solution of 3,4-dihydroxybenzoic acid (5 g, 32.46 mmol) in acetic acid (20 mL). The reaction mixture was stirred at 50 °C overnight, cooled, and crystallized from ethyl acetate/n-hexane to give compound 2 (2 g, 27%). [M]⁺ = 223; acid, HPLC tR = 0.659 min.

### Synthesis of compound 3

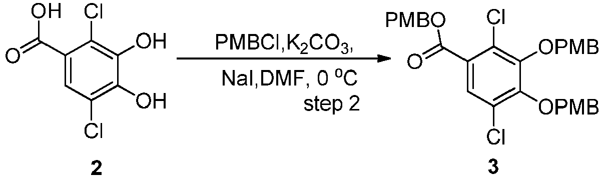

4-methoxybenzyl chloride (5.6 g, 36 mmol) was added to compound 2 (2 g, 9 mmol), potassium carbonate (6.5 g, 47 mmol) and sodium iodide (1.35 g, 9 mmol) in *N,N-*dimethylformamide (50 mL). The reaction mixture was stirred at 50 °C for 6 h. Water (50 mL) was added to the reaction mixture, which was then extracted with ethyl acetate (100 mL). The organic phase was washed with saturated brine, dried over anhydrous sodium sulfate, and concentrated to dryness by rotary evaporation. The residue was purified by column chromatography to give compound 3 (4.88 g, 93%). [M+Na+ACN]+ = 646; acid, HPLC tR = 2.274 min.

### Synthesis of compound 4

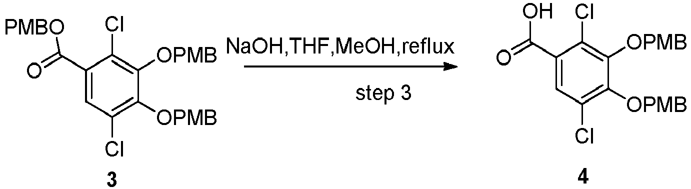

8 mL of sodium hydroxide (2 mol/L, 16 mmol) was added dropwise to a solution of compound 3 (2.4 g, 4.12 mmol) in tetrahydrofuran (8 mL) and methanol (6 mL). The reaction mixture was refluxed for 1.5 h. The solvent was removed by rotary evaporation. IPE and 2 mol/L hydrochloric acid (8.5 mL, 17 mmol) were added to the mixture, and a solid was collected by filtration and dried to give compound 4 (1.57 g, 82.6%). m/z (ES+), [M+Na]+ = 486; acid, HPLC tR = 1.505 min.

### Synthesis of compound 5

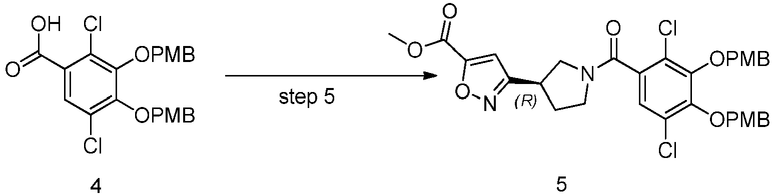

Compound 4 (462 mg,1 mmol), amine (232 mg,1 mmol), HOBt (275 mg, 2 mmol), triethylamine (412 mg, 4 mmol) and EDC·HCl (384 mg, 2 mmol) were added to tetrahydrofuran (5 mL). The reaction mixture was allowed to react at room temperature for 3 h. Water was added to the mixture, which was then extracted 3 times with ethyl acetate. The organic phase was washed with saturated brine, dried over anhydrous sodium sulfate, and concentrated to dryness by rotary evaporation. The residue was purified by column chromatography to give compound 6 as a yellow oil (318 mg, 50%). m/z (ES+), [M+H]⁺ = 641; acid, HPLC tR = 1.955 min.

### Synthesis of compound 6

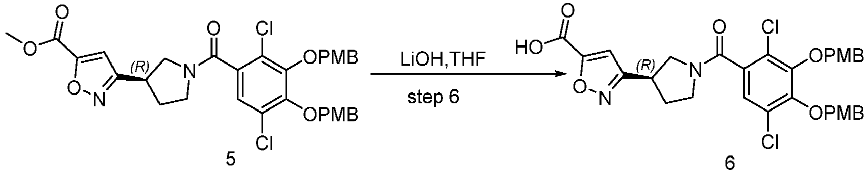

An aqueous solution of lithium hydroxide (1 mL, 2 mol/L) was added dropwise to compound 6 (318 mg, 0.5 mmol) in methanol (3 mL). The reaction mixture was allowed to react at room temperature for 2 h and concentrated by rotary evaporation to remove methanol. The aqueous phase was adjusted to pH 2.5 with hydrochloric acid (1 mol/L) and extracted with ethyl acetate. The organic phase was concentrated to dryness by rotary evaporation to give compound 7 as a white solid (276 mg, 88.7%). m/z (ES+), [M+H]⁺ = 627; acid, HPLC tR = 1.793 min.

### Synthesis of compound 7

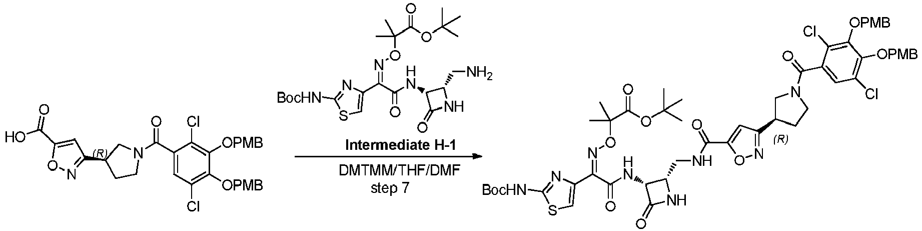

Compound 6 (276 mg, 0.44 mmol) and amine (232 mg, 0.44 mmol) were added to tetrahydrofuran (1.6 mL) and *N,N*-dimethylformamide (1 mL), and DMTMM (200 mg, 0.66 mmol) was added under nitrogen to the mixture. The reaction mixture was stirred at room temperature for 2 h and purified by reversed-phase chromatography to give white compound 7 (270 mg, 55%). m/z (ES+), [M]⁺ = 1136; acid, HPLC tR = 2.094 min.

### Synthesis of compound 8

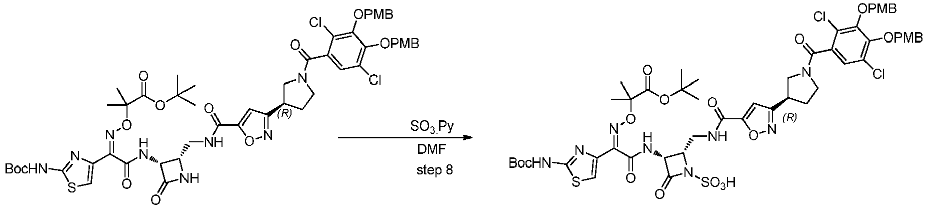

Sulfur trioxide pyridine complex (300 mg, 1.89 mmol, 8 eq) was added to compound 7 (270 mg, 0.238 mmol, 1.00 eq) in *N,N*-dimethylformamide (3 mL). The reaction mixture was stirred at 45 °C under nitrogen for 3 h and purified by reversed-phase chromatography to give compound 8 as a pale yellow solid (200 mg, 69%). m/z (ES+), [M]⁺ = 1216; acid, HPLC tR = 1.199 min.

### Synthesis of compound 1-19

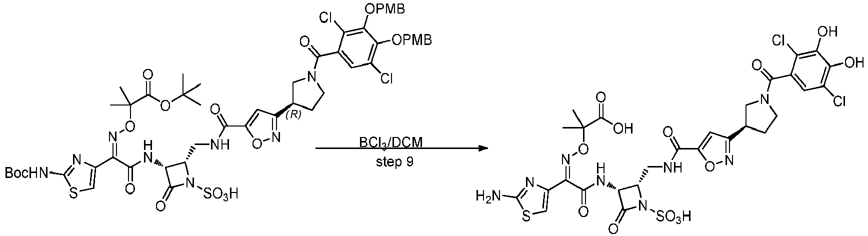

Boron trichloride (0.63 mL, 0.63 mmol) was added dropwise at 0 °C under nitrogen to compound 8 (50 mg, 0.042 mmol, 1.00 eq) in dichloromethane (2 mL). The reaction mixture was stirred at 0 °C for 4 h and blow-dried with nitrogen to remove the solvent. The residue was washed three times with diethyl ether by centrifugation. The solid was purified by preparative high-pressure liquid chromatography (column: XSelect CSH preparative C18 OBD column, 19 × 250 mm, 5 µm; mobile phase A: water (0.1% FA); mobile phase B: ACN; flowrate: 25 mL/min; gradient: from 5 B to 30 B over 10 min; 254/220 nm;) to give compound 1-19 as a white solid (1.9 mg). m/z (ES+), [M]+ = 819; acid, HPLC tR = 0.740 min. ¹H NMR (D₂O, 400 MHz): δ (ppm) 1.31-1.62 (m, 6H), 2.00-2.48 (m, 2H), 3.25 -3.95 (m, 7H), 4.45-4.60 (m, 1H), 5.33 (t, *J* = 5.9 Hz, 1H), 6.71-6.95 (m, 2H), 6.98-7.08 (m, 1H).

### Example 20. Synthesis of Compound 1-20

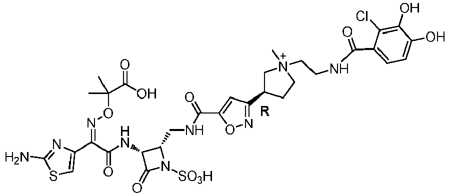

### Synthesis of compound 2

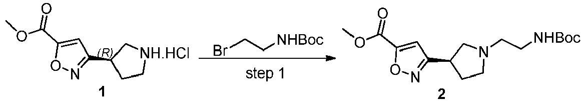

Compound 1 (2.1 g, 9 mmol), *N,N*-diisopropylethylamine (3.48 g, 27 mmol) and N-Boc-bromoethylamine (2.02 g, 9 mmol) were dissolved in acetonitrile (20 mL). The reaction mixture was stirred at 60 °C for 2 h, cooled to room temperature, and concentrated to dryness by rotary evaporation. The residue was purified by silica gel thin layer chromatography to give compound 2 as a yellow oil (1.26 g, 41.2% yield). m/z (ES+), [M+1] = 340; acid, HPLC RT = 1.137 min. ¹H NMR (300 MHz, DMSO-*d*₆) δ 1.43 (s, 9H), 1.90-2.08 (m, 1H), 2.33 (ddt, J = 6.7, 9.8, 13.2 Hz, 1H), 2.55-2.85 (m, 5H), 3.04 (dd, J = 8.0, 9.6 Hz, 1H), 3.21 (t, J = 6.8 Hz, 2H), 3.47-3.66 (m, 1H), 3.97 (s, 3H), 7.09 (s, 1H).

### Synthesis of compound 3

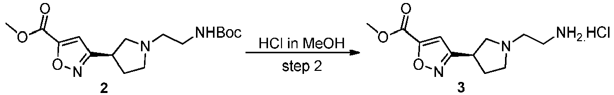

Compound 2 (1.26 g, 3.71 mmol) was dissolved in methanol (10 mL), and a solution of hydrogen chloride in methanol (4 M, 20 mL) was added slowly. The reaction mixture was stirred at room temperature for 8 h and concentrated to dryness by rotary evaporation to give compound 3 as a brown oily crude product (1.19 g), which was directly used in the next step without purification. m/z (ES+), [M+H]⁺ = 240; acid, HPLC RT = 0.523 min.

### Synthesis of compound 4

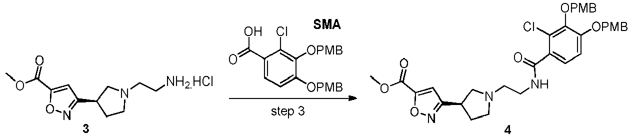

Starting material A (1.85 g, 4.31 mmol) was dissolved in *N,N*-dimethylformamide (20 mL), and HATU (3.28 g, 8.62 mmol), *N,N*-diisopropylethylamine (2.22 g, 17.24 mmol) and compound 3 (1.19 g, 4.31 mmol) were slowly added. The reaction mixture was stirred at room temperature for 3 h, quenched with water, and extracted three times with ethyl acetate. The organic phase was washed three times with saturated brine, dried, and concentrated to dryness by rotary evaporation. The residue was purified by silica gel column chromatography to give compound 4 as a pale yellow solid (1.75 g, 62.9% yield). m/z (ES+), [M]+ = 650; acid, HPLC RT = 1.775 min.

### Synthesis of compound 5

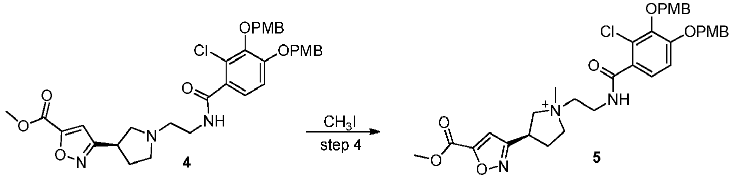

Compound 4 (1.5 g, 2.3 mmol) was dissolved in N,N-dimethylformamide (20 mL), and iodomethane (327 mg, 2.3 mmol) was slowly added dropwise to the solution. The reaction mixture was stirred at 80 °C for 4 h, directly purified through a C18 column, and lyophilized to give compound 5 as a yellow oil (860 mg, 56.2% yield). m/z (ES+), [M]⁺ = 665; acid, HPLC RT = 1.557 min.

### Synthesis of compound 6

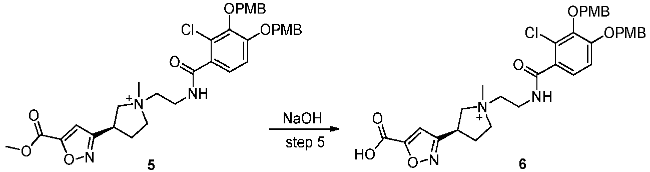

Compound 5 (860 mg, 1.29 mmol) was dissolved in methanol/tetrahydrofuran (5 mL/5 mL), and a 1 M aqueous solution of sodium hydroxide (2.5 mL, 2.58 mmol) was slowly added dropwise. The reaction mixture was stirred at room temperature for 3 h and concentrated, and the residue was dissolved in water. The aqueous solution was adjusted to pH 3-4 with 1 M hydrochloric acid and lyophilized to give compound 6 as a yellow solid crude product (850 mg), which was directly used in the next step without purification. m/z (ES+), [M]⁺ = 651; acid, HPLC RT = 1.474 min.

### Synthesis of compound 7

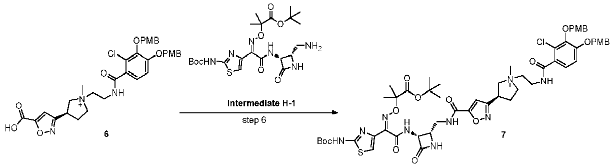

Compound 6 (850 mg, 1.31 mmol) and compound H-1 (690 mg, 1.31 mmol) were dissolved in *N,N*-dimethylformamide/tetrahydrofuran (6 mL/9 mL), and DMTMM (773 mg, 2.62 mmol) was added slowly. The reaction mixture was stirred at room temperature for 2 h, directly purified through a C18 column, and lyophilized to give compound 7 as a yellow solid (388 mg, 25.5% yield). m/z (ES+), [M]⁺ = 1159; acid, HPLC RT = 2.217 min.

### Synthesis of compound 8

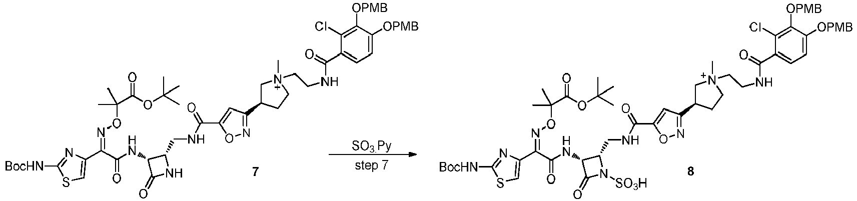

Compound 7 (150 mg, 0.13 mmol) was dissolved in *N,N*-dimethylformamide (3 mL), and sulfur trioxide pyridine complex (205 mg, 1.3 mmol) was slowly added. The reaction mixture was stirred at 45 °C for 6 h, directly purified through a C18 column, and lyophilized to give compound 8 as a yellow solid (60 mg, 37.5% yield). m/z (ES+), [M]⁺ = 1239; acid, HPLC RT = 2.250 min.

### Synthesis of compound 1-20

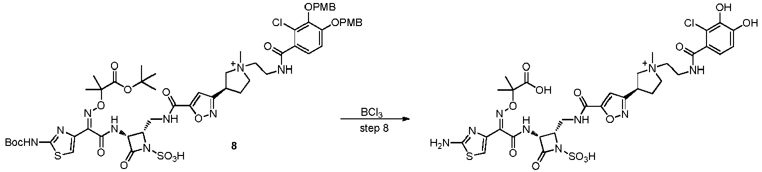

Compound 8 (60 mg, 0.048 mmol) was dissolved in dichloromethane (3 mL), and the system was purged three times with nitrogen. A solution of boron trichloride in dichloromethane (1 M, 2 mL) was slowly added dropwise in an ice bath under nitrogen. The reaction mixture was stirred in the ice bath for 4 h. After the reaction was completed, the reaction was blow-dried with nitrogen. The residue was washed three times with diethyl ether by centrifugation. The crude product was lyophilized and purified by preparative high-pressure liquid chromatography to give compound 1-20 as an off-white solid (2 mg, 4.9% yield). m/z (ES+), [M]⁺ = 843; acid, HPLC RT = 0.981 min. ¹H NMR (400 MHz, D₂O) δ 1.47 (d, J = 5.6 Hz, 6H), 2.44-2.62 (m, 1H), 2.82 (d, *J* = 10.4 Hz, 1H), 3.31 (d, *J* = 13.3 Hz, 3H), 3.72-4.00 (m, 9H), 4.08-4.25 (m, 2H), 4.52-4.67 (m, 1H), 5.41 (dd, *J* = 2.5, 5.7 Hz, 1H), 6.85-7.00 (m, 2H), 7.00-7.08 (m, 2H).

### Example 21. Synthesis of Compound 1-21

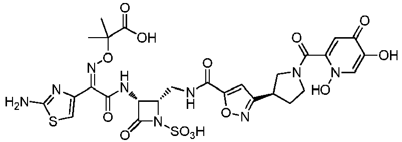

### Synthesis of compound 3:

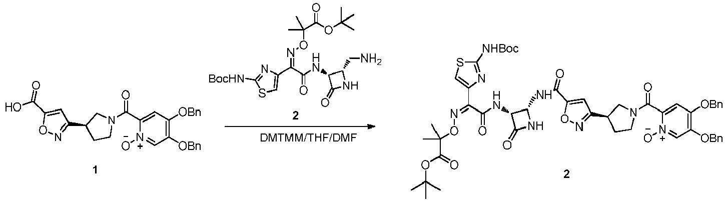

4-(4,6-Dimethoxytriazin-2-yl)-4-methylmorpholinium hydrochloride (100 mg, 0.34 mmol) was added under nitrogen to compound 1 (110 mg, 0.21 mmol) and compound H-1 (100 mg, 0.19 mmol) in a solvent mixture of THF (1.6 mL)/DMF (1 mL). The reaction system was stirred at room temperature for 2 h. The reaction mixture was directly purified by reversed-phase column chromatography to give 2 as a white solid (130 mg, 54.6%). m/z (ES⁺), [M]⁺ = 1024.

### Synthesis of compound 4:

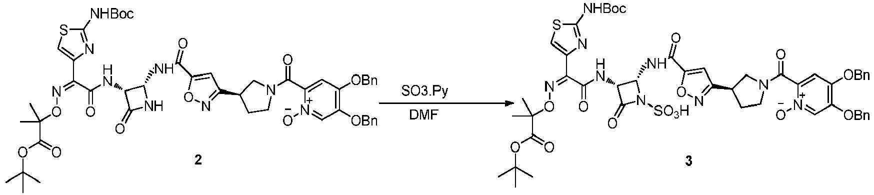

SO₃.Py (200 mg, 1.14 mmol) was added at 0 °C to compound 2(108 mg, 0.1 mmol) in *N,N-*dimethylformamide (3 mL). The reaction system was stirred at room temperature for 20 h, and the reaction mixture was directly purified by reversed-phase column chromatography to give 3 as a pale yellow solid (56 mg, 50%). m/z (ES⁺), [M]⁺ = 1104.

### Synthesis of compound 1-21:

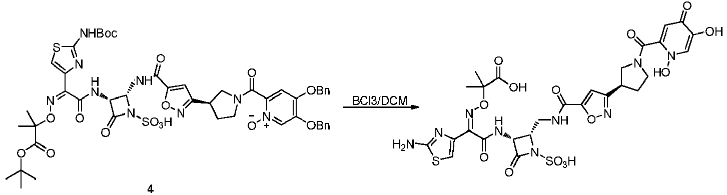

Boron trichloride (1 mol/L in dichloromethane) was added at 0 °C under nitrogen to a solution of compound 4 (92 mg, 0.083 mmol) in dichloromethane (7 mL). The reaction system was stirred at 0 °C for 1 h and blow-dried with nitrogen to remove the solvent, followed by the addition of diethyl ether. The mixture was centrifuged, and the supernatant was removed. The solid was purified by reversed-phase column chromatography, and the product was directly lyophilized to give the final product 1-21 as a white solid (12.7 mg, 19.85%). ¹H NMR (400 MHz, D₂O) δ 1.32-1.55 (m, 6H), 2.16 (dq, *J* = 20.4, 6.8, 5.7 Hz, 1H), 2.42 (ddd, *J* = 25.8, 12.6, 6.2 Hz, 1H), 3.35-3.54 (m, 2H), 3.54-3.88 (m, 5H), 4.53-4.60 (m, 1H), 5.37 (t, *J* = 5.7 Hz, 1H), 6.90-7.09 (m, 2H), 7.01-7.04 (m, 1H), 7.87 (d, *J* = 4.6 Hz, 1H). m/z (ES⁺), [M+H]⁺ = 768; [M-H]⁻ = 766.

### Example 22. Synthesis of Compound 1-22

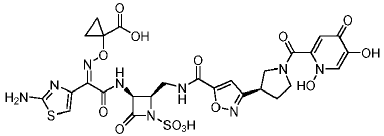

### Synthesis of compound 1

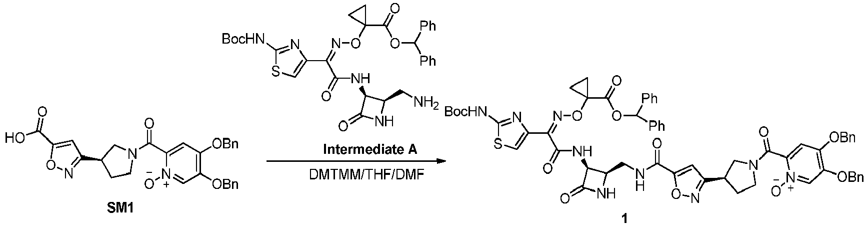

Starting material 1 (140 mg, 0.27 mmol) and compound A (170 mg, 0.27 mmol) were added to tetrahydrofuran (3.2 mL) and *N,N*-dimethylformamide (2 mL), and DMTMM (120 mg, 0.4 mmol) was added under nitrogen to the mixture. The reaction mixture was stirred at room temperature for 2 h and purified by reversed-phase chromatography to give white compound 1 (150 mg, 49%). m/z (ES+), [M]+ = 1132; acid, HPLC tR = 1.91 min.

### Synthesis of compound 2

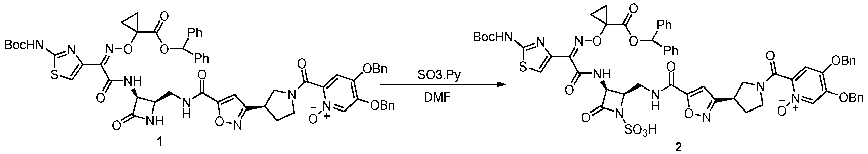

Sulfur trioxide pyridine complex (200 mg, 1.266 mmol, 9.7 eq) was added to compound 1 (150 mg, 0.132 mmol, 1.00 eq) in *N,N*-dimethylformamide (3 mL). The reaction mixture was stirred at 45 °C under nitrogen for 3 h and purified by reversed-phase chromatography to give pale yellow compound 2 (120 mg, 78%). m/z (ES+), [M]+ = 1212; acid, HPLC tR = 1.843 min.

### Synthesis of compound 1-22

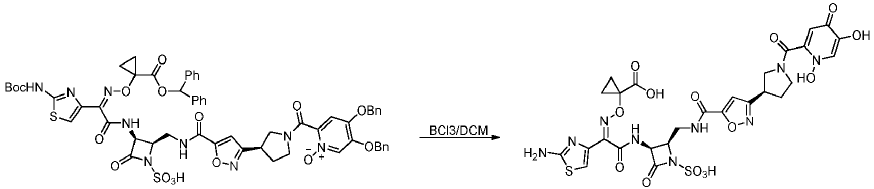

Boron trichloride (1 mL, 1 mmol) was added dropwise at 0 °C under nitrogen to compound 2 (70 mg, 0.058 mmol, 1.00 eq) in dichloromethane (2 mL). The reaction mixture was stirred at 0 °C for 4 h and blow-dried with nitrogen to remove the solvent. The solid was washed three times with diethyl ether by centrifugation and purified by preparative high-pressure liquid chromatography (column: Atlantis preparative T3 OBD column, 19 × 250 mm 10 u; mobile phase A: water (0.1% FA); mobile phase B: ACN; flowrate: 25 mL/min; gradient: from 10 B to 25 B over 10 min; 254/220 nm;) to give white compound 1-22 (11.2 mg). m/z (ES+), [M+H]+ = 766; acid, HPLC tR = 0.869 min. NMR (DMSO-*d*₆, 400 MHz): δ (ppm) 1.18-1.42 (m, 4H), 2.06 (d, *J* = 7.8 Hz, 1H), 2.37 (d, *J* = 7.7 Hz, 1H), 3.44-3.46 (m, 3H), 3.55-3.60 (m, 3H), 3.85-3.90(m, 1H), 4.03-4.27 (m, 1H), 5.19-5.25(m, 1H), 6.71-7.15 (m, 3H), 7.95(s, 1H).

### Example 23. Synthesis of Compound 1-23

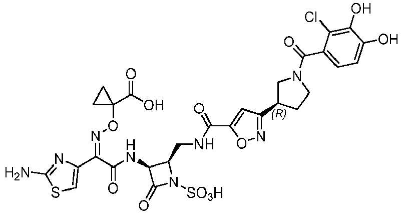

### Synthesis of compound 2

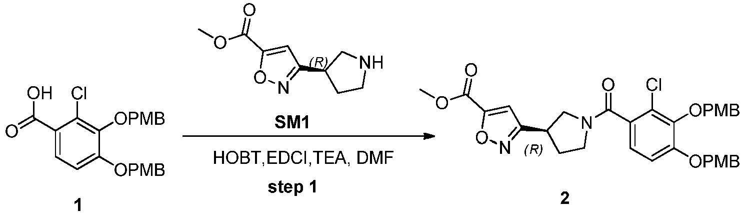

Compound 1 (428 mg,1 mmol) and starting material 1 (232 mg,1 mmol) were dissolved in tetrahydrofuran (5 mL), and HOBt (275 mg, 2 mmol), triethylamine (412 mg, 4 mmol) and EDC·HCl (384 mg, 2 mmol) were added successively. The reaction mixture was stirred at room temperature for 3 h. After the reaction was completed, the reaction mixture was diluted successively with water and ethyl acetate and extracted with ethyl acetate. The organic phases were combined, washed with saturated brine, dried over anhydrous magnesium sulfate, and finally concentrated to dryness. The resulting crude product was purified by silica gel column chromatography to give final product 2 as a yellow oil (606 mg, 50%). m/z (ES+), [M]+ = 607; acid, HPLC tR = 1.180 min.

### Synthesis of compound 3

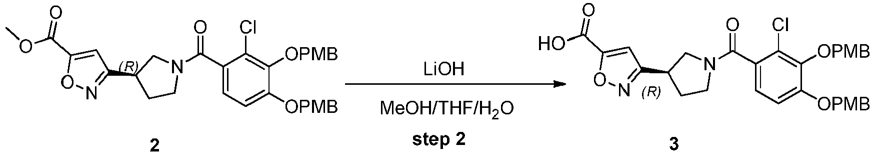

Compound **2** (303 mg, 0.5 mmol) was dissolved in methanol (3.0 mL) and tetrahydrofuran (3.0 mL), and then an aqueous solution of lithium hydroxide (1 mL, 2 mol/L) was added. The reaction mixture was stirred at room temperature for 2 h. After the reaction was completed, methanol and tetrahydrofuran were removed by rotary evaporation, and the remaining aqueous solution was adjusted to pH 2.5 with hydrochloric acid (1 mol/L) and then extracted with ethyl acetate. The organic phase was concentrated to dryness by rotary evaporation to give final product **3** as a white solid (262 mg, 88.7%). m/z (ES+), [M]+ = 593; acid, HPLC tR = 1.104 min.

### Synthesis of compound 4

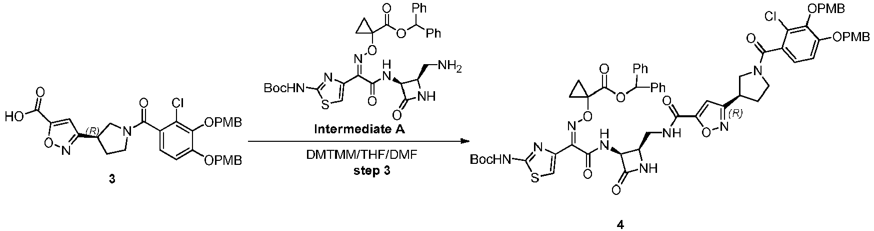

Compound 3 (261 mg, 0.44 mmol) and compound A (279 mg, 0.44 mmol) were dissolved in tetrahydrofuran (1.6 mL) and N,N-dimethylformamide (1 mL), and DMTMM (200 mg, 0.66 mmol) was added slowly. The reaction mixture was stirred under nitrogen at room temperature for 2 h. After the reaction was completed, the crude product was purified by reversed-phase column chromatography to give final product **4** as a white solid (293 mg, 55%). m/z (ES+), [M+H]+ = 1210; acid, HPLC tR = 2.528 min.

### Synthesis of compound 5

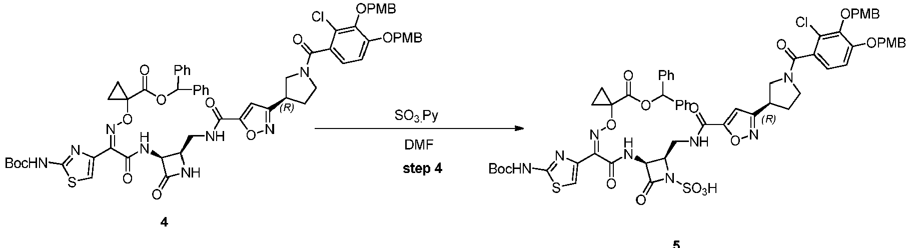

Compound **4** (288 mg, 0.238 mmol, 1.0 eq) was dissolved in *N,N*-dimethylformamide (3 mL), and sulfur trioxide pyridine complex (300 mg, 1.89 mmol, 8.0 eq) was slowly added. Then the reaction mixture was stirred at 45 °C for 3 h. After the reaction was completed, the crude product was purified by reversed-phase column chromatography to give final product 5 as an off-white solid (211 mg, 69%). m/z (ES+), [M+H]+ = 1290; acid, HPLC tR = 1.275 min.

### Synthesis of compound 1-23

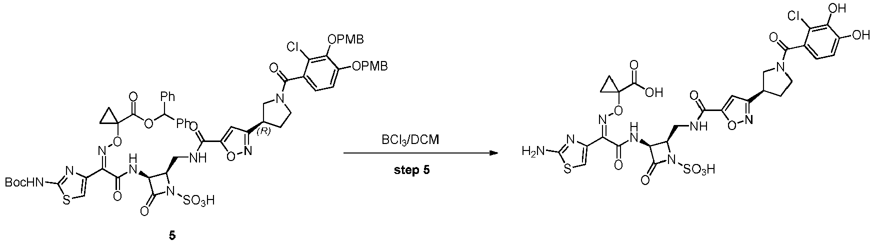

Compound 5 (50 mg, 0.042 mmol, 1.00 eq) was dissolved in dichloromethane (2 mL), and the reaction flask was purged 3 times with nitrogen. A solution of boron trichloride in dichloromethane (0.63 mL, 0.63 mmol) was slowly added dropwise in an ice bath under nitrogen. The reaction mixture was stirred in an ice bath for 4 h and blow-dried with nitrogen. The residue was washed three times with diethyl ether by centrifugation. The resulting crude product was lyophilized and purified by preparative high-pressure liquid chromatography to give compound **1-23** as a light gray solid (4.1 mg). m/z (ES+), [M]+ = 783; acid, HPLC tR = 1.054 min. ¹H NMR (400 MHz, DMSO-*d*₆) δ 8.60 (s, 1H), 7.07-6.69 (m, 3H), 6.64 (dd, *J* = 8.2, 5.0 Hz, 1H), 5.21 (t, J = 5.4 Hz, 1H), 4.25-4.10 (m, 1H), 3.50 (s, 5H), 3.32-3.19 (m, 1H), 2.40-2.24 (m, 1H), 2.04 (ddd, *J* = 19.8, 12.4, 7.3 Hz, 1H), 1.52-1.04 (m, 4H).

### Example 24. Synthesis of Compound 1-24

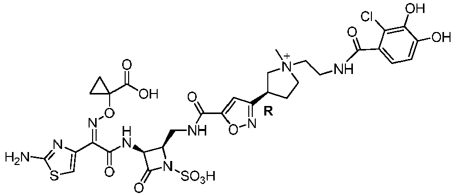

### Synthesis of compound 2

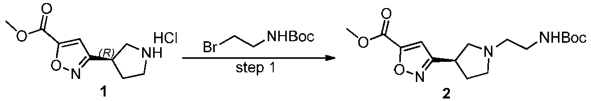

Compound 1 (1.9 g, 8.2 mmol), *N,N*-diisopropylethylamine (3.17 g, 24.6 mmol) and *N-*Boc-bromoethylamine (1.84 g, 8.2 mmol) were dissolved in acetonitrile (10 mL). The reaction mixture was stirred at 60°C for 2 h, cooled to room temperature, and concentrated to dryness by rotary evaporation. The residue was purified by silica gel thin layer chromatography to give compound 2 as a yellow oil (800 mg, 28.7% yield). m/z (ES+), [M+1]=340, acid, HPLC RT = 0.803 min. ¹H NMR (300 MHz, methanol-*d*₄) δ 1.43 (s, 9H), 1.98 (ddd, *J* = 7.2, 13.2, 14.4 Hz, 1H), 2.33 (ddt, J = 6.7, 9.8, 13.2 Hz, 1H), 2.54-2.81 (m, 5H), 3.03 (dd, *J* = 8.0, 9.6 Hz, 1H), 3.21 (t, *J* = 6.8 Hz, 2H), 3.55 (ddt, *J* = 6.7, 7.9, 9.8 Hz, 1H), 3.93 (s, 3H), 7.09 (s, 1H).

### Synthesis of compound 3

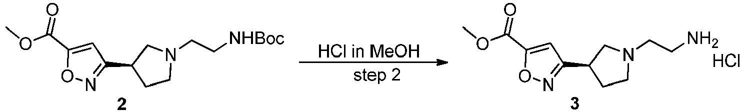

Compound 2 (800 mg, 2.36 mmol) was dissolved in methanol (5 mL), and a solution of hydrogen chloride in methanol (4 M, 10 mL) was added slowly. The reaction mixture was stirred at room temperature for 8 h and concentrated to dryness by rotary evaporation to give compound 3 as a brown semi-solid crude product (750 mg), which was directly used in the next step without purification. m/z (ES+), [M+H]+ = 240, acid, HPLC RT = 0.506 min. ¹H NMR (300 MHz, methanol-*d*₄) δ 2.35 (s, 1H), 2.65 (s, 1H), 3.46 (t, *J* = 7.0 Hz, 3H), 3.68 (t, *J* = 7.0 Hz, 3H), 3.95 (s, 6H), 7.25 (s, 1H).

### Synthesis of compound 4

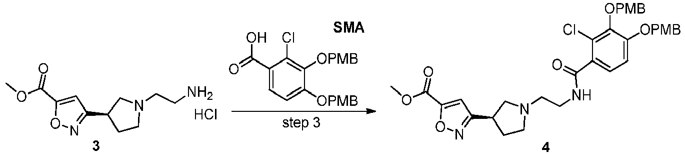

Starting material A (750 mg, 2.72 mmol) was dissolved in *N,N*-dimethylformamide (10 mL), and HATU (2.07 g, 5.44 mmol), *N,N*-diisopropylethylamine (1.40 g, 10.88 mmol) and compound 3 (1.17 g, 2.72 mmol) were slowly added. The reaction mixture was stirred at room temperature for 3 h, quenched with water, and extracted three times with ethyl acetate. The organic phase was washed three times with saturated brine, dried, and concentrated to dryness by rotary evaporation. The residue was purified by silica gel column chromatography to give compound 4 as a pale yellow solid (680 mg, 44.4% yield, 96.94% ee value). m/z (ES+), [M]+ = 650, acid, HPLC RT = 1.400 min. ¹H NMR (300 MHz, methanol-*d*₄) δ 1.90-2.09 (m, 1H), 2.22-2.44 (m, 1H), 2.69-2.96 (m, 5H), 2.97-3.09 (m, 1H), 3.54 (dt, *J* = 7.5, 13.4 Hz, 3H), 3.77 (s, 3H), 3.81 (s, 3H), 3.89 (s, 3H), 4.92 (s, 2H), 5.11 (s, 2H), 6.77-6.85 (m, 2H), 6.92-6.98 (m, 2H), 7.11 (d, *J* = 9.9 Hz, 2H), 7.19 (d, *J* = 8.5 Hz, 1H), 7.25-7.32 (m, 2H), 7.36-7.44 (m, 2H).

### Synthesis of compound 5

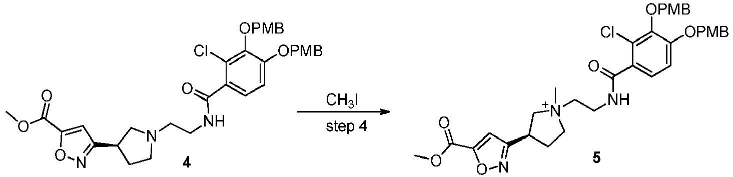

Compound 4 (680 mg, 1.05 mmol) was dissolved in *N,N*-dimethylformamide (10 mL), and iodomethane (746 mg, 5.25 mmol) was slowly added dropwise. The reaction mixture was stirred at 80 °C for 4 h, directly purified through a C18 column, and lyophilized to give compound 5 as a yellow oil (570 mg, 82% yield). m/z (ES+), [M]+ = 665, acid, HPLC RT = 1.681 min. ¹H NMR (300 MHz, methanol-*d*₄) δ 2.51 (dt, *J* = 7.7, 14.5 Hz, 1H), 2.81 (dd, *J* = 7.7, 14.7 Hz, 1H), 3.34 (d, *J* = 5.6 Hz, 3H), 3.67-4.03 (m, 16H), 4.08-4.29 (m, 2H), 4.94 (d, *J* = 2.5 Hz, 2H), 5.12 (s, 2H), 6.77-6.85 (m, 2H), 6.91-6.99 (m, 2H), 7.14 (dd, *J* = 2.6, 8.6 Hz, 1H), 7.18-7.32 (m, 4H), 7.40 (d, *J* = 8.5 Hz, 2H).

### Synthesis of compound 6

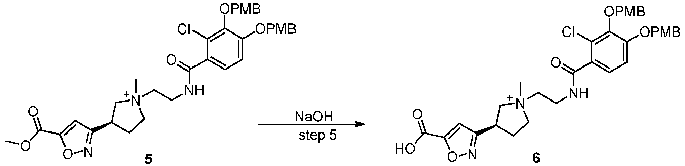

Compound 5 (300 mg, 0.45 mmol) was dissolved in methanol/tetrahydrofuran (2.5 mL/2.5 mL), and a 1 M aqueous solution of sodium hydroxide (1 mL, 0.92 mmol) was slowly added dropwise. The reaction mixture was stirred at room temperature for 3 h and concentrated, and the residue was dissolved in water. The aqueous solution was adjusted to pH 3-4 with 1 M hydrochloric acid and lyophilized to give compound 6 as a yellow solid crude product (350 mg), which was directly used in the next step without purification. m/z (ES+), [M]⁺ = 651, acid, HPLC RT = 1.100 min.

### Synthesis of compound 7

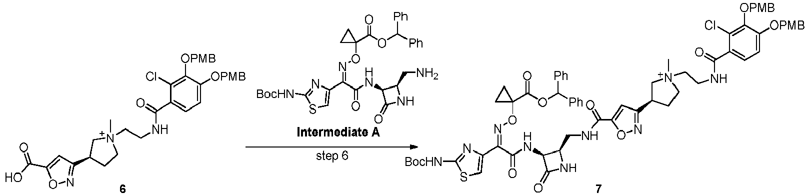

Compound 6 (350 mg, 0.54 mmol) and starting material B (343 mg, 0.54 mmol) were dissolved in *N,N*-dimethylformamide/tetrahydrofuran (2 mL/3 mL), and DMTMM (319 mg, 1.08 mmol) was added slowly. The reaction mixture was stirred at room temperature for 2 h, purified through a C18 column, and lyophilized to give compound 7 as a yellow solid (150 mg, 22% yield). m/z (ES+), [M]+ = 1267, acid, HPLC RT = 1.275 min. ¹H NMR (400 MHz, methanol-*d*₄) δ 1.40-1.64 (m, 13H), 2.39 (td, *J* = 7.1, 14.0, 14.5 Hz, 1H), 2.61-2.84 (m, 1H), 3.25 (s, 1H), 3.42-3.54 (m, 1H), 3.60-4.18 (m, 17H), 4.93 (d, J = 6.4 Hz, 2H), 5.11 (d, J = 6.6 Hz, 2H), 5.26 (dd, J = 4.8, 8.3 Hz, 1H), 6.74-6.83 (m, 2H), 6.87 (d, J = 4.4 Hz, 1H), 6.94 (dq, *J* = 2.6, 3.1, 9.7 Hz, 3H), 7.13 (dd, *J* = 6.5, 8.6 Hz, 1H), 7.17-7.48 (m, 16H), 8.27 (s, 2H).

### Synthesis of compound 8

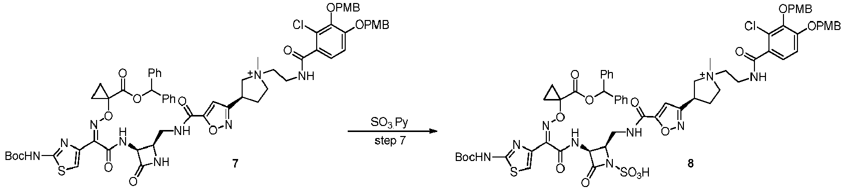

Compound 7 (150 mg, 0.12 mmol) was dissolved in *N,N*-dimethylformamide (3 mL), and sulfur trioxide pyridine complex (380 mg, 2.4 mmol) was slowly added. The reaction mixture was stirred at 45 °C for 6 h and purified through a C18 column to give compound 8 as a yellow solid (60 mg, 37.7% yield). m/z (ES+), [M]+ = 1347, acid, HPLC RT = 2.236 min.

### Synthesis of compound 1-24

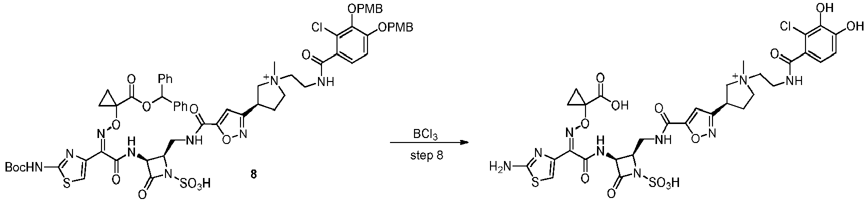

Compound 8 (40 mg, 0.03 mmol) was dissolved in dichloromethane (3 mL), and the system was purged three times with nitrogen. A solution of boron trichloride in dichloromethane (1 M, 0.6 mL) was slowly added dropwise at 0 °C under nitrogen. The reaction mixture was stirred in an ice bath for 4 h. After the reaction was completed, the reaction was blow-dried with nitrogen. The residue was washed three times with diethyl ether by centrifugation. The crude product was lyophilized and purified by preparative high-pressure liquid chromatography to give compound **1-24** as an off-white solid (4.2 mg, 16.8% yield). m/z (ES+), [M]⁺ = 841, acid, HPLC RT = 1.118 min. ¹H NMR (400 MHz, DMSO-*d*₆) δ 1.26 (d, *J* = 27.5 Hz, 4H), 2.23-2.36 (m, 1H), 2.62-2.71 (m, 1H), 3.20 (d, *J* = 2.0 Hz, 3H), 3.37-3.44 (m, 1H), 3.56-3.62 (m, 1H), 3.62-3.69 (m, 3H), 3.69-3.84 (m, 4H), 4.11 (ddd, *J* = 6.1, 10.9, 22.6 Hz, 3H), 5.22 (dd, *J* = 1.4, 5.7 Hz, 1H), 6.78 (d, *J* = 7.5 Hz, 2H), 6.83 (s, 1H), 7.21 (d, *J* = 17.6 Hz, 1H).

### Example 25. Synthesis of Compound 1-25

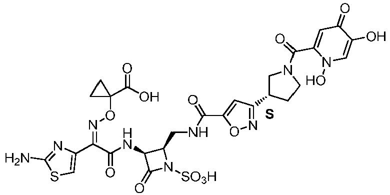

### Synthesis of compound 1:

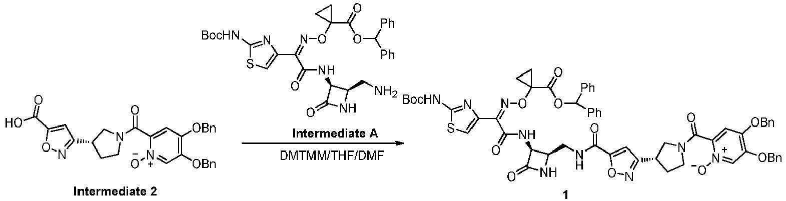

Compound 2 (200 mg, 0.388 mmol) and compound A (246 mg, 0.388 mmol) were dissolved in *N,N*-dimethylformamide/tetrahydrofuran (2 mL/3.2 mL), and 4-(4,6-dimethoxytriazin-2-yl)-4-methylmorpholinium hydrochloride (170 mg, 0.58 mmol) was added slowly. The reaction mixture was stirred at room temperature for 2 h and purified by reversed-phase column chromatography to give compound 1 as an off-white solid (300 mg, 68.3%). m/z (ES⁺), [M+H]⁺ = 1133.

### Synthesis of compound 2:

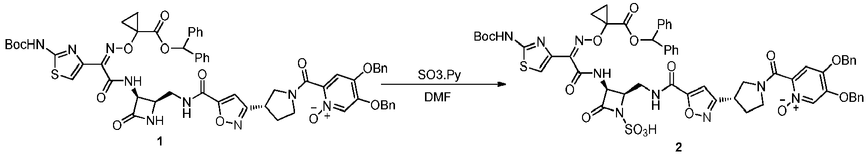

Compound 1 (150 mg, 0.132 mmol) was dissolved in *N,N*-dimethylformamide (3 mL), and sulfur trioxide pyridine complex (200 mg, 1.266 mmol) was slowly added. The reaction mixture was stirred at 45 °C for 3 h and purified by reversed-phase column chromatography to give compound 2 as a pale yellow solid (128 mg, 78%). m/z (ES⁺), [M]+ = 1212.60.

### Synthesis of compound 1-25:

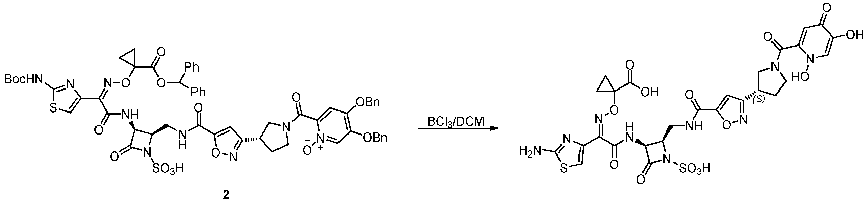

Compound 2 (70 mg, 0.058 mmol) was dissolved in dichloromethane (2 mL), and a solution of boron trichloride in dichloromethane (1 M, 1 mL) was slowly added dropwise in an ice bath under nitrogen. The reaction mixture was stirred in the ice bath for 4 h and blow-dried with nitrogen to remove the solvent. The residue was washed three times with diethyl ether by centrifugation. The resulting crude product was purified by reversed-phase column chromatography to give compound **1-25** as an off-white solid (2.8 mg, 6.3%). m/z (ES⁺), [M+H]⁺ = 766.25. ¹H NMR (400 MHz, DMSO-*d*₆) δ (ppm) 1.18-1.42 (m, 4H), 2.06 (d, *J* = 7.8 Hz, 1H), 2.37 (d, *J* = 7.7 Hz, 1H), 3.44-3.46 (m, 3H), 3.55-3.60 (m, 3H), 3.85-3.90 (m, 1H), 4.03-4.27 (m, 1H), 5.19-5.25 (m, 1H), 6.85 (d, J = 3.0 Hz, 1H), 7.08-6.92 (m, 2H), 7.95(s, 1H).

### Example 26. Synthesis of Compound 1-26

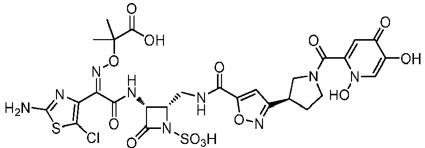

### Synthesis of compound 1

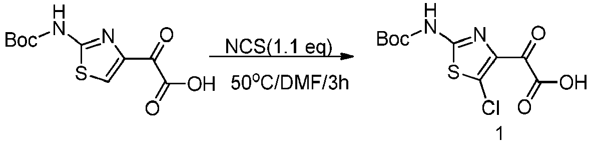

*N*-chlorosuccinimide (12.98 g, 97.24 mmol) was added to 2-(2-((*tert-*butoxycarbonyl)amino)thiazol-4-yl)-2-oxoacetic acid (22 g, 81 mmol) in *N,N-*dimethylformamide (200 mL). The reaction mixture was allowed to react at 50 °C for 3 h and cooled to room temperature. Water was added to the reaction mixture, and the aqueous phase was extracted with ethyl acetate. The organic phase was dried over anhydrous sodium sulfate and concentrated to dryness by rotary evaporation. The residue was purified by silica gel column chromatography to give compound 1 as a yellow oil (16.1 g, 65%). m/z (ES+), [M+Na]+ = 329; acid, HPLC tR = 1.11 min.

### Synthesis of compound 2

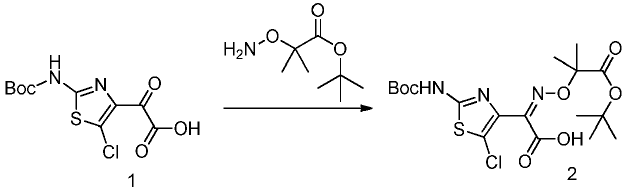

Compound 1 (1.93 g, 6.29 mmol) was added to amine (1 g) in ethyl acetate (20 mL). The reaction mixture was stirred at room temperature for 2 h and concentrated to dryness by rotary evaporation. The residue was purified by silica gel column chromatography to give compound 2 as a yellow solid (2.77 g, crude). m/z (ES+), [M+H]+ = 464; acid, HPLC tR = 1.442 min.

### Synthesis of compound 4

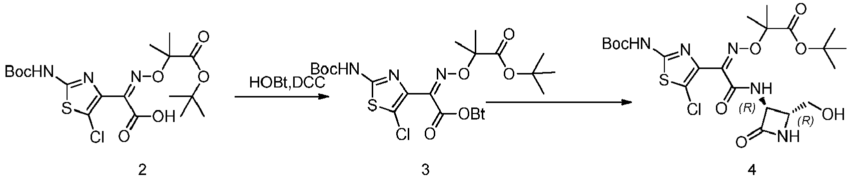

Compound 2 (2 g, crude, 3 mmol) was dissolved in a solvent mixture of dichloromethane (18 mL) and N,N-dimethylformamide (9 mL), and HOBt (434 mg, 3.2 mmol) and DCC (661 mg, 3.2 mmol) were added at 0 °C (N2) to the reaction mixture. The reaction mixture was stirred at 0 °C for 2 h. Dichloromethane (14 mL) and triethylamine (1.072 g, 10 mmol) were added at 0 °C to the reaction mixture. Amine (330 mg, 2.84 mmol) was dissolved in *N,N-*dimethylformamide (10 mL), and the resulting solution was added dropwise at 0 °C to the reaction mixture, which was then allowed to react overnight (from 0 °C to room temperature). NaH₂PO₄ (100 mL) was added to the reaction mixture, which was then extracted with ethyl acetate. The organic phase was washed with saturated brine, dried over anhydrous sodium sulfate, and concentrated to dryness by rotary evaporation. The residue was purified by silica gel column chromatography to give compound 4 as a white solid (1.63 g, 76%). m/z (ES+), [M]+ = 562; acid, HPLC tR = 1.712 min.

### Synthesis of compound 5

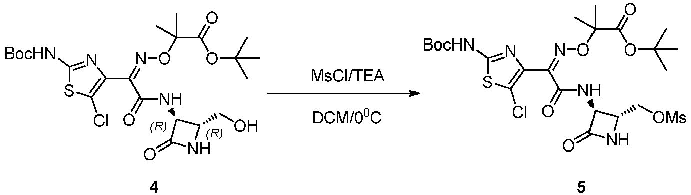

Triethylamine (0.6 g, 6 mmol) was added dropwise at 0 °C (N₂) to compound 4 (1.12 g, 2 mmol) in dichloromethane (15 mL), and MSCl (343 mg, 3 mmol) was added dropwise at 0 °C to the reaction mixture. The reaction mixture was allowed to react at 0 °C for 30 min. Ice water (15 mL) was added to the reaction mixture. The organic phase was separated, washed with saturated brine, dried over anhydrous sodium sulfate, and concentrated to dryness by rotary evaporation to give compound 5 as a brown solid. m/z (ES+), [M]+ = 640; acid, HPLC tR = 1.843 min.

### Synthesis of compound 6

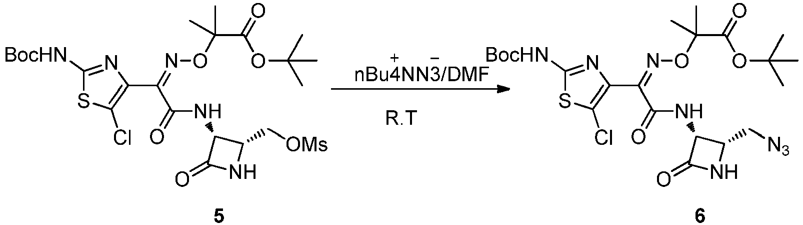

TBAA (1.7 g, 6 mmol) was added to compound 5 (1.3 g, 2 mmol) in *N,N-*dimethylformamide (15 mL). The reaction mixture was stirred at 62 °C under N2 overnight and quenched with a cold 4% solution of NaHCO₃ (200 mL). The aqueous phase was extracted with ethyl acetate (150 mL × 2), and the organic phase was washed with NaCl, dried over anhydrous sodium sulfate, and concentrated to dryness by rotary evaporation. The residue was purified by silica gel column chromatography to give compound 6 as a pale brown solid (561 mg, 48%). m/z (ES+), [M]+ = 587; acid, HPLC tR = 1.895 min.

### Synthesis of compound 7

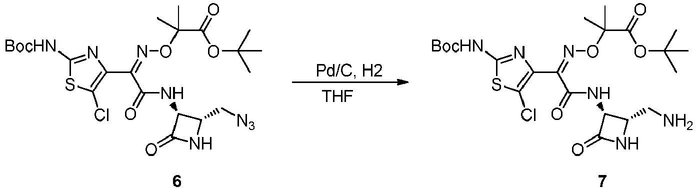

Pd/C (60 mg, 0.566 mmol) was added to compound 6 (561 mg, 0.96 mmol) in tetrahydrofuran (20 mL). The reaction mixture was allowed to react under H2 at room temperature for 2 h. Pd/C was filtered out, and the filtrate was concentrated to dryness by rotary evaporation to give compound 7 as a white solid (480 mg, 89.6%). m/z (ES+), [M]+ = 561; acid, HPLC tR = 1.514 min.

### Synthesis of compound 8

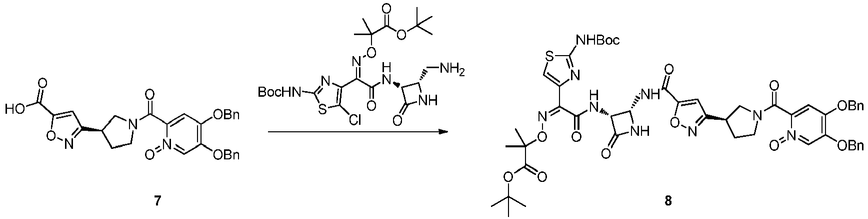

Compound 7 (400 mg, 0.776 mmol) and amine (430 mg, 0.767 mmol) were added to tetrahydrofuran (1.6 mL) and N,N-dimethylformamide (1 mL), and DMTMM (340 mg, 1.15 mmol) was added to the reaction system, which was then stirred at room temperature under nitrogen for 2 h and purified by reversed-phase chromatography to give compound 8 as a white solid (368 mg, 45.3%). m/z (ES+), [M+Na+H]+ = 1058; acid, HPLC tR = 1.974 min.

### Synthesis of compound 9

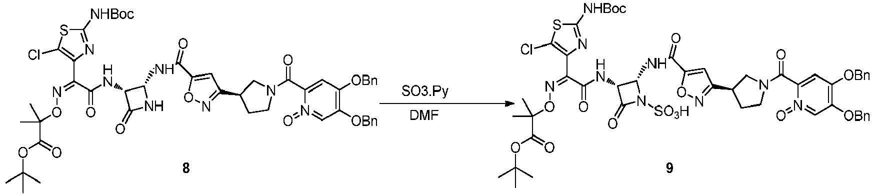

Sulfur trioxide pyridine complex (500 mg, 3.16 mmol) was added to a solution of compound 7 (368 mg, 0.348 mmol, 1.00 equiv) in *N,N*-dimethylformamide (3 mL). The reaction mixture was stirred at 45 °C under nitrogen for 4 h and purified by reversed-phase chromatography to give compound 8 as a white solid. m/z (ES+), [M]+ = 1138; acid, HPLC tR = 1.900 min.

### Synthesis of compound 1-26

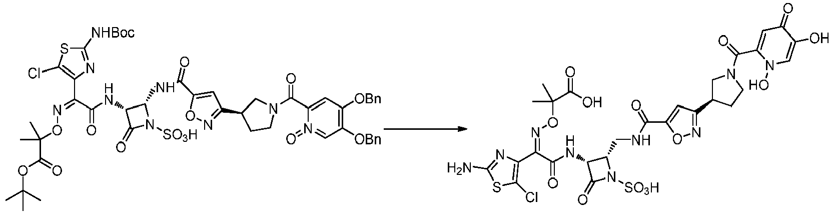

A 1 mol/L solution of boron trichloride (0.5 mL) in dichloromethane was added dropwise at 0 °C under nitrogen to compound 9 (50 mg, 0.044 mmol) in dichloromethane (2.5 mL). The reaction mixture was allowed to react at 0 °C for 1 h and blow-dried to remove the solvent. The residue was washed with diethyl ether by centrifugation, and the resulting solid was purified by preparative chromatography (C18 column: Atlantis preparative T3 OBD column, 19 × 250 mm 10 u; mobile phase A: water (0.1% formic acid); mobile phase B: ACN; flowrate: 25 mL/min; gradient: from 25 B to 35 B over 7 min; 254/220 nm) to give product 1-26 as a while solid (3.8 mg). m/z (ES+), [M]+ = 802; acid, HPLC tR = 0.676 min. ¹H NMR (D₂O, 400 MHz): δ (ppm) 1.50 (s, 6H), 2.11 (ddd, J = 20.9, 13.0, 7.3 Hz, 1H), 2.39 (ddd, J = 25.0, 12.1, 5.8 Hz, 1H), 3.43 (dq, *J* = 18.5, 6.1 Hz, 2H), 3.55-3.80 (m, 4H), 3.80-4.08 (m, 1H), 4.52 (t, *J* = 6.0 Hz, 1H), 5.29 (t, *J* = 5.5 Hz, 1H), 6.80-7.06 (m, 2H), 7.87 (d, *J* = 2.1 Hz, 1H).

### Example 27. Synthesis of Compound 1-27

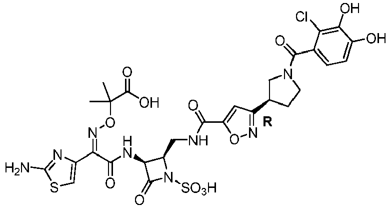

### Synthesis of compound 1:

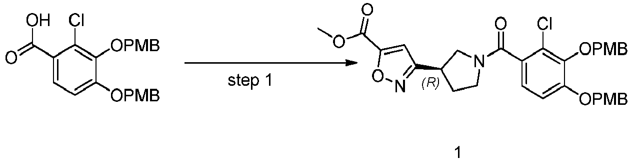

To a solution of 2-chloro-3,4-bis((4-methoxybenzyl)oxy)benzoic acid (1 g, 2.33 mmol) and methyl (*R*)-3-(pyrrolidin-3-yl)isoxazole-5-carboxylate hydrochloride (541 mg, 2.33 mmol) in tetrahydrofuran (50 mL) were added 1-hydroxybenzotriazole (630 mg, 4.66 mmol), triethylamine (940 mg, 9.32 mmol) and 1-ethyl-(3-dimethylaminopropyl)carbodiimide hydrochloride (895 mg, 4.66 mol). The reaction mixture was stirred at room temperature for 3 h. The reaction system was diluted with water and ethyl acetate. The organic layer was separated, and the aqueous layer was extracted with ethyl acetate. The extracts were combined, washed with brine, dried over anhydrous magnesium sulfate, and filtered. The filtrate was concentrated *in vacuo.* The crude product was purified by column chromatography to give compound 1 as a yellow oil (720 mg, 52%). m/z (ES+), [M]+ = 607.

### Synthesis of compound 2:

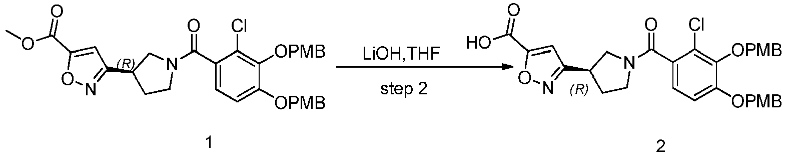

To a solution of compound 1 (440 mg, 0.73 mmol) in methanol (6 mL) was added an aqueous solution of lithium hydroxide (4 mL, 2 mol/L). The reaction mixture was stirred at room temperature for 2 h. The reaction system was concentrated to remove methanol and acidified to pH 2.5 with a solution of hydrochloric acid (1 mol/L), and the aqueous phase was extracted with ethyl acetate. The organic phases were combined and concentrated to give compound 2 as a white solid (650 mg, 92.4%). m/z (ES+), [M]+ = 593.

### Synthesis of compound 3:

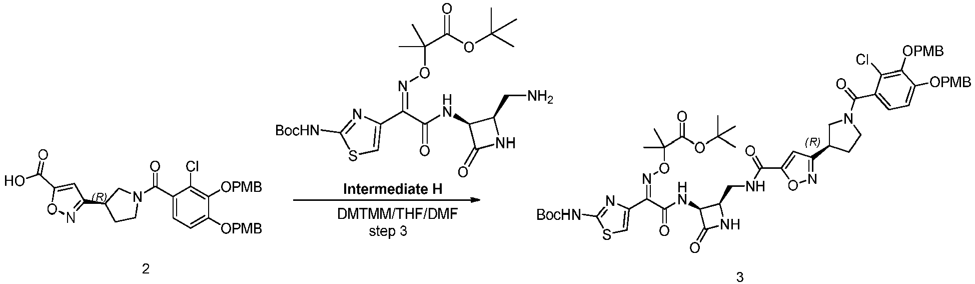

To a 10 mL round-bottom flask was added a solution of compound 2 (323 mg, 0.54 mmol) and compound **H** (286 mg, 0.54 mmol) in tetrahydrofuran/*N,N*-dimethylformamide (1.6 mL/1 mL). 4-(4,6-dimethoxytriazin-2-yl)-4-methylmorpholinium hydrochloride (240 mg, 0.81 mmol) was added under nitrogen. The reaction mixture was stirred at room temperature for 2 h. After the reaction was completed, the crude product was purified by column chromatography to give compound 3 as a white solid (350 mg, 53.8%). m/z (ES⁺), [M]⁺ = 1101.

### Synthesis of compound 4:

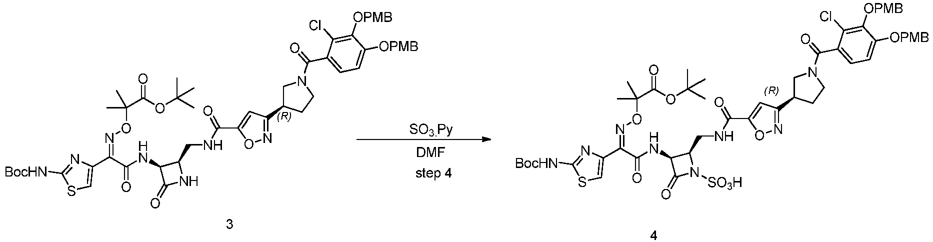

To a solution of compound 3 (350 mg, 0.318 mmol) in *N,N*-dimethylformamide (3 mL) was added sulfur trioxide pyridine complex (350 mg, 2.21 mmol). The reaction system was stirred at 45 °C for 3 h. The reaction mixture was purified by reversed-phase column chromatography to give compound **4** (270 mg, 72%).

m/z (ES+), [M]⁺ = 1181.

### Synthesis of compound 1-27:

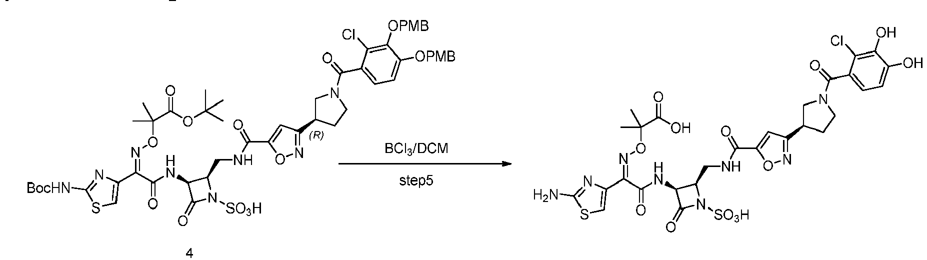

To a solution of compound 4 (780 mg, 0.66 mmol) in dichloromethane was added a solution of boron trichloride (0.7 mL). The reaction mixture was stirred at 0 °C under nitrogen for 4 h and blow-dried with nitrogen to remove the solvent. The solid residue was washed 3 times with diethyl ether and centrifuged. The crude product was purified by reversed-phase column chromatography to give compound **1-27** as an off-white solid (149 mg). m/z (ES⁺), [M-H]⁺ = 783.

¹H NMR (400 MHz, DMSO-*d*₆) δ 6.92 (d, J = 31.5 Hz, 1H), 6.84-6.71 (m, 2H), 6.61 (dd, J = 8.2, 6.0 Hz, 1H), 5.17 (t, J = 5.5 Hz, 1H), 4.20-4.10 (m, 1H), 3.84-3.39 (m, 5H), 3.34-3.10 (m, 2H), 2.01 (d, J = 16.8 Hz, 1H), 1.39 (dd, J = 6.5, 4.9 Hz, 6H), 1.12-1.02 (m, 1H).

### Example 28. Synthesis of Compound 1-28

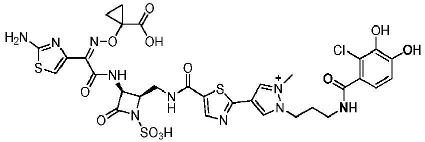

### Synthesis of compound 1:

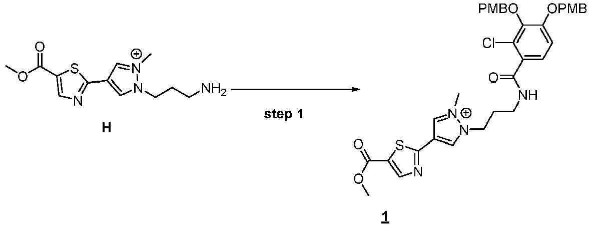

Compound H (1 g, 3.56 mmol) was dissolved in tetrahydrofuran (30 mL), and 1-(3-dimethylaminopropyl)-3-ethylcarbodiimide hydrochloride (1.367 g, 7.11 mmol), 1-hydroxybenzotriazole (0.961 g, 7.11 mmol) and triethylamine (1.438 g, 14.23 mmol) were added, followed by the addition of 2-chloro-3,4-bis(4-methoxybenzyl)benzoic acid (1.523 g, 3.56 mmol). The reaction mixture was stirred at room temperature for 2 h and purified by reversed-phase column chromatography to give compound 1 as a white solid (600 mg, 24.4%). [M-H]+ = 691.

### Synthesis of compound 2:

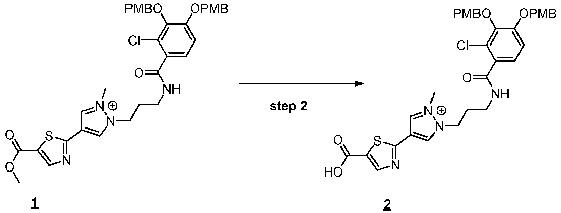

Compound 1 (200 mg, 0.289 mmol) was dissolved in an aqueous solution of tetrahydrofuran (10 mL), and lithium hydroxide (13.9 mg, 0.579 mmol) was added at 0 °C. The reaction mixture was stirred at room temperature for 1 h and concentrated by rotary evaporation to remove tetrahydrofuran. The residue was lyophilized to give compound 2 as a white solid (160 mg, crude), which was directly used in the next step without purification. m/z (ES⁺), [M-H]⁺ = 677.

### Synthesis of compound 3:

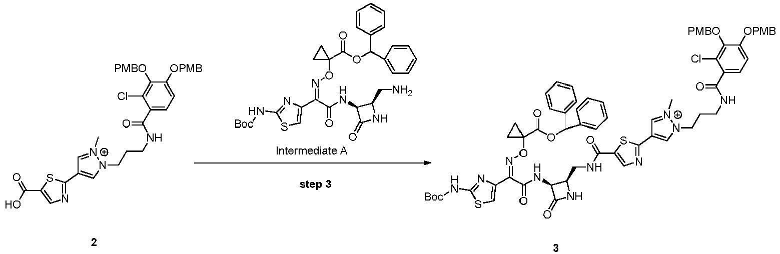

Compound 2 (160 mg, 0.263 mmol), reactant A (180 mg, 0.284 mmol) and 4-(4,6-dimethoxytriazin-2-yl)-4-methylmorpholinium hydrochloride (139 mg, 0.473 mmol) were dissolved at room temperature in a solution of tetrahydrofuran/*N,N*-dimethylformamide (3.2 mL : 2 mL). The reaction mixture was stirred at room temperature for 2 h and purified by reversed-phase column chromatography to give compound 3 as a white solid (40 mg, 13.1%). m/z (ES⁺), [M+H]⁺ = 1294.

### Synthesis of compound 4:

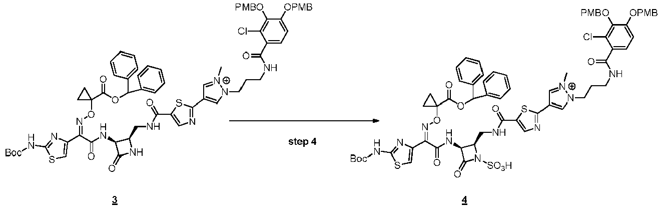

Sulfur trioxide pyridine complex (71 mg, 0.39 mmol) was added to a solution of compound 3 (50 mg, 0.039 mmol) in dry *N,N*-dimethylformamide (3 mL). The reaction system was stirred at room temperature for 20 h, and the crude product was purified by reversed-phase column chromatography to give compound 4 as a pale yellow solid (30 mg, 56.5%). m/z (ES⁺), [M]⁺ = 1374.

### Synthesis of compound 1-28:

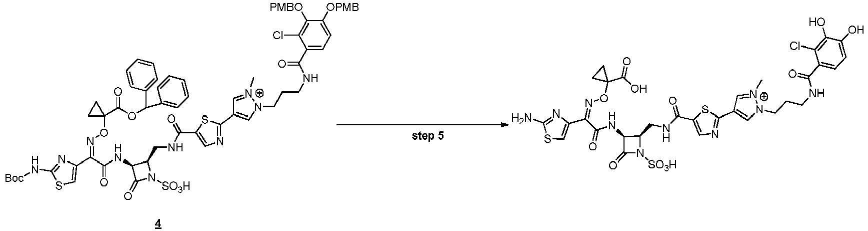

Compound 4 (30 mg, 0.022 mmol) was dissolved in dichloromethane (2 mL) in a 10 mL round-bottom flask, and a solution of boron trichloride (0.22 mL) was added dropwise at 0 °C. The reaction system was stirred at 0 °C for 4 h. After the reaction was completed, the solvent was removed by blow-drying with nitrogen. Diethyl ether was added to the residue, and the mixture was centrifuged to give a solid crude product, which was then purified by reversed-phase column chromatography to give compound **1-28** as a white solid (3.8 mg, 20.1%). m/z (ES⁺), [M+H]⁺ = 869. ¹H NMR (400 MHz, DMSO-*d*₆) δ 9.21 (d, *J* = 26.6 Hz, 2H), 8.23 (s, 1H), 6.86 (s, 1H), 6.80-6.73 (m, 2H), 5.21 (d, *J* = 5.6 Hz, 1H), 4.54 (t, *J* = 6.9 Hz, 2H), 4.17 (s, 4H), 3.30 (t, *J* = 6.4 Hz, 2H), 2.16 (t, *J* = 6.8 Hz, 1H), 2.16 (t, *J* = 6.8 Hz, 1H), 2.16 (t, *J* = 6.8 Hz, 2H), 1.32 (d, *J* = 11.5 Hz, 4H).

### Example 29. Synthesis of Compound 1-29

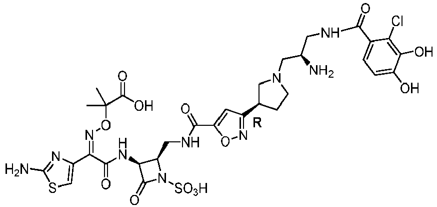

### Synthesis of compound 1

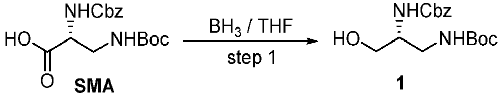

Starting material A (20 g, 0.06 mol) was dissolved in tetrahydrofuran (50 mL), and a solution of borane-tetrahydrofuran (300 mL) was slowly added dropwise in an ice bath. The reaction mixture was stirred in the ice bath for 4 h and slowly added dropwise to ice water for quenching. The mixture was extracted 3 times with ethyl acetate, and the organic phases were collected, washed 3 times with saturated brine, dried over anhydrous sodium sulfate, and concentrated. The residue was purified by silica gel column chromatography to give compound 1 as an off-white solid (9.4 g, 49% yield). m/z (ES+), [M+Na]+ = 347, acid, HPLC RT = 1.156 min. ¹H NMR (400 MHz, chloroform-d) δ 1.44 (s, 9H), 3.14-3.41 (m, 2H), 3.41-3.79 (m, 4H), 4.94 (s, 1H), 5.09 (s, 2H), 5.37 (d, J = 8.3 Hz, 1H), 7.28-7.39 (m, 5H).

### Synthesis of compound 2

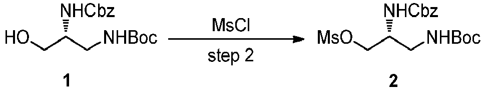

Compound 1 (2 g, 6.2 mmol) and triethylamine (1.25 g, 12.4 mmol) were dissolved in dichloromethane (20 mL), and methanesulfonyl chloride (1.07 g, 9.3 mmol) was slowly added dropwise in an ice bath. The reaction mixture was stirred in the ice bath for 1 h, quenched with water, and extracted 3 times with dichloromethane. The organic phases were collected, washed 3 times with saturated brine, dried over anhydrous sodium sulfate, and concentrated to dryness by rotary evaporation to give compound 2 as a yellow solid crude product (2.33 g), which was directly used in the next step without purification. m/z (ES+), [M+Na]+ = 425, acid, HPLC RT = 1.242 min.

### Synthesis of compound 3

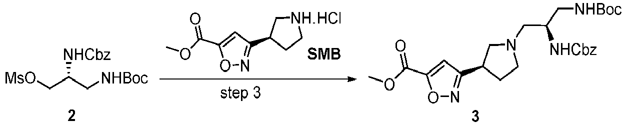

Starting material B (500 mg, 2.15 mmol) and triethylamine (1.3 g, 12.9 mmol) were dissolved in acetonitrile (10 mL). After the solution was stirred for 20 min, compound 2 (1.73 g, 4.3 mmol) was added slowly. The reaction mixture was stirred at 60 °C overnight and concentrated. The residue was purified by silica gel thin layer chromatography to give compound 3 as a yellow oil (360 mg, 33.3% yield). m/z (ES+), [M+H]+ = 503, acid, HPLC RT = 1.522 min.

### Synthesis of compound 4

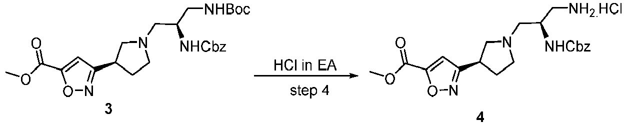

Compound 3 (360 mg, 0.72 mmol) was dissolved in ethyl acetate (3 mL), and a solution of hydrogen chloride in ethyl acetate (4 M, 20 mL) was added slowly. The reaction mixture was stirred at room temperature for 2 h and concentrated, and the residue was lyophilized to give compound 4 as a yellow solid crude product (400 mg), which was directly used in the next step without purification. m/z (ES+), [M+H]+ = 403, acid, HPLC RT = 0.875 min.

### Synthesis of compound 5

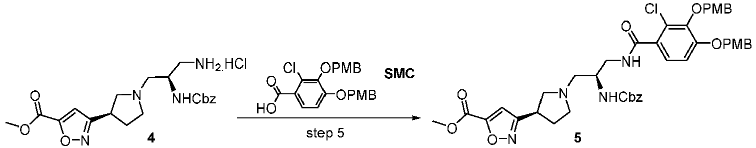

Starting material C (373 mg, 0.87 mmol), HATU (661 mg, 1.74 mmol) and *N,N-*diisopropylethylamine (449 mg, 3.48 mmol) were dissolved in DMF (5 mL), and then compound 4 (380 mg, 0.87 mmol) was added slowly. The reaction mixture was stirred at room temperature for 3 h, quenched with water, and extracted 3 times with ethyl acetate. The organic phases were collected, washed with saturated brine, dried over anhydrous sodium sulfate, and concentrated. The residue was purified through a C18 column and lyophilized to give compound 5 as a pale yellow solid (320 mg, 45.3% yield, 100% ee value). m/z (ES+), [M]+=813, acid, HPLC RT = 2.011 min. ¹H NMR (400 MHz, chloroform-d) δ 1.25 (s, 1H), 1.93 (s, 1H), 2.31 (s, 1H), 2.71 (s, 4H), 3.00 (s, 1H), 3.55 (s, 2H), 3.80 (s, 4H), 3.83 (s, 3H), 3.94 (s, 4H), 4.94 (s, 2H), 5.04-5.17 (m, 4H), 6.79-6.87 (m, 3H), 6.92 (dd, J = 2.0, 8.7 Hz, 3H), 7.28-7.41 (m, 10H).

### Synthesis of compound 6

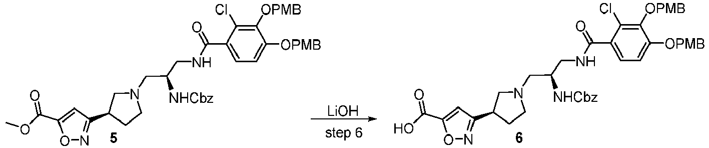

Compound 5 (300 mg, 0.37 mmol) was dissolved in methanol/tetrahydrofuran (5 mL/5 mL), and a 0.5 M aqueous solution of lithium hydroxide (3 mL, 1.48 mmol) was slowly added dropwise. The reaction mixture was stirred at room temperature for 3 h and concentrated, and the residue was dissolved in water. The solution was adjusted to pH 3-4 with 1 M hydrochloric acid and lyophilized to give compound 6 as a white solid crude product (270 mg), which was directly used in the next step without purification. m/z (ES+), [M]+ = 799, acid, HPLC RT = 1.753 min.

### Synthesis of compound 7

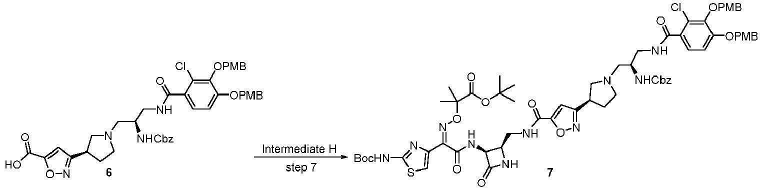

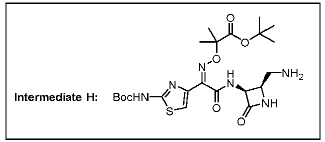

Compound 6 (250 mg, 0.31 mmol) was dissolved in *N,N-*dimethylformamide/tetrahydrofuran (2 mL/3 mL), and DMTMM (274 mg, 0.93 mmol) was added slowly. The reaction mixture was stirred at room temperature for 2 h, purified through a C18 column, and lyophilized to give compound 7 as a white solid (190 mg, 46.4% yield). m/z (ES+), [M]+ = 1307, acid, HPLC RT = 1.946 min.

### Synthesis of compound 8

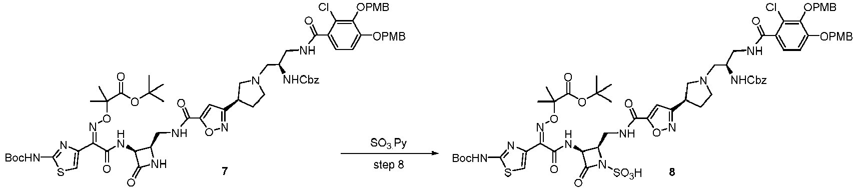

Compound 7 (180 mg, 0.14 mmol) was dissolved in *N,N*-dimethylformamide (3 mL), and sulfur trioxide pyridine complex (442 mg, 2.8 mmol) was slowly added. The reaction mixture was stirred at 45 °C for 3 h, purified through a C18 column, and lyophilized to give compound 8 as a yellow solid (145 mg, 75.9% yield). m/z (ES+), [M]+ = 1387, acid, HPLC RT = 3.419 min.

### Synthesis of compound 1-29

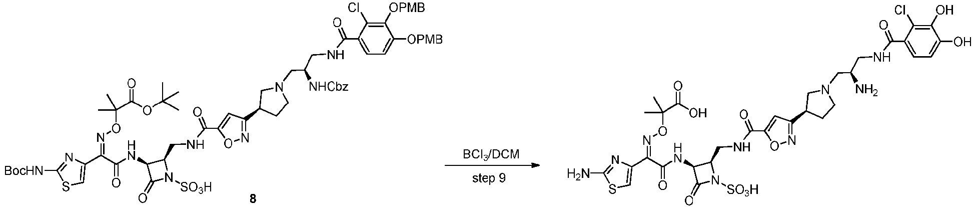

Compound 8 (65 mg, 0.047 mmol) was dissolved in dichloromethane (2 mL), and the reaction flask was purged 3 times with nitrogen. A solution of boron trichloride in dichloromethane (1 M, 0.5 mL) was slowly added dropwise in an ice bath under nitrogen. The reaction mixture was stirred in an ice bath for 2 h and blow-dried with nitrogen. The residue was washed three times with diethyl ether by centrifugation. The resulting crude product was lyophilized and purified by preparative high-pressure liquid chromatography to give compound **1-29** as a light gray solid (3.1 mg, 7.7% yield). m/z (ES+), [M]+ = 857, acid, HPLC RT = 0.928 min. ¹H NMR (400 MHz, DMSO-*d*₆) δ 1.37 (d, J = 6.0 Hz, 6H), 1.74 (s, 1H), 2.16-2.30 (m, 1H), 2.55-2.65 (m, 2H), 2.73 (t, J = 11.4 Hz, 1H), 2.86 (d, J = 27.6 Hz, 2H), 3.39 (d, J = 25.0 Hz, 5H), 3.61 (s, 2H), 4.15 (s, 1H), 5.15 (d, J = 5.6 Hz, 1H), 6.71-6.81 (m, 2H), 6.85 (d, J = 8.3 Hz, 1H), 6.93 (s, 1H).

### Example 30. Synthesis of Compound 1-30

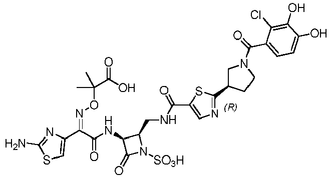

### Synthesis of compound 1

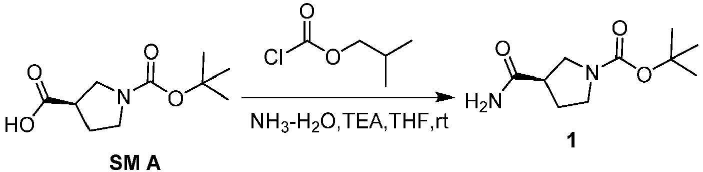

Starting material A (5 g, 23.23 mmol) was dissolved in tetrahydrofuran (100 mL), and triethylamine (4.69 g,46.46 mmol) and isobutyl chloroformate (3.8 g, 27.87 mmol) were successively added slowly in an ice bath. The reaction mixture was stirred in the ice bath for 20 min. Then ammonia solution was added, and finally, the reaction mixture was stirred at room temperature for 2 h. After the reaction was completed, the reaction mixture was diluted with ethyl acetate (30 mL), then washed twice with a saturated solution of citric acid (2 × 20 mL), and then washed twice with a saturated solution of sodium carbonate (2 × 20 mL). Finally, the organic phase was dried over anhydrous sodium sulfate and concentrated to dryness by rotary evaporation to give a crude product as a yellow oil (2.39 g, 49%). MS (ESI): m/z = 214.

### Synthesis of compound 2

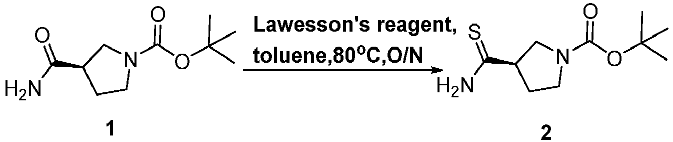

Compound 1 (0.76 g, 3.55 mmol) was dissolved in dry toluene (20 mL), and Lawesson's reagent (0.71 g, 1.77 mmol) was added at room temperature. The reaction mixture was stirred at 80 °C under nitrogen for 16 h overnight. After the reaction was completed, the reaction mixture was diluted with ethyl acetate (30 mL), and then washed twice with a saturated solution of sodium carbonate (2 × 20 mL) and twice with saturated brine (2 × 20 mL). Finally, the organic phase was dried over anhydrous sodium sulfate and concentrated to dryness by rotary evaporation. The resulting crude product was purified by silica gel column chromatography (petroleum ether/ethyl acetate = 6:1) to give the final product as brown oil (230 mg, 28%). MS (ESI): *m*/*z* = 231.

### Synthesis of compound 3

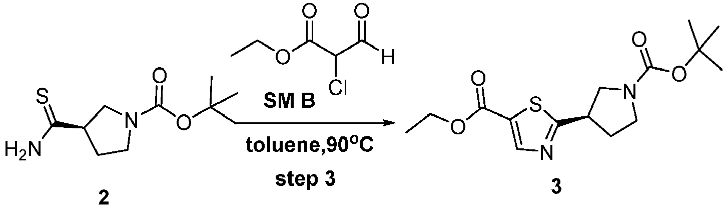

Compound 2 (360 mg, 0.1 mmol) and starting material B (150 mg, 0.1 mmol) were dissolved in toluene (10 mL). The reaction mixture was stirred at 90 °C for 16 h. After the reaction was completed, the reaction mixture was diluted with ethyl acetate (30 mL) and then washed twice with water (2 × 20 mL) and twice with saturated brine (2 × 20 mL). Finally, the organic phase was dried over anhydrous sodium sulfate and concentrated to dryness by rotary evaporation. The crude product was purified by normal-phase column chromatography to give the final product as a brown oil (100 mg, 20%). MS (ESI): *m*/*z* = 326.

### Synthesis of compound 4

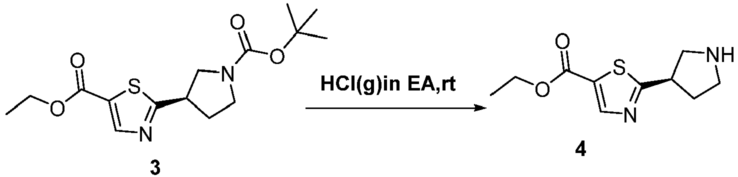

Compound 3 (326 mg, 1.0 mmol) was dissolved in ethyl acetate (10.0 mL), and then hydrogen chloride (2.0 mL, 4.0 M/L) was added in an ice bath. Then the reaction mixture was stirred at room temperature for 2 h. After the reaction was completed, the organic phase was concentrated to give crude product 4 (200 mg, 88%). MS (ESI): *m*/*z* = 227.

### Synthesis of compound 5

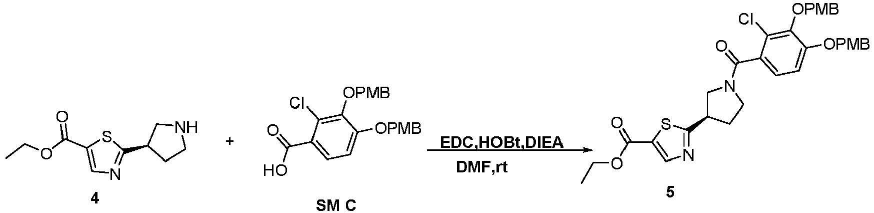

Compound C (428 mg,1 mmol) and compound 4 (226 mg,1 mmol) were dissolved in DMF (5 mL), and HOBt (275 mg, 2 mmol), DIEA (516 mg, 4 mmol) and EDC·HCl (384 mg, 2 mmol) were added successively. The reaction mixture was allowed to react at room temperature for 3 h. After the reaction was completed, the reaction mixture was diluted successively with water and ethyl acetate and extracted with ethyl acetate. The organic phases were combined, washed with saturated brine, dried over anhydrous magnesium sulfate, and finally concentrated to dryness. The resulting crude product was purified by silica gel column chromatography to give the final product as a yellow solid (426 mg, 67%). m/z (ES+), [M]+ = 637.

### Synthesis of compound 6

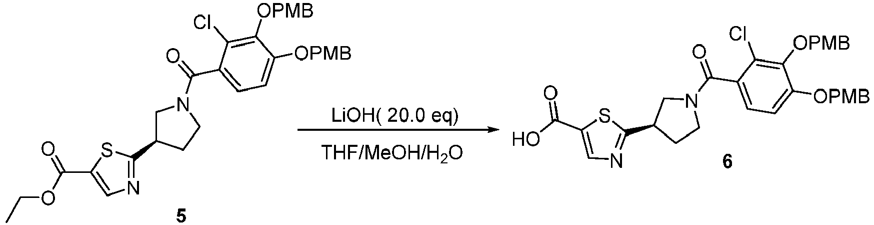

Compound 5 (318 mg, 0.5 mmol) was dissolved in methanol (5.0 mL) and tetrahydrofuran (5.0 mL), and then an aqueous solution of lithium hydroxide (10 mL, 2 mol/L) was added. The reaction mixture was stirred at room temperature for 2 h. After the reaction was completed, methanol and tetrahydrofuran were removed by rotary evaporation, and the remaining aqueous solution was adjusted to pH 2.5 with hydrochloric acid (1 mol/L) and then extracted with ethyl acetate. The organic phase was concentrated to dryness by rotary evaporation to give final product compound **6** as a white solid (295 mg, 97%). m/z (ES+), [M]+ = 609.

### Synthesis of compound 7

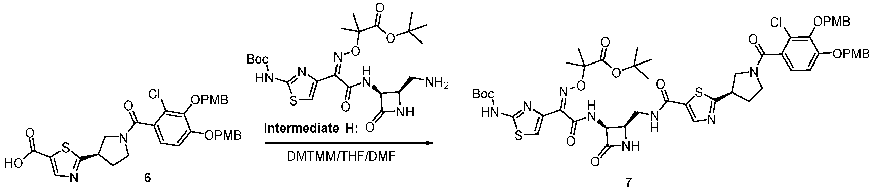

Compound **6** (267 mg, 0.44 mmol) and compound **H** (231 mg, 0.44 mmol) were dissolved in tetrahydrofuran (1.6 mL) and N,N-dimethylformamide (1 mL), and DMTMM (200 mg, 0.66 mmol) was added slowly. The reaction mixture was stirred under nitrogen at room temperature for 2 h. After the reaction was completed, the crude product was purified by reversed-phase column chromatography to give the final product as a white solid (270 mg, 55%). m/z (ES+), [M]+ = 1117.

### Synthesis of compound 8

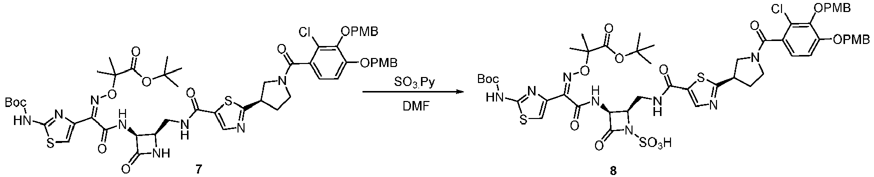

Compound **7** (265 mg, 0.238 mmol, 1.0 eq) was dissolved in *N,N*-dimethylformamide (3 mL), and sulfur trioxide pyridine complex (300 mg, 1.89 mmol, 8.0 eq) was slowly added. Then the reaction mixture was stirred at 45 °C for 3 h. After the reaction was completed, the crude product was purified by reversed-phase column chromatography to give final product 8 as a white solid (185 mg, 69%). m/z (ES+), [M]+ = 1197.

### Synthesis of compound 1-30

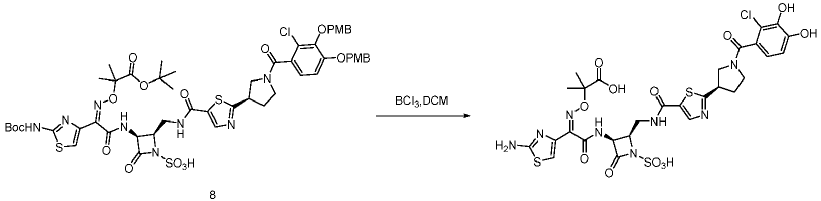

Compound 8 (20 mg, 0.017 mmol, 1.00 eq) was dissolved in dichloromethane (2 mL), and the reaction flask was purged 3 times with nitrogen. A solution of boron trichloride in dichloromethane (0.63 mL, 0.63 mmol) was slowly added dropwise in an ice bath under nitrogen. The reaction mixture was stirred in an ice bath for 2 h and blow-dried with nitrogen. The residue was washed three times with diethyl ether by centrifugation. The resulting crude product was lyophilized and purified by preparative high-pressure liquid chromatography to give compound **1-30** as a light gray solid (4.1 mg). m/z (ES+), [M]+ = 801. ¹H NMR (400 MHz, DMSO-*d*₆) δ 8.05 (d, *J* = 21.2 Hz, 1H), 6.95-6.72 (m, 2H), 6.63 (dd, *J* = 8.1, 4.7 Hz, 1H), 5.19 (t, *J* =5.3 Hz, 1H), 4.25-4.11 (m, 1H), 385-3.90(m, 1H), 3.63 (d, *J* = 6.2 Hz, 2H), 3.58-3.44 (m, 2H), 3.36 (dd, J = 10.6, 6.4 Hz, 1H), 3.25 (t, J = 7.0 Hz, 1H), 2.40 (dd, J = 15.3, 8.9 Hz, 1H), 2.24-2.04 (m, 1H), 1.42 (dd, *J* = 9.6, 4.1 Hz, 6H).

### Example 31. Synthesis of Compound 1-31

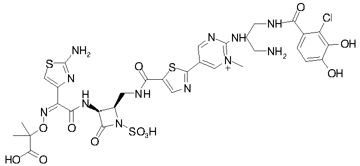

### Synthesis of compound 1:

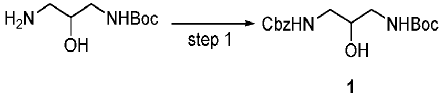

To a solution of *tert-butyl* (3-amino-2-hydroxypropyl)carbamate (5 g, 26.3 mmol, 1 eq) in dichloromethane (100 mL) were added triethylamine (5.3 g, 52.6 mmol, 2 eq) and Cbz-Cl (5.36 g, 31.5 mmol, 1.2 eq). The mixture was stirred at room temperature for 3 h. The resulting residue was diluted with water and ethyl acetate. The organic layer was separated, and the extracts were combined, washed with brine, dried over anhydrous magnesium sulfate, and filtered. The filtrate was concentrated *in vacuo.* The resulting residue was purified by silica gel chromatography to obtain compound **1** as a colorless oil (6.2 g, 72.9%). m/z (ES+), [M+H]+ = 325; acid, HPLC tR = 0.87 min.

### Synthesis of compound 2:

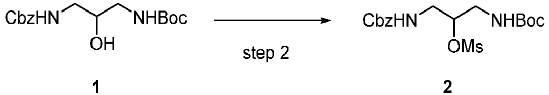

To a solution of compound **1** (6.2 g, 19 mmol, 1 eq) in DCM (100 mL) was added at 0 °C TEA (2.99 g, 29.6 mol, 1.5 eq), and methanesulfonyl chloride (3.39 g, 29.6 mol, 1.5 eq) was added dropwise. The resulting mixture was stirred at room temperature for 2 h and washed successively with hydrochloric acid solution (1 M) and sodium bicarbonate (aqueous, 5%). The organic phase was dried over anhydrous magnesium sulfate, and filtered. The filtrate was concentrated *in vacuo* to give compound **2** as a colorless oil (6.2 g, 81%). m/z (ES+), [M+Na]+ = 425; acid, HPLC tR = 1.229 min.

### Synthesis of compound 3:

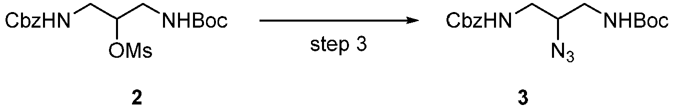

To a solution of compound 2 (6.2 g, 16 mmol, 1 eq) in DMF (50 mL) was added sodium azide (2.1 g, 30 mmol, 2 eq). The resulting mixture was stirred at 70 °C for 4 h. Ice water was added to the resulting mixture, which was then extracted with ethyl acetate. The extracts were combined, washed with brine, dried over anhydrous Na₂SO₄, and filtered. The filtrate was concentrated *in vacuo* to give compound **3** as a colorless oil (6.1 g, crude). m/z (ES+), [M+Na]+ = 372; acid, HPLC tR = 1.301 min.

### Synthesis of compound 4:

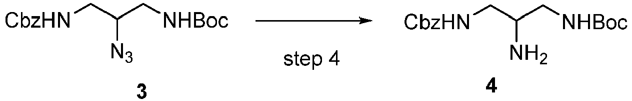

To a solution of compound 3 (6.1 g, 17.4 mmol, 1 eq) in MeOH (50 mL) were added triphenylphosphane (6.86 g, 26.2 mol, 1.5 eq) and H₂O (15 mL). The resulting mixture was stirred at 70 °C for 4 h. The solution was evaporated, and the resulting mixture was washed 3 times with petroleum ether to give compound **4** (7 g, crude). m/z (ES+), [M+H]+ = 324; acid, HPLC tR = 1.599 min.

### Synthesis of compound 5:

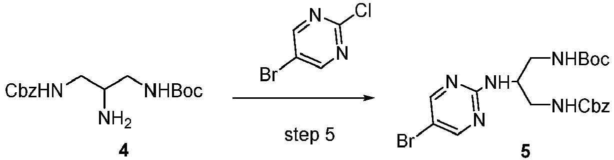

To a solution of compound **4** (7 g, crude) in DMF (50 mL) were added TEA (5.78 g, 57.3 mmol, 3 eq) and 5-bromo-2-chloropyrimidine (7.3 g, 38.2 mmol, 2 eq). The resulting mixture was stirred at 45 °C for 3 h. Ice water was added to the reaction mixture, which was then extracted with ethyl acetate. The organic phase was combined, washed with brine, dried over anhydrous magnesium sulfate, and filtered. The filtrate was concentrated *in vacuo,* and the resulting residue was purified by silica gel chromatography to give compound **5** as a brown oil (3 g, 37.7% overall yield for 3 steps). m/z (ES+), [M+H]+ = 481; acid, HPLC tR = 0.968 min.

### Synthesis of compound 6:

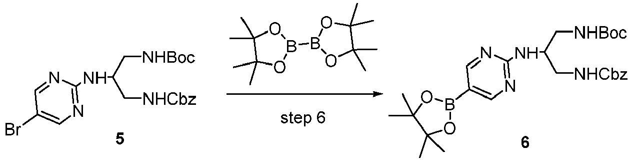

To a solution of compound 5 (3 g, 6.26 mmol) in 1,4-dioxane (50 mL) were added bis(pinacolato)diboron (3.18 g, 12.5 mmol, 2 eq), potassium acetate (1.22 g, 12.5 mmol, 2 eq) and Pd(dppf)Cl₂ (0.458 g, 6.26 mmol, 0.1 eq). The reaction system was purged with nitrogen and heated at 80 °C under nitrogen for 2 h. After the reaction was completed, the reaction mixture was filtered, and the filtrate was concentrated *in vacuo.* The resulting crude product was purified by silica gel column chromatography with ethyl acetate/petroleum ether to give compound 6 as a yellow oil (1.2 g, 36.37% yield). m/z (ES+), [M+H]+ = 528; base, HPLC tR = 1.055 min.

### Synthesis of compound 7:

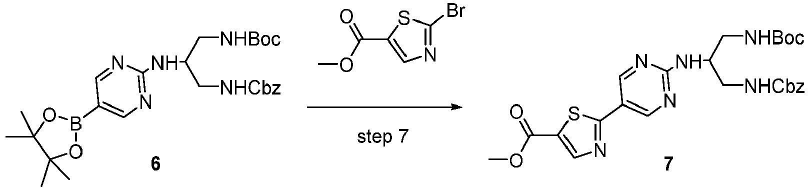

To a solution of compound 6 (0.4 g, 0.759 mmol) in 1,4-dioxane and water (5 mL : 0.5 mL) were added methyl 2-bromothiazole-5-carboxylate (0.218 g, 0.986 mmol, 1.3 eq), potassium phosphate (0.48 g, 2.27 mmol, 3 eq) and Pd(dppf)Cl₂ (0.111 g, 0.152 mmol, 0.2 eq). The reaction system was purged with nitrogen and heated at 80 °C under nitrogen for 3 h. After the reaction was completed, the reaction mixture was concentrated *in vacuo.* The resulting crude product was purified by silica gel column chromatography with ethyl acetate/petroleum ether to give compound 7 as a yellow oil (0.11 g, 26.8%). m/z (ES+), [M+H]+ = 543; acid, HPLC tR = 1.316 min.

### Synthesis of compound 8:

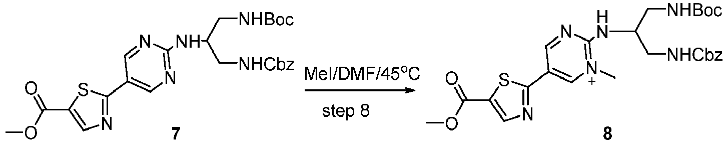

To a solution of compound 7 (0.3 g, 0.55 mmol, 1 eq) in DMF (1 mL) was added iodomethane (0.39 g, 2.76 mmol, 5 eq). The reaction system was heated at 45 °C for 3 h. After the reaction was completed, the reaction mixture was concentrated *in vacuo.* The resulting crude product was purified by reversed-phase chromatography using a C18 column to give compound **8** as a white solid (260 mg, 81%). m/z (ES+), [M]+ = 557; acid, HPLC tR = 0.774 min.

### Synthesis of compound 9:

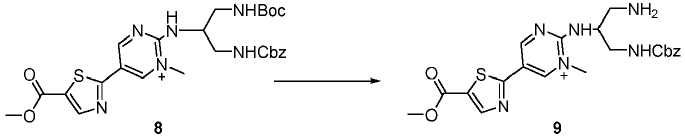

To a solution of compound **8** (0.26 g) in ethyl acetate (3 mL) was added at 0 °C a solution of HCl/EA (4 M) (3 mL). The reaction system was stirred at 0 °C for 3 h. After the reaction was completed, the system was diluted with petroleum ether, and a precipitate was generated. The mixture was filtered, and the filter cake was dried to give compound **9** as a yellow solid (180 mg, 84.5%). m/z (ES+), [M]+ = 457; acid, HPLC tR = 0.902 min.

### Synthesis of compound 10:

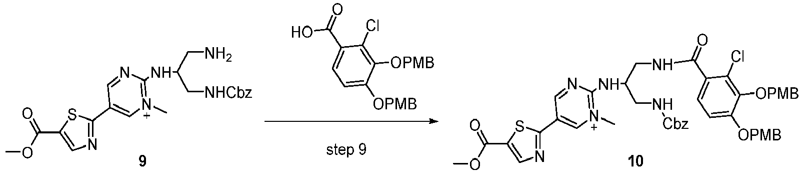

To a solution of 2-chloro-3,4-bis((4-methoxybenzyl)oxy)benzoic acid (0.185 g, 0.433 mmol, 1.1 eq) in DMF (2 mL) were added HATU (0.449 g, 1.18 mmol, 3 eq), DIEA (0.152 g, 1.18 mmol, 3 eq) and compound 9 (0.18 g, 0.393 mmol, 1 eq). The reaction system was stirred at room temperature for 2 h, After the reaction was completed, the mixture was poured into ice water and extracted with ethyl acetate. The organic phases were combined and concentrated, and the resulting crude product was purified by silica gel column chromatography with ethyl acetate/petroleum ether to give compound **10** as a yellow solid (300 mg, 88%). m/z (ES+), [M]+ = 868; acid, HPLC tR = 0.748 min.

### Synthesis of compound 11:

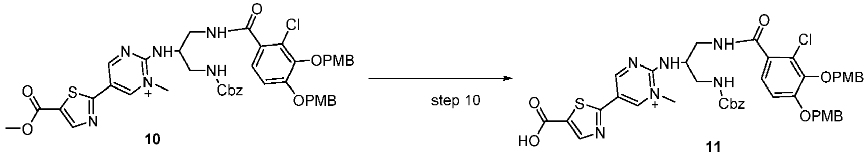

A solution of lithium hydroxide solid (0.039 g, 1.38 mmol, 4 eq) in water (0.6 mL) was added to a solution of compound 10 (300 mg, 0.346 mmol, 1 eq) in methanol (3 mL). The reaction system was stirred at room temperature for 2 h and concentrated by rotary evaporation to remove methanol. The residue was acidified to pH 3 with a solution of hydrochloric acid (1 mol/L). The system was extracted with ethyl acetate, and the organic phase was concentrated to give compound 11 as a yellow solid (200 mg, 67.7%). m/z (ES+), [M]+ = 854; acid, HPLC tR = 1.204 min.

### Synthesis of compound 12:

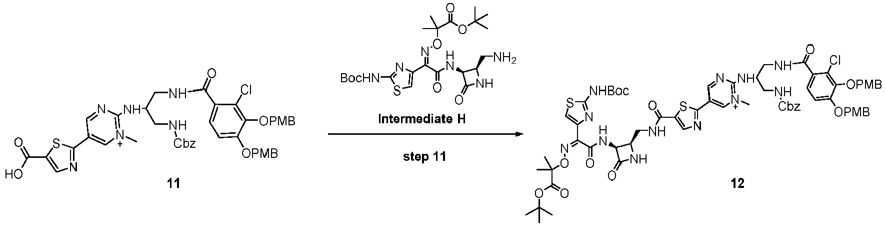

To a solution of compound **11** (220 mg, 0.257 mmol) in THF (1.6 mL) and DMF (1 mL) were added intermediate H (149 mg, 0.283 mmol, 1.1 eq) and DMTMM (114 mg, 0.386 mmol, 1.5 eq). The reaction mixture was stirred at room temperature for 2 h and purified by preparative HPLC to give compound **12** as a white solid (20 mg, 53.8%). m/z (ES+), [M]+ = 1362; acid, HPLC tR = 1.330 min.

### Synthesis of compound 13:

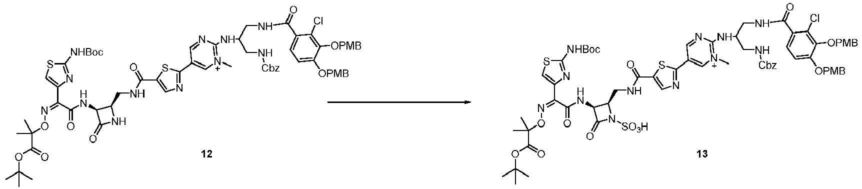

In a 10 mL round-bottom flask, sulfur trioxide pyridine complex (20 mg) was added to a solution of compound 12 (20 mg) in DMF (1 mL). The reaction system was stirred at 45 °C for 3 h. After the reaction was completed, the mixture was directly purified by preparative HPLC to give compound **13** as a white solid (12 mg, 56%). m/z (ES+), [M]+ = 1443; acid, HPLC tR = 1.415 min.

### Synthesis of compound 1-31:

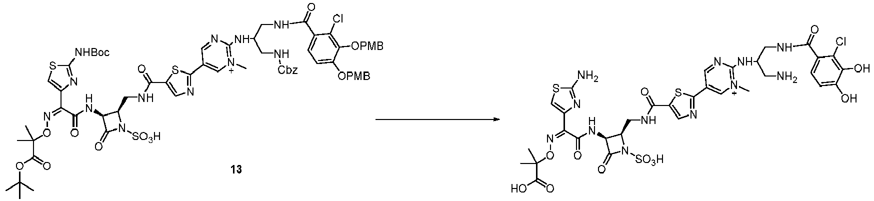

To a solution of compound 13 (12 mg) in dichloromethane (1 mL) was added at 0 °C a solution of boron trichloride (0.2 mL). The reaction system was stirred at 0 °C under nitrogen for 4 h. After the reaction was completed, the residual boron trichloride was removed under a stream of nitrogen. The crude product was purified by preparative HPLC (column: XSelect CSH preparative C18 OBD column, 19 × 250 mm, 5 µm; mobile phase A: water (0.1% FA); mobile phase B: CAN; flowrate: 25 mL/min; gradient: from 5 B to 30 B over 7 min; 254/220 nm) to give compound **1-31** as a white solid (2 mg, 26.4%). m/z (ES+), [M]+ = 912; ¹H NMR (400 MHz, DMSO-*d*₆): δ (ppm) 1.36 (t, *J* = 6.9 Hz, 6H), 3.23 (d, *J* = 35.4 Hz, 3H), 3.41-3.55 (m, 2H), 3.61 (s, 3H), 4.17-4.32 (m, 2H), 5.16 (d, *J* = 5.5 Hz, 1H), 6.66-6.81 (m, 3H), 7.97 (s, 1H), 8.19 (s, 1H), 8.27 (s, 3H).

### Example 32. Synthesis of Compound 1-32

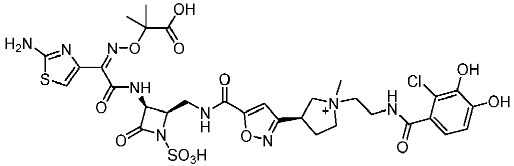

### The compound was synthesized by referring to Example 24.

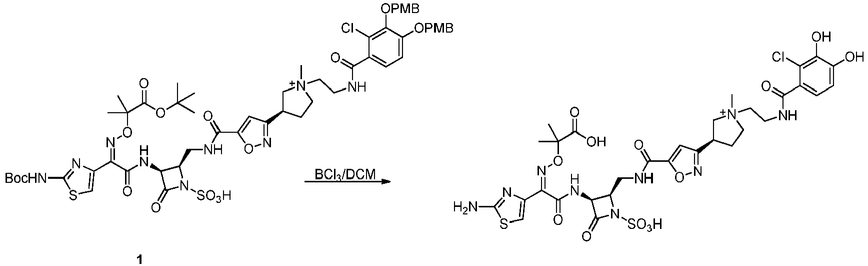

m/z (ES+), [M]+ = 843; acid, HPLC tR = 0.801 min. ¹H NMR (D₂O, 400 MHz): δ (ppm) 1.42 (d, *J* = 5.6 Hz, 6H), 2.49 (tt, *J* = 13.5, 7.6 Hz, 1H), 2.70-2.89 (m, 1H), 3.25 (d, *J* = 12.6 Hz, 3H), 3.66-3.96 (m, 9H), 3.99-4.24 (m, 2H), 4.57 (p, *J* = 6.2 Hz, 1H), 5.35 (dd, *J* = 5.7, 3.4 Hz, 1H), 6.76-6.97 (m, 2H), 6.94-7.04 (m, 2H).

### Example 33. Synthesis of Compound 1-33

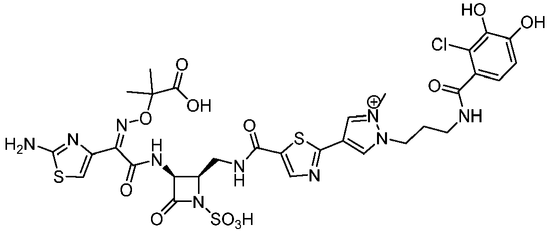

### Synthesis of compound 1

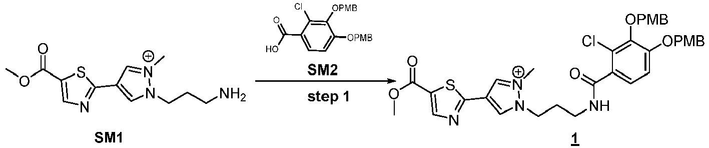

In a 50 mL single-necked flask, SM1 (1.2 g, 4.27 mmol, 1.00 equiv) and SM2 (1.83 g, 4.27 mmol, 1 equiv) were added to a solution of DIPEA (1.65 g, 12.81 mmol, 3.00 equiv) and HATU (2.6 g, 6.83 mmol, 1.6 equiv) in tetrahydrofuran (10 mL). The reaction mixture was stirred at room temperature for 2 h, quenched with water, extracted with ethyl acetate, and concentrated by rotary evaporation to remove the solvent. The residue was purified by reversed-phase column chromatography to give compound 1 as a crude product (700 mg). m/z (ES+), [M-H]+ = 691; acid, HPLC tR = 0.801 min.

### Synthesis of compound 2

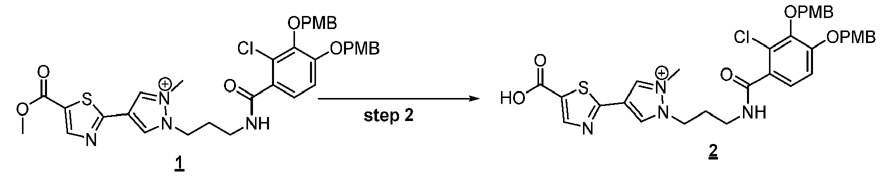

An aqueous solution of LiOH (2 mL, 2 mol/L) was added dropwise to intermediate 1 (300 mg, 0.43 mmol) in THF (3 mL). The reaction mixture was allowed to react at room temperature for 2 h and concentrated by rotary evaporation to remove tetrahydrofuran. The pH of the solution was adjusted to about 2.5 with HCl (1 mol/L), and the aqueous phase was extracted with ethyl acetate. The organic phase was concentrated to dryness by rotary evaporation to give compound 2 as a white solid (60 mg, 20%). m/z (ES+), [M-H]+ = 677; acid, HPLC tR = 2.545 min.

### Synthesis of compound 3

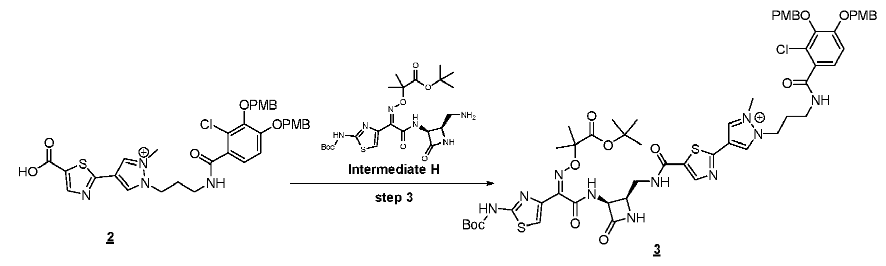

In a 10 mL round-bottom flask, compound 2 (60 mg, 0.088 mmol) and intermediate H (47 mg, 0.088 mmol) were dissolved in a solvent mixture of THF (1.6 mL) and DMF (1 mL), and DMTMM (39 mg, 0.132 mmol) was added under nitrogen. The reaction mixture was stirred at room temperature for 2 h. The reaction system was directly purified by reversed-phase chromatography to give compound 3 as a white solid (45 mg, 42.8%). m/z (ES+), [M+H]+ = 1186; acid, HPLC tR = 1.287 min.

### Synthesis of compound 4:

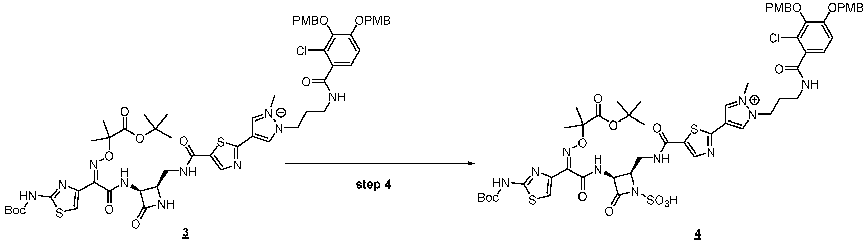

In a 10 mL round-bottom flask, compound 3 (40 mg, 0.034 mmol, 1.00 eq) was dissolved in DMF (2 mL), and SO₃.Py (120 mg, 0.76 mmol, 22 eq) was added. The reaction system was allowed to react at 45 °C for 3 h. The reaction mixture was directly purified by reversed-phase column chromatography to give 4 as a pale yellow solid (25 mg, 62%). m/z (ES+), [M-H]+ = 1265; acid, HPLC tR = 1.201 min.

### Synthesis of compound 1-33:

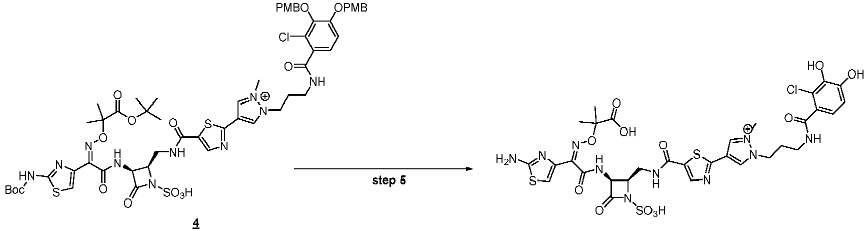

In a 25 mL round-bottom flask, compound 4 (25 mg, 0.02 mmol, 1.00 eq) was dissolved in dichloromethane (2 mL), and BCl₃ (0.5 mL, 0.5 mmol) was added at 0 °C under nitrogen. The reaction system was stirred at 0 °C for 4 h. After the reaction was completed, dichloromethane was removed by blow-drying with nitrogen, and the solid was washed with diethyl ether and purified by preparative high-pressure liquid chromatography (under the following conditions: column: Sunfire preparative C18 OBD column, 10 µm,19 × 250 mm; mobile phase A: water (0.1% FA); mobile phase B: CAN; flowrate: 20 mL/min; gradient: from 5 B to 10 B over 0.5 min; 254/220 nm) to give final product 1-33 as an off-white solid (2.1 mg). m/z (ES+), [M+H]+ = 869; acid, HPLC tR = 0.87 min. ¹H NMR(D₂O, 400 MHz): δ (ppm) 1.68 (d, J = 4.5 Hz, 6H), 2.48 (s, 3H), 3.66 (s, 2H), 3.96 (s, 2H), 4.40 (s, 3H), 4.65 (t, *J* = 5.9 Hz, 2H), 5.50 (d, *J* = 5.5 Hz, 1H), 7.02 (d, *J* = 8.1 Hz, 1H), 7.05-7.17 (m, 2H), 8.47 (s, 1H), 9.08 (s, 1H), 9.18 (s, 1H).

### Example 34. Synthesis of Compound 1-34

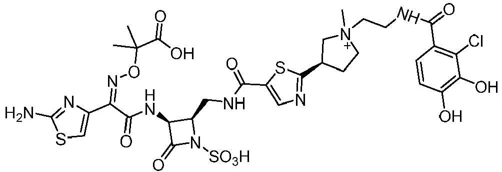

### Synthesis of compound 2:

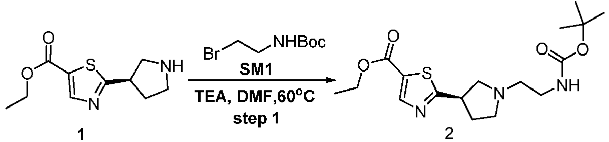

Compound 1 (452 mg, 2.0 mmol) was dissolved in dry *N*,*N*-dimethylformamide (10 mL), and then triethylamine (606 mg, 6.0 mmol) and compound *N*-Boc-bromoethylamine (535.2 mg, 2.4 mmol) were added successively at room temperature. Finally, the reaction mixture was stirred at 60 °C for 3 h. After the reaction was completed, the reaction mixture was diluted with ethyl acetate and washed with a saturated solution of sodium carbonate (2 × 20 mL). The organic phase was dried over anhydrous sodium sulfate and concentrated to dryness by rotary evaporation. The resulting crude product was purified by silica gel column chromatography to give compound **2** as a yellow oil (400 mg, 49%). m/z (ES+), [M]+ = 370.

### Synthesis of compound 3:

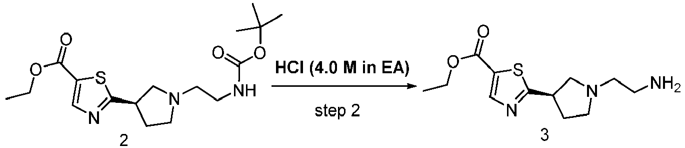

Compound **2** (380 mg, 1.02 mmol) was dissolved in dry ethyl acetate (10 mL), and 5.0 mL of HCl (4.0 M in EA) was added at room temperature. The reaction mixture was stirred at room temperature for 2 h. After the reaction was completed, the reaction mixture was directly concentrated to dryness by rotary evaporation to give compound **3** as a brown solid (300 mg, 95.5%), which was directly used in the next step without further purification. m/z (ES+), [M]+ = 269.

### Synthesis of compound 4:

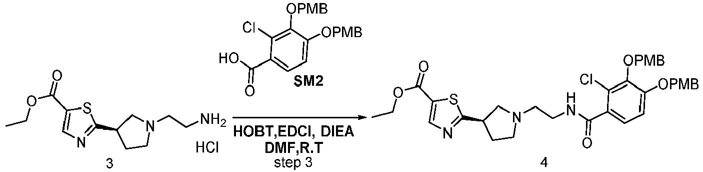

Compound **SM2** (428 mg, 1 mmol) and compound **3** (269 mg,1 mmol) were dissolved in *N,N*-dimethylformamide (5 mL), and HOBt (275 mg, 2 mmol), DIEA (516 mg, 4 mmol) and EDC·HCl (384 mg, 2 mmol) were successively added at room temperature. After addition, the reaction mixture was stirred at room temperature for 3 h. After the reaction was completed, the reaction mixture was diluted successively with water and ethyl acetate and extracted with ethyl acetate. The organic phases were combined, washed with saturated brine, dried over anhydrous magnesium sulfate, and finally concentrated to dryness. The resulting crude product was purified by silica gel column chromatography to give the final product, compound **4** as a yellow solid (360 mg, 48%). m/z (ES+), [M]+ = 680.

### Synthesis of compound 5:

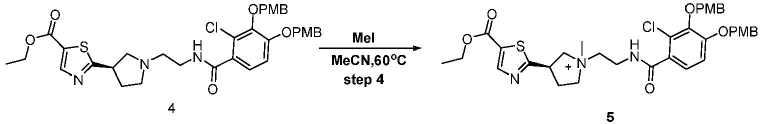

Compound **4** (340 mg, 0.5 mmol) was dissolved in dry acetonitrile (10 mL), and iodomethane (355 mg, 2.5 mmol) was added at room temperature. After addition, the reaction mixture was stirred at 60 °C for 3 h. After the reaction was completed, the reaction mixture was directly concentrated to dryness by rotary evaporation to give compound **5** as a crude product (300 mg, 95.5%).

m/z (ES+), [M]+ = 694.

### Synthesis of compound 6:

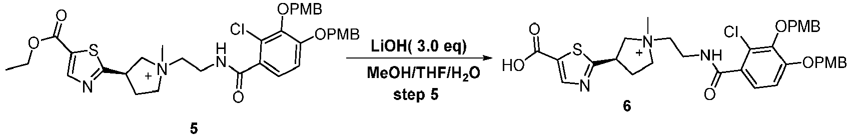

Compound **5** (347 mg, 0.5 mmol) was dissolved in methanol (5.0 mL) and tetrahydrofuran (5.0 mL), and then an aqueous solution of lithium hydroxide (10 mL, 1.5 mol/L) was added. The reaction mixture was stirred at room temperature for 2 h. After the reaction was completed, methanol and tetrahydrofuran were removed by rotary evaporation, and the remaining aqueous solution was adjusted to pH 2.5 with hydrochloric acid (1 mol/L) and then extracted with ethyl acetate. The organic phase was concentrated to dryness by rotary evaporation to give final product **6** as a white solid (200 mg, 97%). m/z (ES+), [M+H]+ = 668.

### Synthesis of compound 7:

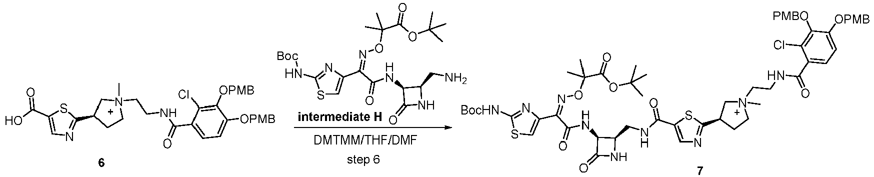

Compound **6** (200 mg, 0.30 mmol) and intermediate H (158 mg, 0.30 mmol) were dissolved in tetrahydrofuran (1.6 mL) and N,N-dimethylformamide (1 mL), and DMTMM (132 mg, 0.45 mmol) was added slowly. The reaction mixture was stirred under nitrogen at room temperature for 2 h. After the reaction was completed, the crude product was purified by reversed-phase column chromatography to give compound **7** as a white solid (200 mg, 57%). m/z (ES+), [M]+ = 1174.

### Synthesis of compound 8:

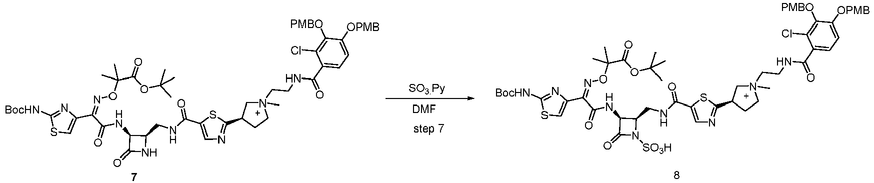

The compound 7 (200 mg, 0.17 mmol, 1.0 eq) was dissolved in dry *N,N-*dimethylformamide (3 mL), and sulfur trioxide pyridine complex (537 mg, 3.4 mmol, 20.0 eq) was added at room temperature. The reaction system was stirred at 45 °C for 3 h. After the reaction was completed, the reaction system was purified by reversed-phase column chromatography to give compound **8** as a white solid (165 mg, 69%). m/z (ES+), [M]+ = 1255.

### Synthesis of compound I-34:

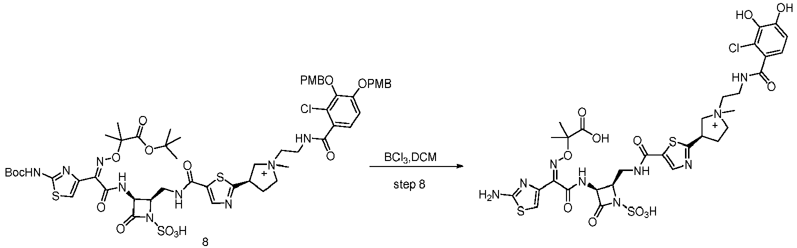

Compound **8** (100 mg, 0.0797 mmol, 1.00 eq) was dissolved in dichloromethane (2 mL), and the reaction system was purged 3 times with nitrogen. A solution of boron trichloride in dichloromethane (0.63 mL, 0.63 mmol) was slowly added dropwise in an ice bath under nitrogen. The reaction mixture was stirred in an ice bath for 2 h and blow-dried with nitrogen. The residue was washed three times with diethyl ether by centrifugation. The resulting crude product was lyophilized and purified by preparative high-pressure liquid chromatography to give compound **I-34** as a white solid (22.7 mg, 33%). m/z (ES+), [M]+ = 859; acid, 1H NMR (400 MHz, DMSO-*d*₆) δ 8.13 (d, J = 4.8 Hz, 1H), 6.79 (pd, J = 8.2, 3.9 Hz, 3H), 6.03 (d, J = 4.7 Hz, 1H), 5.66 (d, J = 4.3 Hz, 1H), 5.17 (d, J = 5.5 Hz, 1H), 4.19 (q, J = 6.2 Hz, 1H), 3.70-3.49 (m, 4H), 3.33 (d, J = 29.1 Hz, 4H), 3.15-2.56 (m, 6H), 1.40 (d, J = 7.4 Hz, 6H).

### Example 35. Synthesis of Compound I-35

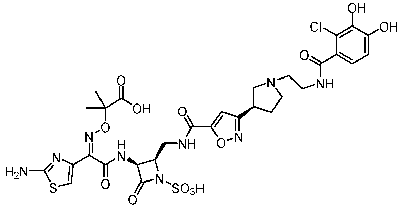

### Synthesis of compound 2:

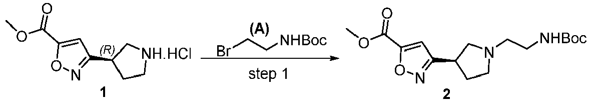

Compound 1 (4.5 g, 19.3 mmol), *N*,*N*-diisopropylethylamine (10 g, 77.2 mmol) and *N-*Boc-bromoethylamine (4.3 g, 19.3 mmol) were dissolved in acetonitrile (80 mL). The reaction mixture was stirred at 60 °C for 2 h, cooled to room temperature, and concentrated to dryness by rotary evaporation. The residue was dissolved in ethyl acetate, and the resulting solution was washed 3 times with saturated brine, dried, and concentrated. The residue was purified by silica gel column chromatography to give compound 2 as a yellow oil (4.1 g, 62.4% yield). m/z (ES+), [M+H] = 340; acid, HPLC RT = 1.354 min.

### Synthesis of compound 3:

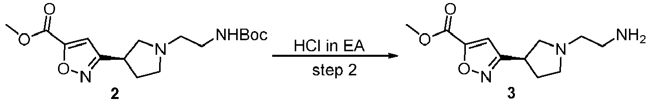

Compound 2 (4.1 g, 12.1 mmol) was dissolved in ethyl acetate (20 mL), and a solution of hydrogen chloride in ethyl acetate (4 M, 50 mL) was slowly added dropwise. The reaction mixture was stirred at room temperature for 2 h, and concentrated to dryness by rotary evaporation to give compound 3 as a brown oil (3.9 g, crude), which was directly used in the next step without purification. m/z (ES+), [M+H] = 240; acid, HPLC RT = 0.708 min.

### Synthesis of compound 4:

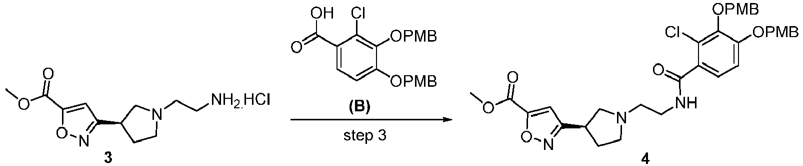

Compound B (5.92 g, 13.8 mmol) was dissolved in *N*,*N*-dimethylformamide (50 mL), and HATU (10.5 g, 27.6 mmol), *N*,*N*-diisopropylethylamine (7.12 g, 55.2 mmol) and compound 3 (3.8 g, 13.8 mmol) were slowly added. The reaction mixture was stirred at room temperature for 2 h, quenched with water, and extracted three times with ethyl acetate. The organic phase was washed three times with saturated brine, dried, and concentrated to dryness by rotary evaporation. The residue was purified by silica gel column chromatography to give compound 4 as a brown solid (6.1 g, 68.5% yield). m/z (ES+), [M]+ = 650; acid, HPLC RT = 1.471 min.

### Synthesis of compound 5:

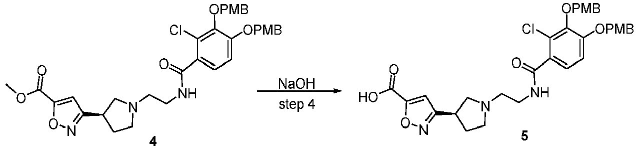

Compound 4 (2 g, 3 mmol) was dissolved in tetrahydrofuran (50 mL), and an aqueous solution of sodium hydroxide (6 mL, 1 M, 6 mmol) was slowly added dropwise. The reaction mixture was stirred at room temperature for 2 h and concentrated. The residue was dissolved in water, and the resulting solution was adjusted to pH 3-4 with 1 M hydrochloric acid and lyophilized to give compound 5 as a yellow solid (1.9 g, crude). m/z (ES+), [M]+ = 636; acid, HPLC RT = 1.709 min.

### Synthesis of compound 6:

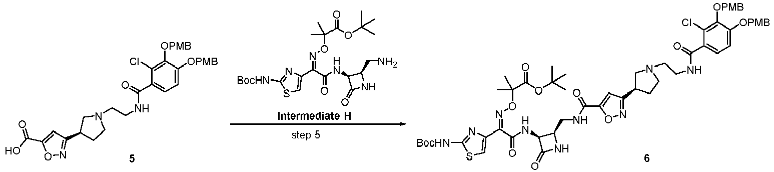

Compound 5 (1.6 g, 2.5 mmol) and intermediate H (1.32 g, 2.5 mmol) were dissolved in N,N-dimethylformamide (20 mL), and DMTMM (1.48 g, 5 mmol) was added slowly. The reaction mixture was stirred at room temperature for 2 h, directly purified by reversed-phase chromatography without workup, and lyophilized to give compound 6 as a pale yellow solid (1.67 g, 57.5% yield). m/z (ES+), [M]+ = 1144; acid, HPLC RT = 2.367 min.

### Synthesis of compound 7:

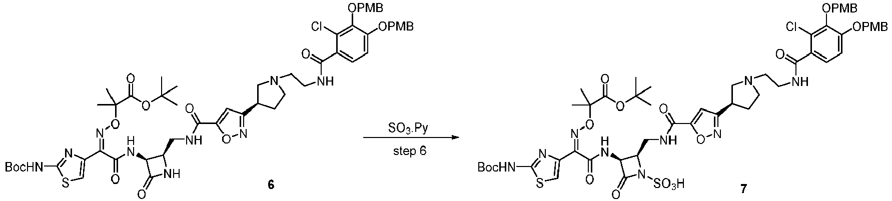

Compound 6 (1.67 g, 1.46 mmol) was dissolved in N,N-dimethylformamide (20 mL), and sulfur trioxide pyridine complex (4.64 g, 29.2 mmol) was slowly added. The reaction mixture was stirred at 45 °C for 2 h, directly purified by reversed-phase chromatography without workup, and lyophilized to give compound 7 as an off-white solid (1.45 g, 81.4% yield). m/z (ES+), [M+H]+ = 1225; acid, HPLC RT = 2.484 min.

### Synthesis of compound I-35:

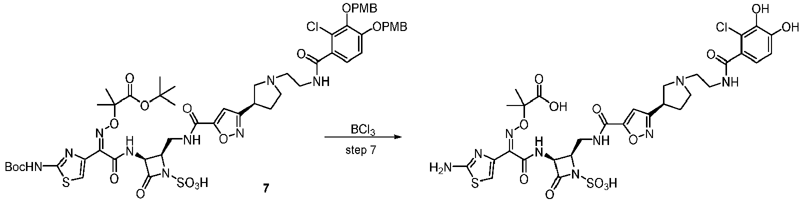

Compound 7 (300 mg, 0.25 mmol) was dissolved in dichloromethane (5 mL), and the reaction flask was purged 3 times with nitrogen. A solution of boron trichloride in dichloromethane (1.75 mL, 1.75 mmol) was slowly added dropwise in an ice bath under nitrogen. The reaction mixture was stirred in an ice bath for 2 h and blow-dried with nitrogen. The residue was washed three times with diethyl ether by centrifugation. The resulting crude product was purified by preparative high-pressure liquid chromatography and lyophilized to give compound **I-35** as a white solid (59.9 mg, 29.6% yield). m/z (ES-), [M+H]+ = 829; acid, HPLC RT = 0.796 min. ¹H NMR (400 MHz, DMSO-*d*₆) δ 1.40 (s, 6H), 2.28 (s, 1H), 2.62 (d, J = 23.6 Hz, 1H), 3.52 (t, J = 6.1 Hz, 3H), 3.69-3.78 (m, 4H), 3.83 (dd, J = 7.4, 14.5 Hz, 2H), 3.90 (s, 2H), 4.57 (q, J = 6.0 Hz, 1H), 5.33 (d, J = 5.6 Hz, 1H), 6.83 (d, J = 8.4 Hz, 1H), 6.92-6.99 (m, 3H).

### Example 36. Synthesis of Compound 1-36

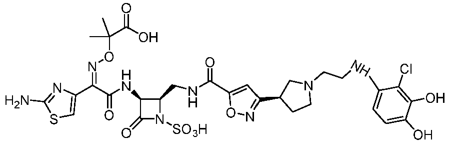

### Synthesis of compound 1

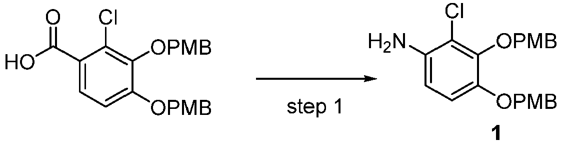

2-Chloro-3,4-bis((4-methoxybenzyl)oxo))benzoic acid (10 g, 23.3 mmol), triethylamine (2.8 g, 28 mmol), diphenylphosphoryl azide (8 g, 28 mmol), 9-fluorenylmethanol (6.8 g, 35 mmol) and piperidine (10 g, 116.5 mmol) were added to toluene (100 mL). The reaction mixture was stirred at 100 °C for 16 h and concentrated. Water was added, and then the mixture was extracted with ethyl acetate. The organic phase was washed with 2 M hydrochloric acid, a saturated aqueous solution of sodium bicarbonate and saturated brine, dried over anhydrous sodium sulfate, and concentrated to dryness by rotary evaporation to give compound 1 as an off-white solid (6 g, 64%). m/z (ES+), [M+H]+ = 400, acid.

### Synthesis of compound 2

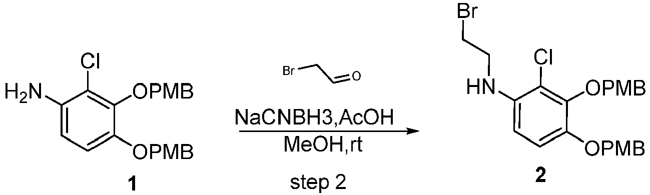

Compound 1 (8 g, 20 mmol), 2-bromoacetaldehyde (4.89 g, 40 mmol) and *N,N-*diisopropylethylamine (766 mg, 5.94 mmol) were added to methanol (20 mL). The reaction mixture was stirred at room temperature for 2 h, and then sodium cyanoborohydride (3.789 g, 60 mmol) and acetic acid (0.5 mL) were added thereto. The reaction mixture was stirred at room temperature for 18 h, then quenched with water, and extracted with ethyl acetate (2 × 100 mL). The organic phase was concentrated to dryness by rotary evaporation, and the residue was purified by column chromatography to give compound 2 (3 g, 29.5%). m/z (ES+), [M+H]+ = 507, acid.

### Synthesis of compound 3

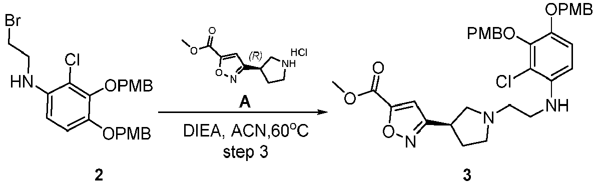

Compound 2 (1 g, 1.98 mmol), amine hydrochloride (459 mg, 1.98 mmol) and *N,N-*diisopropylethylamine (766 mg, 5.94 mmol) were added to acetonitrile (20 mL). The reaction mixture was stirred under nitrogen at 60 °C for 5 h and concentrated, and the residue was purified by column chromatography to give compound 3 (500 mg, 40.5%). m/z (ES+), [M+H]+ = 623, acid.

### Synthesis of compound 4

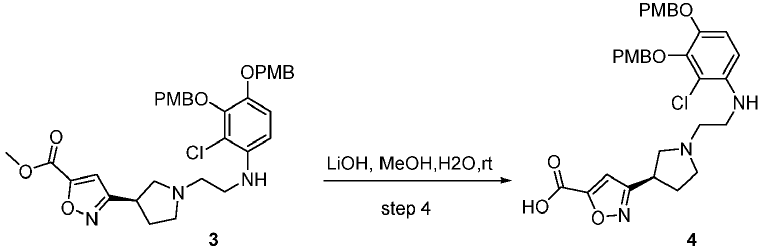

Lithium hydroxide (288 mg, 10.3 mmol) was added to compound 4 (1.6 g, 2.57 mmol) in a solvent mixture of methanol (5 mL)/tetrahydrofuran (5 mL)/water (5 mL). The reaction mixture was allowed to react at room temperature for 3 h, and the reaction system was purified by reversed-phase chromatography to give compound 4 (1.4 g, 22.3%). m/z (ES+), [M]+ = 608, acid.

### Synthesis of compound 5

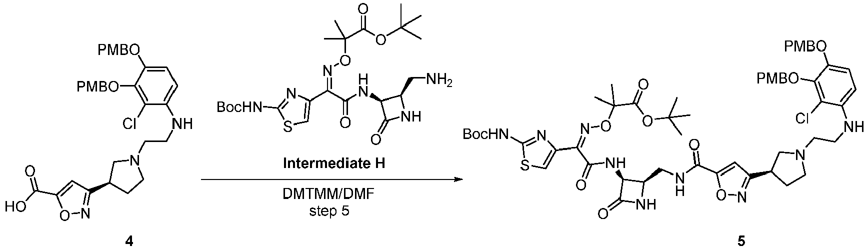

Compound 4 (1.4 g, 2.3 mmol) and intermediate H (1.21 g, 2.3 mmol) were dissolved in a solvent mixture of tetrahydrofuran (12 mL) and *N*,*N*-dimethylformamide (8 mL). The solution was stirred for 10 min, and then 4-(4,6-dimethoxytriazin-2-yl)-4-methylmorpholinium hydrochloride (718 mg, 3.45 mmol) was added thereto. The reaction mixture was stirred at room temperature under nitrogen for 1.5 h, and the reaction system was concentrated. The residue was purified by reversed-phase chromatography to give compound 5 as a white solid (900 mg, 35%). m/z (ES+), [M+H]+ = 1117.

### Synthesis of compound 6

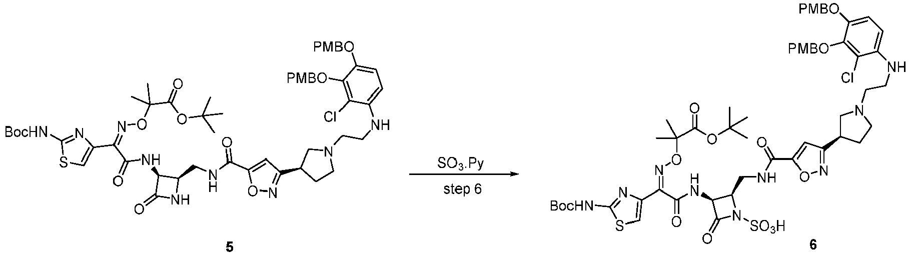

Compound 5 (900 mg, 0.8 mmol, 1.00 eq) was dissolved in N,N-dimethylformamide (15 mL), and sulfur trioxide pyridine complex (2.0 g, 11.4 mmol) was added under nitrogen to the solution. The reaction mixture was stirred at room temperature for 2 h and purified by reversed-phase chromatography to give compound 6 as a white solid (420 mg, 94%). (ES+), [M+H]+ = 1197.

### Synthesis of compound I-36

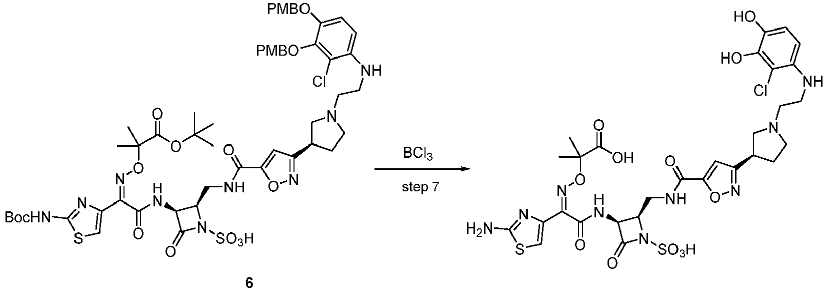

Boron trichloride (5 mL) was added at 0 °C under nitrogen to compound 6 (400 mg, 0.334 mmol, 1.00 eq) in dichloromethane (10 mL). The reaction mixture was stirred at 0 °C for 6 h and blow-dried with nitrogen to remove the solvent. The residue was washed four times with diethyl ether by centrifugation, and the resulting solid was purified by preparative high-pressure liquid chromatography (column: Xselect CSH OBD column 30 × 150 mm 5 µm, n; mobile phase A: water (0.1% FA); mobile phase B: CAN; flowrate: 60 mL/min; gradient: from 5% B to 30% B, 30% B over 7 min; wavelength: 254/220 nm) to give compound 1-36 as an off-white solid (72.3 mg, 27%). (ES+), [M+H]+ = 800. ¹H NMR (400 MHz, DMSO-*d*₆): δ (ppm) 1.41 (d, *J* = 6.2 Hz, 6H), 2.14 (s, 1H), 3.04-3.21 (m, 4H), 3.41 (d, *J* = 37.7 Hz, 8H), 3.85 (d, *J* = 13.1 Hz, 1H), 5.20 (d, *J* = 5.7 Hz, 1H), 6.10 (d, *J* = 8.7 Hz, 1H), 6.60 (d, *J* = 8.7 Hz, 1H), 6.74 (s, 1H), 7.07 (s, 1H).

### Example 37. Synthesis of Compound 1-37

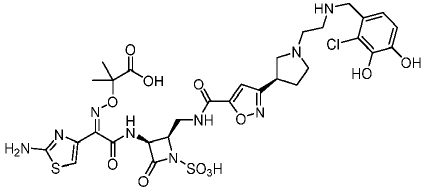

### Synthesis of compound 1:

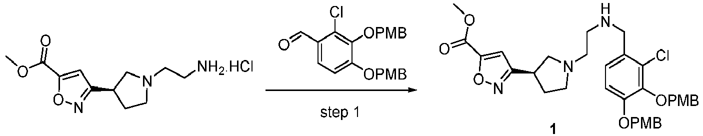

Compound SM (550 mg, 1.33 mmol), triethylamine (336 mg, 3.36 mmol) and aldehyde (550 mg, 1.33 mmol) were added under nitrogen to methanol (10 mL). The reaction mixture was stirred at room temperature for 5 h. Sodium borohydride (152 mg, 4 mmol) was added in batches at 0 °C, and the reaction mixture was allowed to react at room temperature for 3 h and purified by reversed-phase chromatography to give compound 1 as a white solid (260 mg, 10.3%). m/z (ES+), [M]+ = 636, acid.

### Synthesis of compound 2:

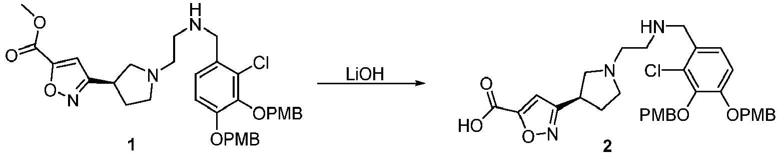

Lithium hydroxide (30 mg, 1.25 mmol) was added at room temperature to compound 1 (260 mg, 0.41 mmol) in a solvent mixture of methanol (2 mL)/tetrahydrofuran (2 mL)/water (4 mL). The reaction mixture was stirred at room temperature for 3 h and purified by reversed-phase chromatography to give compound 2 as a white solid (200 mg, 78%). m/z (ES+), [M]+ = 622, acid.

### Synthesis of compound 3

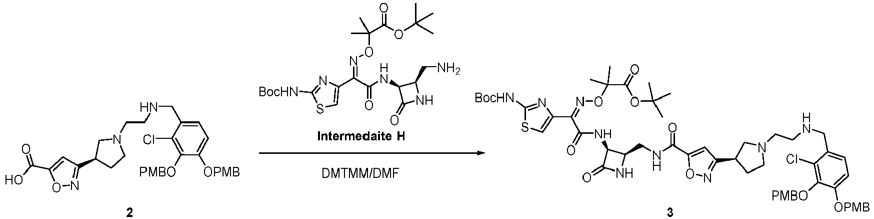

Compound 2 (340 mg, 0.55 mmol) and intermediate H (287 mg, 0.55 mmol) were added to tetrahydrofuran (6 mL) and *N*,*N*-dimethylformamide (4 mL). The mixture was stirred at room temperature for 10 min, and 4-(4,6-dimethoxytriazin-2-yl)-4-methylmorpholinium hydrochloride (200 mg, 0.96 mmol) was added. The reaction mixture was stirred at room temperature under nitrogen for 1.5 h and concentrated, and the residue was purified by reversed-phase chromatography to give compound 3 as a white solid (110 mg, 17.7%). m/z (ES+), [M]+ = 1130, acid.

### Synthesis of compound 4

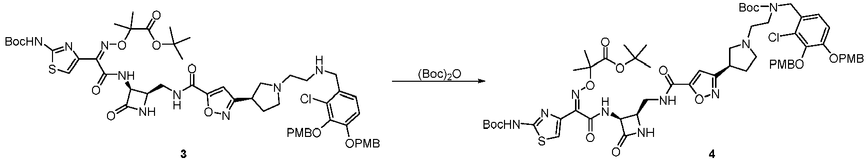

Compound 3 (110 mg, 0.53 mmol), di-*tert*-butyl dicarbonate (346 mg, 1.59 mmol) and triethylamine (267 mg, 2.65 mmol) were dissolved in dichloromethane (5 mL). The reaction mixture was stirred at room temperature for 4 h and concentrated to remove the solvent, and the residue was purified by reversed-phase chromatography to give compound 4 as a white solid (100 mg, 15.3%). (ES+), [M]+ = 1230, acid.

### Synthesis of compound 5:

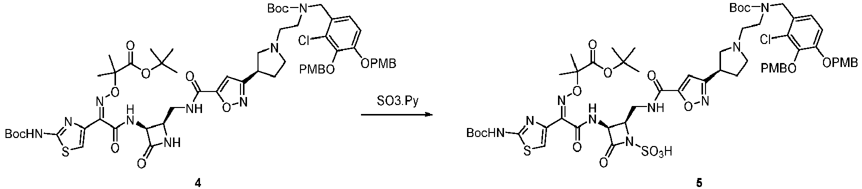

Sulfur trioxide pyridine complex (200 mg, 1.14 mmol) was added under nitrogen to compound 4 (100 mg, 0.081 mmol, 1.00 eq) in *N*,*N*-dimethylformamide (3 mL). The reaction mixture was stirred at room temperature for 2 h and purified by reversed-phase chromatography to give compound 5 as a white solid (100 mg, 93.8%). (ES+), [M+H]+ = 1311.

### Synthesis of compound I-37:

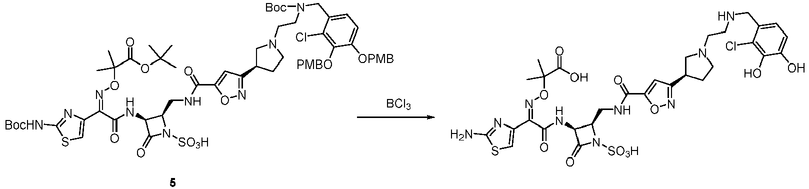

Boron trichloride (2 mL) was added dropwise at 0-5 °C under nitrogen to a solution of compound 5 (100 mg, 0.076 mmol, 1.00 eq) in dichloromethane (4 mL). The reaction mixture was stirred at 0 °C for 6 h and blow-dried with nitrogen to remove the solvent. The residue was washed four times with diethyl ether by centrifugation to give a solid, which was then purified by preparative high-pressure liquid chromatography (column: XSelect CSH preparative C18 OBD column, 19 × 250 mm, 5 µm; mobile phase A: water (0.1% FA); mobile phase B: ACN; flowrate: 25 mL/min; 254/220 nm) to give compound 1-37 as an off-white solid (4.5 mg, 7.2%). (ES+), [M+H]+ = 814. ¹H NMR(DMSO-*d*₆, 400 MHz): δ (ppm) 1.42 (d, *J* = 6.6 Hz, 6H), 1.80-2.00 (m, 1H), 2.18-2.38 (m, 1H), 2.43 (s, 3H), 2.65-2.89 (m, 4H), 3.00 (t, *J* = 10.1 Hz, 3H), 3.78 (dd, *J* = 14.3, 4.3 Hz, 1H), 4.12 (d, *J* = 6.5 Hz, 3H), 5.22 (d, *J* = 5.7 Hz, 1H), 6.62-6.84 (m, 2H), 6.89 (d, *J* = 8.3 Hz, 1H), 7.03 (s, 1H).

### Example 38. Synthesis of Compound 1-38

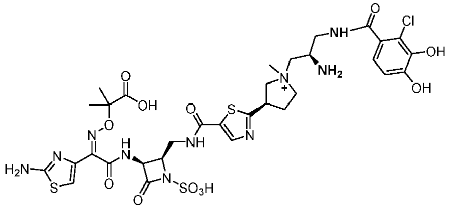

### Synthesis of compound 2:

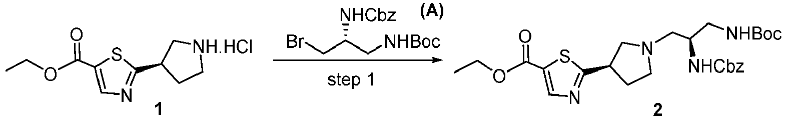

Compound 1 (500 mg, 1.9 mmol) was dissolved in *N*,*N*-dimethylformamide (10 mL), and *N*,*N*-diisopropylethylamine (1.47 g, 11.4 mmol) and compound A (1.47 g, 3.8 mmol) were slowly added. The reaction mixture was stirred at 60 °C overnight, quenched with water, and extracted three times with ethyl acetate. The organic phases were combined, washed three times with saturated brine, dried over anhydrous sodium sulfate, and concentrated. The residue was purified by silica gel thin layer chromatography to give compound 2 as a yellow oil (250 mg, 24% yield). m/z (ES+), [M+H]+ = 533; acid, HPLC RT = 1.181 min.

### Synthesis of compound 3:

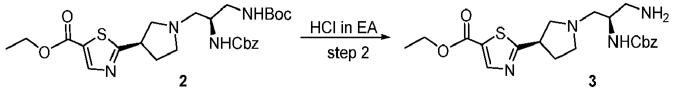

Compound 2 (250 mg, 0.47 mmol) was dissolved in a small amount of ethyl acetate (3 mL), and a solution of hydrogen chloride in ethyl acetate (4 M, 10 mL) was slowly added dropwise. The reaction mixture was stirred at room temperature for 2 h and concentrated to give compound 3 as a yellow solid (210 mg, crude). m/z (ES+), [M+H]⁺ = 433; acid, HPLC RT = 0.964 min.

### Synthesis of compound 4:

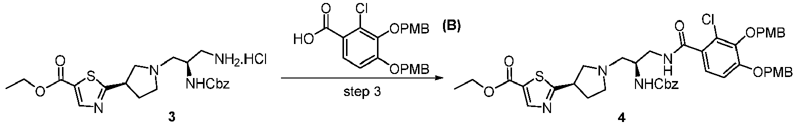

Compound B (193 mg, 0.45 mmol) was dissolved in DMF (3 mL), and HATU (342 mg, 0.9 mmol) and DIEA (232 mg, 1.8 mmol) were slowly added. The mixture was stirred for 10 min, and compound 3 (210 mg, 0.45 mmol) was slowly added. The reaction mixture was stirred at room temperature for 2 h, quenched with water, and extracted with ethyl acetate (3 × 20 mL). The organic phases were combined, washed with saturated brine, dried, and concentrated. The residue was purified by silica gel thin layer chromatography and lyophilized to give compound 4 as a pale yellow solid (150 mg, 39% yield). m/z (ES+), [M+H]⁺ = 845, acid, HPLC RT = 2.284 min.

### Synthesis of compound 5:

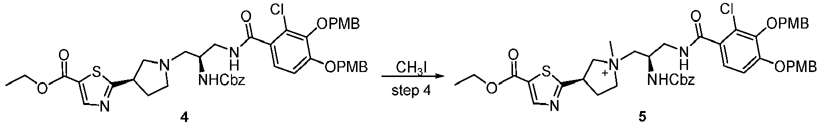

Compound 4 (150 mg, 0.18 mmol) was dissolved in *N*,*N*-dimethylformamide (3 mL), and iodomethane (126 mg, 0.9 mmol) was slowly added. The reaction mixture was stirred at 80 °C for 3 h, directly purified by reversed-phase chromatography without workup, and lyophilized to give compound 5 as a yellow solid (100 mg, 65% yield). m/z (ES+), [M]+ = 857; acid, HPLC RT = 1.640 min.

### Synthesis of compound 6:

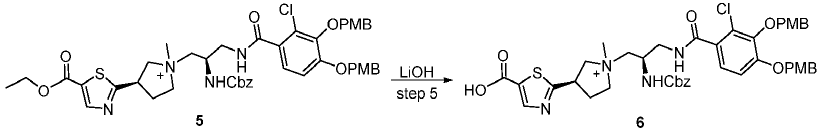

Compound 5 (100 mg, 0.117 mmol) was dissolved in tetrahydrofuran (3 mL), and an aqueous solution of lithium hydroxide (1.2 mL, 0.5 M, 0.585 mmol) was slowly add dropwise. The reaction mixture was stirred at room temperature for 1 h. After the reaction was completed, the reaction solution was concentrated, and the residue was dissolved in water. The aqueous phase was adjusted to pH 3-4 with 1 M HCl and lyophilized to give compound 6 as a pale yellow solid (110 mg, crude). m/z (ES+), [M]+ = 829, acid, HPLC RT = 1.690 min.

### Synthesis of compound 7:

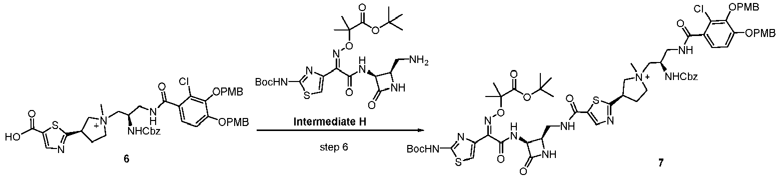

Compound 6 (90 mg, 0.11 mmol) and intermediate H (58 mg, 0.11 mmol) were dissolved in *N*,*N*-dimethylformamide (2 mL), and DMTMM (65 mg, 0.22 mmol) was added slowly. The reaction mixture was stirred at room temperature for 2 h, directly purified by preparative high-pressure liquid chromatography, and lyophilized to give compound 7 as a white solid (23 mg, 15.8% yield). m/z (ES+), [M]+ = 1337, acid, HPLC RT = 2.723 min.

### Synthesis of compound 8:

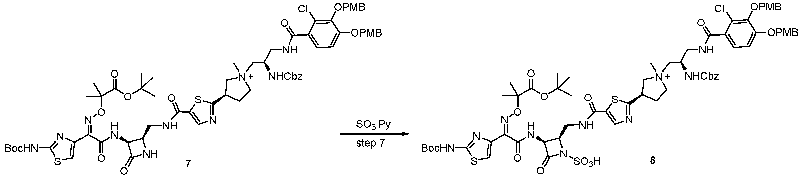

Compound 7 (20 mg, 0.015 mmol) was dissolved in *N*,*N*-dimethylformamide (2 mL), and sulfur trioxide pyridine complex (48 mg, 0.3 mmol) was slowly added. The reaction mixture was stirred at 45 °C for 2 h, directly purified through a C18 column without workup, and lyophilized to give compound 8 as a white solid (18 mg, 85.7% yield). m/z (ES+), [M+H]⁺ = 1420; acid, HPLC RT = 2.726 min.

### Synthesis of compound I-38:

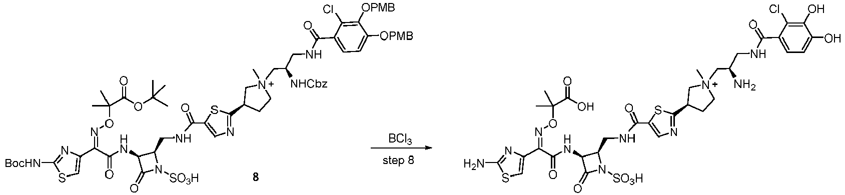

Compound 8 (18 mg, 0.013 mmol) was dissolved in dichloromethane (1 mL), and the reaction system was purged 3 times with nitrogen. A solution of boron trichloride in dichloromethane (1 M, 0.5 mL) was slowly added dropwise in an ice bath under nitrogen. The reaction mixture was stirred in an ice bath for 2 h. After the reaction was completed, the reaction mixture was blow-dried with nitrogen. The residue was washed three times with diethyl ether by centrifugation. The resulting crude product was purified by preparative high-pressure liquid chromatography (column: X Select CSH preparative C18 OBD column, 19 × 250 mm, 5 µm; mobile phase A: water (0.1% FA); mobile phase B: ACN; flowrate: 25 mL/min; gradient: from 10% B to 30% B over 7 min; 254/220 nm) to give a compound as a white solid (2 mg, 18.1% yield). m/z (ES+), [M]+ = 887; acid, HPLC RT = 0.772 min.

### Example 39. Synthesis of Compound 1-39

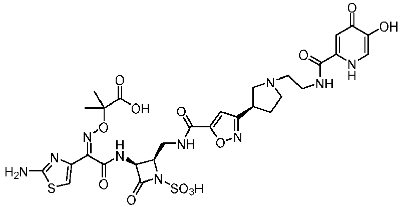

### Synthesis of compound 1:

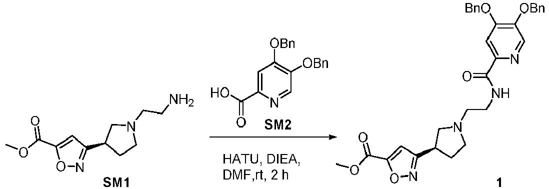

In a 50 mL single-neck flask, SM1 (1.2 g, 4.26 mmol, 1.43 eq) was dissolved by magnetic stirring in DMF (12 mL), and **SM2** (1.0 g, 2.98 mmol, 1.0 eq), HATU (2.1 g, 5.54 mmol, 1.86 eq) and *N*,*N*-diisopropylethylamine (1.7 g, 12.78 mmol, 4.29 eq) were added. After addition, the reaction mixture was stirred at room temperature for 2 h. After the reaction was completed, ice water (120 mL) was added dropwise to the system, and a solid precipitated. The mixture was filtered under vacuum, and the filter cake was washed with water (20 mL × 2) and dried to give **compound 1** as an off-white solid (crude, 2.2 g, 88% purity). MS = 557.2 (M+H)⁺.

### Synthesis of compound 2:

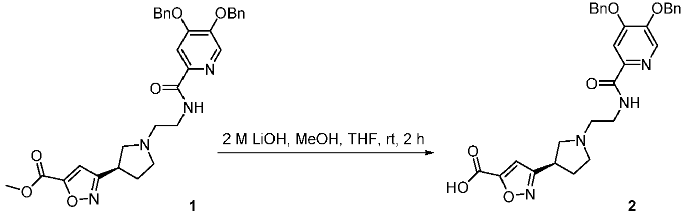

In a 50 mL single-neck flask, **compound 1** (2.0 g, 3.6 mmol, 1.0 eq) was dissolved by magnetic stirring in a mixture of methanol (12 mL) and tetrahydrofuran (12 mL), and a 2 M aqueous solution of lithium hydroxide (4 mL) was added dropwise. After addition, the reaction mixture was stirred at room temperature for 2 h. After the reaction was completed, the reaction system was concentrated, and water (10 mL) was added. The pH was adjusted to about 2 with a 2 N solution of hydrochloric acid, and a solid precipitated. The mixture was filtered under vacuum, and the filter cake was washed with water (20 mL × 2) and dried to give **compound 2** as an off-white solid crude product (1.85 g, 91% purity, 94.9% yield). MS = 543.2 (M+H)⁺.

### Synthesis of compound 3:

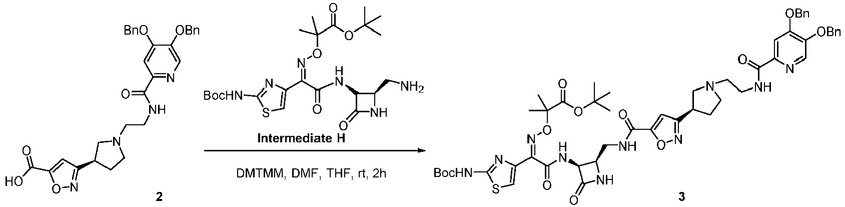

In a 50 mL four-necked flask, **compound 2** (1.3 g, 2.40 mmol, 1.0 eq) was dissolved under N2 by magnetic stirring in a mixture of DMF (5 mL) and tetrahydrofuran (9.8 mL), and **intermediate H** (1.51 g, 2.88 mmol, 1.2 eq) was added, followed by the addition of DMTMM (1.10 g, 3.60 mmol, 1.5 eq). The reaction mixture was stirred at room temperature for 2 h. After the reaction was completed, water (100 mL) was added to the system, and the mixture was extracted with dichloromethane (100 mL × 3). The organic phase was washed with saturated brine, dried, and concentrated. The residue was purified by silica gel column chromatography (dichloromethane/methanol = 20:1) to give **compound 3** as a pale yellow solid (1.02 g, 93% purity, 40.8% yield). MS = 1051.4 (M+H)⁺.

### Synthesis of compound 4:

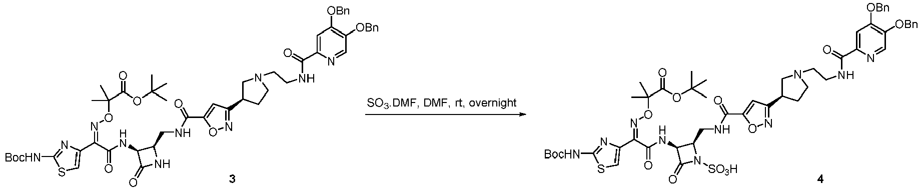

**Compound 3** (500.0 mg, 0.48 mmol, 1.0 eq) was dissolved under nitrogen in DMF (5 mL), and SO₃.DMF (364.3 mg, 2.38 mmol, 5.0 eq) was added. The reaction mixture was stirred at room temperature overnight. After the reaction was completed, the system was added dropwise to 50 mL of ice water, and a solid precipitated. The mixture was filtered under vacuum, and the filter cake was lyophilized to give **compound 4** as a white solid (501.0 mg, 91% purity, 93% yield). MS = 1131.4 (M+H)⁺.

### Synthesis of compound I-39:

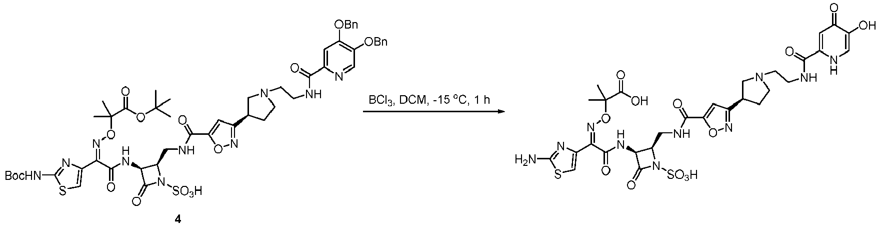

**Compound** 4 (200.0 mg, 0.194 mmol, 1.0 eq) was dissolved under nitrogen in dichloromethane (4 mL). The solution was cooled to -15 °C, and a solution of boron trichloride in dichloromethane was added dropwise. The reaction mixture was stirred at -15 °C for 1 h. After the reaction was completed, potassium bicarbonate was added in batches at -15 °C, followed by the addition of 0.2 mL of water and 12 mL of methyl *tert-butyl* ether. The mixture was stirred for 30 min and then filtered under vacuum and nitrogen. The filter cake was rinsed with methyl *tert-butyl* ether, blow-dried with nitrogen, and slurried with 1.2 mL of methyl *tert-*butyl ether and 1.2 mL of ethanol for 1 h. The slurry was filtered under vacuum, and the filter cake was blow-dried with nitrogen and purified by preparative HPLC to give **compound I-39** as an off-white solid (42 mg, 98% purity, 29.9% yield).

MS = 795.1 (M+H)⁺, ¹HNMR (400 MHz, DMSO-*d₆*): *δ* (ppm) 10.61 (brs, 1H), 9.90 (brs, 1H), 9.34 (d, *J* = 8.4 Hz, 1H), 8.75-8.66 (m, 2H), 7.98 (s, 1H), 7.48 (s, 1H), 7.26 (s, 2H), 7.09 (s, 1H), 6.73 (s, 1H), 5.23-5.19 (m, 1H), 4.12 (s, 1H), 3.82-3.33 (m, 4H), 2.10-2.07 (m, 2H), 1.43 (s, 3H), 1.41 (s, 3H).

### Example 40. Synthesis of Compound I-40

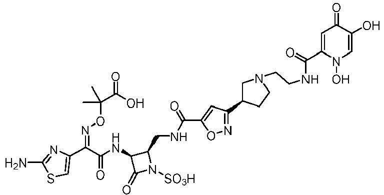

### Synthesis of compound 1:

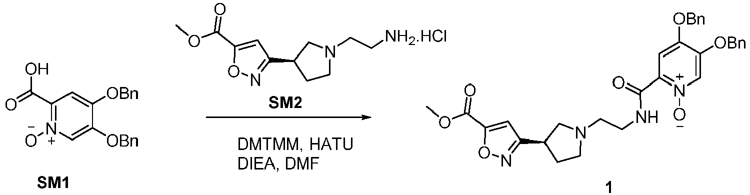

SM1 (500 mg, 1.42 mmol), DMTMM (592 mg, 2.13 mmol) and DIPEA (733 mg, 5.68 mmol) were dissolved in DMF (50 mL), and the system was purged with nitrogen and then warmed to 50 °C. A solution of **SM2** (628 mg, 2.13 mmol) in DMF (5 mL) was added dropwise in batches. The solution was stirred at 50 °C for 2 h and then gradually became clear. HATU (500 mg, 1.31 mmol) was added, and the heating was stopped. The mixture was stirred at room temperature overnight and concentrated under reduced pressure to remove DMF. Ethyl acetate and water were added, and the organic phase was separated, concentrated under reduced pressure to dryness to give a crude product, which was then purified by column chromatography (DCM/MeOH) to give purified compound 1 (710 mg, 93% purity, 87% yield). LCMS: [M+H]⁺ = 573.

### Synthesis of compound 2:

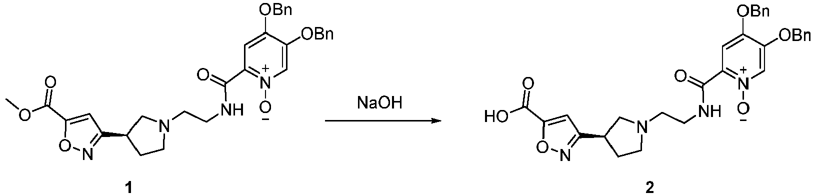

Compound 1 (710 mg, 1.24 mmol) was dissolved in THF/MeOH (7 mL/7 mL), and a 1 M solution of lithium hydroxide (2 mL) was added at room temperature. The reaction mixture was stirred at room temperature for 3 h, and the reaction was completed under intermediate control. The reaction mixture was concentrated under reduced pressure to remove the organic solvent. The pH was adjusted to 2 with 2 M hydrochloric acid, and then a large amount of insoluble matter precipitated. The mixture was extracted three times with ethyl acetate, and the organic phase was concentrated under reduced pressure to dryness to give compound **2** as a crude product (480 mg, 93% purity, 69% yield). LCMS: [M+H]+ = 559.

### Synthesis of compound 3:

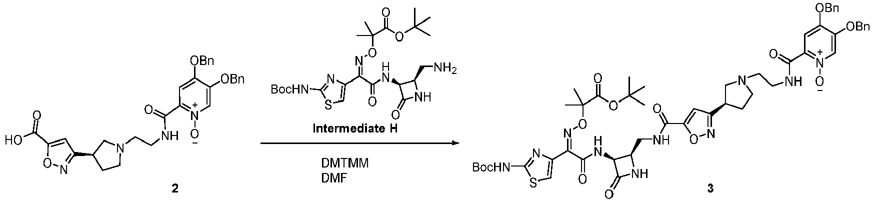

Compound **2** (350 mg, 0.627 mmol) and **intermediate H** (264 mg, 0.502 mmol) were dissolved in a solvent mixture of DMF/THF, and DMTMM (203 mg, 0.690 mmol) was added. The reaction mixture was stirred at room temperature under nitrogen for 2 h, and the reaction was completed under intermediate control. The reaction mixture was concentrated under reduced pressure to remove tetrahydrofuran, and water and ethyl acetate were added. The organic phase was separated and concentrated under reduced pressure. The residue was purified by normal-phase column chromatography (DCM/MeOH system) to give purified compound **3** (330 mg, 95% purity, 49% yield). LCMS: [M+H]+ = 1067.

### Synthesis of compound 4:

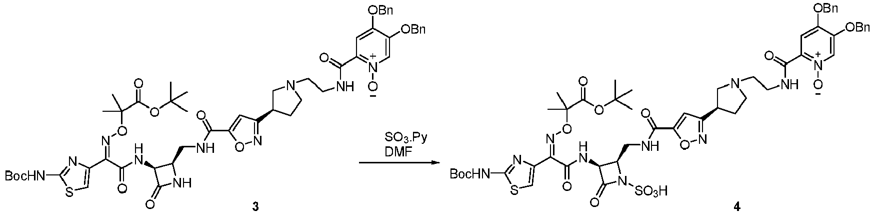

Compound 3 (120 mg, 0.113 mmol) was dissolved in anhydrous DMF (3 mL), and sulfur trioxide pyridine complex (286 mg, 1.180 mmol) was added. Under nitrogen, the mixture was warmed to 45 °C and stirred overnight, and the reaction was completed under intermediate control. Water was added to the system, and the mixture was extracted twice with ethyl acetate. The organic phases were combined and concentrated under reduced pressure to dryness, and the residue was purified by normal-phase column chromatography (DCM/MeOH system) to give compound **4** as a solid (77 mg, 96% purity, 60% yield). LCMS: [M+H]+ = 1146.

### Synthesis of compound I-40:

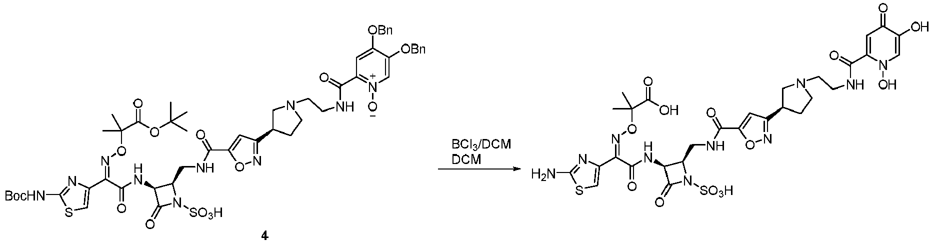

Compound **4** (77 mg, 0.067 mmol) was dissolved in dichloromethane (2 mL), and the reaction flask was purged 3 times with nitrogen. The system was cooled to below -20 °C. A solution of BCl₃.DCM (0.92 mL) was slowly added dropwise, with the internal temperature not higher than -20 °C. After addition, the reaction mixture was stirred at -20 °C for 1 h, and the reaction was completed under intermediate control. Solid NaHCO₃ (492 mg) was added in batches at -20 °C to the system, followed by the addition of water (77 mg) and then the slow addition of methyl *tert-butyl* ether TBME (4.5 mL). The mixture was stirred at -5 °C for another 30 min and filtered under nitrogen, and the filter cake was rinsed with a small amount of TBME and then blow-dried with nitrogen. The solid was slurried at room temperature with TBME (1.2 mL)/EtOH (1.2 mL) for 1 h, and the slurry was filtered. The filter cake was blow-dried with nitrogen to give a solid crude product (400 mg), which was then purified by preparative high-pressure liquid chromatography to give **compound I-40** (24.2 mg, 98.8% purity, 45% yield). LCMS: [M+H]+ = 811.

¹H NMR (CD₃OD-*d*₄, 400 MHz): δ (ppm) 1.61 (s, 6H), 2.25-2.34 (m, 1H), 2.51-2.62 (m, 1H), 3.57-3.59 (m, 2H), 3.67-3.81 (m, 2H), 3.81-3.98 (m, 7H), 4.47 (dd, *J* = 12.0, 8.0 Hz, 1H), 5.39-5.40 (m, 1H), 6.97 (s, 1H), 7.01 (s, 1H), 7.71 (s, 1H), 7.93 (s, 1H).

### Example 41. Synthesis of Compound 1-41

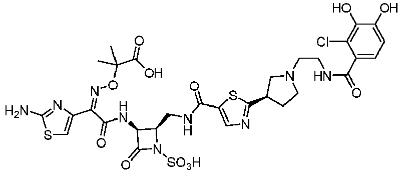

### Synthesis of compound 1:

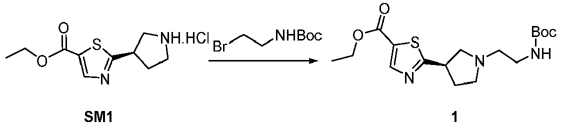

To acetonitrile (60 mL) were added **SM1** (6.0 g, 26.4 mmol, 1.0 eq), *N*-Boc-3-aminoethyl bromide (6.5 g, 29.1 mmol, 1.1 eq) and triethylamine (5.4 g, 52.8 mmol, 2.0 eq). The reaction mixture was warmed to 50 °C and then stirred overnight. After the reaction was completed, the reaction mixture was concentrated under reduced pressure to remove the solvent. The residue was dissolved in ethyl acetate, and the solution was rinsed with water and concentrated to remove the solvent. The resulting crude product was purified by column chromatography to give **compound 1** (2.2 g, 93% purity, 23% yield).

### Synthesis of compound 2:

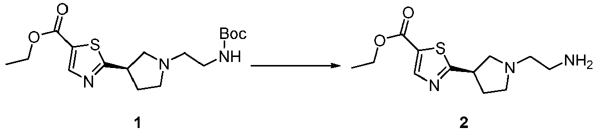

**Compound 1** (2.2 g, 5.9 mmol, 1.0 eq) was dissolved in 1,4-dioxane (10 mL), and a solution of hydrogen chloride/dioxane (10 mL) was added at room temperature. The reaction mixture was stirred at room temperature for 1 h. After the reaction was completed, the reaction mixture was concentrated under reduced pressure to dryness to give **compound 2** (3.0 g, 93% purity), which was directly used in the next step.

### Synthesis of compound 3:

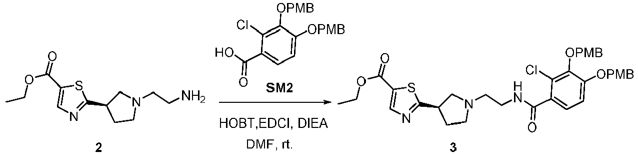

**Compound** 2 (1.6 g, 5.9 mmol, 1.0 eq), **SM2** (2.1 g, 4.7 mmol, 0.8 eq), EDCI (2.3 g, 11.8 mmol, 2.0 eq), HOBT (1.6 g, 11.8 mmol, 2.0 eq) and *N*,*N*-diisopropylethylamine (3.1 g, 23.7 mmol, 4.0 eq) were dissolved in DMF (20 mL). The reaction mixture was stirred at room temperature overnight. After the reaction was completed, ethyl acetate and water were added to the system. The organic phase was separated, reversely washed with water, concentrated under reduced pressure to dryness to give **compound 3** as a crude product (3.4 g, 85% purity, 85% yield), which was directly used in the next step without purification.

### Synthesis of compound 4:

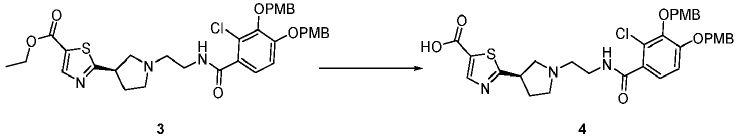

**Compound 3** (3.4 g, 5.0 mmol, 1.0 eq.) was dissolved in a solution of tetrahydrofuran/methanol (30 mL/30 mL), and a 2 M solution of lithium hydroxide was added at room temperature. The reaction mixture was stirred at room temperature for 1 h. After the reaction was completed, the reaction mixture was concentrated under reduced pressure to remove the organic solvent. The pH was adjusted to 3-4 with 6 M hydrochloric acid, and the mixture was extracted with dichloromethane (containing 10% methanol), and reversely washed with water. The organic phase was concentrated under reduced pressure to dryness to give **compound 4** as an oil (2.8 g, 85% purity, 99% yield).

### Synthesis of compound 5:

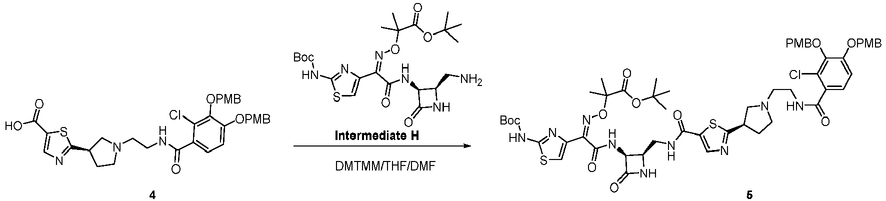

**Compound 4** (2.8 g, 4.3 mmol, 1.0 eq.) and **intermediate H** (1.8 g, 3.4 mmol, 0.8 eq.) were dissolved in a mixture of DMF/tetrahydrofuran (12 mL/18 mL). The reaction mixture was stirred at room temperature under nitrogen for 1 h. After the reaction was completed, water and ethyl acetate were added to the system. The aqueous phase was separated and reversely extracted with ethyl acetate, and the organic phases were combined, washed with water, and concentrated under reduced pressure to dryness. The crude product was purified by reversed-phase column chromatography (acetonitrile/water system) to give **compound 5** as a white solid (1.0 g, 95% purity, 20% yield).

### Synthesis of compound 6:

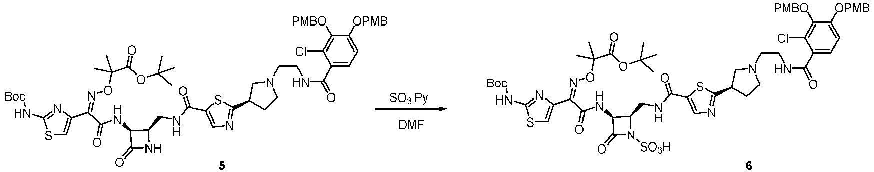

**Compound 5** (1.0 g, 0.86 mmol, 1.0 eq.) was dissolved in DMF (10 mL), and sulfur trioxide pyridine complex (2.2 g, 13.8 mmol, 16.0 eq.) was added. Under nitrogen, the reaction mixture was warmed to 45 °C and stirred overnight. After the reaction was completed, water and ethyl acetate were added to the system. The aqueous phase was separated and reversely extracted with ethyl acetate, and the organic phases were combined, reversely washed with water, and concentrated under reduced pressure to dryness. The residue was purified by column chromatography (dichloromethane/methanol system) to give **compound 6** as a white solid (0.7 g, 98% purity, 65% yield).

### Synthesis of compound 1-41:

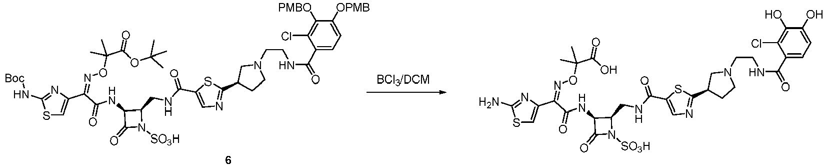

**Compound 6** (0.7 g, 0.56 mmol, 1.0 eq) was dissolved in dichloromethane (16 mL), and the reaction system was purged 3 times with nitrogen and cooled to below -20 °C. A solution of BCl₃.DCM was slowly added dropwise, with the internal temperature not higher than -20 °C. After addition, the reaction mixture was stirred at -20 °C for 1 h. After the reaction was completed, solid sodium bicarbonate (4.5 g) was added in batches at -20 °C to the system, followed by the addition of water (0.7 g). After quenching, methyl *tert-butyl* ether (42 mL) was slowly added to the system, which was then stirred at -5 °C for another 30 min and filtered under nitrogen. The filter cake was rinsed with a small amount of methyl *tert-butyl* ether and blow-dried with nitrogen. The crude product was slurried at room temperature with methyl *tert-*butyl ether (6 mL)/methanol (3 mL) for 1 h, and the slurry was filtered. The filter cake was blow-dried with nitrogen, and the resulting product was purified by preparative high-pressure liquid chromatography to give purified **compound I-41** (253 mg, 97% purity, 53% yield). (ES+), [M+H]+ = 845; ¹H NMR (400 MHz, DMSO-*d*₆): δ (ppm) 10.07 (s, 1H), 9.37 (d, *J* = 8.4 Hz, 1H), 9.29 (s, 1H), 8.49 (dd, *J* = 7.2, 3.6 Hz, 1H), 8.38 (t, *J* = 5.7 Hz, 1H), 8.15 (s, 1H), 7.27 (s, 2H), 6.86 (d, *J* = 8.2 Hz, 1H), 6.83-6.64 (m, 2H), 5.22 (dd, *J* = 8.4, 5.6 Hz, 1H), 4.15 (dt, *J* = 9.7, 5.0 Hz, 2H), 3.84-3.69 (m, 2H), 3.57 (d, *J* = 6.0 Hz, 3H), 1.45 (d, *J* = 9.0 Hz, 6H).

### Example 42. Synthesis of Compound 1-42

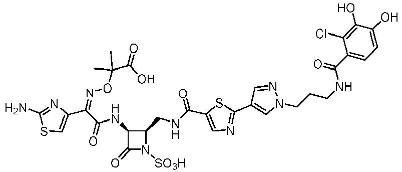

### Synthesis of compound 1:

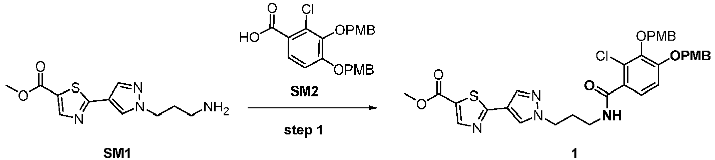

**SM1** (3.7 g, 14.0 mmol, 1.0 eq.), **SM2** (6.0 g, 14.0 mmol, 1.0 eq.) and HATU (5.9 g, 15.4 mmol, 1.1 eq.) were dissolved in DMF (40 mL), and *N*,*N*-diisopropylethylamine (5.4 g, 42.0 mmol, 3.0 eq.) was added. The reaction mixture was stirred at room temperature for 1 h, and the reaction was completed as indicated by LCMS. Water and ethyl acetate were added to the system, and the aqueous phase was separated and reversely extracted once. The organic phases were combined, reversely washed with water, and concentrated under reduced pressure to give **compound 1** as a solid (13.3 g, 80% purity), which was directly used in the next step.

### Synthesis of compound 2:

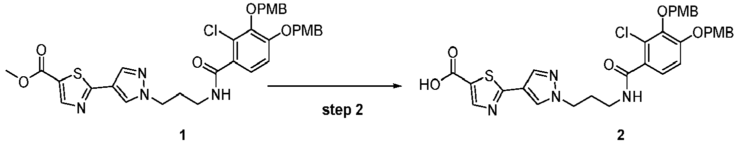

**Compound 1** (13.3 g, 197 mmol, 1.0 eq.) was dissolved in a solution of tetrahydrofuran/methanol (100 mL/100 mL), and a 2 M solution of lithium hydroxide (200 mL) was added at room temperature. The reaction mixture was stirred at room temperature for 1 h. After the reaction was completed, the reaction mixture was concentrated under reduced pressure to remove the organic solvent, and the pH was adjusted to 3-4 with 6 M hydrochloric acid. The solid precipitate was collected by filtration under vacuum, and the filter cake was rinsed with water and dried to give **compound 2** as an off-white solid (13.7 g, 80% purity, 99% yield).

### Synthesis of compound 3:

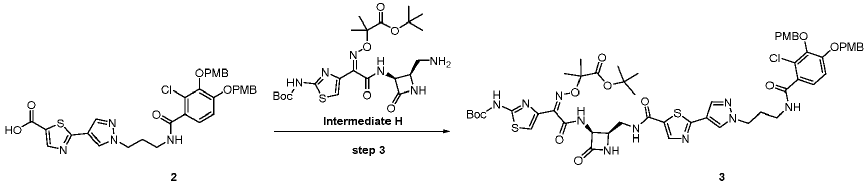

**Compound 2** (6.8 g, 10.27 mmol, 1.0 eq.) and **intermediate H** (4.1 g, 7.7 mmol, 0.75 eq.) were dissolved in a mixture of DMF/tetrahydrofuran (27 mL/40 mL). The reaction mixture was stirred at room temperature under nitrogen for 1 h. After the reaction was completed, water and ethyl acetate were added to the system. The aqueous phase was separated and reversely extracted with ethyl acetate, and the organic phases were combined, reversely washed with water, and concentrated under reduced pressure to dryness. The residue was purified by column chromatography (dichloromethane/methanol system) to give **compound 3** as a white solid (6.5 g, 97% purity, 54% yield).

### Synthesis of compound 4:

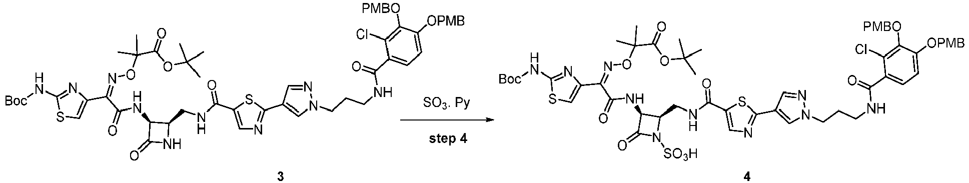

**Compound 3** (1.0 g, 0.85 mmol, 1.0 eq.) was dissolved in DMF (10 mL), and sulfur trioxide pyridine complex (1.4 g, 8.5 mmol, 10 eq.) was added. Under nitrogen, the reaction mixture was warmed to 45 °C and stirred overnight. After the reaction was completed, water and ethyl acetate were added to the system. The aqueous phase was separated and reversely extracted with ethyl acetate, and the organic phases were combined, reversely washed with water, and concentrated under reduced pressure to dryness. The residue was purified by column chromatography (dichloromethane/methanol system) to give **compound 4** as a white solid (0.8 g, 97% purity, 72% yield).

### Synthesis of compound 1-42:

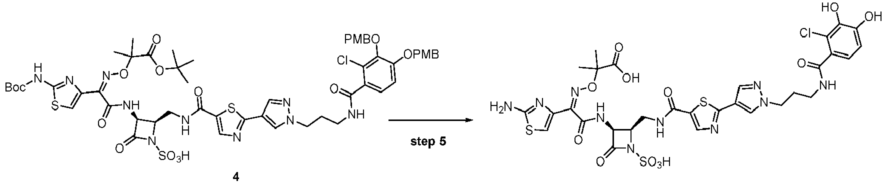

**Compound 4** (1.0 g, 0.81 mmol, 1.0 eq) was dissolved in dichloromethane (24 mL), and the reaction system was purged 3 times with nitrogen and then cooled to below -20 °C. A solution of BCl₃.DCM was slowly added dropwise, with the internal temperature not higher than -20 °C. After addition, the reaction mixture was stirred at -20 °C for 1 h. After the reaction was completed, solid sodium bicarbonate (6.4 g) was added in batches at -20 °C to the system, followed by the addition of water (1 g) and then the slow addition of methyl *tert-butyl* ether (60 mL). The mixture was stirred at -5 °C for another 30 min and filtered under nitrogen. The filter cake was rinsed with a small amount of methyl *tert-butyl* ether and blow-dried with nitrogen. The solid was slurried at room temperature with methyl *tert-butyl* ether (8 mL)/methanol (4 mL) for 1 h, and the slurry was filtered. The filter cake was blow-dried with nitrogen, and the resulting crude product was purified by preparative high-pressure liquid chromatography to give **compound I-42** (288 mg, 95% purity, 41% yield). (ES+), [M+H]⁺ = 856; ¹H NMR (400 MHz, DMSO-*d*₆): δ (ppm) 9.46 (d, *J* = 8.5 Hz, 1H), 8.48 (s, 1H), 8.43 (dd, *J* = 7.0, 3.4 Hz, 1H), 8.25 (t, *J* = 5.7 Hz, 1H), 8.13 (s, 1H), 8.02 (s, 1H), 5.28 (dd, *J* = 8.5, 5.6 Hz, 1H), 4.26 (t, *J* = 6.8 Hz, 2H), 4.23-4.09 (m, 1H), 3.76 (dt, *J* = 12.9, 6.5 Hz, 1H), 3.52 (ddd, *J* = 13.9, 8.3, 3.3 Hz, 1H), 3.22 (q, *J* = 6.4 Hz, 2H), 2.06 (t, *J* = 6.8 Hz, 2H), 1.51 (d, *J* = 10.4 Hz, 6H).

### Example 43. Synthesis of Compound I-43

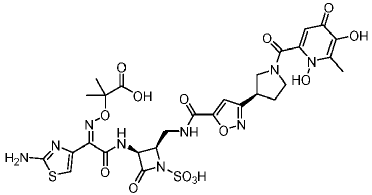

### Synthesis of compound 1:

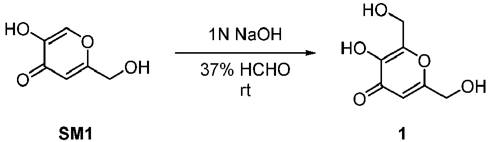

In a 3 L four-necked flask, NaOH (96.8 g, 2.32 mol) was added and completely dissolved in water (2200 mL) by magnetic stirring, and **SM1** (300 g, 2.11 mol) was added at room temperature and completely dissolved by stirring. The mixture was stirred in an ice-water bath, and yellow insoluble matter precipitated. 37% HCHO (197 g, 2.43 mol) was added dropwise over 25 min. After addition, The reaction mixture was naturally warmed to room temperature and stirred overnight. The starting material **SM1** was completely consumed as indicated by TLC (DCM/MeOH = 10/1, UV 254 nm). The reaction mixture was cooled in a low-temperature bath at -5 °C and mechanically stirred. The pH was adjusted to about 1 by dropwise adding concentrated hydrochloric acid (219 mL, 2.628 mol). After addition, the stirring at low temperature was continued, and insoluble matter precipitated. The mixture was stirred at -5 °C for another 1.5 h and filtered under vacuum. The filtrate was separately collected. The filter cake was washed with petroleum ether (250 mL × 2) and dried under vacuum overnight to give a pale yellow solid (268 g). The aqueous filtrate was concentrated to precipitate insoluble matter. The mixture was stirred in an ice water bath for 1 h and filtered under vacuum. The filter cake was washed with petroleum ether (250 mL × 2), collected, and dehydrated by rotary evaporation to give compound **1** as a yellow solid (81.7 g, 96% yield). MS = 172.81(M+H)⁺.

### Synthesis of compound 2:

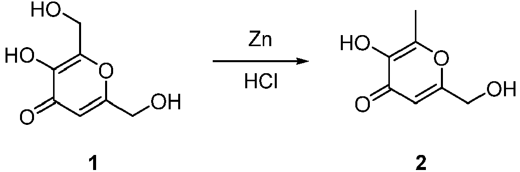

In a 3 L four-necked flask, compound **1** (349.7 g, 2.03 mol) was added and mechanically stirred in water (1170 mL), not completely dissolved. Zinc powder (266 g, 4.06 mol) was added at room temperature, and concentrated HCl (609 mL, 7.31 mol) was added dropwise, with the internal temperature gradually increasing. The mixture was heated in a water bath at 40 °C, with the internal temperature at 40-60 °C during the dropwise addition. The dropwise addition of concentrated hydrochloric acid was completed in about 1.5 h. Immediate LC-MS monitoring indicated that compound 1 had been consumed. The reaction mixture was cooled and then filtered under vacuum through filter-paper, and the filter cake was washed with water (50 mL). The filtrate was transferred to a 3 L four-necked flask, and stirred in a cooling bath at -5 °C. After 40 min, insoluble matter precipitated. The stirring was performed under cooling for a total of 3 h, and then the mixture was filtered under vacuum overnight. The filter cake was collected and dehydrated by rotary evaporation to give **compound 2** as a yellow solid (144 g, 45% yield). MS = 156.81 (M+H)⁺.

### Synthesis of compound 3:

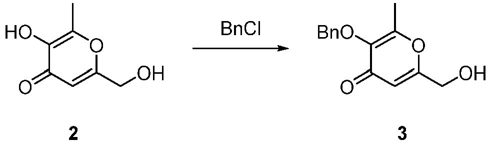

In a 3 L four-necked flask, potassium carbonate (178 g, 1.287 mol) was added and completely dissolved in water (804 mL) by mechanical stirring. **Compound 2** (134 g, 0.858 mol) and methanol (1876 mL) were added at room temperature, and BnCl (119.5 g, 0.944 mol) was added dropwise at room temperature over 10 min, with the internal temperature not significantly increasing. The mixture was stirred at room temperature (25 °C) overnight. The reaction mixture was transferred to a single-necked flask and concentrated to remove methanol. Ice water was added to the residue until the total volume reached about 3500 mL. The precipitate was an oil. The mixture was extracted with ethyl acetate (800 mL × 3), and the organic phase was dried over anhydrous sodium sulfate and filtered. The filtrate was concentrated to dryness by rotary evaporation to give **compound 3** as a brown oil (192 g, 91% yield). MS = 246.92 (M+H)⁺.

### Synthesis of compound 4:

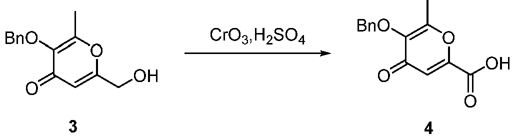

In a 500 mL four-necked flask A, chromium trioxide (13.76 g, 0.138 mol) was added and completely dissolved in water (19 mL) by stirring, and the mixture was cooled to -10 °C. Concentrated sulfuric acid (12.1 mL) was added dropwise, and insoluble matter precipitated. The mixture was diluted with water (8.5 mL), and the stirring was continued at -10 °C.

In another 500 mL four-necked flask B, **compound 3** (22.6 g, 0.092 mol) was added and completely dissolved in acetone (465 mL). The mixture in flask A was slowly added dropwise to flask B in an ice-salt bath at -5 °C over 20 min, with the internal temperature maintained at < 10 °C. After addition, the mixture was naturally warmed to room temperature and stirred overnight. The reaction mixture was transferred to a single-necked flask and concentrated by rotary evaporation to remove acetone. Water (1000 mL) was added to the residue, and a yellow viscous oil precipitated. The mixture was extracted with ethyl acetate (200 mL × 3). The organic phase was dried over anhydrous sodium sulfate overnight and filtered, and the filtrate was concentrated by rotary evaporation to give a yellow solid (20.7 g). The solid was stirred and slurried with *n*-pentane/dichloromethane (3/1, 150 mL) for 6 h, and the slurry was filtered under vacuum. The filter cake was washed with *n*-pentane/dichloromethane (3/1, 20 mL × 2), collected, and dried by rotary evaporation to give **compound 4** as a yellow solid (11.5 g, 48% yield). MS = 261.01 (M+H)⁺.

### Synthesis of compound 5:

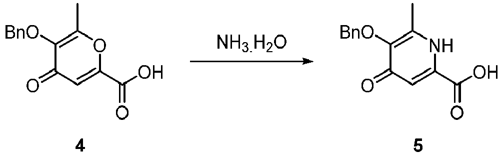

To a 200 mL glass tube were added **compound 4** (11.5 g) and 25% ammonia solution (92 mL). After the tube was sealed, the mixture was stirred at room temperature for 1 h, then warmed to 80 °C, stirred for 10 h, and naturally cooled to room temperature. The reaction mixture was washed 3 times with ethyl acetate (80 mL × 3), with insoluble flocculent matter present, and the organic phase was discarded. Dichloromethane (80 mL × 1) was added to the aqueous phase, and the mixture was stirred and then filtered under vacuum to remove the insoluble matter. The aqueous phase was separated, washed with dichloromethane (80 mL × 2), adjusted to about pH 4 by adding concentrated hydrochloric acid under stirring, cooled to room temperature, and filtered under vacuum overnight to give **compound 5** as a light gray solid (the filter cake, 11.9 g, 85.8% yield). MS = 259.90 (M+H)⁺.

### Synthesis of compound 6:

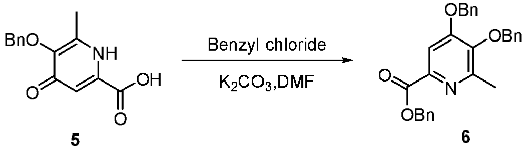

Benzyl chloride (6.1 g, 48 mmol) was added dropwise to **compound 5** (5 g, 19 mmol) and potassium carbonate (6.66 g, 48 mmol) in *N*,*N*-dimethylformamide (80 mL). The reaction mixture was reacted at 80 °C overnight and cooled to room temperature. Water was added, and the mixture was extracted twice with ethyl acetate. The organic phase was dried over anhydrous sodium sulfate and concentrated to dryness by rotary evaporation. The residue was purified by normal-phase silica gel column chromatography to give **compound 6** as a white solid (6 g, 97% yield). LCMS: [M+H]+ = 440.01.

### Synthesis of compound 7:

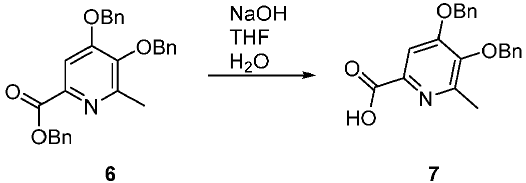

An aqueous solution of sodium hydroxide (20 mL, 1 mol/L) was added dropwise to **compound 6** (6 g, 13.6 mmol) in tetrahydrofuran (100 mL). The reaction mixture was allowed to react at room temperature for 2 h, and water (200 mL) was added thereto. The pH was adjusted to 2-3 with 1 N hydrochloric acid, and a solid precipitated. The mixture was filtered, and the filter cake was rinsed with water (3 × 25 mL) and dried to give **compound 7** as a white solid (5 g, 100% yield). LCMS: [M+H]⁺= 350.16.

### Synthesis of compound 8:

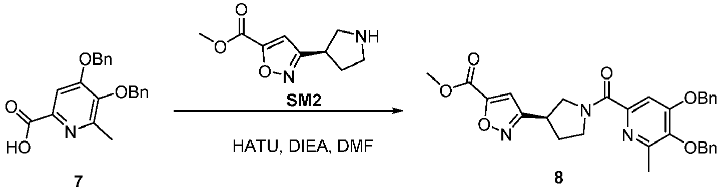

To *N*,*N*-dimethylformamide (250 mL) were added **compound 7** (2.5 g, 7.1 mmol), **SM2** (1.7 g, 7.1 mmol), HATU (3.5 g, 9.23 mmol) and DIEA (2.8 g, 22 mmol). The reaction mixture was allowed to react at room temperature for 2 h. Water (200 mL) was added to the system, and a large amount of viscous matter precipitated. The supernatant was removed, and the viscous matter was dried under vacuum to give **compound 8** as a crude product (5 g, 92% purity, 100% yield). LCMS: [M+H]+ = 528.07.

### Synthesis of compound 9:

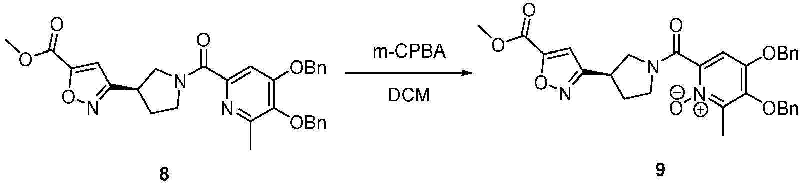

*m*-CPBA (7 g, 33 mmol) was dissolved in dichloromethane (60 mL), and the solution was added dropwise to a solution of **compound 8** (6 g, 11 mmol) in dichloromethane (60 mL) in an ice bath. The reaction mixture was allowed to react at room temperature for 2 h. Dichloromethane (200 mL) and saturated sodium bicarbonate (200 mL) were added to the reaction mixture. The organic phase was washed with sodium chloride (20 mL) and concentrated to dryness by rotary evaporation. The residue was purified by normal-phase column chromatography (DCM : MeOH = 10:1) to give **compound 9** as a white solid (4 g, 95% purity, 85.7% yield). LCMS: [M+H]+ = 544.12.

### Synthesis of compound 10:

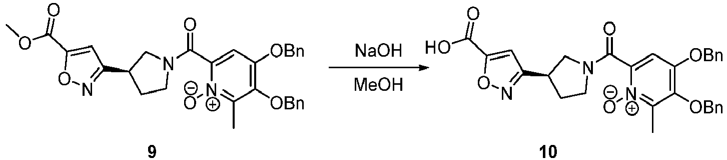

**Compound** 9 (4 g, 7.3 mmol) was dissolved in a solution of methanol (40 mL), and a 2 M solution of lithium hydroxide (20 mL) was added at room temperature. The reaction mixture was stirred at room temperature for 1 h, and the reaction was completed under intermediate control. The reaction mixture was concentrated under reduced pressure to remove the organic solvent, and 20 mL of water was added. The pH was adjusted to 2 with 2 M hydrochloric acid, and a solid was precipitated in large quantities. The solid was collected by filtration and dried to give **compound 10** as a crude product (3.6 g, 92% purity, 92% yield). LCMS: [M+H]⁺ = 530.05.

### Synthesis of compound 11:

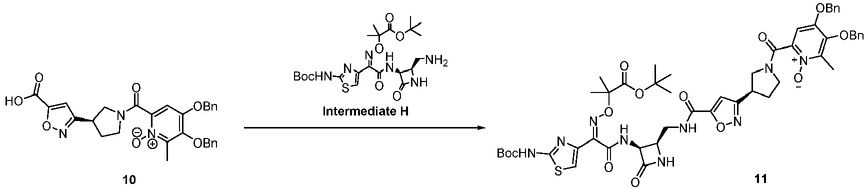

**Compound 10** (3.6 g, 6.8 mmol) and **intermediate H** (3.6 g, 6.8 mmol) were dissolved in a solvent mixture of DMF/THF (20 mL/20 mL), and DMTMM (2.7 g, 8.8 mmol) was added. The reaction mixture was stirred at room temperature under nitrogen for 2 h, and the reaction was completed under intermediate control. Water (400 mL) was added to the system, and a large amount of viscous matter precipitated. The supernatant was removed, and the viscous matter was dissolved in dichloromethane. The organic phase was concentrated to dryness by rotary evaporation, and the residue was purified by normal-phase column chromatography (dichloromethane/methanol system) to give **compound 11** (5 g, 95% purity, 70% yield) as a pale yellow solid. LCMS: [M+H]+ = 1038.33.

### Synthesis of compound 12:

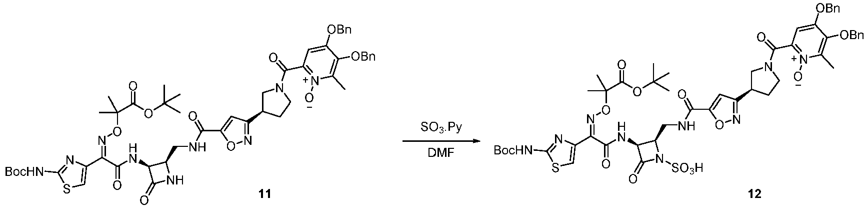

**Compound 11** (2.5 g, 2.4 mmol) was dissolved in DMF (50 mL), and sulfur trioxide pyridine complex (5.7 g, 36 mmol) was added. Under nitrogen, the mixture was warmed to 45 °C and stirred overnight, and the reaction was completed under intermediate control. Water (200 mL) was added to the system, and a solid precipitated in large quantities. The solid was collected by filtration and dried to give a crude product, which was then purified by normal-phase column chromatography (DCM/MeOH system) to give **compound 12** as a pale yellow solid (2.3 g, 95% purity, 85% yield). LCMS: [M+H]+ = 1118.30.

### Synthesis of compound I-43:

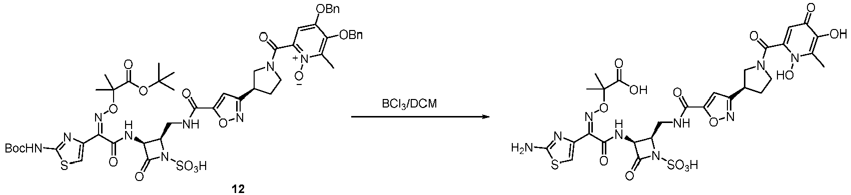

**Compound 12** (2.2 g, 1.9 mmol) was dissolved in dichloromethane (45 mL), and the reaction flask was purged 3 times with nitrogen. The system was cooled to below -20 °C. A solution of BCl₃.DCM (20 mL) was slowly added dropwise, with the internal temperature not higher than -20 °C. After addition, the reaction mixture was stirred at -20 °C for 1 h, and the reaction was completed under intermediate control. Solid sodium bicarbonate (6.4 g) was added in batches at -20 °C to the system, followed by the addition of water (2 g) and then the slow addition of methyl *tert-butyl* ether (120 mL). The mixture was stirred for another 30 min at a temperature not higher than -5 °C and filtered under nitrogen. The filter cake was rinsed with a small amount of methyl *tert-butyl* ether and then blow-dried with nitrogen. The solid was slurried at room temperature with methyl *tert-butyl* ether (20 mL)/methanol (20 mL) for 1 h, and the slurry was filtered. The filter cake was blow-dried with nitrogen to give a solid crude product (11 g), 3.5 g of which was purified by preparative high-pressure liquid chromatography to give purified **compound I-43** (270 mg, 98.1% purity, 50% yield). LCMS: [M+H]+ = 782.19. ¹H NMR (CD₃OD-*d*₄, 400 MHz): δ (ppm) 1.43-1.45 (m, 6H), 2.08-2.13 (m, 1H), 2.34-2.37 (m, 1H), 2.42 (s, 3H), 3.40-3.52 (m, 3H), 3.54-3.70 (m, 4H), 4.17-4.22 (m, 1H), 5.22-5.24 (m, 1H), 6.85 (s, 1H), 6.99 (s, 1H), 7.06 (s, 1H).

### Example 44. Synthesis of Compound I-44

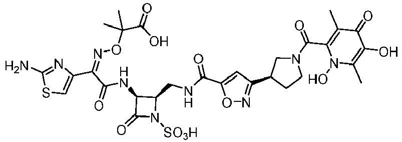

### Synthesis of compound 1:

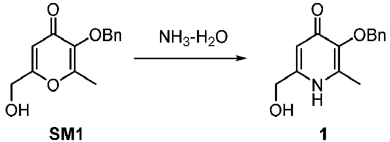

Compound SM1 (10 g, 40.61 mmol), 25% ammonia solution (60 mL, 6 V) was added to a glass tube, which was then sealed. The mixture was warmed to 80 °C in an oil bath, stirred for 12 h, and cooled to room temperature. The reaction was completed as indicated by LC-MS. The reaction mixture was transferred to a flask and concentrated under reduced pressure for dehydration. The solid residue was stirred and slurried with acetonitrile (40 mL) for 30 min, and the slurry was filtered under vacuum. The filter cake was washed with acetonitrile (15 mL × 2), collected, and dried to give **compound 1** as an ocher yellow solid (7.6 g, 76% yield). MS = 246.1 (M+H)⁺.

### Synthesis of compound 2:

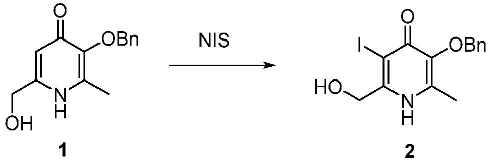

**Compound 1** (7.1 g, 28.95 mmol) was suspended in acetonitrile (75 mL), and NIS (7.16 g, 31.84 mmol) was added at room temperature. The reaction mixture was stirred at room temperature overnight for 17 h and filtered under vacuum. The filter cake was washed with acetonitrile (10 mL × 3) and collected to give **compound 2** as a yellow solid (9.6 g, 89% yield). MS = 371.8 (M+H)⁺.

### Synthesis of compound 3:

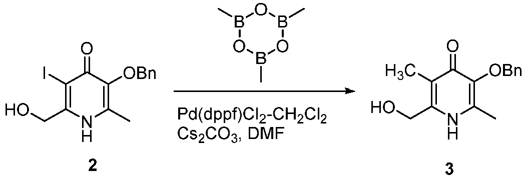

**Compound 2** (6.6 g, 17.78 mmol), cesium carbonate (17.4 g, 53.34 mmol), Pd(dppf)Cl₂-CH₂Cl₂ (1.3 g, 1.78 mmol), DMF (130 mL) and trimethylboroxine (3.5 M, 15 mL) were added to a reaction flask. The reaction mixture was stirred in an oil bath at 100 °C under nitrogen overnight for 16 h, concentrated under reduced pressure by using an oil pump to remove DMF, and concentrated with 40 g of silica gel to dryness by rotary evaporation. The residue was purified by column chromatography (220 g of silica gel, 0-25% (dichloromethane/methanol = 2/1)/DCM) to give compound 3 as a brown solid (2.4 g, 52% yield). MS = 260.14(M+H)⁺.

### Synthesis of compound 4:

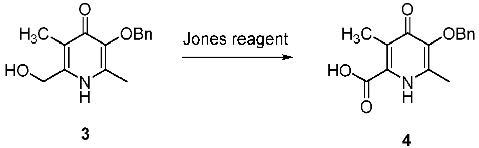

In a reaction flask A, chromium trioxide (1.94 g, 19.44 mmol) was added and completely dissolved in water (2.7 mL), and the solution was stirred in a dry ice-ethanol bath (the external temperature was at -30 °C). Concentrated sulfuric acid (1.7 mL, 31.92 mmol) was slowly added dropwise, and insoluble matter precipitated. Water (1.2 mL) was slowly added dropwise to dilute the mixture for later use.

In a reaction flask B, **compound 3** (2.8 g, 10.80 mmol) and acetone (150 mL) were added, and the mixture was stirred in a dry ice-ethanol bath (the external temperature was at -10 °C). The oxidizing agent in flask A was slowly added to flask B. After addition, the mixture was naturally warm to room temperature, stirred for 1 h, concentrated under reduced pressure to remove the solvent, and concentrated under reduced pressure by using an oil pump to remove the remaining small amount of water to give **compound 4** as a crude product (2.95 g), which was directly used in the next step.

### Synthesis of compound 5:

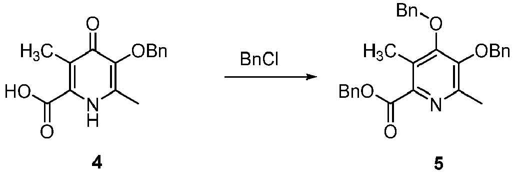

**Compound 4** (2.95 g, 10.80 mmol) was dissolved in *N*,*N*-dimethylformamide (50 mL), and potassium carbonate (9.0 g, 64.80 mmol) and benzyl chloride (4.1 g, 32.40 mmol) were added at room temperature. The reaction mixture was stirred at 80 °C overnight. Methyl *tert-*butyl ether (100 mL) was added at room temperature to the reaction mixture, which was then stirred for 15 min and filtered under vacuum to remove insoluble matter. Water (250 mL) was added to the filtrate, and the aqueous phase was separated and extracted with methyl *tert-butyl* ether (100 mL × 2). There was still a product remaining in the aqueous phase. Saturated brine (100 mL) was added to the aqueous phase, and the mixture was extracted with methyl *tert-butyl* ether (100 mL × 2). The organic phases were combined and dried over anhydrous sodium sulfate. 9 g of silica gel was added to the filtrate, and the mixture was concentrated under reduced pressure to dryness. The residue was purified by column chromatography (40 g of silica gel, 0-20% ethyl acetate/petroleum ether) to give **compound 5** as a pale yellow oil (2.8 g, 57% yield for two steps). MS = 454.3 (M+H)⁺.

### Synthesis of compound 6:

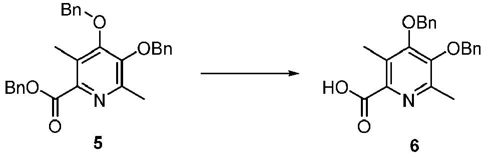

**Compound 5** (2.8 g, 6.17 mmol) was dissolved in tetrahydrofuran (40 mL), and a 1.0 M solution of sodium hydroxide [(321 mg, 8.03 mmol) + water (8 mL)] was added at room temperature. The reaction mixture was stirred at room temperature overnight. The pH of the reaction mixture was adjusted to about 5-6 with 1 M hydrochloric acid, and no solid precipitated. Water (50 mL) was added, and the mixture was extracted with dichloromethane (50 mL × 4). The organic phases were combined and dried over anhydrous sodium sulfate. The filtrate was concentrated under reduced pressure to dryness, and the residue was purified by column chromatography (20 g of silica gel, 0-25% (dichloromethane/methanol = 2/1)) to give **compound 6** as a pale yellow oil (2.04 g, 91% yield), which became a waxy solid after being dried under vacuum overnight. MS = 364.2 (M+H)⁺.

### Synthesis of compound 7:

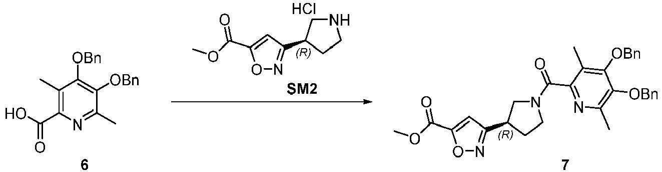

To a reaction vessel were added **compound 6** (1.136 g, 3.13 mmol), **SM2** (700 mg, 3.01 mmol), *N*,*N*-dimethylformamide (20 mL) and *N*,*N*-diisopropylethylamine (1.41 g, 10.91 mmol). The mixture was stirred in an ice-water bath, and HATU (1.25 g, 3.29 mmol) was added. The reaction mixture was naturally warmed to room temperature and stirred for 1.5 h, and the reaction was completed as indicated by LC-MS. Another tried reaction (142 mg, 1310A3) was combined with this reaction. Water (200 mL) was added, and the mixture was extracted with MTBE (50 mL × 2). The organic phases were combined, dried over anhydrous sodium sulfate, and filtered. The filtrate was concentrated under reduced pressure to dryness, and the residue was purified by column chromatography (20 g of silica gel, 0-100% ethyl acetate/petroleum ether) to give **compound 7** as a pale brown oil (1.4 g, 83% yield). MS = 542.2 (M+H)⁺.

### Synthesis of compound 8:

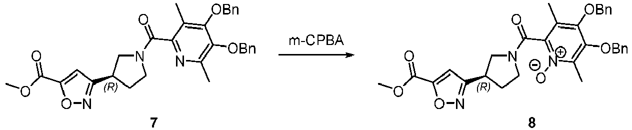

**Compound 7** (1.4 g, 2.585 mmol) was added to a reaction flask, followed by the addition of dichloromethane (24 mL). A solution of m-CPBA (900 mg, 4.433 mmol) in dichloromethane (24 mL) was added dropwise with stirring in an ice-water bath over 5 min. The reaction mixture was naturally warmed to room temperature and stirred for 1.5 h. After the reaction was completed, the system was concentrated under reduced pressure, and the residue was purified by column chromatography (40 g of silica gel, 0-25% (ethyl acetate/methanol = 2/1)) to give **compound 8** as a colorless oil (1.2 g, 82% yield). MS = 558.1 (M+H)⁺.

### Synthesis of compound 9:

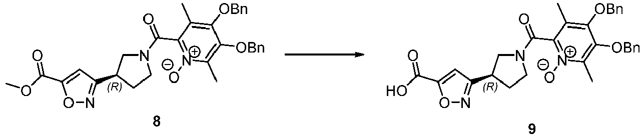

**Compound 8** (1.2 g, 2.15 mmol) was added to a reaction flask, and methanol (30 mL) was added. 2 M sodium hydroxide (5 mL, 10 mmol) was added with stirring at room temperature. The reaction mixture was stirred at room temperature for 30 min. After the reaction was completed, the reaction mixture was concentrated under reduced pressure to remove methanol. Water (40 mL) was added, and the mixture was washed with methyl *tert-butyl* ether (50 mL). Saturated brine (10 mL) was added, and the mixture was let stand for 30 min. The aqueous phase was separated and concentrated under reduced pressure to remove methyl *tert-butyl* ether. The pH of the remaining aqueous phase was adjusted to about 4 with an aqueous solution of citric acid (3 g of citric acid + 15 mL of water), and insoluble matter precipitated in clusters. With the flask open to the air, the mixture was stirred at room temperature for 45 min to disperse the precipitate and filtered under vacuum. The filter cake was washed with water until the washing water was near neutral, then washed with petroleum ether (70 mL), dried under vacuum, collected, and dehydrated under reduced pressure by using an oil pump to give **compound 9** as a pale yellow solid (1.02 g, 87% yield). MS = 544.0 (M+H)⁺.

### Synthesis of compound 10:

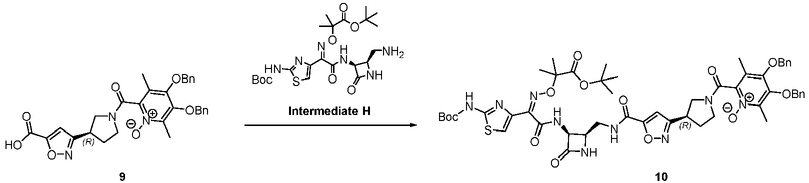

Intermediate H (910 mg, 1.729 mmol) and **compound 9** (920 mg, 1.692 mmol) were added to a reaction flask. Under nitrogen, tetrahydrofuran/DMF (16 mL/10 mL) was added, and DMTMM (544 mg, 1.846 mmol) was added with stirring at room temperature. The reaction mixture was stirred overnight. After the reaction was completed, the reaction mixture was concentrated under reduced pressure to remove THF and DMF. The residue was dissolved in dichloromethane/methanol (10/1), and silica gel was added. The mixture was concentrated to dryness by rotary evaporation, and the residue was purified by column chromatography (40 g of silica gel, 0-27% (dichloromethane/methanol = 2/1)) to give **compound 10** as a yellow solid (2.0 g). MS = 1052.3 (M+H)⁺.

### Synthesis of compound 11:

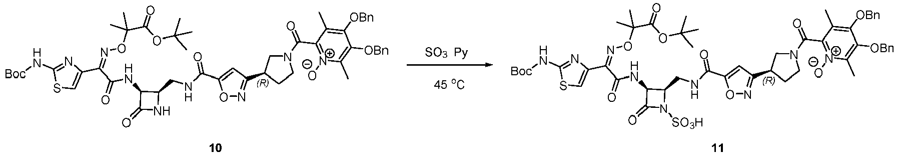

**Compound 10** (1.19 g, 1.13 mmol) was added to a reaction flask, and DMF (24 mL) was added. The mixture was stirred in an ice-water bath under nitrogen. Sulfur trioxide-pyridine complex (2.88 g, 18.10 mmol) was added in one batch. The reaction mixture was stirred in the ice-water bath for 10 min and then stirred at 45 °C overnight. After the reaction was completed, water (250 mL) was added to the reaction mixture, which was then extracted with ethyl acetate (60 mL × 3). The organic phases were combined and washed with water (50 mL) and saturated brine (10 mL). The separation into layers was slow. Part of the aqueous phase was separated and then washed with water (50 mL) and saturated brine (30 mL). The separation into layers was slow. Part of the aqueous phase was separated and then washed with saturated brine (50 mL × 2). The organic phase was dried over anhydrous sodium sulfate, and the filtrate was concentrated under reduced pressure to dryness. The residue was purified by column chromatography (20 g of silica gel, 0-27% (dichloromethane/methanol = 2/1)) to give **compound 10** as a yellow solid (642 mg). Another column chromatography purification (40 g of silica gel, 0-27% (dichloromethane/methanol = 2/1)) was performed to give **compound 11** as a yellow solid (409 mg, 32% yield). MS = 1132.3 (M+H)⁺.

### Synthesis of compound 1-44:

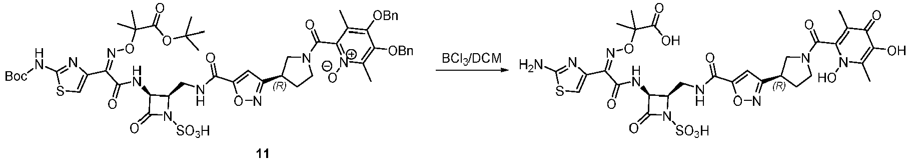

To a 50 mL three-necked flask internally provided with a thermometer was added with **compound 11** (409 mg, 1.236 mmol), and dichloromethane (9 mL) was added under nitrogen for complete dissolution. The solution was stirred in a cooling dry ice-ethanol bath, with the internal temperature at -30 °C. A 1.0 M solution of boron trichloride in dichloromethane (3.6 mL, 3.6 mmol) was added dropwise over 10 min, with the internal temperature controlled at no higher than -25 °C, and pale brown insoluble matter precipitated. The mixture was stirred at low temperature for another 30 min. After the reaction was completed, with the internal temperature maintained at -35 °C, solid sodium bicarbonate (2.45 g) was added in one batch to the reaction flask. The internal temperature was raised to -30 °C, and water (400 mL) was added dropwise. The mixture was gradually warmed to -5 °C-0 °C and stirred for 30 min. The temperature was reduced to -20 °C, and methyl *tert-butyl* ether (25 mL) was added dropwise. The internal temperature was controlled at below 0 °C, and the mixture was stirred for 10 min and filtered under vacuum and nitrogen. The filter cake was rinsed with methyl *tert-butyl* ether (20 mL), dried under vacuum, collected, and slurried under nitrogen with methyl *tert-butyl* ether/ethanol (1/1, 5 mL) for 30 min. The slurry was filtered under vacuum and nitrogen, and the filter cake was rinsed with methyl *tert-butyl* ether, dried under vacuum, and collected to give a salt-containing crude product (2.55 g), which was then purified by preparative high-pressure HPLC. The eluate was collected and directly lyophilized overnight to give **compound I-44** as an off-white solid (151 mg). MS = 796.10 (M+H)⁺, HPLC: 97.1%, ¹HNMR (DMSO-*d*₆, 400MHz): δ (ppm) 9.39 (dd, 1H), 8.72-8.64 (m, 1H), 7.17-7.03 (m, 1H), 6.80 (dd, 1H), 5.28-5.23 (m, 1H), 4.19-4.12 (m, 1H), 4.02-3.20 (m, 7H), 2.48-2.28 (m, 4H), 2.24-2.00 (m, 4H), 1.51-1.35 (m, 6H).

### Example 45. Synthesis of Compound I-45

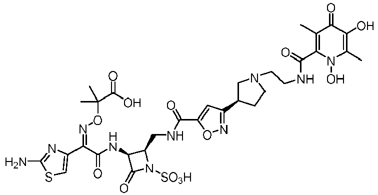

### Synthesis of compound 1:

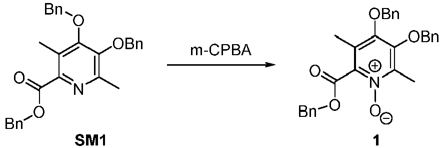

Compound **SM1** (2.2 g, 4.85 mmol) was added to a reaction flask, and dichloromethane (45 mL) was added. *m*-CPBA (1.97 g, 9.70 mmol) was added at room temperature. The reaction mixture was stirred at room temperature overnight, and insoluble matter precipitated. After the reaction was completed, 6 g of silica gel was added. The mixture was concentrated under reduced pressure to dryness, and the residue was purified by column chromatography (80 g of silica gel, dichloromethane/methanol = 10/1) to give **compound 1** as a pale yellow oil (2.29 g, 100% yield). MS = 470.2 (M+H)⁺.

### Synthesis of compound 2:

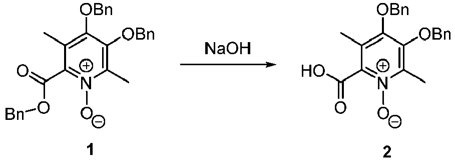

**Compound 1** (2.29 g, 4.88 mmol) was dissolved in tetrahydrofuran (35 mL) and methanol (35 mL), and a 2 M solution of sodium hydroxide (22 mL) was added at room temperature. The reaction mixture was stirred at room temperature at 20 °C for 2 d. After the reaction was completed, the reaction mixture was concentrated under reduced pressure to remove tetrahydrofuran and methanol. Water (20 mL) was added to the residue, and the mixture was stirred in a cold water bath. The pH was adjusted to 1-2 with 1 M hydrochloric acid, and insoluble matter precipitated. The mixture was stirred for 1 h and filtered under vacuum. The filter cake was washed with water until the washing water was near neutral, then collected, and dehydrated under reduced pressure by using an oil pump to give **compound 2** as an off-white solid (1.7 g, 92% yield). MS = 380.2 (M+H)⁺.

### Synthesis of compound 3:

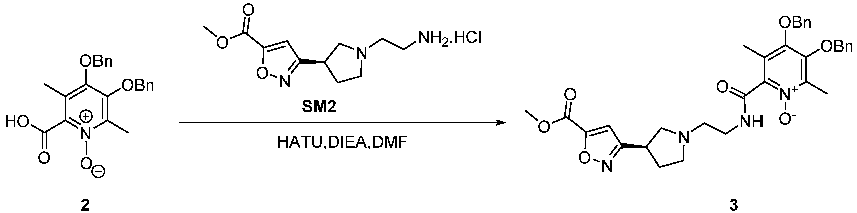

**Compound 2** (1.7 g, 4.48 mmol) was added to a reaction flask and completely dissolved in DMF (30 mL), and the solution was stirred in an ice-water bath. *N*,*N*-diisopropylethylamine (1.5 g, 11.61 mmol) and HATU (2.05 g, 5.38 mmol) were added, and the mixture was stirred in an ice-water bath for 30 min. A mixture of **SM2** (1.36 g, 4.93 mmol), *N,N-*diisopropylethylamine (1.5 g, 11.61 mmol) and DMF (20 mL) was slowly added dropwise at room temperature under nitrogen over 2.5 h. After addition, the reaction mixture was stirred at room temperature. After the reaction was completed, the reaction mixture was concentrated under reduced pressure to remove DMF to give a brown oil. Water (100 mL) and a saturated aqueous solution of sodium bicarbonate (100 mL) were added, and the mixture was extracted with ethyl acetate (100 mL × 3). The organic phases were combined, washed with a saturated solution of sodium chloride (100 mL), and dried over anhydrous sodium sulfate. The filtrate was concentrated under reduced pressure to dryness, and the residue was purified by column chromatography (80 g of silica gel, 0-50% (dichloromethane/methanol = 10/1)) to give **compound 3** as a pale brown oil (1.76 g, 65% yield). MS = 601.3 (M+H)⁺.

### Synthesis of compound 4:

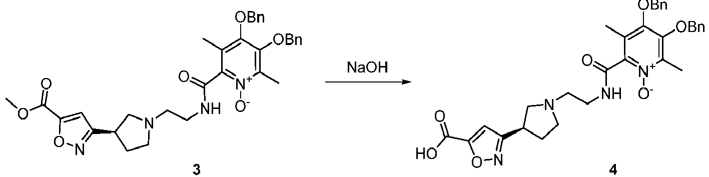

**Compound 3** (1.76 g, 2.93 mmol) was added to a reaction flask, and tetrahydrofuran (30 mL) and methanol (30 mL) were added. A 2 M solution of sodium hydroxide (6 mL, 12 mmol) was added with stirring at room temperature. The reaction mixture was stirred at room temperature for 45 min and concentrated under reduced pressure to remove tetrahydrofuran and methanol. Water (100 mL) and a saturated aqueous solution of sodium chloride (50 mL) were added. The mixture was washed with methyl *tert-butyl* ether (100 mL), and liquid separation was performed twice. The aqueous phases were combined, adjusted to pH 6.2-6.4 with a 1 M solution of hydrochloric acid, extracted with dichloromethane/methanol (10/1, 200 mL), and let stand for a long time, and then liquid separation was performed. The aqueous phase was extracted with ethyl acetate (200 mL × 3), and the organic phases were combined and dried over anhydrous sodium sulfate. The filtrate was concentrated under reduce pressure to give **compound 4** as a pale brown oil (1.4 g, 81% yield). MS = 587.2 (M+H)⁺.

### Synthesis of compound 5:

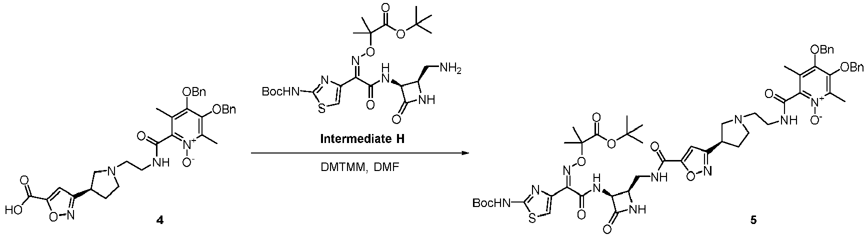

**Compound 4** (1.53 g, 2.61 mmol) was added to a reaction flask, and tetrahydrofuran/DMF (24 mL/15 mL) was added. **Intermediate H** (1.14 g, 2.169 mmol) and DMTMM (767 mg, 2.603 mmol) were added with stirring at room temperature. The reaction mixture was stirred at room temperature under nitrogen for 1.5 h. After the reaction was completed, the reaction mixture was concentrated under reduced pressure to remove tetrahydrofuran and DMF. The residue was dissolved in dichloromethane/methanol (10/1), and 6 g of silica gel was added. The mixture was concentrated under reduced pressure to dryness, and the residue was purified by column chromatography (80 g of silica gel, methanol/ethyl acetate) to give **compound 5** as a pale brown foam (1.15 g, 49% yield). MS = 1095.32 (M+H)⁺.

### Synthesis of compound 6:

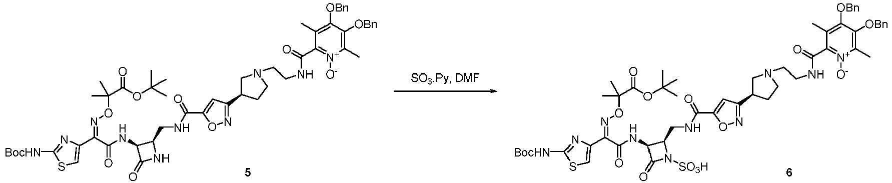

**Compound 5** (700 mg, 0.639 mmol) was added to a reaction flask, and DMF (14 mL) was added. The mixture was stirred in an ice-water bath under nitrogen. Sulfur trioxide-pyridine complex (1.63 g, 10.226 mmol) was added in one batch. Under nitrogen, the reaction mixture was stirred in the ice-water bath for 10 min and then stirred at 45 °C overnight. After the reaction was completed, ice water (50 mL) was added to the reaction mixture, and insoluble matter precipitated. The mixture was stirred for 5 min and filtered under vacuum, and the filter cake was rinsed with ice water (10 mL × 5) until the filtrate was neutral, then collected, and concentrated under reduced pressure by using an oil pump for dehydration to give a crude product, which was then purified by column chromatography (20 g of silica gel, dichloromethane/methanol = 2/1) to give **compound 6** as a pale brown foam (370 mg, 49% yield). MS = 1175.6 (M+H)⁺.

### Synthesis of compound I-45:

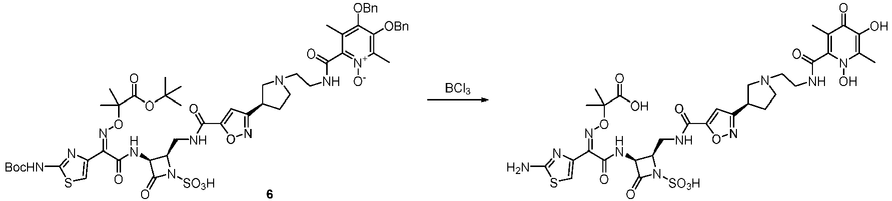

To a 50 mL three-necked flask internally provided with a thermometer was added with **compound 6** (365 mg, 0.311 mmol), and dichloromethane (8 mL) was added under nitrogen for complete dissolution. The solution was stirred in a cooling dry ice-ethanol bath, with the internal temperature at -30 °C. A 1.0 M solution of boron trichloride in dichloromethane (3.1 mL, 3.1 mmol) was added dropwise over 10 min, with the internal temperature controlled at no higher than -25 °C, and pale brown insoluble matter precipitated. The mixture was stirred at low temperature for another 20 min. After the reaction was completed, with the internal temperature maintained at -35 °C, solid sodium bicarbonate (2.34 g) was added in one batch to the reaction flask. The internal temperature was raised to -30 °C, and water (365 mg) was added dropwise. The mixture was gradually warmed to -5 °C-0 °C and stirred for 30 min. The temperature was reduced to -30 °C, and methyl *tert-butyl* ether (22 mL) was added dropwise. The internal temperature was controlled at below 0 °C, and the mixture was stirred for 10 min and filtered under vacuum and nitrogen. The filter cake was washed with methyl *tert-butyl* ether (18 mL), dried under vacuum, collected, and slurried under nitrogen with methyl *tert-butyl* ether/ethanol (1/1, 4.5 mL) for 30 min, and methyl *tert-butyl* ether (20 mL) was added. The mixture was filtered under vacuum and nitrogen, and the filter cake was rinsed with methyl *tert-butyl* ether (15 mL × 3), dried under vacuum, and collected to give a salt-containing crude product (2.4 g), which was then purified by preparative high-pressure liquid chromatography. The eluate was collected and directly lyophilized for about 48 h to give **compound I-45** as a white solid (128 mg). MS = 839.33 (M+H)⁺, HPLC: 96.7%, ¹H NMR (400 MHz, CD₃OD): δ (ppm) 7.08 (s, 1H), 6.96 (s, 1H), 5.38 (d, *J* = 5.6 Hz, 1H), 4.45 (dt, *J* = 8.0, 5.5 Hz, 1H), 4.03-3.53 (m, 11H), 2.72-2.62 (m, 1H), 2.47 (s, 3H), 2.45-2.30 (m, 1H), 2.21 (s, 3H), 1.60 (s, 6H).

### Example 46. Synthesis of Compound I-46

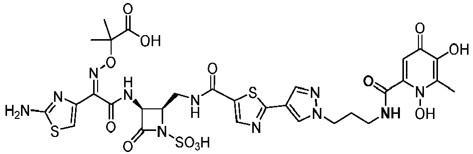

### Synthesis of compound 1:

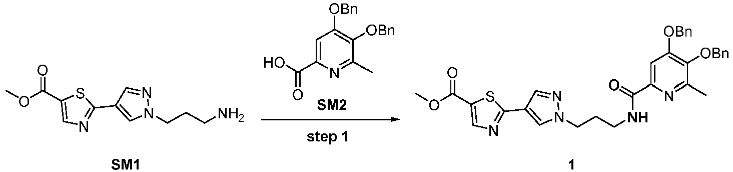

**SM1** (2.0 g, 6.7 mmol, 1.0 eq.), **SM2** (1.6 g, 4.7 mmol, 0.7 eq.) and HATU (2.8 g, 7.4 mmol, 1.1 eq.) were dissolved in DMF (20 mL), and *N*,*N*-diisopropylethylamine (2.6 g, 20.1 mmol, 3.0 eq.) was added. The reaction system was stirred at room temperature for 1 h. After the reaction was completed, water and ethyl acetate were added to the system, and the aqueous phase was separated and reversely extracted once. The organic phases were combined, reversely washed with water, and concentrated under reduced pressure to give **compound 1** as a solid (6.4 g, 90% purity).

### Synthesis of compound 2:

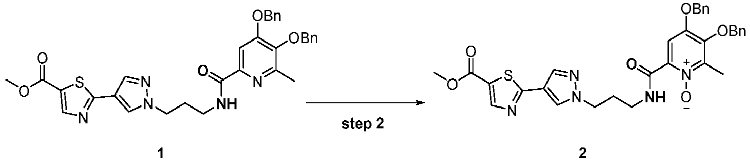

**Compound 1** (5.9 g, 9.88 mmol, 1.0 eq.) was dissolved in dichloromethane (60 mL), and a solution of *m*-CPBA (6.8 g, 39.53 mmol, 4.0 eq.) in dichloromethane (60 mL) was added in an ice bath. The reaction mixture was allowed to react at room temperature for 2 h. After the reaction was completed, a saturated solution of sodium bicarbonate (60 mL) was added to the system. The organic phase was separated, washed once with a saturated brine, and then concentrated under reduced pressure, and the resulting crude product was purified by reversed-phase column chromatography (acetonitrile/water system) and lyophilized to give **compound 2** (2.6 g, 95% purity, 42% yield).

### Synthesis of compound 3:

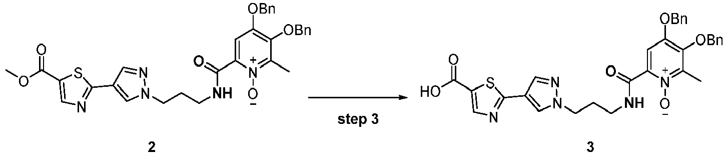

**Compound 2** (2.0 g, 3.26 mmol, 1.0 eq.) was dissolved in a solution of tetrahydrofuran/methanol (20 mL/20 mL), and a 2 M solution of lithium hydroxide (40 mL) was added at room temperature. The reaction mixture was stirred at room temperature for 1 h. After the reaction was completed, the reaction mixture was concentrated under reduced pressure to remove the organic solvent. The pH was adjusted to 3-4 with 6 M hydrochloric acid, and the mixture was extracted with ethyl acetate. The organic phase was separated, washed with water, and concentrated under reduced pressure to dryness to give **compound 3** as a yellowish solid (1.0 g, 96% purity, 51% yield).

### Synthesis of compound 4:

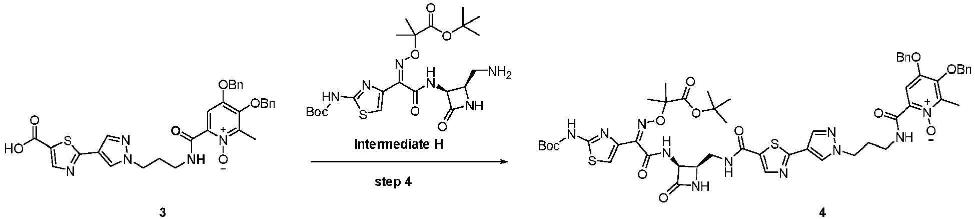

**Compound** 3 (800 mg, 1.34 mmol, 1.0 eq.) and **intermediate H** (772 mg, 1.47 mmol, 1.1 eq.) were dissolved in a mixture of DMF/tetrahydrofuran (32 mL/48 mL), and DMTMM (434 mg, 1.47 mmol, 1.1 eq.) was added. The reaction system was stirred at room temperature under nitrogen for 1 h. After the reaction was completed, water and ethyl acetate were added to the system. The aqueous phase was separated and reversely extracted with ethyl acetate, and the organic phases were combined, reversely washed with water, and concentrated under reduced pressure. The residue was purified by column chromatography (dichloromethane/methanol system) to give **compound 4** as a white solid (1.0 g, 96% purity, 66% yield).

### Synthesis of compound 5:

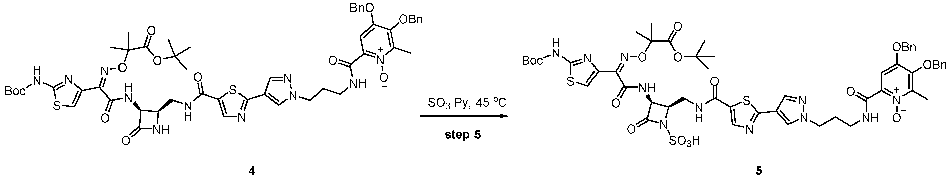

**Compound 4** (1.2 g, 1.08 mmol, 1.0 eq.) was dissolved in DMF (12 mL), and sulfur trioxide pyridine complex (2.8 g, 17.33 mmol, 16 eq.) was added. Under nitrogen, the reaction system was warmed to 45 °C and stirred overnight. After the reaction was completed, water and ethyl acetate were added to the system. The aqueous phase was separated and reversely extracted with ethyl acetate, and the organic phases were combined, reversely washed with water, and concentrated under reduced pressure. The residue was purified by column chromatography (dichloromethane/methanol system) to give **compound 5** as a white solid (1.1 g, 95% purity, 86% yield).

### Synthesis of compound I-46:

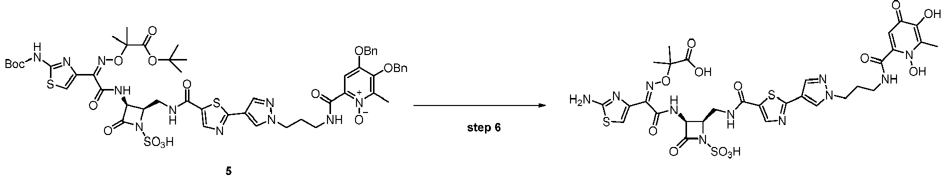

**Compound 5** (1.1 g, 0.92 mmol, 1.0 eq) was dissolved in dichloromethane (26 mL), and the reaction system was purged 3 times with nitrogen and cooled to below -20 °C. A solution of BCl₃.DCM (1 M, 13.2 mL) was slowly added dropwise, with the internal temperature not higher than -20 °C. After addition, the reaction mixture was stirred at -20 °C for 1 h. After the reaction was completed, solid sodium bicarbonate (6.4 g) was added in batches at -20 °C to the system, followed by the addition of water (1 g) and then the slow addition of methyl *tert-butyl* ether (60 mL). The mixture was stirred at -5 °C for another 30 min and filtered under nitrogen. The filter cake was rinsed with a small amount of methyl *tert-butyl* ether and blow-dried with nitrogen. The solid was slurried at room temperature with methyl *tert-butyl* ether (8 mL)/methanol (4 mL) for 1 h, and the slurry was filtered. The filter cake was blow-dried with nitrogen, and the resulting crude product was purified by preparative high-pressure liquid chromatography to give **compound I-46** (347 mg, 95.7% purity, 41% yield). (ES+), [M+H]⁺ = 852; ¹H NMR (400 MHz, DMSO-*d*₆): δ (ppm) 11.39 (t, *J* = 5.8 Hz, 1H), 9.44 (d, *J* = 8.5 Hz, 1H), 8.61-8.29 (m, 2H), 8.10 (s, 1H), 7.98 (s, 1H), 7.51 (s, 1H), 6.92 (s, 1H), 5.25 (dd, *J* = 8.5, 5.6 Hz, 1H), 4.20 (dt, *J* = 24.1, 6.1 Hz, 3H), 3.88-3.61 (m, 1H), 3.49 (ddd, *J* = 14.0, 8.5, 3.3 Hz, 1H), 3.33 (q, *J* = 6.5 Hz, 2H), 2.35 (s, 3H), 2.23-1.90 (m, 2H), 1.49 (d, *J* = 10.4 Hz, 6H).

### Biological Evaluation

### 1. Materials

**Table 1. Screening combinations**

| Bacterial species | Strain number _{[note]} |
|---|---|
| *Escherichia coli* | HDBEC 30008 |
| *Escherichia coli* | HDBEC 30016 |
| *Escherichia coli* | HDBEC 30048 |
| *Enterobacter cloacae* | HDBECL 30030 |
| *Klebsiella pneumoniae* | HDBKP 9153 |
| *Klebsiella pneumoniae* | HDBKP 9249 |
| *Klebsiella pneumoniae* | HDBKP 30006 |
| *Proteus mirabilis* | HDBPM 40 |
| *Pseudomonas aeruginosa* | HDBPA 5679 |
| *Acinetobacter baumannii* | ABA-0003YK |

Note: All of the above strains were derived from HD Biosciences (Shanghai) Co., Ltd.

### 2. Medium

Trypticase soy agar (Trypticase soy agar, TSA) (BD BBL 236950)
Cation-adjusted Mueller Hinton Broth (CAMHB) (BD BBL 212322)
Maconkey agar (MA) (Hope Bio HB6238)

### 3. Reagents and consumables

Aztreonam (MCE HY-B0129)
Disposable shake flask, 250 mL (Corning 430183)
Disposable plate, 90 mm (Nest 752001)
96-well microtiter plate (Greiner 650162)

### 4. Method

### Thawing of bacterial strains

103 bacterial strains used for the minimum inhibitory concentration test were frozen in a - 80 °C ULT freezer and thawed 2 days before use. A small amount of frozen microorganism bacteria were scraped using a sterile inoculating loop, streaked onto a suitable solid culture dish for inoculation, and then cultured for about 20 h at 35 ± 2 °C in a normal incubator.
- *Proteus mirabilis* HDBPM 40: MA, normal atmospheric environment
- The remaining 102 strains of bacteria: TSA, normal atmospheric environment

5-10 colonies with similar morphology were picked from the culture dish using a sterile inoculating loop and then re-streaked onto a corresponding solid culture dish. Then the dish was incubated in a normal incubator at 35 ± 2 °C for about 20 h.

### Preparation of inoculum bacteria

The 1.02x CAMHB was taken out of a 4 °C freezer and left to warm at room temperature.

5-10 single microbial colonies were picked from the solid culture dish, resuspended in 500 µL of sterile normal saline (0.9% NaCl), and adjusted to an OD₆₀₀ of about 0.15 with a spectrophotometer.

The bacteria were then diluted 300-fold with 1.02x CAMHB to an inoculation concentration of 2 × 10⁵ CFU/mL.

Notice:
- The prepared microbial inocula were to be inoculated into a 96-well assay plate within 15 min.
- The number of inoculated microorganisms could be obtained by plate colony counting.

### Preparation of assay plates

Assay plate patterns
- There were a total of 3 assay plate patterns, one of which was used for each assay.
- 8 rows of the assay plate (rows A-H) were for 8 test compounds, respectively. The highest test concentration of the compounds was 16, 32 or 64 µg/mL, and each compound was diluted 2-fold.
- Growth control (GC): a medium containing microbial inoculum (1.02× CAMHB) and dimethylsulfoxide (DMSO) but no compound.

Dilution of compounds:
The test and reference compounds were dissolved in DMSO or H₂O to prepare 6.4 mg/mL stock solutions. The stock solutions were diluted to prepare standby solutions with certain concentrations, and the volumes of the standby solutions were determined according to the experimental design. Corresponding volumes of the standby solutions were transferred to the starting wells of a dilution plate (A1-H1), and half the starting-well volume of DMSO to the other wells. Each compound was diluted 2-fold sequentially from column 1 to column 11 (e.g., transferring 30 µL of DMSO to columns 2-12 first, then pipetting 30 µL of the compounds from column 1 to column 2 and mixing them well, then pipetting 30 µL of the compounds from column 2 to column 3 and mixing them well, then pipetting 30 µL of the compounds from column 3 to column 4 and mixing them well, and so on to column 11).

1 or 2 µL of the compounds and DMSO were transferred from the compound plate to corresponding wells of the assay plates.

98 µL of corresponding bacterial inocula were added to the assay plates.

After addition, the assay plates were covered with sterile covers. The mixtures in the assay plates were centrifuged in a centrifuge at 800 rpm for 30 s, mixed well on a microplate shaker at 400 rpm for 1 min, and incubated in a normal incubator at 35 ± 2 °C for 20-24 h.

Notice: The dissolution of the compounds in the original tube and the assay plates after the addition of microbial inocula was carefully examined, and the occurrence of precipitates may lead to erroneous judgment. It was most desirable that the compounds be completely soluble in the solvent at each concentration.

### Colony counting

The inoculated bacteria (4-6 strains were selected) were diluted from 10⁻¹ to 10⁻³ with a liquid medium (such as 100 µL of bacterial inocula + 900 µL of 1.02x CAMHB).

100 µL of the bacterial dilution described above was spread evenly onto TSA plates, 2 replicates per dilution. After uptake of the culture medium by TSA for 10 min, the plates were incubated in an incubator upside down at 35 ± 2 °C for 24 h.

Minimum inhibitory concentration recording and colony counting

The compound management system was turned on to check whether the barcode and compound assignment of each assay plate were correct or not.

The assay plates were placed on a plate reading device, and the speculum was adjusted to observe and record the growth condition of bacteria in each well. In the mean time, each assay plate was photographed with QCount system.

The minimum inhibitory concentration of each compound was recorded by reference to guidelines from the Clinical and Laboratory Standards Institute.

The colonies formed by different dilutions of bacterial inocula on the TSA plates were counted and the bacterial load was calculated.

### Results

The minimum inhibitory concentrations of the compounds against experimental strains were tested in this study by reference to the medium microdilution method as indicated by the Clinical and Laboratory Standards Institute. Compounds were diluted two-fold in 96-well plates from the highest detection concentration of 16, 32 or 64 µg/mL. The bacterial inocula in the assay plates were thawed from corresponding medium plates and diluted in 1.02x CAMHB, while growth controls (GC wells) were set up in the assay plates. The minimum inhibitory concentration of each compound against different bacteria was observed and recorded after the assay plates were incubated in a normal incubator at 35 ± 2 °C for about 20 h. The minimum inhibitory concentration values for the different compounds are listed in the list below.

**Table 2. MIC values of the compounds of the present invention against bacterial strains (µg/mL)**

| Compound | HDBEC 30008 | HDBEC 30016 | HDBEC 30048 | HDBECL 30030 | HDBKP 9153 | HDBKP 9249 | HDBKP 30006 | HDBPM 40 | HDBPA 5679 |
|---|---|---|---|---|---|---|---|---|---|
| I-1 | B | D | D | A | D | A | B | A | D |
| I-2 | A | D | D | A | A | A | A | A | D |
| I-3 | A | D | D | A | D | A | B | A | D |
| I-4 | B | D | D | B | D | A | D | A | D |
| I-5 | A | D | D | A | D | A | D | A | D |
| I-6 | A | D | D | A | D | A | D | A | D |
| I-7 | A | B | A | A | A | D | D | A | A |
| I-8 | A | A | A | A | A | B | B | A | A |
| I-9 | A | D | D | A | C | A | B | A | D |
| I-11 | A | C | C | A | A | A | A | A | D |
| I-12 | A | A | A | A | A | B | C | A | A |
| I-13 | C | D | D | B | B | A | C | A | D |
| I-14 | A | D | A | A | A | D | D | A | A |
| I-15 | B | D | D | B | D | D | D | A | D |
| I-16 | C | D | D | D | D | C | D | A | D |
| I-17 | A | B | A | A | A | A | D | A | A |
| I-18 | A | C | A | A | B | D | D | A | B |
| I-19 | A | B | A | A | A | C | D | A | B |
| I-20 | A | D | C | A | C | D | D | A | C |
| I-21 | A | D | A | A | A | D | D | A | A |
| I-22 | A | B | A | A | A | B | D | A | A |
| I-23 | A | A | A | A | A | A | C | A | A |
| I-24 | A | C | A | A | A | A | C | A | A |
| I-25 | A | B | A | A | A | B | D | A | A |
| I-26 | D | D | D | D | D | D | D | B | D |
| I-27 | A | A | A | A | A | B | B | A | A |
| I-28 | A | A | A | A | A | A | B | A | A |
| I-30 | A | A | A | A | A | B | B | A | A |
| I-32 | A | B | A | A | A | A | C | A | A |
| I-33 | A | A | A | A | A | B | C | A | A |
| I-34 | A | A | A | A | A | C | B | A | A |
| I-35 | A | A | A | A | A | B | A | A | A |
| I-36 | A | B | A | A | A | A | C | A | A |
| I-38 | A | A | A | A | A | C | C | A | A |
| I-39 | A | D | A | A | A | C | C | A | A |
| I-40 | A | A | A | A | A | C | C | A | A |
| I-41 | A | A | A | A | A | B | A | A | A |
| I-42 | A | A | A | A | A | C | A | A | A |
| I-43 | A | A | A | A | A | C | C | A | A |
| I-44 | A | B | A | A | A | C | C | A | A |
| I-45 | A | C | A | A | A | C | C | A | A |
| I-46 | A | A | A | A | A | C | C | A | A |
| Aztreonam | C | D | D | D | D | D | D | C | D |

**Table 3. MIC values (µg/mL) of the compounds of the present invention against the metallo-β-lactamase-expressing Acinetobacter baumannii strain**

| | 1-7 | 1-27 | 1-28 | 1-30 | 1-32 | 1-33 | Aztreonam |
|---|---|---|---|---|---|---|---|
| *A. baumannii* ABA-0003YK | A | A | A | A | A | A | D |

In Tables 2 and 3: A: MIC ≤ 4; B: 4 < MIC ≤ 8; C: 8 < MIC ≤ 16; D: MIC > 16; NA = no data.

### Pharmacokinetic Evaluation of Compounds in Mice

### Administration and sampling

Male CD-1 (ICR) mice were purchased and then acclimatized in this laboratory for at least 3 days. Administration was performed to 3 mice by tail vein injection (not fasted, given *ad libitum* access to water) at a dose of 10 mg/kg. Blood samples were collected from the saphenous vein at 0.0833 h, 0.25 h, 0.5 h, 1 h, 2 h, 4 h, 8 h and 24 h following the tail vein injection administration. The volume of blood collection at each time point of the blood collection was about 30 µL. The blood samples were placed in centrifuge tubes containing anticoagulant EDTA-K2 and centrifuged at 3,200 g at 4 °C for 10 min, and then the upper plasma samples were collected and preserved in a freezer at -70±10 °C for later tests. The experimental design is summarized in the table below.

**Table 4. Pharmacokinetic study of the compounds in CD-1 (ICR)**

| **Route of administration** | **Dose** | **Concentration of preparation** | **Dosing volume** | **Vehicle** | **Testing animals** | **Sample size** | **Blood sampling time point** |
|---|---|---|---|---|---|---|---|
| Intravenous injection | 10 mg/kg | 2 mg/mL | 5 ml/kg | 2% DMSO 20% CremophorEL 78% normal saline | Male CD-1 mice | N=3 | 0.0833, 0.25, 0.5,1,2,4,8 and 24 h |

### Biological sample treatment

### 1. Protein precipitation:

1) To 1.5 mL centrifuge tubes were added a 6 µL aliquot of an unknown sample, a standard curve, a quality control, a dilution quality control, single blank and double blank samples.
2) Each sample (except for the double blank sample) was treated with 120 µL of IS 1-containing precipitant (the double blank sample was treated with 120 µL of pure MeOH for precipitation), and the mixtures were well mixed (for at least 15 s) using a vortexer and centrifuged at 12,000 g at 4 °C for 15 min.
3) 60 µL of the supernatants were transferred to a 96-well plate and centrifuged at 4 °C at 3220 g for 5 min, and 6 µL of the supernatants were injected for LC-MS/MS quantitative analysis.

### 2. Dilution process:

1) The 1 h-plasma samples from the 3 mice were diluted 10-fold: 2 µL of the samples were added to 18 uL of blank matrix.
2) The 0.0833 h to 0.5 h-plasma samples from the 3 mice were diluted 100-fold: 18 µL of blank matrix was added to 2 µL of the samples to obtain 10-fold diluted samples. 2 µL of the 10-fold diluted samples were added to 18 µL of blank matrix to obtain 100-fold diluted analysis samples.

LC-MS/MS analysis conditions: chromatography column: ACQUITY UPLC HSS T3 (2.1 × 50 mm, 1.8 µm); column temperature: 50 °C; mobile phase A: a 0.1% aqueous solution of formic acid; mobile phase B: a 0.1% solution of formic acid in acetonitrile; flowrate:
0.65 mL/min. Internal standard: verapamil (100 ng/mL)

### 3. Data analysis:

The concentration of the compounds in plasma was measured by LC-MS/MS (LC-MS/MS-AK_Q-Trap 6500). The pharmacokinetic software WinNonlin [version: Phoenix^{™} WinNonlin^{®} 6.3, manufacturer: Pharsight Corporation] was used to calculate pharmacokinetic parameters (V-Noncompartmental model 201).

### The results are as follows:

| **Compound** | **I-7** | **I-35** | **I-43** | **I-44** |
|---|---|---|---|---|
| **Dose mg/kg mice** | 5 | 10 | 10 | 10 |
| **Cl ml/min/kg** | 19.6 | 19.5 | 17.3 | 9.61 |
| **Vss L/kg** | 0.48 | 0.57 | 0.24 | 0.19 |
| **Tl/2 h** | 0.27 | 1.43 | 0.59 | 0.63 |
| **AUC ng^{∗}h/ml** | 4.24 | 8.53 | 9.66 | 17.3 |

## Claims

1. A compound of formula (I): 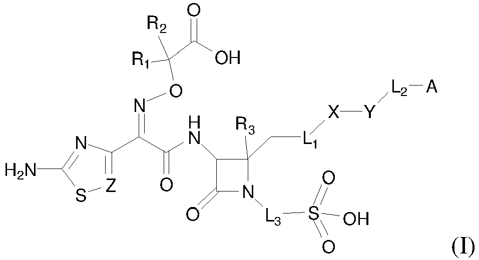 wherein R₁ and R₂ are each independently selected from H, C₁-C₆ alkyl and C₁-C₆ haloalkyl; or R₁ and R₂, together with the carbon atom to which they are attached, form C₃-C₆ cycloalkyl, or R₁ and R₂, together with the carbon atom to which they are attached, form C₃-C₆ heterocycloalkyl; wherein the C₃-C₆ cycloalkyl and C₃-C₆ heterocycloalkyl are optionally substituted with C₁-C₆ alkyl, C₁-C₆ haloalkyl, halogen or hydroxy;
R₃ is selected from H, C₁-C₆ alkyl and halogen, wherein the C₁-C₆ alkyl is optionally substituted with halogen or hydroxy;
L₁ is absent or is selected from -O-T-, -O-C(=O)-T-, -O-S(=O)ᵣ-T-, -S(=O)ᵣ-T-, -S-CH₂-C(O)-T-, -NH-C(=O)-T-, -NH-S(O)ᵣ-T-, -NH-C(=N-CN)-T, -NH-C(=NH)-T-, -NH-T-, -(CH₂)_{P}-S(O)ᵣ-T-, -(CH₂)ₚ-C(O)-T-, -(CH₂)ₚ-S-T- and -(CH₂)ₚ-C(=NH)-T-, wherein the T is absent or is selected from -(CH₂)ₚ-, -(CH₂)ₚ-NH-(CH₂)_{q}-, -(CH₂)ₚ-O-(CH₂)_{q}-, -(CH₂)ₚ-C(=O)-NH-(CH₂)_{q}-, - (CH₂)ₚ-NH-C(=O)-(CH₂)_{q}-, -CH(CH₃)-NH-C(=O)-(CH₂)_{q}-, -(CH₂)ₚ-NH-C(=O)-NH-(CH₂)_{q}-, - CH(CH₃)-NH-C(=O)-NH-(CH₂)_{q}-, -CH(CH₃)-NH-C(=O)-NH-(CH₂)_{q}-, and -(CH₂)ₚ-C(=O)-(CH₂)_{q}-;
wherein the r is 1 or 2; wherein the p and q are 0, 1 or 2;
X is selected from 5-6 membered heteroaryl, 6-10 membered aryl, 4-8 membered cycloalkyl and 4-8 membered heterocycloalkyl; wherein the 5-6 membered heteroaryl or 4-8 membered heterocycloalkyl contains 1, 2 or 3 heteroatoms selected from N, O and S as ring atoms, and the 5-6 membered heteroaryl, 6-10 membered aryl, 4-8 membered cycloalkyl or 4-8 membered heterocycloalkyl is optionally substituted with P, wherein the P is selected from C₁₋₄ alkyl, C₁₋₄ haloalkyl, C₁₋₄ alkoxy, halogen, NR4R4' and hydroxy, wherein the C₁₋₄ alkyl is optionally further substituted with hydroxy, C₁₋₄ alkoxy or NR4R4';
Y is absent or is selected from 5-6 membered heteroaryl, 6-10 membered aryl, 4-8 membered cycloalkyl and 4-8 membered heterocycloalkyl; wherein the 5-6 membered heteroaryl or 4-8 membered heterocycloalkyl contains 1, 2 or 3 heteroatoms selected from N, O and S as ring atoms, and the 5-6 membered heteroaryl, 6-10 membered aryl, 4-8 membered cycloalkyl or 4-8 membered heterocycloalkyl is optionally substituted with Q, wherein the Q is selected from halogen, C₁₋₄ alkyl, NR4R4', hydroxy, C₁₋₄ haloalkyl, C₁₋₄ alkoxy, -C₁₋₄ alkylene-N(R₅)₂, -C₁₋₄ alkylene-NR₅-C(=NR₅)-N(R₅)₂, -C₁₋₄ alkylene-NR₅-CR₅(=NR₅), -C₁₋₄ alkylene (C₁₋₄ alkylene-N(R₅))₂, and -C₁₋₄ alkylene-N⁺(R₇)₃, wherein the C₁₋₄ alkyl is optionally substituted with hydroxy, C₁₋₄ alkoxy or NR4R4', and the C₁₋₄ alkylene is optionally substituted with NR4R4', hydroxy or halogen;
R₄ and R₄' are each independently selected from H and C₁₋₄ alkyl;
R₅ is selected from H and C₁₋₄ alkyl optionally substituted with -OR₆ or -NR₆R₆'; or two R₅ on the same N may form 4-6 membered heterocycloalkyl optionally substituted with C₁₋₄ alkyl, C₁₋₄ alkoxy, hydroxy, amino or oxo, by cyclization;
R6 and R₆' are each independently selected from H and C₁₋₄ alkyl optionally substituted with hydroxy, C₁₋₄ alkoxy or NR4R4';
R₇ is selected from H, C₁₋₄ alkyl and -CH(-C₁₋₄ alkylene-N(R₅)₂)₂;
L2 is absent or is selected from -(NR₄)ₘ-C₁₋₄ alkylene-(NR4)n-, -(NR₄)ₘ-C₁₋₄ alkylene-NR4-C(=NR₄)-NR₄-, and -C₁₋₄ alkylene-NR₄-C₁₋₄ alkylene-; wherein m is 0 or 1, n is 1 or 2, and the C₁₋₄ alkylene is optionally substituted with C₁₋₄ alkyl, NH2, or -C₁₋₄ alkylene-NH₂;
A is selected from H, M and -(CO)-M, wherein the M is selected from 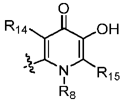 and 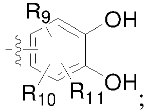 wherein R₈ is selected from H, C₁-C₆ alkyl, hydroxy, and C₁₋₄ alkoxy; R₉, R₁₀ and R₁₁ are each independently selected from H, halogen, CN and OR₁₂, wherein R₁₂ is H, C₁-C₆ alkyl, C₁-C₆ haloalkyl, C₁-C₆ alkylcarbonyl, or -C(O)-NH₂; R₁₄ and R₁₅ are each independently selected from: H, C₁₋₆ alkyl, halogen, cyano and OR₁₆, wherein R₁₆ is selected from H and C₁-C₆ alkyl;
L₃ is absent or is -O-;
Z is selected from N and CR₁₃, wherein R₁₃ is selected from H and halogen;
or an ester, a stereoisomer and a pharmaceutically acceptable salt thereof.

2. The compound according to claim 1, selected from: 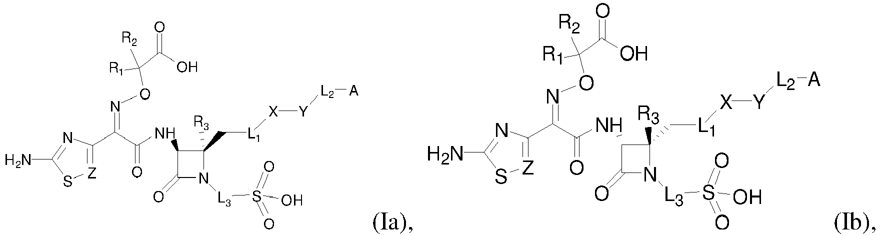 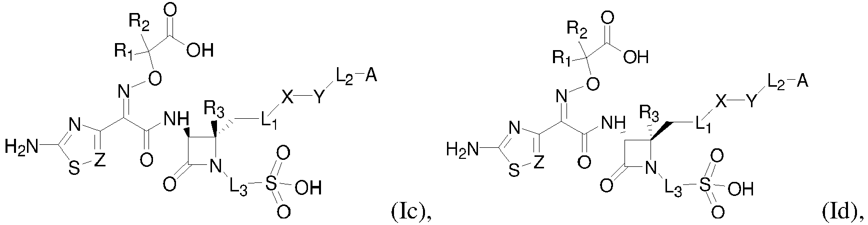 or an ester, a stereoisomer and a pharmaceutically acceptable salt thereof.

3. The compound according to claim 1 or 2, or an ester, a stereoisomer and a pharmaceutically acceptable salt thereof, wherein:
R₃ is selected from H and C₁-C₆ alkyl, wherein the C₁-C₆ alkyl is optionally substituted with halogen;
X is selected from 5-6 membered heteroaryl, 6-10 membered aryl, 4-8 membered cycloalkyl and 4-8 membered heterocycloalkyl; wherein the 5-6 membered heteroaryl or 4-8 membered heterocycloalkyl has at least 1 of heteroatoms selected from an N atom, and the 5-6 membered heteroaryl, 6-10 membered aryl, 4-8 membered cycloalkyl or 4-8 membered heterocycloalkyl is optionally substituted with P, wherein the P is selected from C₁₋₄ alkyl, C₁₋₄ haloalkyl, C₁₋₄ alkoxy, halogen, NR4R4' and hydroxy, wherein the C₁₋₄ alkyl is optionally further substituted with hydroxy, C₁₋₄ alkoxy or NR₄R₄', and R₄ and R₄' are each independently selected from H and C₁₋₄ alkyl.

4. The compound according to claim 3, or an ester, a stereoisomer and a pharmaceutically acceptable salt thereof, wherein:
R₃ is selected from H and C₁-C₆ alkyl, wherein the C₁-C₆ alkyl is optionally substituted with halogen;
X is selected from 5-membered heteroaryl and phenyl; wherein the 5-membered heteroaryl has at least 1 of heteroatoms selected from an N atom, and the 5-membered heteroaryl or phenyl is optionally substituted with P, wherein the P is selected from C₁₋₄ alkyl, C₁₋₄ haloalkyl, C₁₋₄ alkoxy, halogen, NR4R4' and hydroxy, wherein the C₁₋₄ alkyl is optionally further substituted with hydroxy, C₁₋₄ alkoxy or NR4R4', and R₄ and R₄' are each independently selected from H and C₁₋₄ alkyl.

5. The compound according to claim 4, or an ester, a stereoisomer and a pharmaceutically acceptable salt thereof, wherein:
R₃ is selected from H, methyl and halomethyl;
X is selected from oxazolyl, isoxazolyl, thiazolyl, isothiazolyl, imidazolyl, pyrazolyl, triazolyl, oxadiazolyl, thiadiazolyl and phenyl; wherein the groups are optionally substituted with P, wherein the P is selected from C₁₋₄ alkyl, C₁₋₄ haloalkyl, C₁₋₄ alkoxy, halogen, NR4R4' and hydroxy, wherein the C₁₋₄ alkyl is optionally further substituted with hydroxy, C₁₋₄ alkoxy or NR₄R₄', and R₄ and R₄' are each independently selected from H and C₁₋₄ alkyl.

6. The compound according to claim 1 or 2, or an ester, a stereoisomer and a pharmaceutically acceptable salt thereof, wherein:
R₁ and R₂ are each independently selected from H, methyl and halomethyl, or R₁ and R₂, together with the carbon atom to which they are attached, form cyclopropane, cyclobutane, or cyclopentane; wherein the cyclopropane, cyclobutane or cyclopentane is optionally substituted with C₁-C₆ alkyl, C₁-C₆ haloalkyl, halogen or hydroxy.

7. The compound according to claim 6, or an ester, a stereoisomer and
a pharmaceutically acceptable salt thereof, wherein:
R₁ and R₂ are each methyl, or R₁ and R₂, together with the carbon atom to which they are attached, form cyclopropane, cyclobutane, or cyclopentane; wherein the cyclopropane, cyclobutane or cyclopentane is optionally substituted with C₁-C₆ alkyl, C₁-C₆ haloalkyl, halogen or hydroxy;
R₃ is selected from H, methyl and CH₂F;
X is selected from oxazolyl, isoxazolyl, thiazolyl and phenyl, wherein the groups are optionally substituted with P, wherein the P is selected from C₁₋₄ alkyl and NR4R4', wherein R₄ and R₄' are each independently selected from H and C₁₋₄ alkyl;
Y is absent or is selected from 5-6 membered heteroaryl, phenyl and 4-6 membered heterocycloalkyl; wherein the 5-6 membered heteroaryl or 4-6 membered heterocycloalkyl contains 1 or 2 N atoms as ring atoms, and the 5-6 membered heteroaryl, phenyl or 4-6 membered heterocycloalkyl is optionally substituted with Q, wherein the Q is selected from halogen, C₁₋₄ alkyl, NR4R4', hydroxy, C₁₋₄ haloalkyl, C₁₋₄ alkoxy, -C₁₋₄ alkylene-N(R₅)₂, -C₁₋₄ alkylene-NR₅-C(=NR₅)-N(R₅)₂, -C₁₋₄ alkylene-NR₅-CR₅(=NR₅), -C₁₋₄ alkylene (C₁₋₄ alkylene-N(R₅))₂, and -C₁₋₄ alkylene-N⁺(R₇)₃, wherein the C₁₋₄ alkyl is optionally substituted with hydroxy, C₁₋₄ alkoxy or NR4R4', and the C₁₋₄ alkylene is optionally substituted with NR4R4', hydroxy or halogen;
R₄ and R₄' are each independently selected from H and C₁₋₄ alkyl;
R₅ is selected from H and C₁₋₄ alkyl optionally substituted with -OR₆ or -NR₆R₆'; or two R₅ on the same N may form 4-6 membered heterocycloalkyl optionally substituted with C₁₋₄ alkyl, C₁₋₄ alkoxy, hydroxy, amino or oxo, by cyclization;
R6 and R₆' are each independently selected from H and C₁₋₄ alkyl optionally substituted with hydroxy, C₁₋₄ alkoxy or NR4R4';
R₇ is selected from H, C₁₋₄ alkyl and -CH(-C₁₋₄ alkylene-N(R₅)₂)₂.

8. The compound according to claim 7, or an ester, a stereoisomer and
a pharmaceutically acceptable salt thereof, wherein:
X is selected from oxazolyl, isoxazolyl, thiazolyl and phenyl, wherein the groups are optionally substituted with P, wherein the P is methyl;
Y is absent or is selected from pyrazolyl, pyridinyl, pyrrolyl, azetidinyl, pyrimidinyl and phenyl, wherein the groups are optionally substituted with Q, wherein the Q is selected from halogen, C₁₋₄ alkyl, NR4R4', hydroxy, C₁₋₄ haloalkyl, C₁₋₄ alkoxy, -C₁₋₄ alkylene-N(R₅)₂, -C₁₋₄ alkylene-NR₅-C(=NR₅)-N(R₅)₂, -C₁₋₄ alkylene-NR₅-CR₅(=NR₅), -C₁₋₄ alkylene (C₁₋₄ alkylene-N(R₅))₂, and -C₁₋₄ alkylene-N⁺(R₇)₃, wherein the C₁₋₄ alkyl is optionally substituted with hydroxy, C₁₋₄ alkoxy or NR4R4', and the C₁₋₄ alkylene is optionally substituted with NR4R4', hydroxy or halogen;
R₄ and R₄' are each independently selected from H and C₁₋₄ alkyl;
R₅ is selected from H and C₁₋₄ alkyl optionally substituted with -OR₆ or -NR₆R₆'; or two R₅ on the same N may form 4-6 membered heterocycloalkyl optionally substituted with C₁₋₄ alkyl, C₁₋₄ alkoxy, hydroxy, amino or oxo, by cyclization;
R6 and R₆' are each independently selected from H and C₁₋₄ alkyl optionally substituted with hydroxy, C₁₋₄ alkoxy or NR4R4';
R₇ is selected from H, C₁₋₄ alkyl and -CH(-C₁₋₄ alkylene-N(R₅)₂)₂;
A is selected from H, M and -(CO)-M, wherein the M is selected from 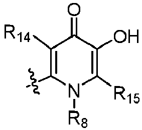 and 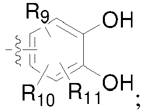 wherein R₈ is hydroxy; R₉, R₁₀ and R₁₁ are each independently selected from H and halogen; R₁₄ and R₁₅ are each independently selected from H, C₁₋₃ alkyl, halogen, cyano and OR₁₆, wherein R₁₆ is selected from H and C₁-C₃ alkyl.

9. The compound according to claim 7 or 8, or an ester, a stereoisomer and
a pharmaceutically acceptable salt thereof, wherein:
R₁ and R₂ are each methyl, or R₁ and R₂, together with the carbon atom to which they are attached, form cyclopropane, cyclobutane or cyclopentane;
R₃ is selected from H, methyl and CH2F;
L₁ is -NH-C(O)-;
Y is absent or is selected from 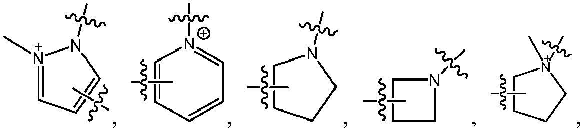 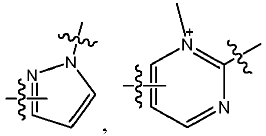 and phenyl;
L2 is absent or is selected from -(NR₄)ₘ-C₁₋₄ alkylene-(NR₄)ₙ-, -(NR₄)ₘ-C₁₋₄ alkylene-NR4-C(=NR₄)-NR₄-, and -C₁₋₄ alkylene-NR₄-C₁₋₄ alkylene-; wherein m is 0 or 1, n is 1 or 2, and the C₁₋₄ alkylene is optionally substituted with C₁₋₄ alkyl, NH2, or -C₁₋₄ alkylene-NH₂;
A is selected from H, M and -(CO)-M, wherein the M is selected from 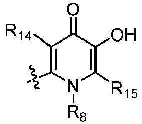 and 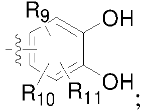 wherein R₈ is hydroxy; R₉, R₁₀ and R₁₁ are each independently selected from H and halogen; R₁₄ and R₁₅ are each independently selected from H and methyl;
L₃ is absent;
Z is CH, N or CCl.

10. The compound according to claim 1 or 2, or an ester, a stereoisomer and
a pharmaceutically acceptable salt thereof, wherein:
R₁ and R₂ are each methyl, or R₁ and R₂, together with the carbon atom to which they are attached, form cyclopropane, cyclobutane or cyclopentane;
R₃ is selected from H, methyl and CH2F;
L₁ is -NH-C(O)-;
X is selected from 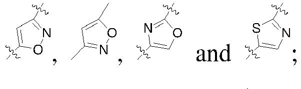
Y is absent or is selected from pyrazolyl, pyridinyl, pyrrolyl, azetidinyl, pyrimidinyl and phenyl, wherein the groups are optionally substituted with Q, wherein the Q is selected from halogen, C₁₋₄ alkyl, NR4R4', hydroxy, C₁₋₄ haloalkyl, C₁₋₄ alkoxy, -C₁₋₄ alkylene-N(R₅)₂, -C₁₋₄ alkylene-NR₅-C(=NR₅)-N(R₅)₂, -C₁₋₄ alkylene-NR₅-CR₅(=NR₅), -C₁₋₄ alkylene (C₁₋₄ alkylene-N(R₅))₂, and -C₁₋₄ alkylene-N⁺(R₇)₃, wherein the C₁₋₄ alkyl is optionally substituted with hydroxy, C₁₋₄ alkoxy or NR4R4', and the C₁₋₄ alkylene is optionally substituted with NR4R4', hydroxy or halogen;
R₄ and R₄' are each independently selected from H and C₁₋₄ alkyl;
R₅ is selected from H and C₁₋₄ alkyl optionally substituted with -OR₆ or -NR₆R₆'; or two R₅ on the same N may form 4-6 membered heterocycloalkyl optionally substituted with C₁₋₄ alkyl, C₁₋₄ alkoxy, hydroxy, amino or oxo, by cyclization;
R6 and R₆' are each independently selected from H and C₁₋₄ alkyl optionally substituted with hydroxy, C₁₋₄ alkoxy or NR4R4';
R₇ is selected from H, C₁₋₄ alkyl and -CH(-C₁₋₄ alkylene-N(R₅)₂)₂;
L2 is absent or is selected from -(NH)ₘ-C₁₋₄ alkylene-(NH)n-, -(NH)ₘ-C₁₋₄ alkylene-NH-C(=NH)-NH-, and -C₁₋₄ alkylene-NH-C₁₋₄ alkylene-; wherein m is 0 or 1, n is 1 or 2, and the C₁₋₄ alkylene is optionally substituted with C₁₋₄ alkyl, NH2, or -C₁₋₄ alkylene-NH₂;
A is selected from H, M and -(CO)-M, wherein M is selected from 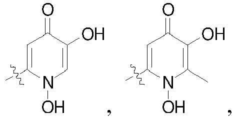 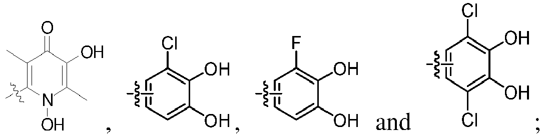
L₃ is absent;
Z is CH, N or CCl.

11. The compound according to claim 1 or 2, or an ester, a stereoisomer and
a pharmaceutically acceptable salt thereof,
wherein: R₁ and R₂ are each methyl, or R₁ and R₂, together with the carbon atom to which they are attached, form cyclopropane, cyclobutane or cyclopentane;
R₃ is selected from H, methyl and CH2F;
L₁ is -NH-C(O)-;
X is selected from 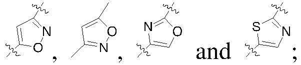
Y is absent or is selected from the following groups: 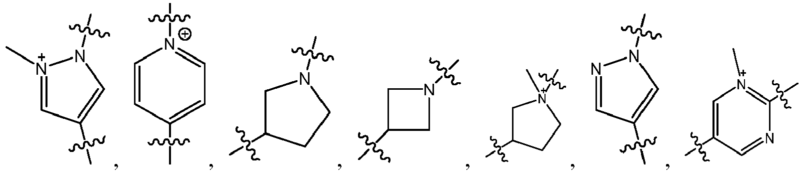 and phenyl;
L₂ is absent or is selected from -C₁₋₄ alkylene-NH-, -C₁₋₄ alkylene-(NH)₂-, -NH-C₁₋₄ alkylene-NH-, -C₁₋₄ alkylene-NH-C(=NH)-NH-, and -C₁₋₄ alkylene-NH-C₁₋₄ alkylene-; wherein the C₁₋₄ alkylene is optionally substituted with NH2 or -CH2-NH2;
A is selected from H, M and -(CO)-M, wherein M is selected from 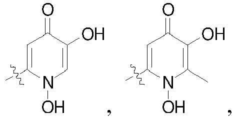 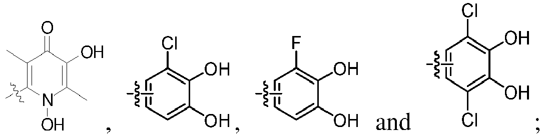
L₃ is absent;
Z is CH, N or CCl.

12. The compound according to claim 1 or 2, selected from: 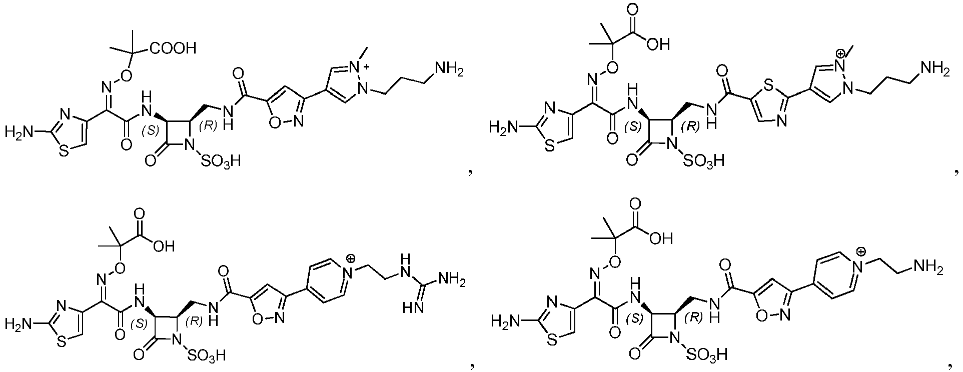 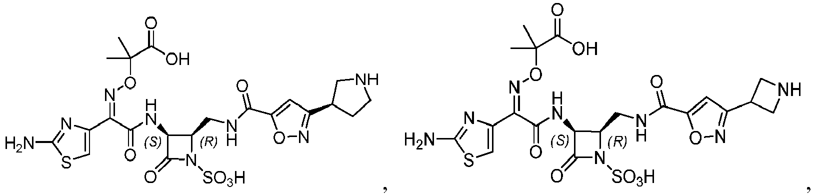 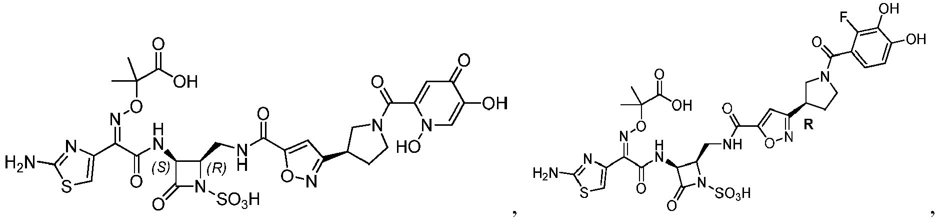 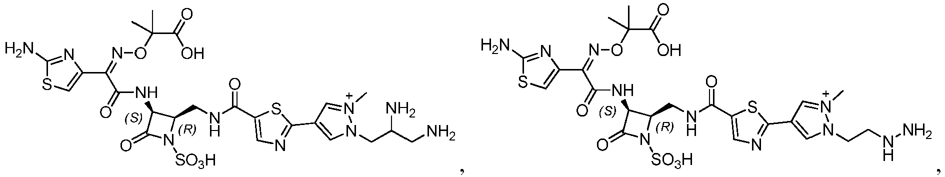 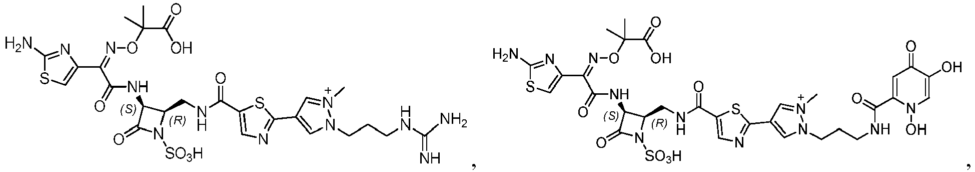 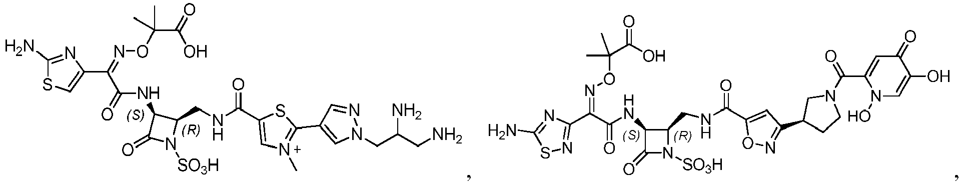 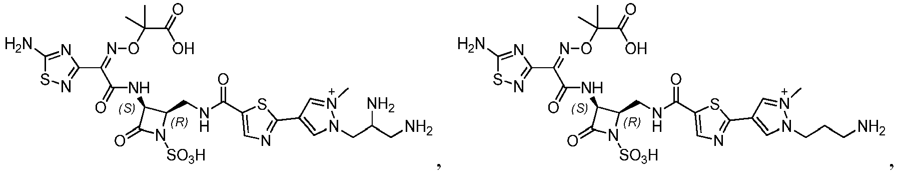 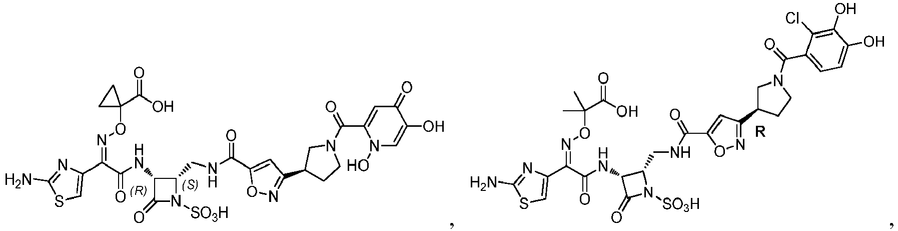 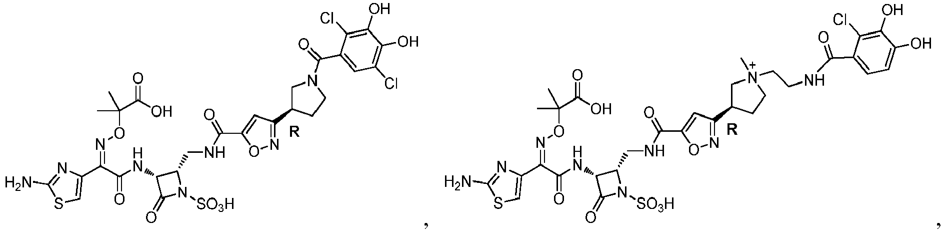 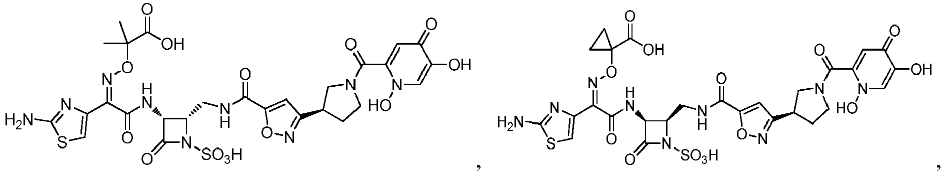 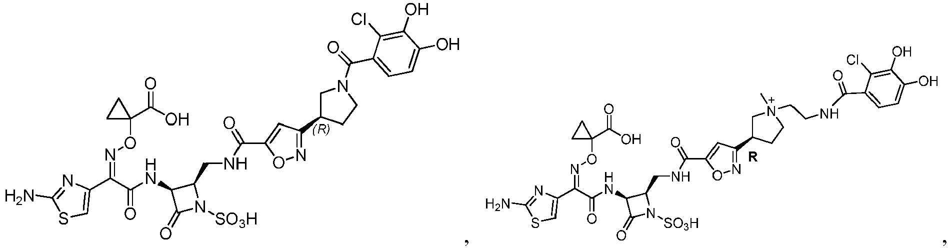 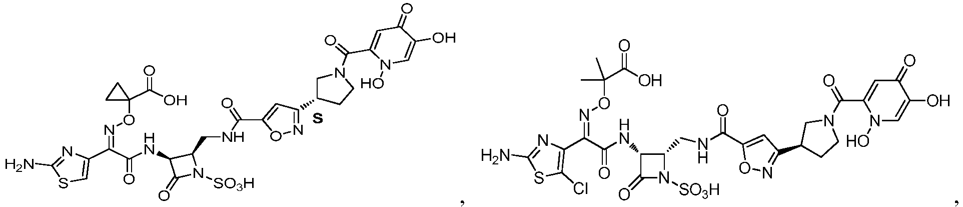 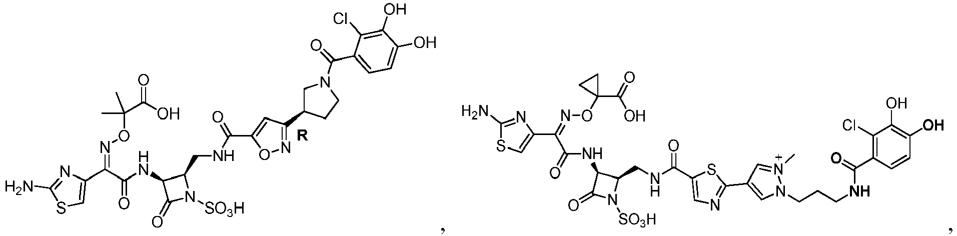 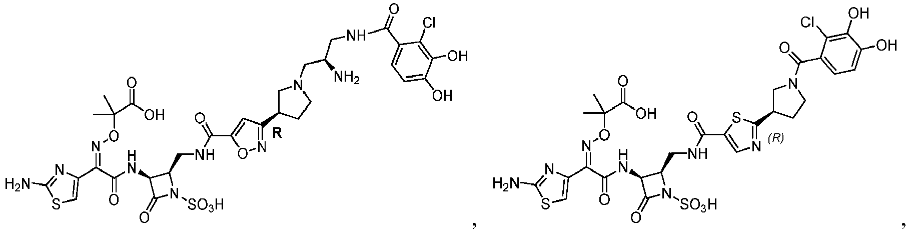 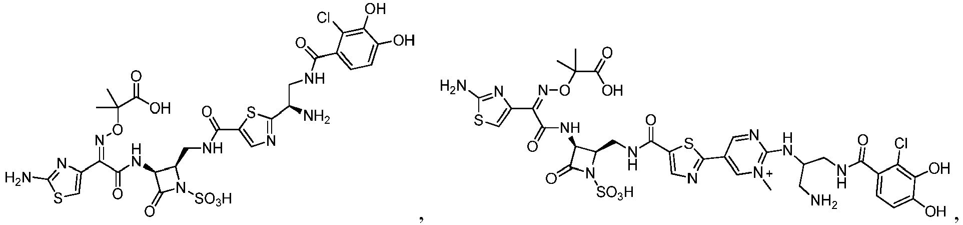 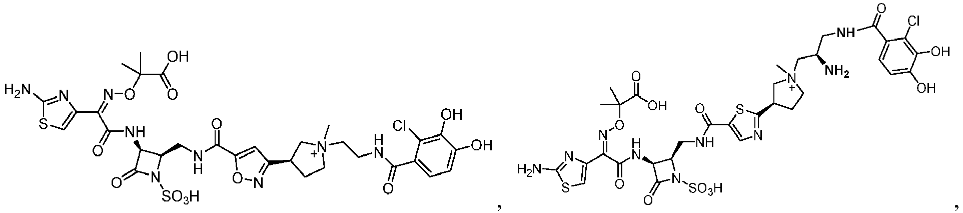 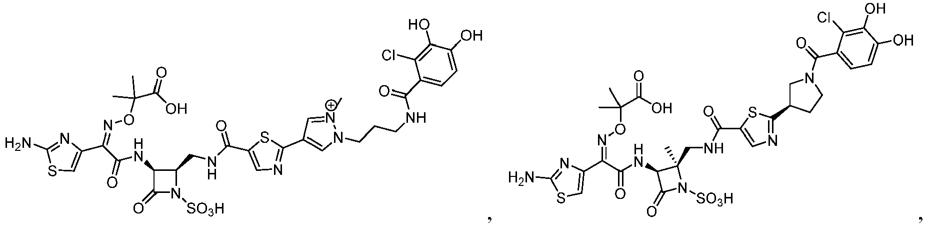 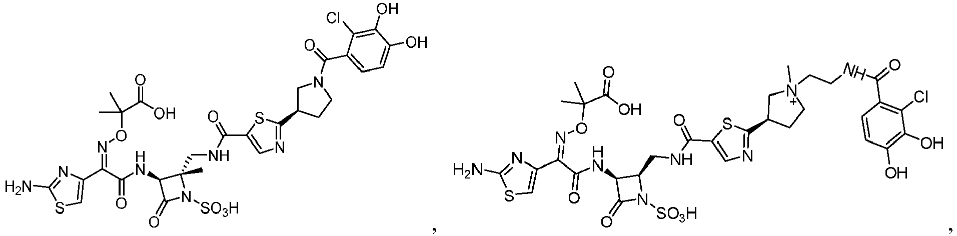 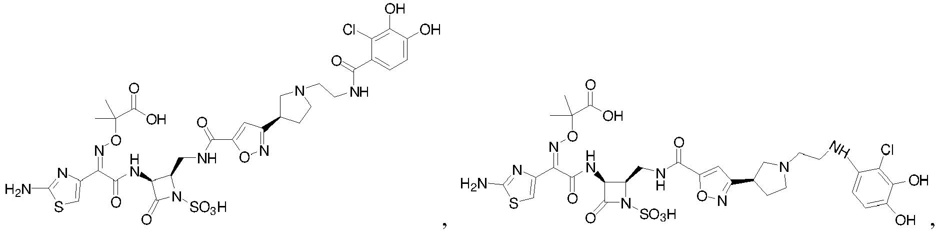 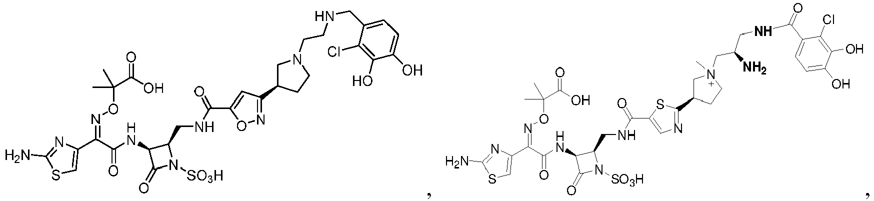 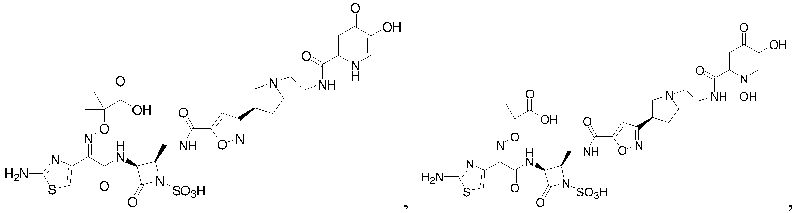 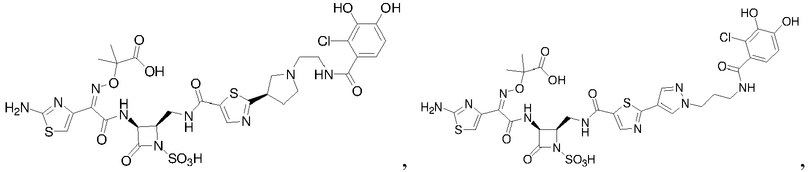 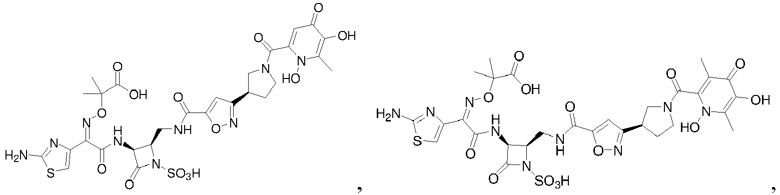 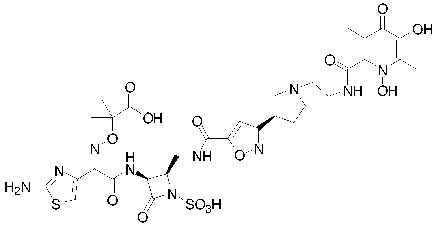 and 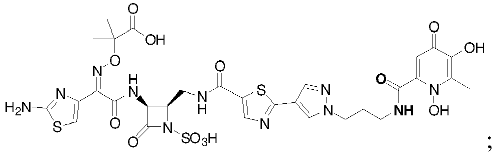 or an ester, a stereoisomer and a pharmaceutically acceptable salt thereof.

13. A pharmaceutical composition comprising the compound according to any one of claims 1-12 and a pharmaceutically acceptable carrier.

14. Use of the compound according to any one of claims 1-12 in the manufacture of a medicament for treating and preventing a disease caused by bacterial infections.

15. The use according to claim 14, wherein the bacteria are gram-positive bacteria or gram-negative bacteria.

16. The use according to claim 15, wherein the bacteria are gram-negative bacteria.

17. The use according to claim 16, wherein the bacteria are selected from: *Escherichia coli, Acinetobacter baumannii, Klebsiella pneumoniae, Pseudomonas aeruginosa, Enterobacter cloacae, Citrobacter* spp., *Citrobacter freundii, Proteus vulgaris* (including *Proteus mirabilis), Shigella* spp., *Serratia marcescens, Moraxella catarrhalis, Neisseria gonorrhoeae, Neisseria meningitidis, Campylobacter* spp., *Helicobacter pylori, Vibrio cholerae, Haemophilus influenzae, Mycobacterium* spp., *Bacteroides fragilis, Bacillus cereus,* and *Stenotrophomonas maltophilia.*

18. The use according to claim 14, wherein the disease is selected from respiratory tract infection, urinary tract infection, central nervous system infection, ear infection, intra-abdominal infection, cardiovascular infection, skin or soft tissue infection, bone and joint infections, genital infection, ocular infection, and oral infection.

19. The use according to claim 18, wherein the respiratory tract infection includes upper respiratory tract infection, lower respiratory tract infection, tuberculosis, and pulmonary infection complicated by pulmonary fibrosis.

20. The use according to claim 19, wherein the upper respiratory tract infection includes pharyngitis, and the lower respiratory infection includes trachitis, bronchitis, and pneumonia caused by *Enterobacter* spp. and *Serratia marcescens.*

21. The use according to claim 18, wherein the urinary tract infection includes uncomplicated and complicated pyelonephritis, recurrent cystitis, complicated urinary tract infection, and uncomplicated urinary tract infection.

22. The use according to claim 19, wherein the central nervous system infection includes encephalitis, meningitis, and encephalopyosis.

23. The use according to claim 19, wherein the ear infection includes otitis externa and otitis media.

24. The use according to claim 19, wherein the intra-abdominal infection includes peritonitis.

25. The use according to claim 19, wherein the cardiovascular infection includes blood infection, endocarditis, myocarditis, and pericarditis.

26. The use according to claim 25, wherein the blood infection includes septicemia and bacteremia.

27. The use according to claim 19, wherein the bone and joint infections include arthritis and osteomyelitis.

28. The use according to claim 19, wherein the genital infection includes genital ulcers, vaginitis and cervicitis.

29. The use according to claim 19, wherein the ocular infection includes conjunctivitis, keratitis and endophthalmitis.

30. The use according to claim 19, wherein the oral infection includes gingivitis, periodontitis and pulpitis.
